# EUROPEAN PATENT APPLICATION

(11) **EP 2 626 371 A1**
(43) Date of publication of application: **14.08.2013**
(21) Application number: 13156225.8
(22) Date of filing: 30.07.2008
(51) Int. Cl.: C07K 16/18, C07K 16/46

(54) **Multispecific epitope binding proteins and uses thereof**

(30) Priority: 31.07.2007 US 935199 P; 10.12.2007 US 12656 P; 20.06.2008 US 74330 P
(62) Divisional of application: 08796901.0
(71) Applicant: MedImmune, LLC, Gaithersburg, MD 20878 (US)
(72) Inventor: Wu, Herren, Gaithersburg, MD Maryland 20878 (US); Gao, Changshou, Gaithersburg, MD Maryland 20878 (US); Hay, Carl, Gaithersburg, MD Maryland 20878 (US); Dimasi, Nazzareno, Gaithersburg, MD Maryland 20878 (US)
(74) Representative: Bates, Rosica Florence

(57) **Abstract**

The present invention relates to multispecific epitope binding proteins, methods of making, and uses thereof in the prevention, management, treatment or diagnosis of acute or chronic diseases.

SEQUENCE LISTING

<110>  MedImmune LLC

<120>  Multispecific Epitope Binding Proteins And Uses Thereof

<130>  AE713PCT

<140>  PCT/US08/071656
<141>  2008-07-30

<150>  US 60/935,199
<151>  2007-07-31

<150>  US 61/012,656
<151>  2007-12-10

<150>  US 61/074.300
<151>  2008-06-20

<160>  4

<170>  PatentIn version 3.3

<210>  1
<211>  5
<212>  PRT
<213>  Unknown

<220>
<223>  Artificial construct

<400>  1

Glu Pro Lys Ser Cys
1               5


<210>  2
<211>  15
<212>  PRT
<213>  Unknown

<220>
<223>  Artificial sequence

<400>  2

Glu Pro Lys Ser Cys Asp Lys Thr His Thr Cys Pro Pro Cys Pro
1               5                   10                  15


<210>  3
<211>  5
<212>  PRT
<213>  Unknown

<220>
<223>  Artificial construct

<400>  3

Glu Pro Lys Ser Ser

1                 5

<210>  4
<211>  5
<212>  PRT
<213>  Unknown

<220>
<223>  Artificial sequence

<400>  4

Gly Gly Gly Gly Ser
1               5

## Description

### 1. Field of the Invention

The present invention relates to multispecific epitope binding proteins, methods of making, and uses thereof in the prevention, management, treatment or diagnosis of acute or chronic diseases.

### 2. Background of the Invention

Antibody fragments, such as Fabs, scFvs, diabodies, tribodies, and tetrabodies capable of binding one or more antigens may prove to be suitable for a number of clinical applications. These types of epitope binding proteins retain binding specificity to antigens, but lack the functional ability to stimulate an immune response directed against the bound antigen, i.e., they lack effector function.

Efforts to increase the valency or the number of antigenic determinants that an individual antibody molecule can bind have lead to the development of bi-specific antibodies (BsAb) (for examples see Jimenez et al. Molecular Cancer Therapeutics 2005:4(3)427-434, Lu et al. J. of Immun. Methods 1999:230, 159-171 and U.S. Patent Publication Nos. 20070014794 and 20050100543). BsAbs are immunoglobulin (Ig)-based molecules that bind to two different epitopes on either the same or distinct antigens. The antibodies, for example, could be specific for a tumor cell antigen and an effector cell such as an activated T-cell or a functional agent such as a cytotoxin. Bispecific T-cell Engagers or BiTE™ molecules are one type of BsAbs, which have been shown to be useful for clinical applications (See U.S. Patent No. 7,112,324 for examples).

Efforts to increase specificity beyond two distinct antigens have lead to the production of a trispecific antibody-like structure composed of three distinct scFv domains flanked by hinge region domains that can be linked via disulfide bonds to circularize the resultant protein (See U.S Patent Pub No. 20050175606). However, the stability of the antibody-like structures are in question due to the reliance on a single disulfide bond for circularization.

A major obstacle in the development of multiple epitope binding antibodies such as BsAbs as therapeutics has been difficulty in producing the antibodies in sufficient quantity and quality for clinical studies. In particular, traditional methods, including hybrid hybridoma, in which two distinct hybridomas are fused to create a cell expressing two sets of heavy and light chains, and chemical conjugation (Carter et al. (1995) J. Hematotherapy 4:463-70) have been inadequate.

For instance, coexpression of two different sets of IgG light and heavy chains in a hybrid hybridoma may produce up to 10 light- and heavy-chain pairs, with only one of these pairs forming the functional bispecific heterodimer (Suresh et al. (1986) Methods Enzymol. 121:210-28). Purification of the antibodies from the non-functional species, such as homodimers and mispaired heterodimers of non-cognate Ig light and heavy chains produced by the hybrid hybridoma is cumbersome and inefficient.

Chemical crosslinking of two IgGs or their fragments is also inefficient and can lead to the loss of antibody activity (Zhu et al. (1994) Cancer Lett. 86:127-34). Like the hybrid hybridoma approach, purification of the antibodies from the non-functional species, such multimeric aggregates resulting from chemical conjugation, is often difficult and the yield is usually low (Cao et al. (1998) Bioconj. Chem. 9:635-44).

To improve efficiency of production of multiple epitope binding antibodies, a variety of recombinant methods have been developed. For example, methods of efficient production of BsAbs have been developed, both as antibody fragments (Carter et al. (1995); Pluckthun et al (1997) Immunotechology 3:83-105; Todorovska et al. (2001) J. Immunol. Methods 248:47-66) and full length IgG formats (Carter (2001) J. Immunol. Methods 248:7-15). For example, production of homogeneous full-length IgG-like BsAbs has been achieved by the so- called "knobs-into-holes" engineering for efficient Ig CH3 domain heterodimerization (Ridgway et al. 1996 Protein Eng. 9:617-21; Merchant et al. 1998 Nat. Biotech. 16:677- 81) and by fusing single chain Fvs (scFv) of different specificities onto either the N- or the C-terminus of a full-length IgG molecule (Zhuang et al. Protein Eng. 2000 13:361-7; Coloma and Morrison Nat. Biotechnol. 199715:159-63). BsAbs have also been constructed by genetically fusing two single chain Fv (scFv) or Fab fragments with or without the use of flexible linkers (Mallender et al. J. Biol. Chem. 1994 269:199- 206; Mack et al. Proc. Natl. Acad. Sci. USA. 1995 92:7021-5; Zapata et al. Protein Eng. 1995 8.1057-62), via a dimerization device such as leucine zipper (Kostelny et al. J. Immunol. 1992148:1 547-53; de Kruif et al. J. Biol. Chem. 1996 271:7630-4) and Ig Cλ/CH1 domains (Muller et al. FEBS Lett. 422:259-64); by diabody (Holliger et al. (1993) Proc. Nat. Acad. Sci. USA. 1998 90:6444-8; Zhu et al. Bio/Technology (NY) 1996 14:192-6); Fab-scFv fusion (Schoonjans et al. J. Immunol. 2000 165:7050-7); and mini-antibody formats (Pack et al. Biochemistry 1992 31:1579-84; Pack et al. Bio/Technology1993 11:1271-7). In the majority of the cases, these recombinant approaches result in the production of divalent bispecific antibody molecules that are monovalent to each of their target antigens.

Antibody alternatives, such as multispecific epitope binding proteins provide many advantages over traditional targeted epitopes for example, but not limited to, access to immunosilent domains, expanded repertoire of targets, new binding specificities, and conjugates to drugs, radionuclides, toxins, enzymes, liposomes and viruses. With these significant advantages, there is a need in the art to construct and efficiently produce functional multivalent and multispecific epitope binding proteins capable of binding at least three or more epitopes with high affinity while retaining the ability to elicit the effector functions of an antibody.

Citation or discussion of a reference herein shall not be construed as an admission that such is prior art to the present invention.

### 3. Summary of the Invention

The present invention provides novel multispecific epitope binding proteins capable of binding multiple epitopes, and which comprise an Fc region of an antibody constant domain. As used herein the term "Fc region" refers to a polypeptide comprising the CH3, CH2 and at least a portion of the hinge region of a constant domain of an antibody. Optionally, an Fc region may include a CH4 domain, present in some antibody classes. An Fc region, as used herein may comprise the entire hinge region of a constant domain of an antibody. In one embodiment, multispecific epitope binding proteins of the invention comprise an Fc region and a CH1 region of an antibody. In another embodiment, multispecific epitope binding proteins of the invention comprise an Fc region, a CH1 region and a Ckappa/lambda region from the constant domain of an antibody. In one embodiment, the multispecific epitope binding proteins and/or polypeptide chains of the invention as described herein do not exist in nature or are not native sequence (composition and orientation) Ig molecules. Additionally, in one embodiment, multispecific epitope binding proteins as described herein are not produced *in vitro* by chemically cross-linking a pair of antibodies or antigen binding fragments thereof.

The multispecific epitope binding proteins of the invention comprise one, two, three, four, or more polypeptide chains. In specific embodiments the epitope binding proteins of the invention comprise between two to four polypeptide chains. Each chain of the multispecific epitope binding protein of the invention may comprise 1, 2, 3, 4, 5, 6, 7, 8 or more epitope binding domains. The epitope binding domains may be scFvs, single chain diabodies, variable regions of antibodies (*e.g.,* heavy chain and/or light chain variable regions), peptidomimetics, or other epitope binding domains known in the art.

The Fc region and the epitope binding domains that are comprised within the polypeptide chains may be linked (as used herein throughout "linked" may refer to directly adjacent to, or indirectly linked with intervening sequences or structures, for example an Fc region linked to an epitope binding domain may be directly adjacent to the epitope binding domain, or the Fc region may be linked through intervening sequences to the epitope binding domain) together in many different orientations. In an embodiment, the epitope binding domains of one or more chains are linked to the C-terminus of the Fc region. In other embodiments, the epitope binding domains of one or more chains are linked to the N-terminus of the Fc region. In other embodiments, the epitope binding domains of one or more chains are linked to both the N-terminus and C-terminus of the Fc region.

The multispecific epitope binding proteins of the invention may be dimers, trimers, tetramers, or higher order multimers and may be homomeric or heteromeric, that is they may comprise multiple polypeptide chains that are the same or different. In one embodiment multispecific epitope binding proteins of the invention may comprise homodimers or heterodimers of two polypeptide chains. In another embodiment, multispecific epitope binding proteins of the invention may comprise homotrimers or heterotrimers of three polypeptide chains. In still another embodiment, multispecific epitope binding proteins of the invention may comprise homotetramers or heterotetramers of four polypeptide chains. The polypeptide chains of multispecific epitope binding proteins of the invention may multimerize (*i.e*., assemble) through the presence of components of the Fc region, namely the CH3, CH2, the hinge region (or a portion thereof) and/or the CH1 region. The polypeptide chains of multispecific epitope binding proteins of the invention may, alternatively or in addition, multimerize (*i.e.,* assemble) through the interaction of other domains present in the constituent polypeptide chains.

In one embodiment, multispecific epitope binding proteins of the invention are capable of binding multiple epitopes concurrently (for example, *in vivo* or *in vitro).* In one embodiment, the epitope binding domains of multispecific epitope binding proteins of the invention are capable of binding at least 1, 2, 3, 4, 5, 6, 7, 8, or more epitopes concurrently (for example, *in vivo* or *in vitro).* In another embodiment, each epitope binding domain is specific for the same epitope. In another embodiment, multispecific epitope binding proteins of the invention comprise one or more epitope binding domains that are specific for distinct epitopes.

In one embodiment, each binding domain within the multispecific epitope binding proteins of the invention are specific for the same or distinct epitopes. In one embodiment, each binding domain within the epitope binding proteins of the invention have different (i.e. a higher or lower) affinity for the antigen compared to the isolated binding domain.

It is known that the immune system inhibits, reduces, or ablates specific targets through the effector function of antibodies. Antibody Dependent Cell-Mediated Toxicity (ADCC) and Complement Dependent Cytotoxicity (CDC) are two of the effector functions regulated by the Fc region of traditional antibodies. In one embodiment, multispecific epitope binding proteins of the invention retain the ability to efficiently stimulate effector function through the Fc region. In some situations, effector function is not desired, for example, when blocking the interaction between ligand and receptor is sufficient to achieve the desired outcome. Accordingly, in some embodiments, multispecific epitope binding proteins of the invention may not elicit effector function directed at a target.

The invention also provides methods of producing multispecific epitope binding proteins of the invention. In one embodiment, the polypeptide chains which form multispecific epitope binding proteins of the invention may be expressed from a single vector, or from multiple vectors. The arrangement of the coding regions of the polypeptide chain binding domains within the vector can be varied. For example, the orientation of the coding region for the Fc region (e.g., the CH3, CH2, the hinge region (or a portion thereof) and/or the CH1 region) may be 5' or 3' to the coding region of any of the epitope binding domains contained within the multispecific epitope binding polypeptide chain. Similarly, the orientation of the coding region of any of the epitope binding domains may be 5' and/or 3' to the coding region for the Fc region. In some embodiments, the coding region of an epitope binding domain is present both 5' and 3' to the coding region for the Fc region. In certain embodiments, a CH1 domain may be present 5' to the coding region for the Fc region.

The multispecific epitope binding proteins of the invention can be produced in many different expression systems. In one embodiment, the multispecific epitope binding proteins of the invention are produced and secreted by mammalian cells. In a specific embodiment, multispecific epitope binding proteins of the invention are produced in and secreted from human cells. In still other embodiments multispecific epitope binding proteins of the invention are produced in and isolated from plant cells.

The invention also provides methods of using multispecific epitope binding proteins of the invention. For example, many cell types express various common surface antigens and it is the specific combination of antigens that distinguish a specific class of cells. Using proteins of the invention, in one embodiment, it is possible to target specific subsets of cells without cross-reacting with other unrelated populations of cells. Also, many cell surface receptors activate or deactivate as a consequence of crosslinking of the subunits. In another embodiment, proteins of the invention may be used to stimulate or inhibit a response in a target cell by crosslinking cell surface receptors. In another embodiment, multispecific epitope binding proteins of the invention may be used to block the interaction of multiple cell surface receptors with antigens. In another embodiment, multispecific epitope binding proteins of the invention may be used to strengthen the interaction of multiple cell surface receptors with antigens. In another embodiment, it may be possible to crosslink homodimers of a cell surface receptor using multispecific epitope binding proteins of the invention containing binding domains that share specificity for the same antigen.

The invention also provides methods of targeting epitopes not easily targeted with traditional antibodies. In one embodiment, multispecific epitope binding proteins of the invention may be used to first target an adjacent antigen and while binding, another binding domain may engage a cryptic epitope, e.g., an epitope not accessible until the first target is bound.

The invention also provides methods of using multispecific epitope binding proteins to bring together (*i.e* in closer proximity) distinct cell types. In one embodiment, proteins of the invention may bind a target cell with one binding domain and recruit another cell via another binding domain. In a specific embodiment, the first cell may be a cancer cell and the second cell is an immune effector cell such as an NK cell. In another embodiment, multispecific epitope binding protein of the invention may be used to strengthen the interaction between two distinct cells, such as an antigen presenting cell and a T cell to possibly boost the immune response.

The present invention also encompasses the use of multispecific epitope binding proteins of the invention for the prevention, management, diagnosis, treatment or amelioration of one or more symptoms associated with diseases, disorders or infections, including but not limited to cancer, inflammatory and autoimmune diseases either alone or in combination with other therapies. The invention also encompasses the use of multispecific epitope binding proteins of the invention conjugated or fused to a moiety (*e.g.,* therapeutic agent or drug) for prevention, management, treatment or amelioration of one or more symptoms associated with diseases, disorders or infections, including but not limited to cancer, inflammatory and autoimmune diseases either alone or in combination with other therapies.

The invention also provides methods of using multispecific epitope binding proteins as diagnostic reagents. The multiple binding specificities may be useful in kits or reagents where different antigens need to be efficiently captured concurrently.

### 4. Brief Description of the Figures:

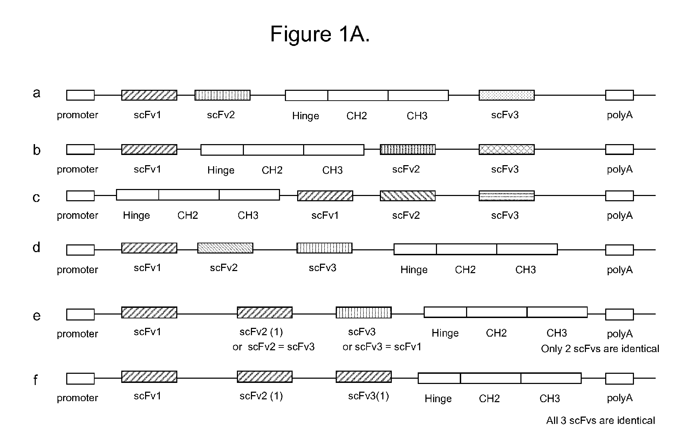

**Figure 1A****.** Presented is a diagram of examples of multispecific epitope binding protein expression vectors comprising a promoter, a polynucleotide sequence encoding three scFvs, a hinge-CH2-CH3 (Fc region) and a poly A tail. The various orientations of the hinge-CH2-CH3 are presented. Regions of the same color or shade are intended to represent identical or duplicate epitope binding domains within a construct. Also, presented is an embodiment comprising duplicated (2) scFvs within the multispecific epitope binding protein (inset e). In addition, presented is an embodiment comprising identical (3) scFvs within the multispecific epitope binding protein (inset f).

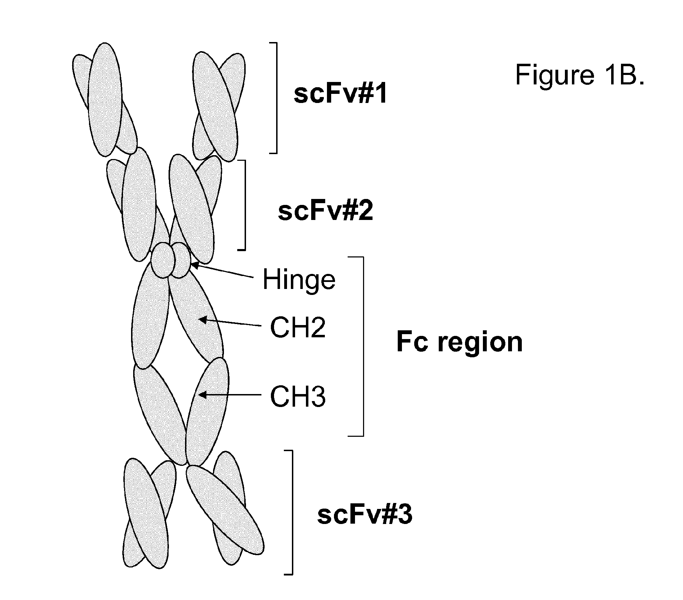

**Figure 1****B.** Presented is a diagram of a multispecific epitope binding protein assembled from a construct presented in Figure 1A (inset a). The epitope binding protein is a homodimer of two polypeptide chains each comprising three scFvs and a hinge-CH2-CH3 (Fc region).

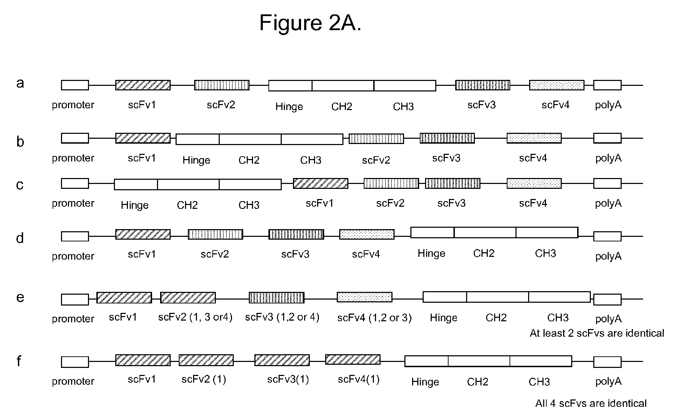

**Figure 2A****.** Presented is a diagram of examples of multispecific epitope binding protein expression vectors comprising a promoter, a polynucleotide sequence encoding four scFvs, a hinge- CH2-CH3 (Fc region) and a poly A tail. The various orientations of the hinge-CH2-CH3 (Fc region) are presented. Regions of the same color or shade are intended to represent identical or duplicate epitope binding domains within a construct. Also, presented is an embodiment comprising duplicated scFvs within the multispecific epitope binding protein (inset e). In addition, an embodiment comprising four identical scFvs is also presented (inset f).

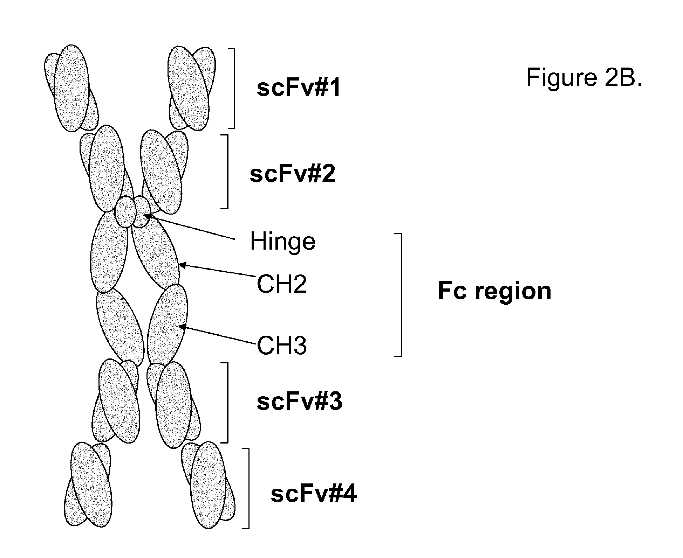

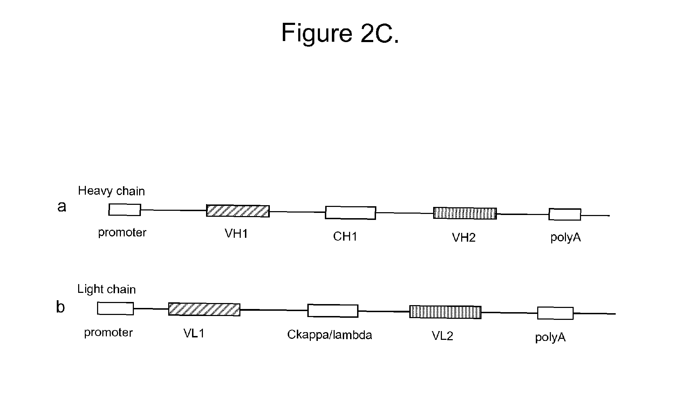

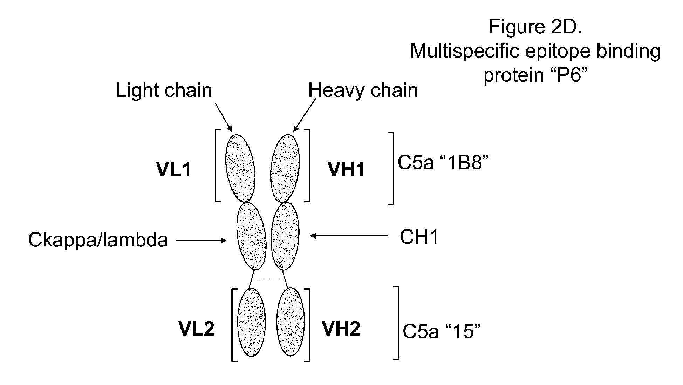

**Figure 2****B.** Presented is a representative diagram of a multispecific epitope binding protein assembled from a construct presented in Figure 2A (inset a). The epitope binding protein is a homodimer of two polypeptide chains each comprising four scFvs and a hinge-CH2-CH3 (Fc region).

**Figure 2C****.** Presented is a diagram of an example of multispecific epitope binding protein expression vectors encoding two distinct polypeptide chains. The heavy chain comprises a promoter, a polynucleotide sequence encoding 2 antibody heavy chain variable regions (VH1, VH2) flanking a CH1 domain and a poly A tail. The light chain comprises a promoter, a polynucleotide sequence encoding 2 antibody light chain variable regions (VL1, VL2) flanking a Ckappa/lambda region and a poly A tail. Regions of the same color or shade are intended to represent identical epitope binding specificities within a construct.

**Figure 2D****.** Presented is a diagram of an example of a multispecific epitope binding protein "P6" assembled from a construct similar to a construct presented in Figure 2C. The multispecific epitope binding protein is a heterodimer of two polypeptide chains. The heavy chain comprises two antibody variable regions (VH1, VH2) flanking a CH1 domain. The light chain comprises two antibody variable regions (VL1, VL2) flanking a Ckappa/lambda domain. The variable regions VH1 and VL1 have been derived from the C5a specific antibody 1B8. The variable regions VH2 and VL1 have been derived from the C5a specific antibody 15. The epitopes for the 1B8 and 15 antibodies are distinct.

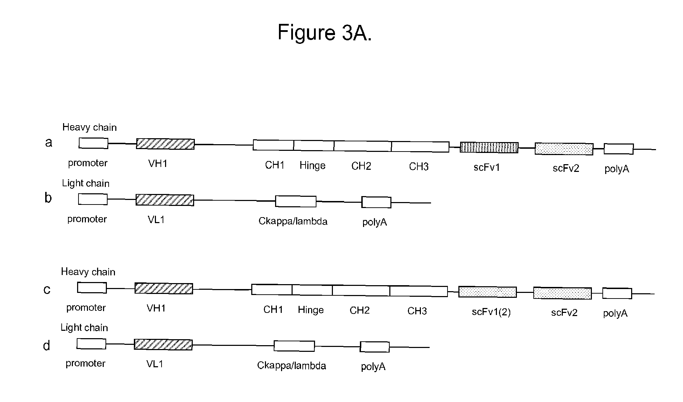

**Figure 3A****.** Presented is a diagram of examples of multispecific epitope binding protein expression vectors comprising a promoter, a polynucleotide sequence encoding two distinct polypeptide chains. The heavy chain comprises a promoter, a polynucleotide sequence encoding a VH domain, an Fc region comprising a Hinge, CH2, and a CH3 (Fc region), A CH1 region, two scFvs and a poly A tail. The light chain comprises a promoter, a polynucleotide sequence encoding a VL domain a Ckappa/lambda region and a poly A tail. Regions of the same color or shade are intended to represent identical or duplicate epitope binding domains within a construct. Also, presented is an embodiment comprising duplicated scFvs within the multispecific epitope binding protein (inset c).

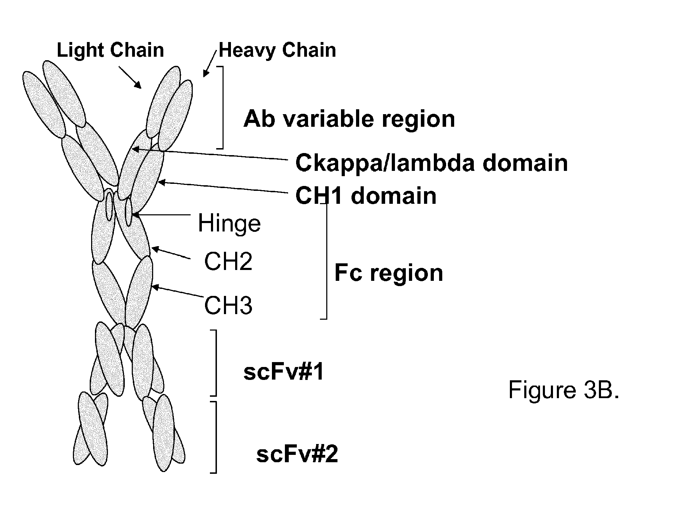

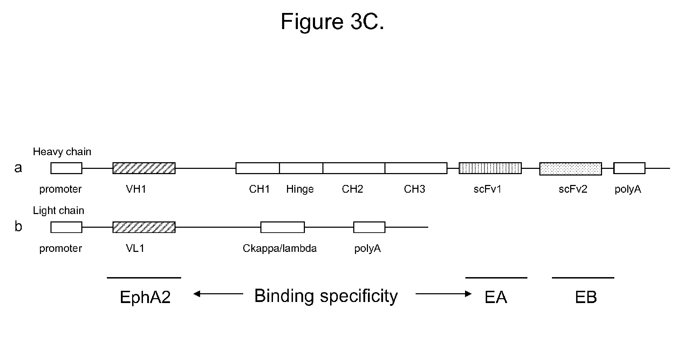

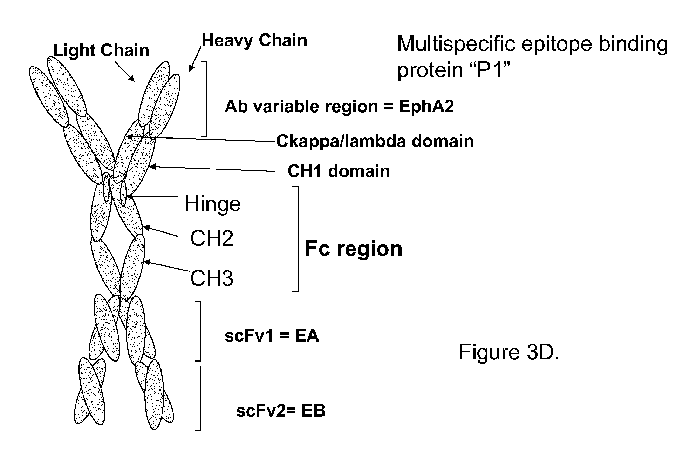

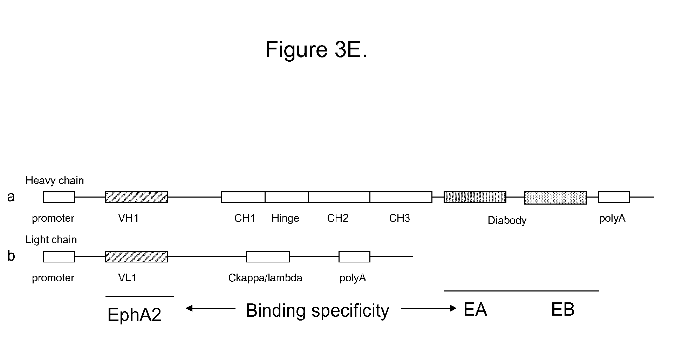

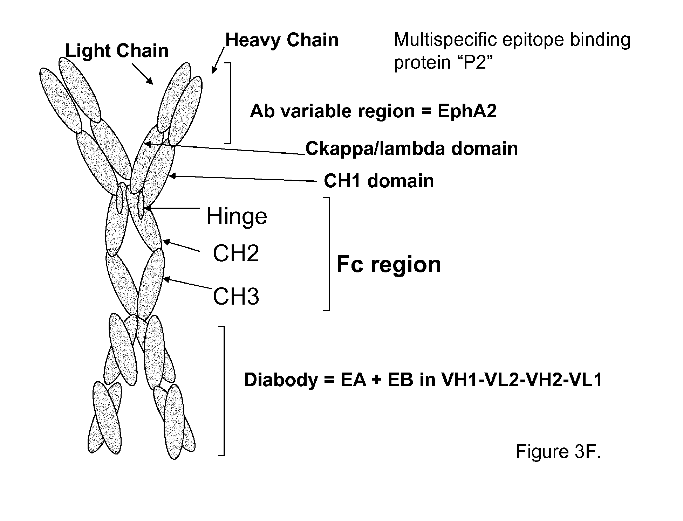

**Figure 3****B.** Presented is a diagram of a multispecific epitope binding protein assembled from a construct presented in Figure 3A (inset a,b). The epitope binding protein is multimer of four polypeptide chains, two heavy chains and two light chains. Each heavy chain comprises VH domains, a Hinge, CH2, and a CH3 (Fc region), a CH1 and two scFvs and each light chain comprises a VL domain and a Ckappa/lambda region.

**Figure 3C****.** Presented is a diagram of an expression vector utilized to produce a multispecific epitope binding protein "P1" as described in the Examples. The heavy chain vector comprises a promoter, a polynucleotide sequence encoding a VH domain, an Fc region comprising a hinge, CH2 and CH3, a CH1, 2 scFvs and a poly A tail. The VH domain is specific for EphA2 while scFv1 represents "EA", an scFv specific for an EphA family RTK and scFv2 represents "EB", an scFv specific for an EphB family RTK. The light chain vector comprises a promoter, a polynucleotide sequence encoding a VL domain, a Ckappa/lambda and a poly A tail. The VL domain present on the light chain encodes a VL domain specific for EphA2.

**Figure 3D****.** Presented is a diagram of a multispecific epitope binding protein assembled form the construct presented in Figure 3C. Protein "P1" comprises four chains, two heavy chains and two light chains. Each heavy chain comprises a VH domain specific for EphA2, a hinge-CH2-CH3 (Fc region), an scFv specific for an EphA family RTK, and an scFv specific for an EphB family RTK. Each light chain comprises a VL domain specific for EphA2 and a Ckappa/lambda domain.

**Figure 3E****.** Presented is a diagram of an expression vector utilized to produce a multispecific epitope binding protein "P2" as described in the Examples. The heavy chain vector comprises a promoter, a polynucleotide sequence encoding a VH domain, a hinge, CH2 and CH3 (Fc region), a CH1, a single chain diabody and a poly A tail. The VH domain is specific for EphA2 while the single chain diabody represents the scFvs "EA" specific for an EphA family RTK and "EB" scFv specific for an EphB family RTK. The light chain vector comprises a promoter, a polynucleotide sequence encoding a VL domain, a Ckappa/lambda and a poly A tail. The VL domain present on the light chain encodes a VL domain specific for EphA2.

**Figure 3F****.** Presented is a diagram of a multispecific epitope binding protein assembled from the construct presented in 3E. Protein "P2" comprises two heavy chains and two light chains, each heavy chain comprising a VH domain specific for EphA2, a hinge, CH2, CH3 (Fc region) and a single chain diabody represents the scFvs "EA" specific for an EphA family RTK and "EB" scFv specific for an EphB family RTK. Each light chain comprises a VL domain specific for EphA2 and a Ckappa/lambda.

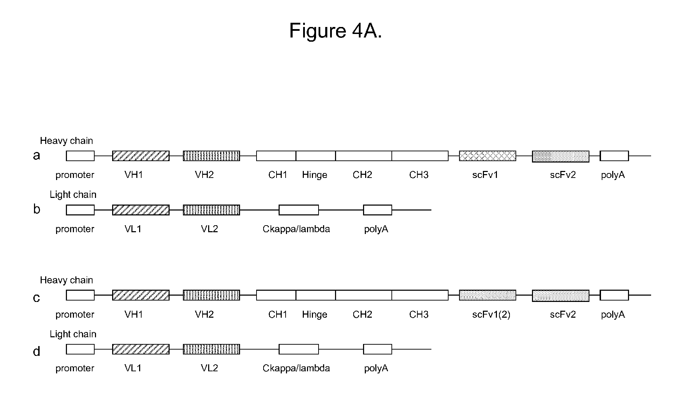

**Figure 4A****.** Presented is a diagram of an example of a multispecific epitope binding protein expression vector comprising a polynucleotide sequence encoding two distinct polypeptide chains. The heavy chain vector comprises a promoter, a polynucleotide sequence encoding two VH domains, an Fc region comprising a CH1, Hinge, CH2, and a CH3 (Fc region), two scFvs and a poly A tail. The light chain vector comprises a promoter, a polynucleotide sequence encoding two VL domains, a Ckappa/lambda and a poly A tail. Regions of the same color or shade are intended to represent identical or duplicate epitope binding domains within a construct. Also, presented is an embodiment comprising duplicated scFvs within the multispecific epitope binding protein (inset c).

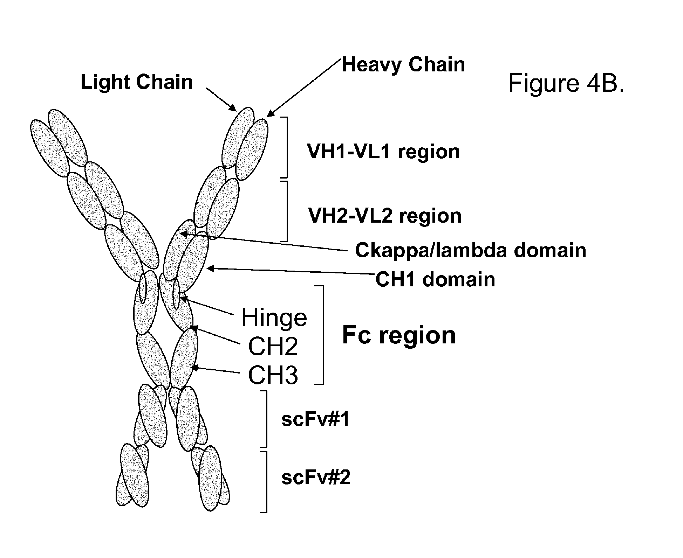

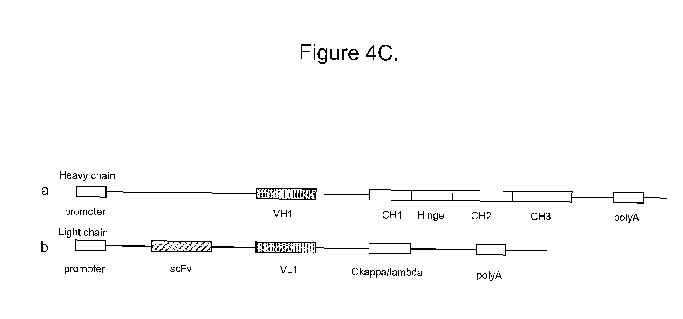

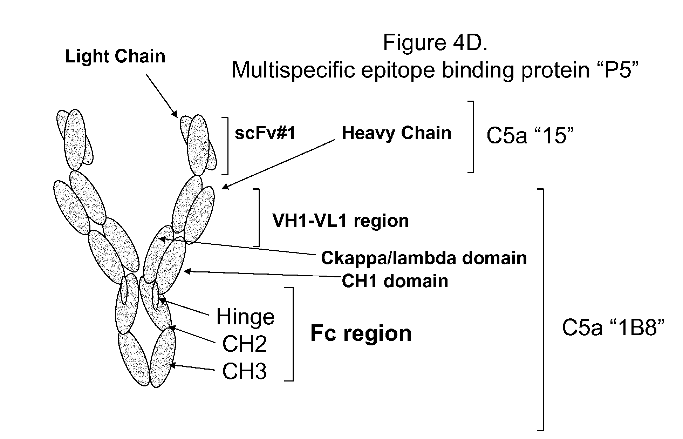

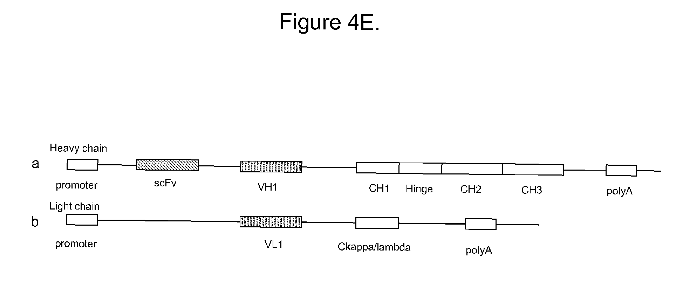

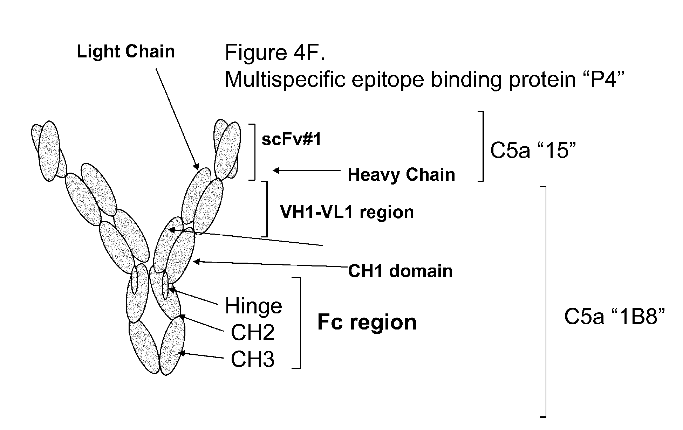

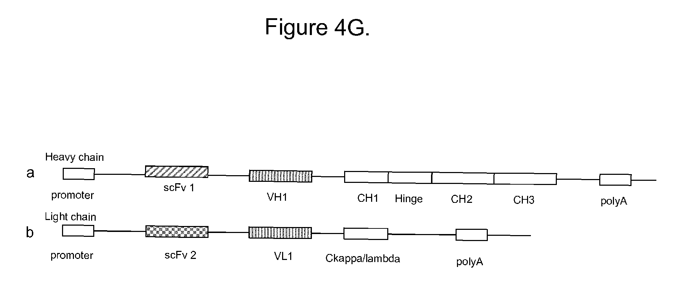

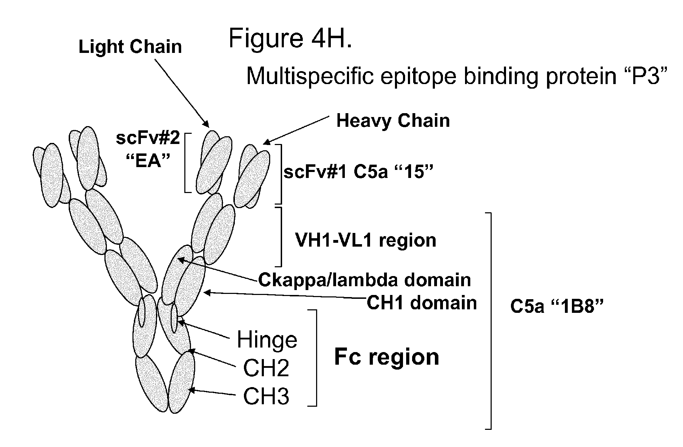

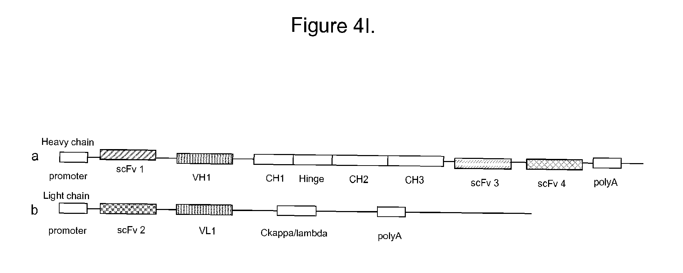

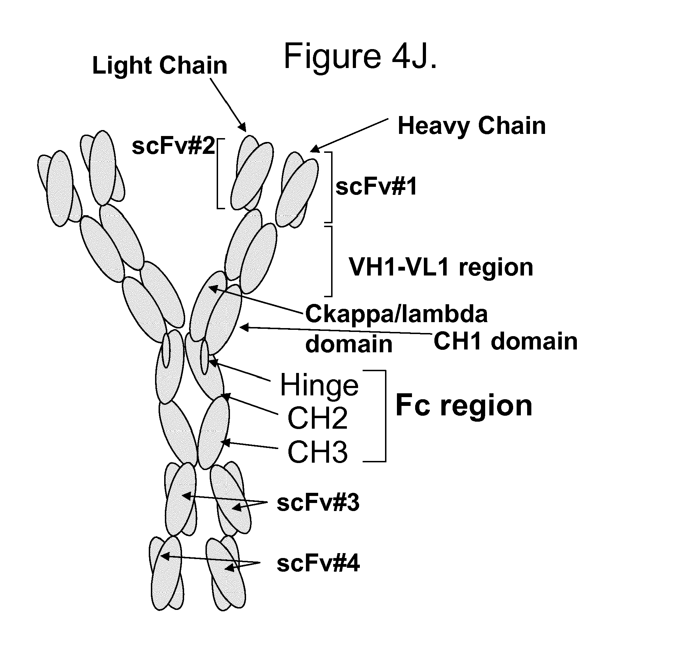

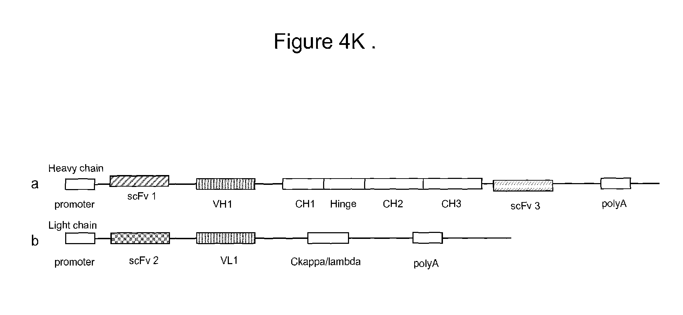

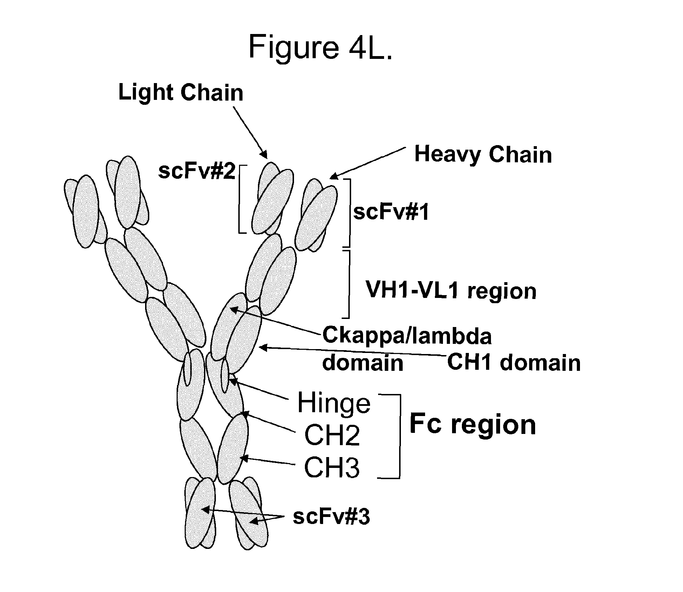

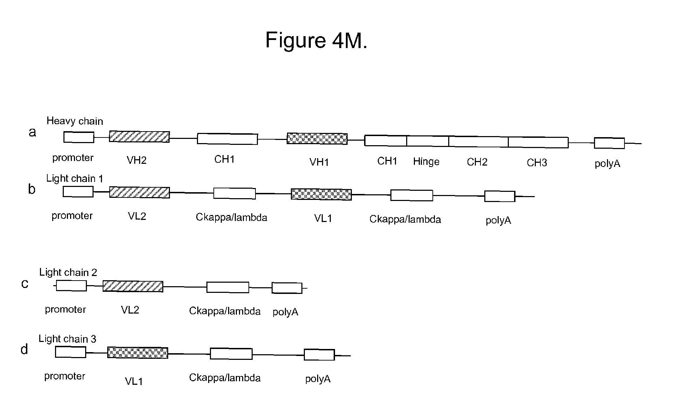

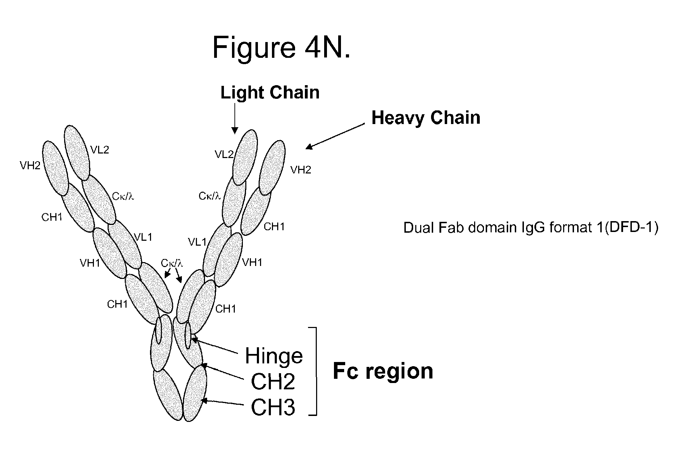

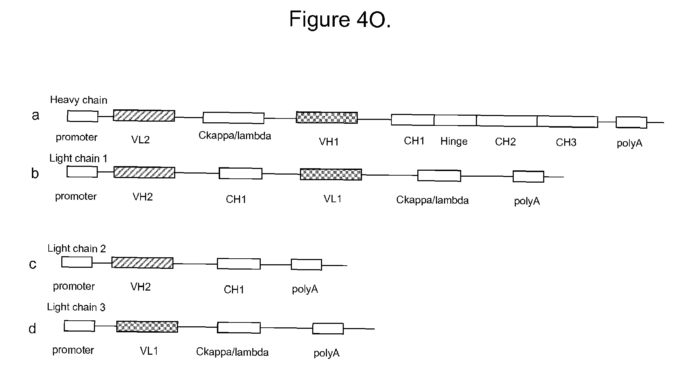

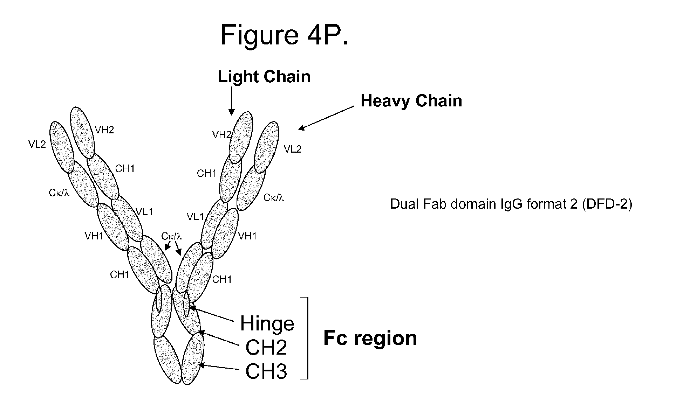

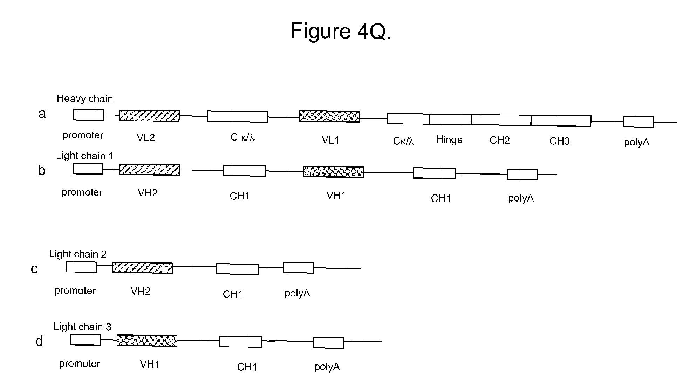

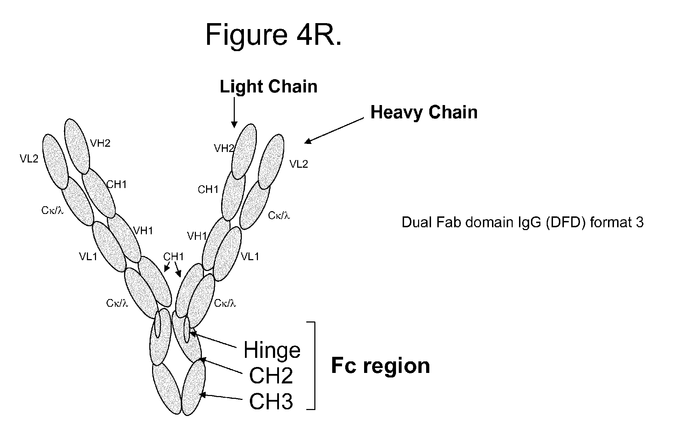

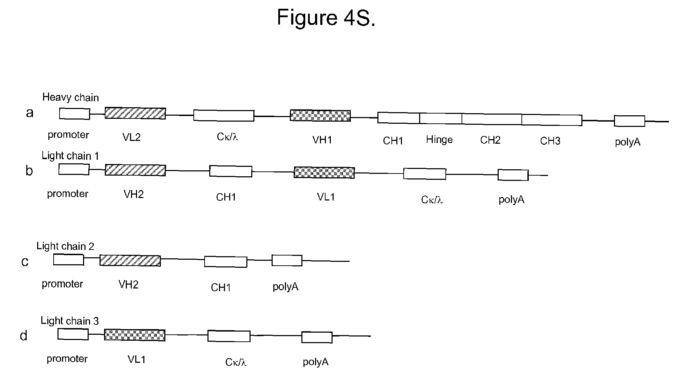

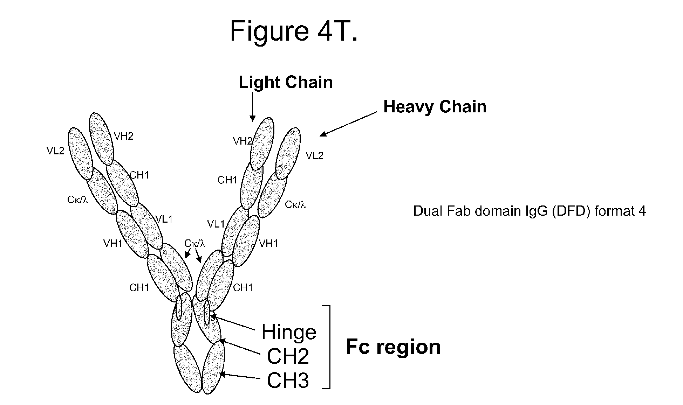

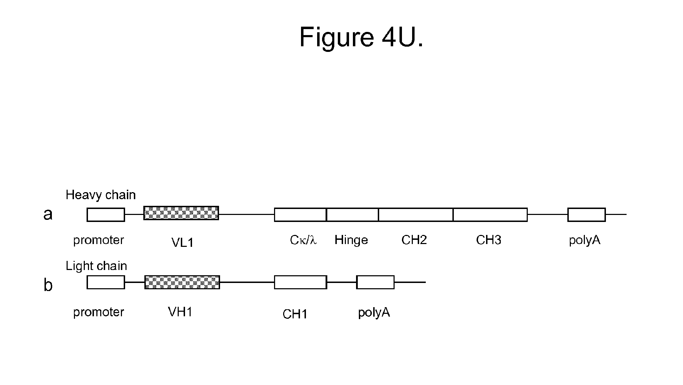

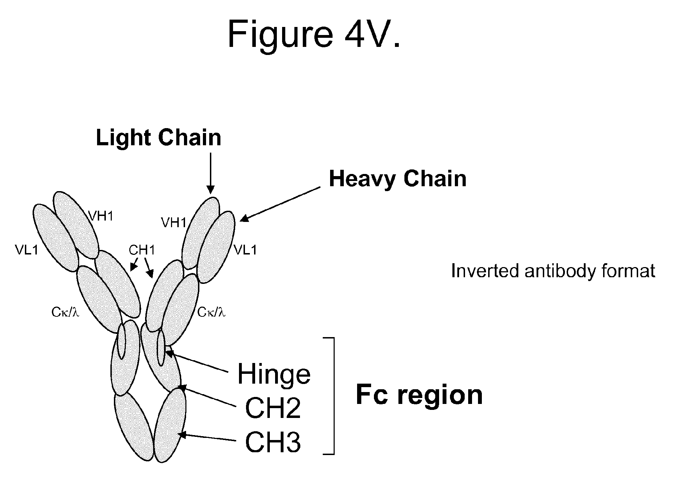

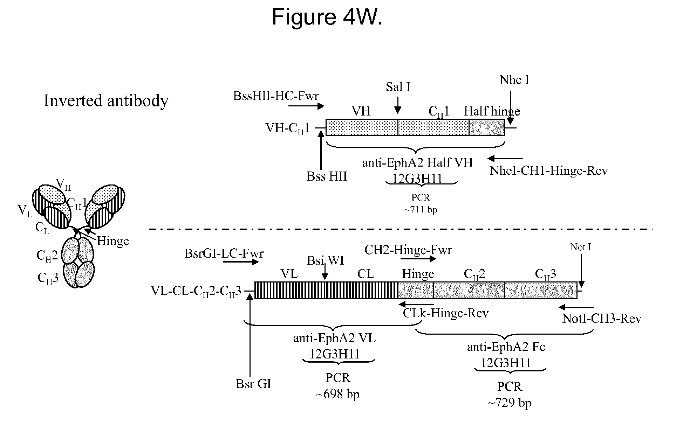

**Figure 4****B.** Presented is a diagram of an example of a multispecific epitope binding protein assembled from a construct presented in Figure 4A (inset a and b). The epitope binding protein is a multimer of four polypeptide chains, two heavy chains each comprising two VH domains, a Fc region comprising a CH1, Hinge, CH2, and a CH3 (Fc region), and two scFvs, and two light chains each comprising two VL domains and a Ckappa/lambda region.

**Figure 4C****.** Presented is a diagram of an example of a multispecific epitope binding protein expression vector comprising a polynucleotide sequence encoding two distinct polypeptide chains. The heavy chain vector comprises a promoter, a polynucleotide sequence encoding a VH domain, an Fc region comprising a CH1, Hinge, CH2, and a CH3 and a poly A tail. The light chain vector comprises a promoter, a polynucleotide sequence encoding an scFv, a VL domain, a Ckappa/lambda and a poly A tail. Regions of the same color or shade are intended to represent domains with identical binding specificities.

**Figure 4D****.** Presented is a diagram of an example of a multispecific epitope binding protein assembled from the construct presented in Figure 4C. The epitope binding protein is a multimer of four polypeptide chains, two heavy chains each comprising a VH1 domain, an Fc region comprising a CH1, Hinge, CH2, and a CH3, and two light chains, each light chain comprising an scFv, a VL and a Ckappa/lambda region.

**Figure 4E****.** Presented is a diagram of an example of a multispecific epitope binding protein expression vector comprising a polynucleotide sequence encoding two distinct polypeptide chains. The heavy chain vector comprises a promoter, a polynucleotide sequence encoding an scFv domain, VH domain, an Fc region comprising a CH1, Hinge, CH2, and a CH3 and a poly A tail. The light chain vector comprises a promoter, a polynucleotide sequence encoding a VL domain, a Ckappa/lambda and a poly A tail. Regions of the same color or shade are intended to represent domains with identical binding specificities.

**Figure 4F****.** Presented is a diagram of an example of a multispecific epitope binding protein assembled from the construct presented in Figure 4E. The epitope binding protein is a multimer of four polypeptide chains, two heavy chains each comprising an scFv domain, a VH1 domain, an Fc region comprising a CH 1, Hinge, CH2, and a CH3, and two light chains, each light chain comprising a VL and a Ckappa/lambda region.

**Figure 4G****.** Presented is a diagram of an example of a multispecific epitope binding protein expression vector comprising a polynucleotide sequence encoding two distinct polypeptide chains. The heavy chain vector comprises a promoter, a polynucleotide sequence encoding an scFv domain, VH domain, an Fc region comprising a CH1, Hinge, CH2, and a CH3 and a poly A tail. The light chain vector comprises a promoter, a polynucleotide sequence encoding an scFv domain, a VL domain, a Ckappa/lambda and a poly A tail. Regions of the same color or shade are intended to represent domains with identical binding specificities.

**Figure 4H****.** Presented is a diagram of an example of a multispecific epitope binding protein assembled from the construct presented in Figure 4G. The epitope binding protein is a multimer of four polypeptide chains, two heavy chains each comprising an scFv domain, a VH1 domain, an Fc region comprising a CH1, Hinge, CH2, and a CH3, and two light chains, each light chain comprising an scFv domain, a VL and a Ckappa/lambda region. This multispecific epitope binding protein "P3" is comprised of an antibody that binds C5a (1B8), an scFv#1(15) that also binds an epitope of C5a, and a scFv#2(EA) that binds an EphA family RTK.

**Figure 4I****.** Presented is a diagram of an example of a multispecific epitope binding protein expression vector comprising a polynucleotide sequence encoding two distinct polypeptide chains. The heavy chain vector comprises a promoter, a polynucleotide sequence encoding an scFv domain, VH domain, an Fc region comprising a CH1, Hinge, CH2, and a CH3, an additional 2 scFvs, and a poly A tail. The light chain vector comprises a promoter, a polynucleotide sequence encoding an scFv domain, a VL domain, a Ckappa/lambda and a poly A tail. Regions of the same color or shade are intended to represent domains with identical binding specificities.

**Figure 4****J.** Presented is a diagram of an example of a multispecific epitope binding protein assembled from the construct presented in Figure 4I. The epitope binding protein is a multimer of four polypeptide chains, two heavy chains each comprising an scFv domain, a VH1 domain, an Fc region comprising a CH1, Hinge, CH2, a CH3, and two additional scFvs and two light chains, each light chain comprising an scFv domain, a VL and a Ckappa/lambda region.

**Figure 4K****.** Presented is a diagram of an example of a multispecific epitope binding protein expression vector comprising a polynucleotide sequence encoding two distinct polypeptide chains. The heavy chain vector comprises a promoter, a polynucleotide sequence encoding an scFv domain, VH domain, an Fc region comprising a CH1, Hinge, CH2, and a CH3, an additional scFv, and a poly A tail. The light chain vector comprises a promoter, a polynucleotide sequence encoding an scFv domain, a VL domain, a Ckappa/lambda and a poly A tail. Regions of the same color or shade are intended to represent domains with identical binding specificities.

**Figure 4L****.** Presented is a diagram of an example of a multispecific epitope binding protein assembled from the construct presented in Figure 4K. The epitope binding protein is a multimer of four polypeptide chains, two heavy chains each comprising an scFv domain, a VH1 domain, an Fc region comprising a CH1, Hinge, CH2, a CH3, an additional scFv and two light chains, each light chain comprising an scFv domain, a VL and a Ckappa/lambda region.

**Figure 4M****.** Presented is a diagram of an example of a multispecific epitope binding protein expression vector comprising a polynucleotide sequence encoding four distinct polypeptide chains. The heavy chain vector (a) comprises a promoter, a polynucleotide sequence encoding a first VH domain (VH2), a first CH1 domain, a second VH domain (VH1), an Fc region comprising a CH1 (second), Hinge, CH2, and a CH3, and a poly A tail. The light chain 1 vector (b) comprises a promoter, a polynucleotide sequence encoding a first VL domain (VL2), a first Ckappa/lambda region, a second VL domain (VL1), a second Ckappa/lambda region and a poly A tail. The light chain 2 vector (c) comprises a promoter, a polynucleotide sequence encoding a VL domain (VL2), a Ckappa/lambda region, and a poly A tail. The light chain 3 vector (d) comprises a promoter, a polynucleotide sequence encoding a VL domain (VL1), a Ckappa/lambda region, and a poly A tail. It is intended that the heavy chain polypeptides encoded by the vector (a) construct may associate with either the polypeptide encoded by the light chain 1 vector (b) or a combination of light chains 2 and 3 (c + d) to form a functional multispecific epitope binding protein as presented in Figure 4N. Regions of the same color or shade are intended to represent domains with identical binding specificities.

**Figure 4N****.** Presented is a diagram of an example of a multispecific epitope binding protein (Dual Fab domain IgG format 1 (DFD-1) assembled from the construct presented in Figure 4M. The epitope binding protein may be a multimer of four polypeptide chains, two heavy chains each comprising a first VH domain (VH2), a first CH1 domain, a second VH domain (VH1) an Fc region comprising a CH1 (second), Hinge, CH2, a CH3 and two light chains, each light chain comprising a first VL domain (VL2), a first Ckappa/lambda region, a second VL domain (VL1), and a second Ckappa/lambda region. Alternatively, the epitope binding protein may be a multimer of six polypeptide chains, two heavy chains, two first light chains and two second light chains. The two heavy chains each comprise a first VH domain (VH2), a first CH1 domain, a second VH domain (VH1), a second CH1 domain and an Fc region comprising a Hinge, CH2, and a CH3. The two first light chains each comprise a VL domain (VL2) and a Ckappa/lambda (see Figure 4N, inset c). The two second light chains each comprise a VL domain (VL1) and a Ckappa/lambda region (see Figure 4N. inset d).

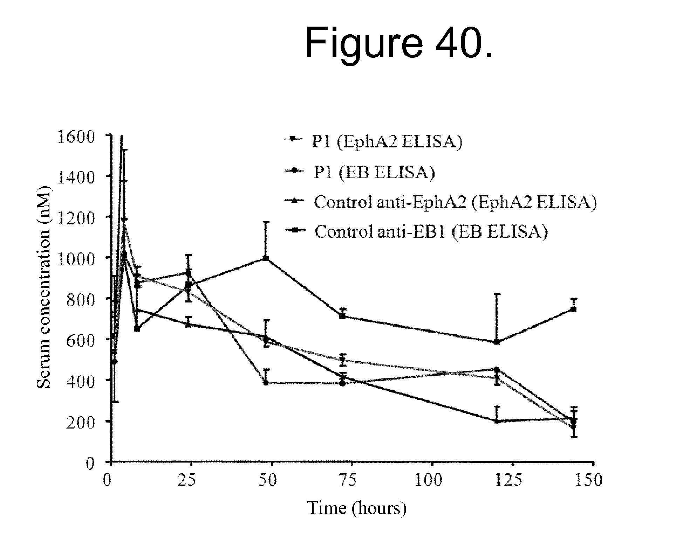

**Figure 40****.** Presented is a diagram of an example of a multispecific epitope binding protein expression vector comprising a polynucleotide sequence encoding four distinct polypeptide chains. The heavy chain vector (a) comprises a promoter, a polynucleotide sequence encoding a VL domain (VL2), a Ckappa/lambda region, a VH domain (VH1), an Fc region comprising a CH1, Hinge, CH2, and a CH3, and a poly A tail. The light chain 1 vector (b) comprises a promoter, a polynucleotide sequence encoding a VH domain (VH2), a CH1, a second VL domain (VL1), a Ckappa/lambda region and a poly A tail. The light chain 2 vector (c) comprises a promoter, a polynucleotide sequence encoding a VH domain (VH2), a CH1 and a poly A tail. The light chain 3 vector (d) comprises a promoter, a polynucleotide sequence encoding a VL domain (VL1), a Ckappa/lambda region, and a poly A tail. It is intended that the heavy chain polypeptides encoded by the vector (a) construct may associate with either the polypeptide encoded by the light chain 1 vector (b) or a combination of light chains 2 and 3 (c + d) to form a functional multispecific epitope binding protein as presented in Figure 4P. Regions of the same color or shade are intended to represent domains with identical binding specificities.

**Figure 4P****.** Presented is a diagram of an example of a multispecific epitope binding protein (Dual Fab domain IgG format 1 (DFD-2) assembled from the construct presented in Figure 40. The epitope binding protein may be a multimer of four polypeptide chains, two heavy chains each comprising a VL domain (VL2), a Ckappa/lambda region, a VH domain (VH1), an Fc region comprising a CH1, Hinge, CH2, a CH3 and two light chains, each light chain comprising a VH domain (VH2), a CH1, a VL domain (VL1), and a Ckappa/lambda region. Alternatively, the epitope binding protein may be a multimer of six polypeptide chains, two heavy chains, two first light chains and two second light chains. The two heavy chains each comprise a VL domain (VL2), a Ckappa/lambda region, a VH domain (VH1), an Fc region comprising a CH1, Hinge, CH2, and a CH3. The two first light chains each comprise a VH domain (VH2) and a CH1 (see Figure 40, inset c). The two second light chains each comprise a VL domain (VL1) and a Ckappa/lambda region (see Figure 40. inset d).

**Figure 4Q****.** Presented is a diagram of an example of a multispecific epitope binding protein expression vector comprising a polynucleotide sequence encoding four distinct polypeptide chains. The heavy chain vector (a) comprises a promoter, a polynucleotide sequence encoding a first VL domain (VL2), a first Ckappa/lambda region, a second VL domain (VL1), a second Ckappa/lambda region, and an Fc region comprising a Hinge, CH2, and a CH3, and a poly A tail. The light chain vector (b, light chain 1) comprises a promoter, a polynucleotide sequence encoding a first VH domain (VH2), a first CH1, a second VH domain (VH1), a second CH1 and a poly A tail. The light chain vector (c, light chain 2) comprises a promoter, a polynucleotide sequence encoding a VH domain (VH2), a CH1, and a poly A tail. The light chain vector (d, light chain 3) comprises a promoter, a polynucleotide sequence encoding a VH domain (VH1), a CH1, and a poly A tail. It is intended that the heavy chain polypeptides encoded by the vector (a) construct may associate with either the polypeptides encoded by the light chain 1 vector (b) or a combination of light chains 2 and 3 vectors (c + d) to form a functional multispecific epitope binding protein as presented in Figure 4R. Regions of the same color or shade are intended to represent domains with identical binding specificities.

**Figure 4R****.** Presented is a diagram of an example of a multispecific epitope binding protein (Dual Fab domain IgG format 1 (DFD-3) assembled from the constructs presented in Figure 4Q. The epitope binding protein may be a multimer of four polypeptide chains, two heavy chains each comprising a first VL domain (VL2), a first Ckappa/lambda region, a second VL domain (VL1), a second Ckappa/lambda region and an Fc region comprising a Hinge, CH2, a CH3 and two light chains, each light chain comprising a first VH domain (VH2), a first CH1, a second VH domain (VH1), and a second CH1. Alternatively, the presented multispecific epitope binding protein may be a multimer of six polypeptide chains, two heavy chains, two first light chains and two second light chains. The two heavy chains each comprise a first VL domain (VL2), a first Ckappa/lambda region, a second VL domain (VL1), a second Ckappa/lambda region and an Fc region comprising a Hinge, CH2, a CH3. The two first light chains each comprise a VH domain (VH2) and a CH1 (see Figure 4Q, inset c). The two second light chains each comprise a VH domain (VH1) and a CH1 (see Figure 4Q. inset d).

**Figure 4S****.** Presented is a diagram of an example of a multispecific epitope binding protein expression vector comprising a polynucleotide sequence encoding four distinct polypeptide chains. The heavy chain vector (a) comprises a promoter, a polynucleotide sequence encoding a VL domain (VL2), a Ckappa/lambda region, a VH domain (VH1), and an Fc region comprising a CH1, Hinge, CH2, and a CH3, and a poly A tail. The light chain vector (b, light chain 1) comprises a promoter, a polynucleotide sequence encoding a VH domain (VH2), a CH1, a VL domain (VL1), a Ckappa/lambda region and a poly A tail. The light chain vector (c, light chain 2) comprises a promoter, a polynucleotide sequence encoding a VH domain (VH2), a CH1, and a poly A tail. The light chain vector (d, light chain 3) comprises a promoter, a polynucleotide sequence encoding a VL domain (VL1), a Ckappa/lambda region, and a poly A tail. It is intended that the heavy chain polypeptides encoded by the vector a construct may associate with either the light chain 1 polypeptides (b) or a combination of light chains 2 and 3 (c + d) to form a functional multispecific epitope binding protein as presented in Figure 4T. Regions of the same color or shade are intended to represent domains with identical binding specificities.

**Figure 4T****.** Presented is a diagram of an example of a multispecific epitope binding protein (Dual Fab domain IgG format 1 (DFD-4) assembled from the constructs presented in Figure 4S. The epitope binding protein may be a multimer of four polypeptide chains, two heavy chains each comprising a VL domain (VL2), a Ckappa/lambda region, a VH domain (VH1), and an Fc region comprising a CH1, Hinge, CH2, a CH3 and two light chains, each light chain comprising a VH domain (VH2), a CH1, a VL domain (VL1), and a Ckappa/lambda region. Alternatively, the presented multispecific epitope binding protein may be a multimer of six polypeptide chains, two heavy chains, two first light chains and two second light chains. The two heavy chains each comprise a VL domain (VL2), a Ckappa/lambda region, a VH domain (VH1), and an Fc region comprising a CH1, Hinge, CH2, and a CH3. The two first light chains each comprise a VH domain (VH2) and a CH1 (see Figure 4S, inset c). The two second light chains each comprise a VL domain (VL1) and a Ckappa/lambda (see Figure 4S. inset d).

**Figure 4U****.** Presented is a diagram of an example of an inverted antibody expression vector comprising a polynucleotide sequence encoding two distinct polypeptide chains. The heavy-like chain or first vector (a) comprises a promoter, a polynucleotide sequence encoding a VL domain (VL1), a Ckappa/lambda region, an Fc region comprising a Hinge, CH2, and a CH3, and a poly A tail. The light-like chain or second vector (b) comprises a promoter, a polynucleotide sequence encoding a VH domain (VH1), a CH1, and a poly A tail. Regions of the same color or shade are intended to represent domains with identical binding specificities.

**Figure 4V****.** Presented is a diagram of an example of an inverted antibody assembled from the constructs presented in Figure 4U. The inverted antibody is a multimer of four polypeptide chains, two heavy-like chains each comprising a first VL domain (VL1), a Ckappa/lambda region, and an Fc region comprising a Hinge, CH2, a CH3 and two light-like chains, each light-like chain comprising a first VH domain (VH1) and a CH1.

**Figure 4W****.** Presented is a diagram representing the construction of an example of a type of inverted antibody as presented in Figure 4U and 4V. Briefly, using the parental anti-EphA2 antibody 12G3H11, a PCR based approach was implemented to aide in the construction of the antibody. Firstly, primers were used to amplify the VH, CH1 and part of the Hinge region from the heavy chain. Secondly, an additional set of primers were used to amplify the VL, CL, and part of the Hinge region, as well as the CH3, CH2, and an overlapping part of the Hinge region. Thirdly, a full length inverted heavy chain was constructed using overlapping PCR fragments and primers to the N-terminus of the VL region and the C-terminus of the CH3 region. Subsequent cloning results in a vector for the production of an "inverted antibody" as represented in Figure 4U and 4V.

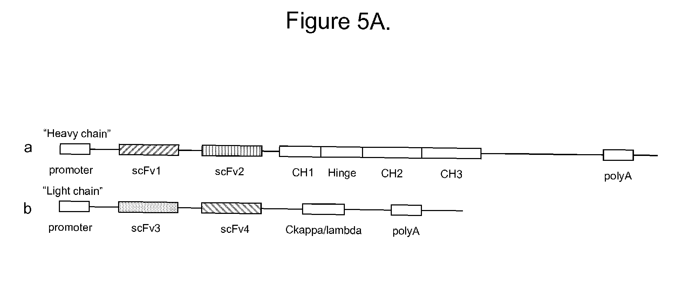

**Figure 5A****.** Presented is a diagram of a multispecific epitope binding protein expression cassette comprising two distinct polypeptide chains. The heavy chain comprises a promoter, two scFv domains, an Fc region comprising a CH1, Hinge, CH2, and a CH3, and a poly A tail. The light chain comprises a promoter, two scFv domains, a Ckappa/lambda region and a poly A tail. Also, presented is an embodiment comprising duplicated scFvs within the multispecific epitope binding protein.

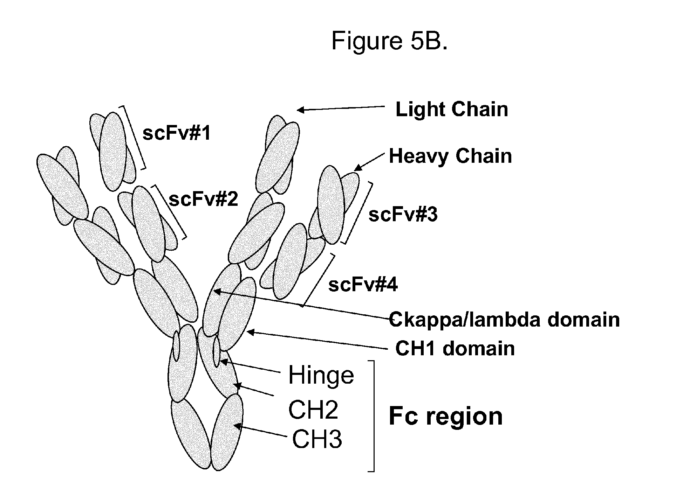

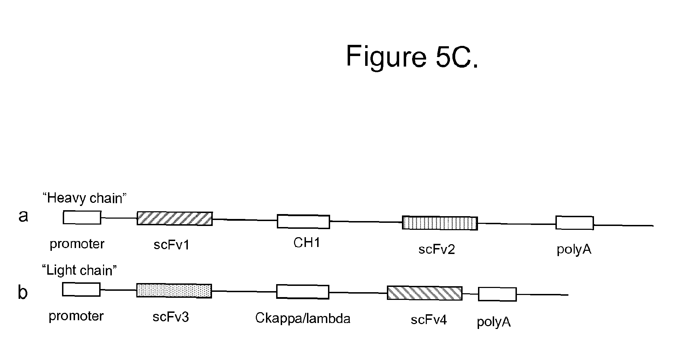

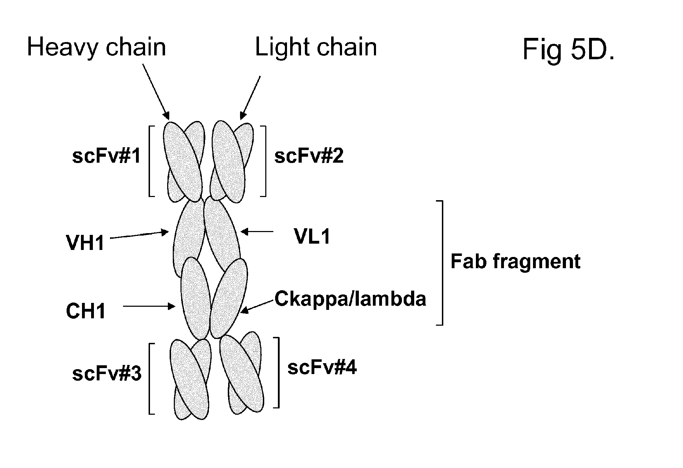

**Figure 5****B.** Presented is a diagram of an example of a multispecific epitope binding protein assembled from a construct presented in Figure 5A. The epitope binding protein is a multimer of four polypeptide chains, two heavy chains and two light chains. Each heavy chain comprises two scFv domains and an Fc region comprising a CH1, Hinge, CH2, and a CH3 domain and each light chain comprises two scFv domains, and a Ckappa/lambda region.

**Figure 5C****.** Presented is a diagram of an example of a multispecific epitope binding protein expression vector comprising a polynucleotide sequence encoding two distinct polypeptide chains. The heavy chain vector comprises a promoter, a polynucleotide sequence encoding an scFv domain, VH domain, a CH1 domain, an additional scFv domain and poly A tail. The light chain vector comprises a promoter, a polynucleotide sequence encoding an scFv domain, a VL domain, a Ckappa/lambda, an additional scFv domain, and a poly A tail.

**Figure 5D****.** Presented is a diagram of an example of a multispecific epitope binding protein assembled from a construct presented in Figure 5C. The epitope binding protein is a dimer of two polypeptide chains, one heavy chain and one light chain. The heavy chain comprises an scFv domain, an Fc region comprising a CH1 and an additional scFv domain and the light chain comprises an scFv domain, a Ckappa/lambda region and an additional scFv domain.

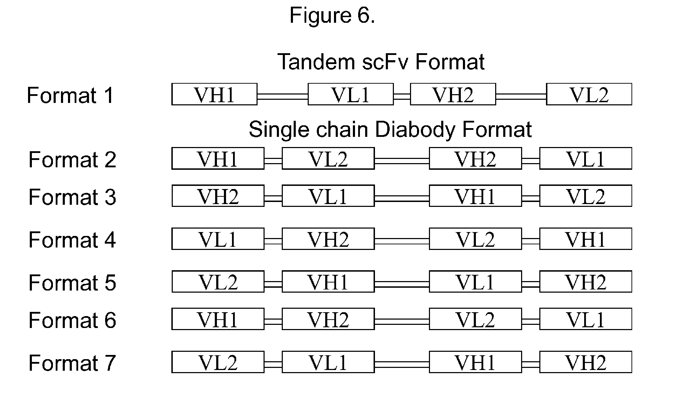

**Figure 6****.** Presented is a pictorial representation of the relative arrangements of VH and VL domains in scFv and single chain diabody formats present in polypeptide chains of the invention. Linker lengths are represented for each of the formats. For the scFv format (inset 1) the linker length between the VH and VL regions of each scFv unit is long (> greater than 5 amino acids) to facility scFv formation. Also the linker between each scFv is long to also facilitate the correct folding of the scFv units. The linker lengths for the single chain diabodies (inset 2-7) represent lengths required to facilitate the "folding over" VH and VL regions to form the single chain diabody structure.

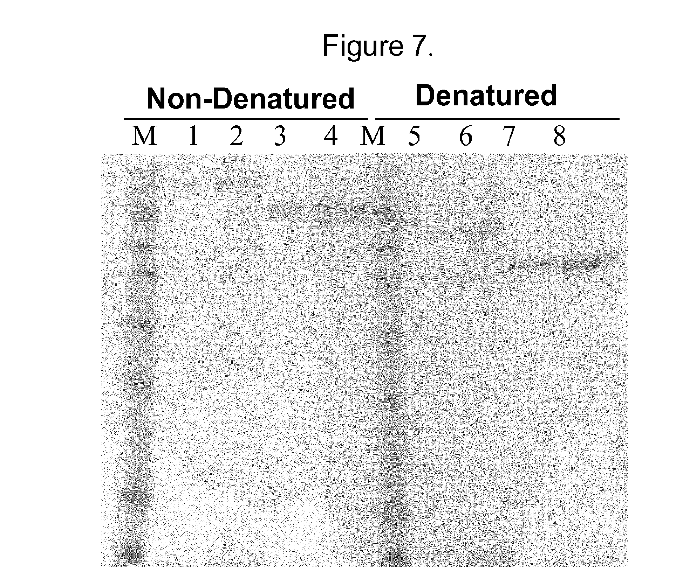

**Figure 7****.** Presented are the results of a PAGE gel experiment wherein various multispecific epitope binding proteins were subjected to (A) Non-denaturing or (B) denaturing conditions. Lanes 1 and 5 represent an scFv-Fc region protein (3F2-522) loaded at 1 µg/well. Lanes 2 and 6 represent an scFv-Fc region protein (3F2-522) loaded at 4 µg/well. Lanes 3 and 7 represent an scFv-Fc region protein (522-Fc) loaded at 1 µg/well. Lanes 4 and 8 represent an scFv-Fc region protein (522-Fc) loaded at 4 µg/well. Lane M represents standard molecular weight markers (SeeBlue 2™).

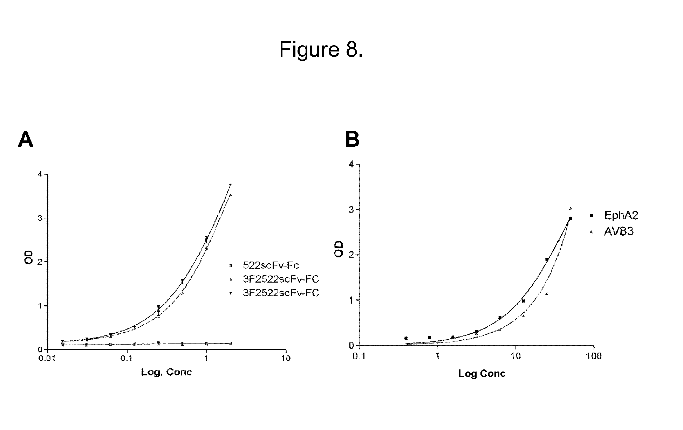

**Figure 8****.** Presented are the results from a co-binding experiment in an ELISA format. The results demonstrate multiple epitope binding proteins retain binding specificity of each isolated functional epitope binding domain. In (A) the ELISA plate is coated with αvβ3 integrin and incubated with 522-Fc region and 3F2522-Fc region. Plate bound proteins were detected by binding of biotinylated EphA2-Fc. In (B) the 3F2522-Fc region protein is captured on the ELISA plate by bound EphA2 or αvβ3 integrin and demonstrates dual specificity binding as measured by optical density.

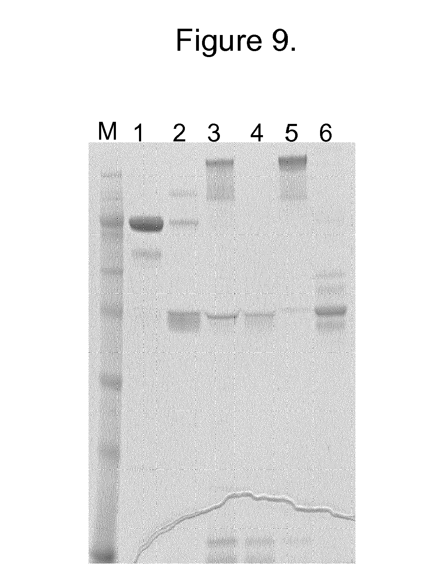

**Figure 9****.** Presented are the results from a polyacrylamide gel electrophoresis experiment of a collection of epitope binding proteins of the invention. Briefly, purified samples of each of the proteins of the invention were loaded and run on a PAGE gel and subsequently stained with Coomassie Blue. The proteins of the invention presented here are as follows: Lane 1 - 522-Fc, Lane 2- 3F2-522-Fc., Lane 3- P1, Lane 4 - 3F2-522-Fc region, Lane 5 -12G3H11-5-8, and Lane 6 - 3F2-522-Fc region.

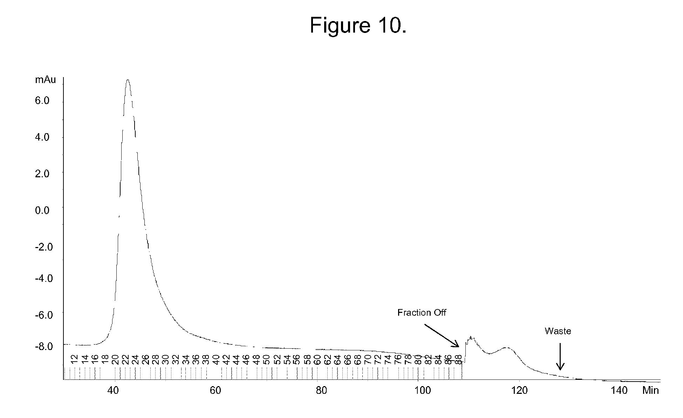

**Figure 10****.** Presented is the elution profile of the multispecific epitope binding protein, P2 from a Size exclusion chromatography column. The tracing represents the relative protein concentration in each column fraction (x axis). The results demonstrate that the P2 multiple epitope binding protein of the invention elutes as a single entity.

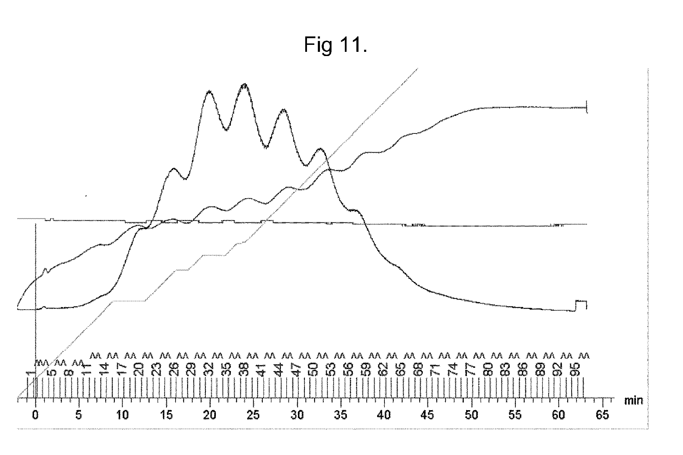

**Figure 11****.** Presented is the elution profile of the multispecific epitope binding protein P1 from a Cation Exchange Chromatography column. The tracing represents the relative protein concentration in each column fraction (x axis).

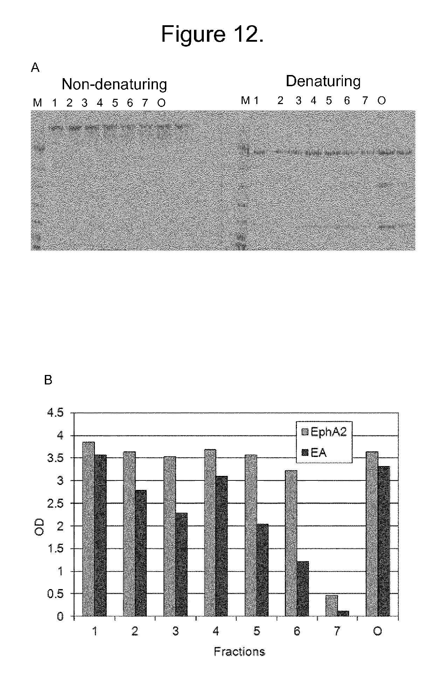

**Figure 12****.** Presented are the results from a PAGE analysis (A and B) and a dual specificity binding analysis (C) of pooled fractions from the Cation Exchange Chromatography presented in Figure 11. Pooled fractions are described in Example 6. In (A) samples were loaded and run on a non-denaturing polyacrylamide gel stained with Coomassie. In (B) the equivalent samples were loaded and run under denaturing conditions and stained with Coomassie. In (C) the equivalent samples were tested for dual binding specificities to EphA2 and another EphA family RTK (EA). Each sample demonstrated binding for both EphA2 and the other EphA family RTK (EA).

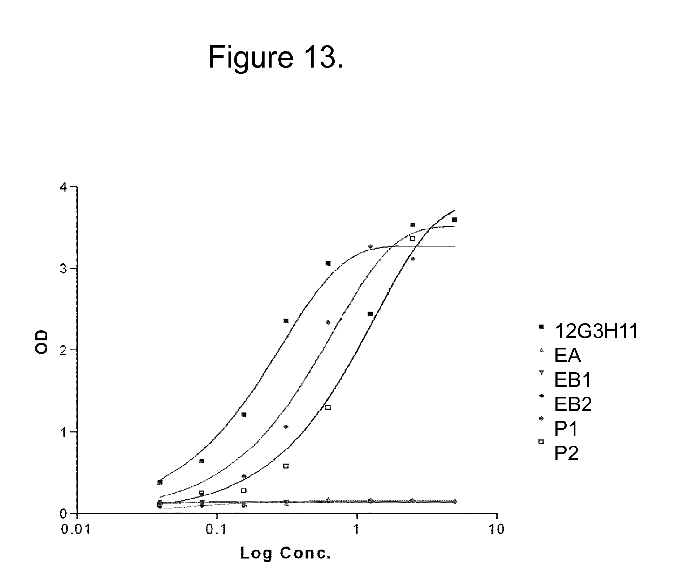

Figure 13. Presented are the results of a binding assay performed on various epitope binding proteins. Specifically demonstrated is the specificity for EphA2 by the antibody 12G3H11 and the multispecific epitope binding proteins P1 and P2. As expected, the EA, EB1 and EB2 epitope binding proteins do not exhibit specificity for EphA2.

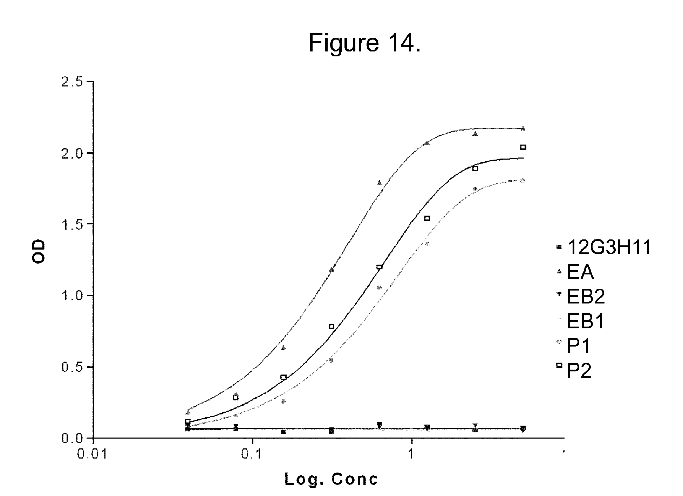

**Figure 14****.** Presented are the results of a binding assay performed on various epitope binding proteins. Specifically demonstrated is the specificity for an Eph A family RTK by the epitope binding protein EA and the multispecific epitope binding proteins P 1 and P2. The antibody 12G3H11 and the epitope binding proteins EB 1 and EB2 do not exhibit specificity for the Eph A family RTK.

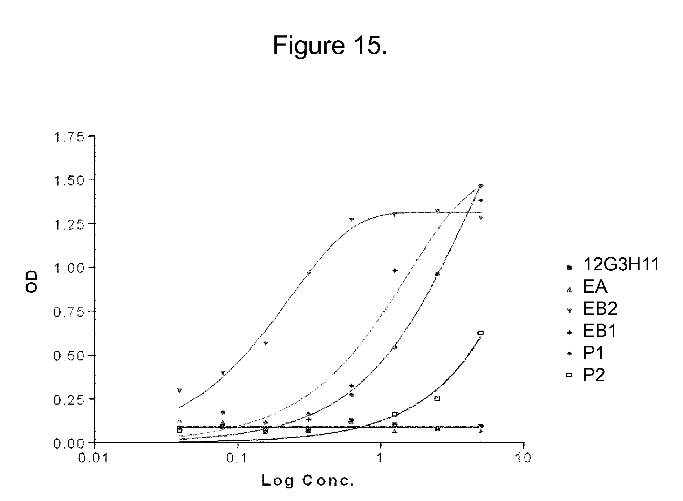

**Figure 15****.** Presented are the results of a binding assay performed on various epitope binding proteins. Specifically demonstrated here is the specificity for an Eph B family RTK by the epitope binding proteins EB 1 and EB2, and the multispecific epitope binding proteins P1 and P2. The antibody 12G3H11 and the epitope binding protein EA do not exhibit specificity for Eph B family RTK.

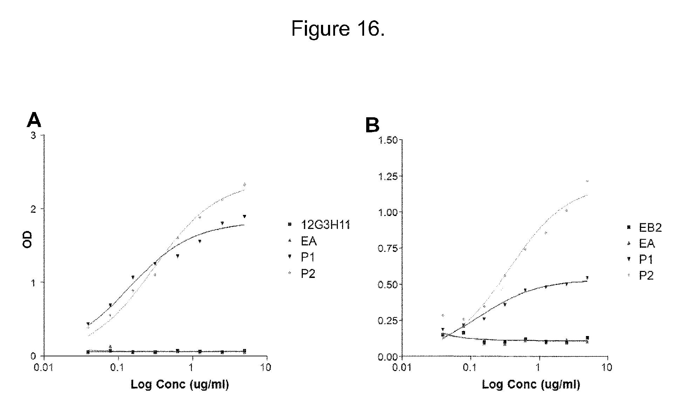

**Figure 16****.** Presented are the results of a dual specificity binding assay performed on various epitope binding proteins. In (A) the multispecific epitope binding proteins P1 and P2 are captured by plate-bound EphA2 and detected with biotinylated Eph A family RTK. This demonstrates that the multispecific epitope binding proteins P1 and P2 are capable of binding EphA2 and the Eph A family RTK concurrently. The antibody 12G3H11 and the monospecific epitope binding protein EA are incapable of binding both epitopes concurrently. In (B) the multispecific epitope binding proteins P1 and P2 are captured by plate-bound EphA2 and detected with biotinylated Eph B family RTK. This demonstrates that the multispecific epitope binding proteins P 1 and P2 are capable of binding EphA2 and the Eph B family RTK concurrently. The monospecific epitope binding proteins EA and EB1 are incapable of binding both epitopes concurrently.

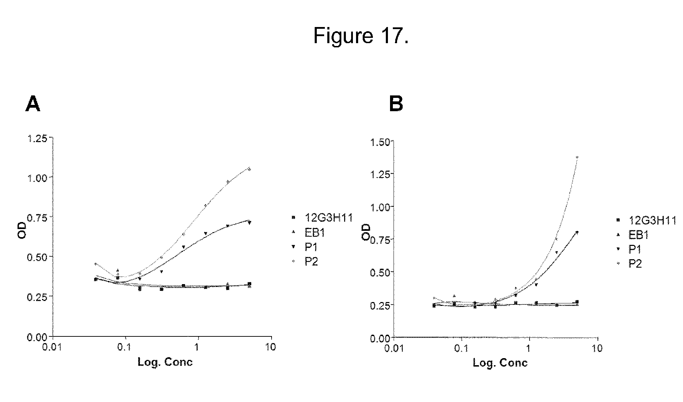

**Figure 17****.** Presented are the results of a dual specificity binding assay performed on various epitope binding proteins. In (A) the multi-specific epitope binding proteins P1 and P2 are captured by plate-bound Eph A family RTK and detected with biotinylated EphA2. This demonstrates that the multispecific epitope binding proteins P1 and P2 are capable of binding EphA2 and the Eph A family RTK concurrently. The antibody 12G3H11 and the monospecific epitope binding protein EB1 are incapable of binding both epitopes concurrently. In (B) the multispecific epitope binding proteins P1 and P2 are captured by plate-bound Eph A family RTK and detected with biotinylated Eph B family RTK. This demonstrates that the multispecific epitope binding proteins P1 and P2 are capable of binding EphA2 and the Eph B family RTK concurrently. The antibody 12G3H11 and the monospecific epitope binding protein EB1 are incapable of binding both epitopes concurrently.

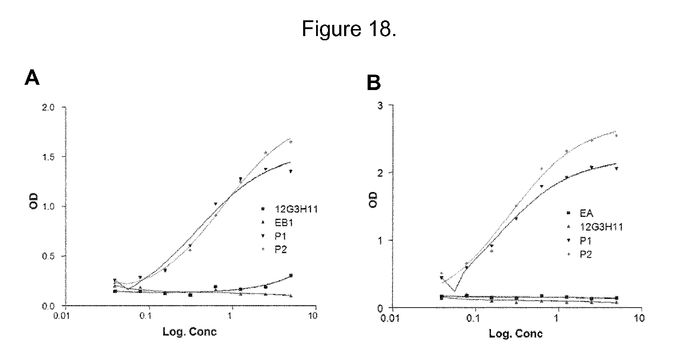

**Figure 18****.** Presented are the results of a dual specificity binding assay performed on various epitope binding proteins. In (A) the multispecific epitope binding proteins P1 and P2 are captured by plate-bound Eph B family RTK and detected with biotinylated EphA2. This demonstrates that the multispecific epitope binding proteins P 1 and P2 are capable of binding EphA2 and the Eph B family RTK concurrently. The antibody 12G3H11 and the monospecific epitope binding protein EB1 are incapable of binding both epitopes concurrently. In (B) the multispecific epitope binding proteins P 1 and P2 are captured by plate-bound Eph B family RTK and detected with biotinylated Eph A family RTK. This demonstrates that the multispecific epitope binding proteins P1 and P2 are capable of binding the Eph A family RTK and the Eph B family RTK concurrently. The antibody 12G3H11 and the monospecific epitope binding protein EA are incapable of binding both epitopes concurrently.

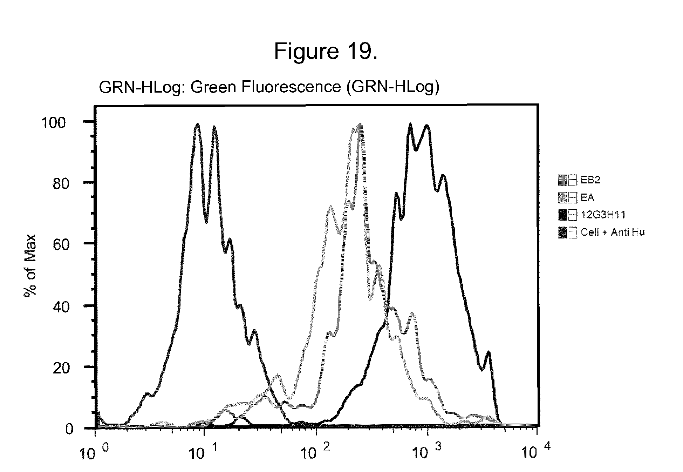

**Figure 19****.** Presented are the results from an analysis of binding specificities for the proteins of the invention to epitopes expressed on live cells. MiaPaCa2 cells were incubated with proteins, EA, EB2 12G3H11 and human IgG. The binding of the proteins of the invention was detected by anti human Fc conjugated to FITC. The binding of the proteins of the invention was analyzed by FACS. The results demonstrate that the MiaPaCa2 cells exhibit specific target epitopes for EA, EB2 and 12G3H11.

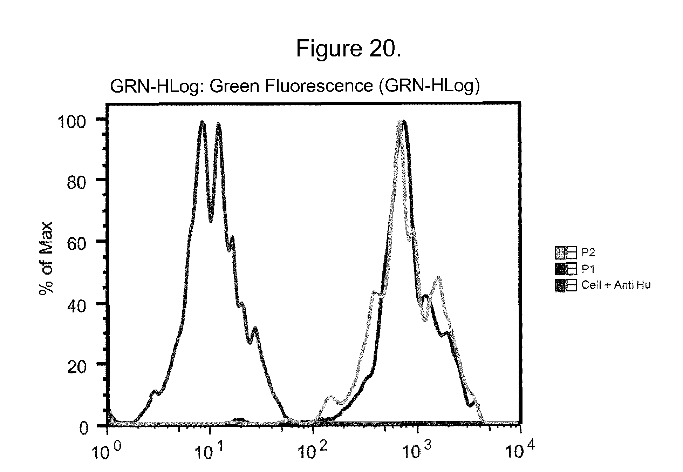

**Figure 20****.** Presented are the results from an analysis of binding specificities for the proteins to epitopes expressed on live cells. MiaPaCa2 cells were incubated with the proteins, P1, P2, and human IgG. The residual protein binding was detected by anti human Fc conjugated to FITC. The extent of protein binding was analyzed by FACS. The results demonstrate that the MiaPaCa2 cells exhibit specific target epitopes for P1, and P2.

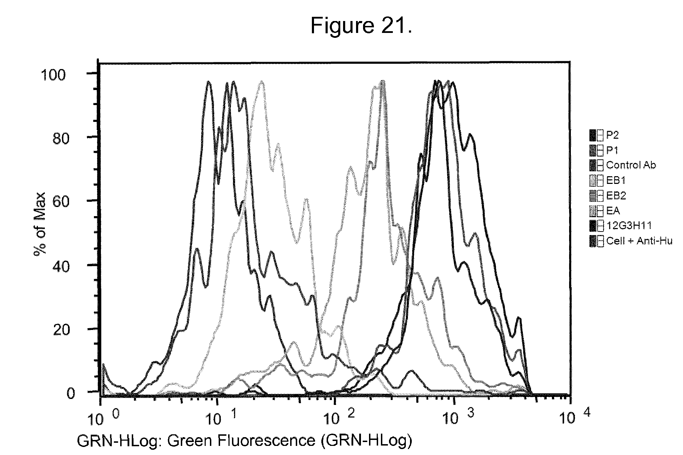

**Figure 21****.** Presented are the results from an analysis of binding specificities for epitope binding proteins to epitopes expressed on live cells. MiaPaCa2 cells were incubated with EA, EB 1, EB2, P1, P2, 12G3H11, a control Ab and human IgG. The residual protein binding was detected by anti human Fc conjugated to FITC. The extent of protein binding was analyzed by FACS. The results demonstrate that the MiaPaCa2 cells exhibit specific target epitopes for 12G3H11, EA, EB1, EB2 P1, and P2.

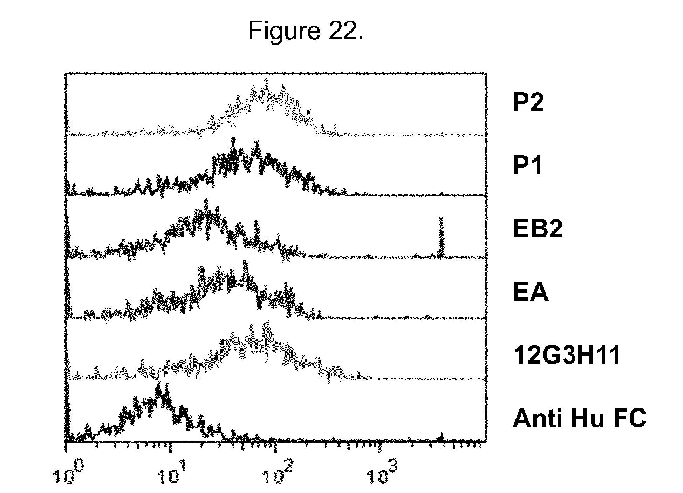

**Figure 22****.** Presented are the results from an analysis of binding specificities for certain epitope binding proteins to epitopes expressed on live cells. MiaPaCa2 cells were incubated with the proteins P1, P2, EB2, EA, 12G3H11 and anti human Fc. The residual protein binding was detected by anti human Fc conjugated to FITC. The extent of binding was analyzed by FACS. The results demonstrate that the MiaPaCa2 cells exhibit specific target epitopes for P1, P2, EA, EB2 and 12G3H11.

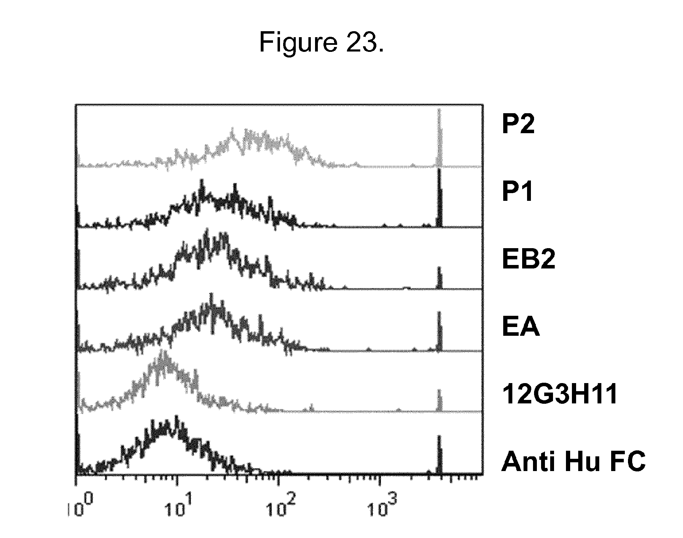

**Figure 23****.** Presented are the results from an analysis of competitive inhibition of binding for certain epitope binding proteins to epitopes expressed on live cells. MiaPaCa2 cells were incubated with the proteins P1, P2, EB2, EA, 12G3H11 and anti human Fc in the presence of excess soluble EphA2-Fc. The residual protein binding was detected by anti human Fc conjugated to FITC. The extent of protein binding was analyzed by FACS. The results demonstrate that soluble EphA2-Fc protein can compete with epitopes on MiaPaCa2 cells for the binding of 12G3H11 but not for P1, P2, EB2 and EA each of which retain binding.

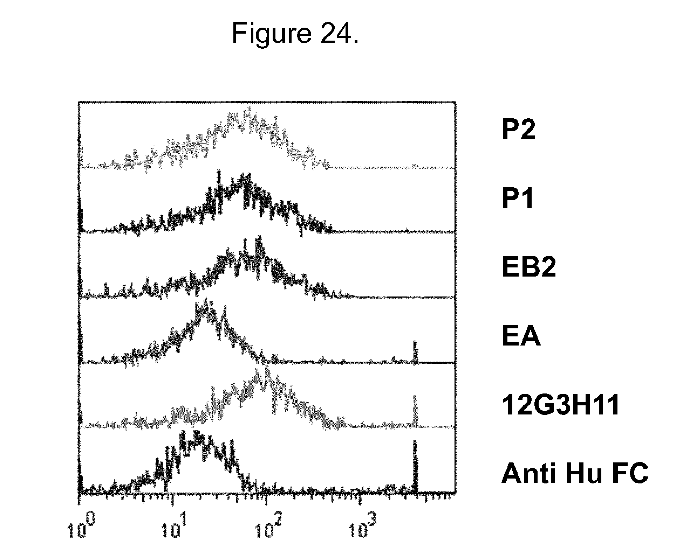

**Figure 24****.** Presented are the results from an analysis of competitive inhibition of binding for certain epitope binding proteins to epitopes expressed on live cells. MiaPaCa2 cells were incubated with proteins of the invention, P1, P2, EB2, EA, 12G3H11 and anti human Fc in the presence of excess soluble Eph A family RTK. The residual protein binding was detected by anti human Fc conjugated to FITC. The extent of protein binding was analyzed by FACS. The results demonstrate that soluble Eph A family RTK protein can compete with epitopes on MiaPaCa2 cells for the binding of EA but not for 12G3H11, P1, P2 and EB2 each of which retain binding.

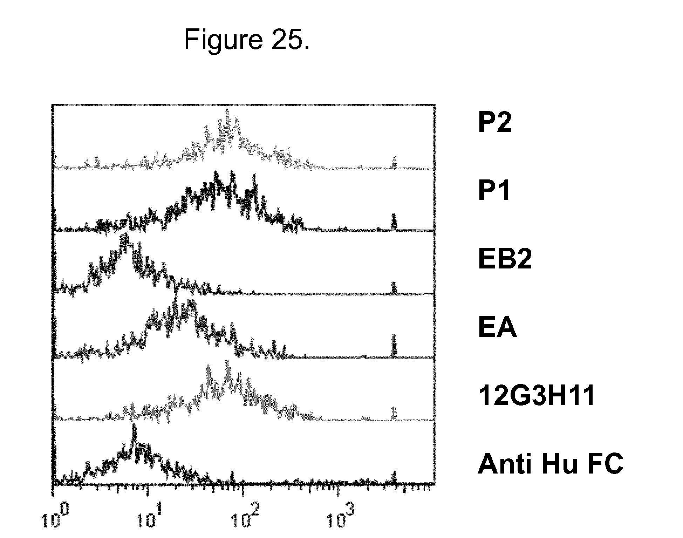

**Figure 25****.** Presented are the results from an analysis of competitive inhibition of binding for certain epitope binding proteins to epitopes expressed on live cells. MiaPaCa2 cells were incubated with proteins of the invention, P1, P2, EB2, EA, 12G3H11 and anti human Fc in the presence of excess soluble Eph B family RTK. The residual binding of the proteins was detected by anti human Fc conjugated to FITC. The extent of protein binding was analyzed by FACS. The results demonstrate that soluble Eph B family RTK protein can compete with epitopes on MiaPaCa2 cells for the binding of EB2 but not for 12G3H11, P1, P2 and EA each of which retain binding.

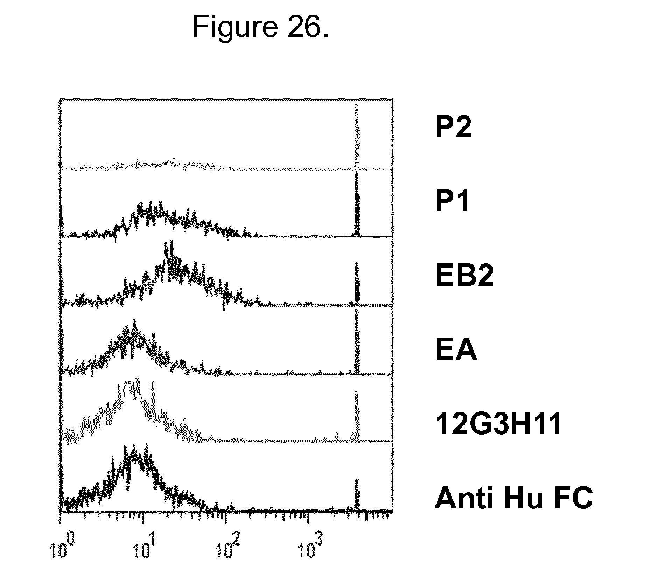

Figure 26. Presented here are the results from an analysis of competitive inhibition of binding for certain epitope binding proteins to epitopes expressed on live cells. MiaPaCa2 cells were incubated with the proteins P1, P2, EB2, EA, 12G3H11 and anti human Fc in the presence of excess soluble EphA2-Fc and Eph B family RTK. The residual binding of the proteins was detected by anti human Fc conjugated to FITC and analyzed by FACS. The results demonstrate that the combination of soluble EphA2-Fc and Eph B family RTK protein can compete with epitopes on MiaPaCa2 cells for the binding of EA and 12G3H11 completely and for P1 and P2 only partially, while only EB2 retained binding.

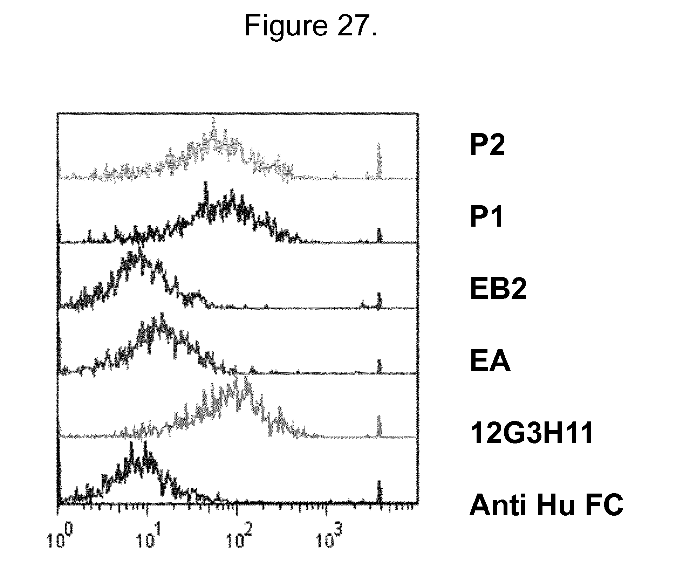

**Figure 27****.** Presented are the results from an analysis of competitive inhibition of binding for certain epitope binding proteins specific to epitopes expressed on live cells. MiaPaCa2 cells were incubated with the proteins P1, P2, EB2, EA, 12G3H11 and anti-human Fc in the presence of excess soluble Eph A family RTK and Eph B family RTK. The residual binding of the proteins was detected by anti human Fc conjugated to FITC and analyzed by FACS. The results demonstrate that the combination of soluble Eph A family RTK and Eph B family RTK protein can compete with epitopes on MiaPaCa2 cells for the binding of EA and EB2 completely while P1, P2 and 12G3H11 retained binding.

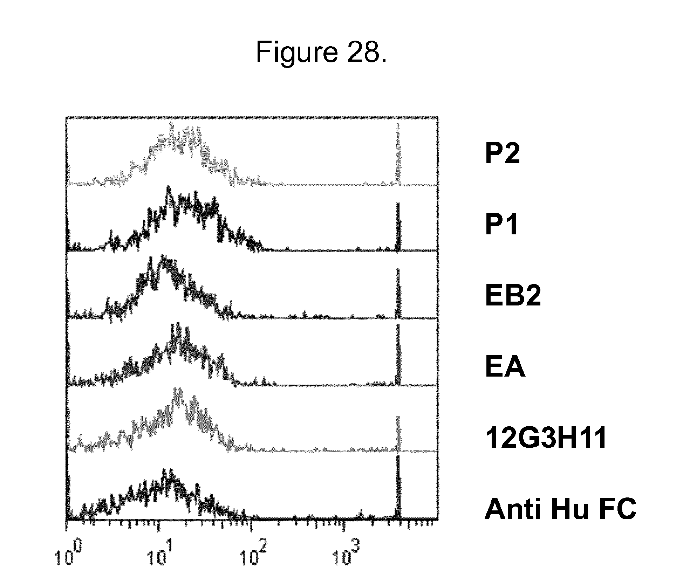

**Figure 28****.** Presented here are the results from an analysis of competitive inhibition of binding for certain epitope binding proteins specific to epitopes expressed on live cells. MiaPaCa2 cells were incubated with proteins of the invention, P1, P2, EB2, EA, 12G3H11 and anti human Fc in the presence of excess soluble EphA2-Fc, Eph A family RTK and Eph B family RTK. The residual binding of the proteins was detected by anti human Fc conjugated to FITC and analyzed by FACS. The results demonstrate that the combination of soluble EphA2-Fc, Eph A family RTK and Eph B family RTK protein can compete with epitopes on MiaPaCa2 cells for the binding of P1, P2 EB2, EA and 12G3H11.

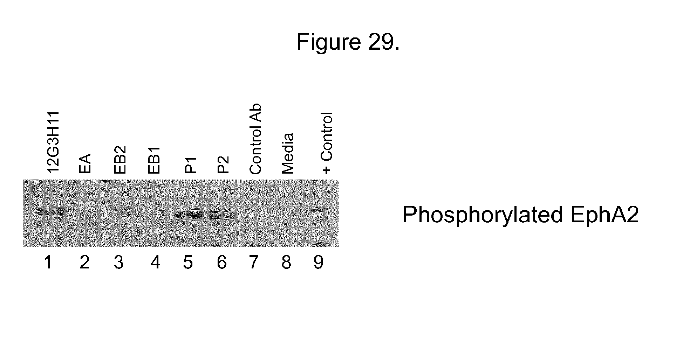

**Figure 29****.** Presented here are the results from an activation assay in which certain epitopes binding proteins are assayed for the ability to stimulate phosphorylation of the target receptor in live cells. The targeted receptors were then immunoprecipitated and analyzed for phosphate content by Western blot. As depicted in the Figure, MiaPaCa cells treated with proteins P1 and P2 activate and phosphorylate EphA2. As a positive control, the EphA2 specific antibody 12G3H11 was included in the study. The EA, EB1, EB2, control Ab and media did not stimulate the activation of EphA2 in the cells.

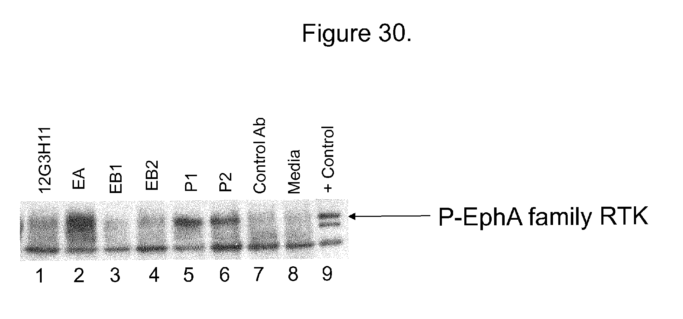

**Figure 30****.** Presented here are the results from an activation assay in which certain epitope binding proteins are assayed for the ability to stimulate phosphorylation of the target receptor in live cells. The targeted receptors were then immunoprecipitated and analyzed for phosphate content by Western blot. As depicted in the Figure 30, MiaPaCa cells treated with proteins if the invention, P1 and P2 activate and phosphorylate the Eph A family RTK. As a positive control, the Eph A family specific antibody EA was included in the study. The 12G3H11, EB1, EB2, control Ab and media did not stimulate the activation of Eph A family RTK in the cells.

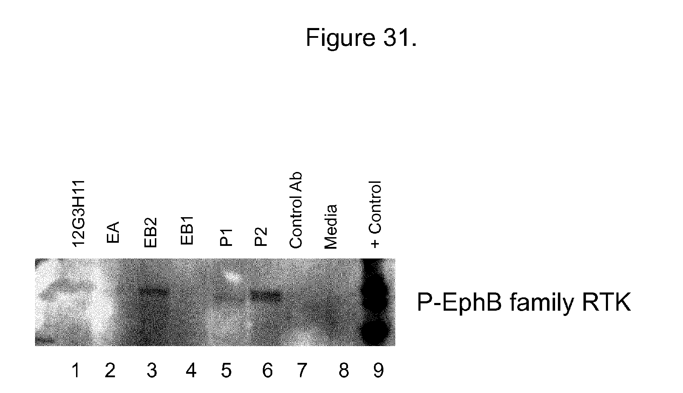

**Figure 31****.** Presented here are the results from an activation assay in which certain epitope binding proteins are assayed for the ability to stimulate phosphorylation of the target receptor in live cells. The targeted receptors were then immunoprecipitated and analyzed for phosphate content by Western blot. As depicted in the Figure 31, MiaPaCa cells treated with proteins P1 and P2 activate and phosphorylate the Eph B family RTK. As a positive control, the Eph B family specific antibody EB2 was included in the study. The 12G3H11, EA, EB2, control Ab and media did not stimulate the activation of Eph B family RTK in the cells.

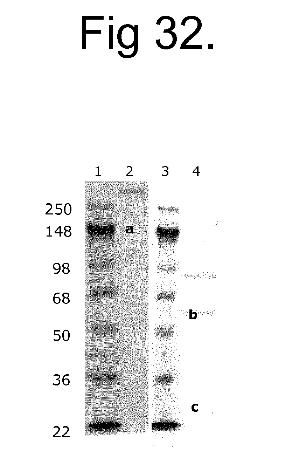

**Figure 32****.** Presented here is a PAGE gel documenting the expression of a trispecific epitope binding protein as presented in figure 4G. In the panel, a non-reducing (lanes 1 and 2) and a denaturing gel (lanes 3 and 4) document the relative molecule weight of the trispecific epitope binding protein under those conditions. In lane 2, the trispecific epitope binding protein exhibits a predicted molecular weight of about 240 kDa which is more than the predicted molecular weigh of a traditional antibody represented by (a) run on a PAGE gel in non-denaturing conditions. In lane 4, the trispecific epitope binding protein exhibits predicted molecular weights to about 75 kDa for the heavy chain and about 50 kDa for the light chain. These values are higher than the predicted molecular weights exhibited by a traditional antibody, including a heavy chain (b) and a light chain (c) run under similar conditions.

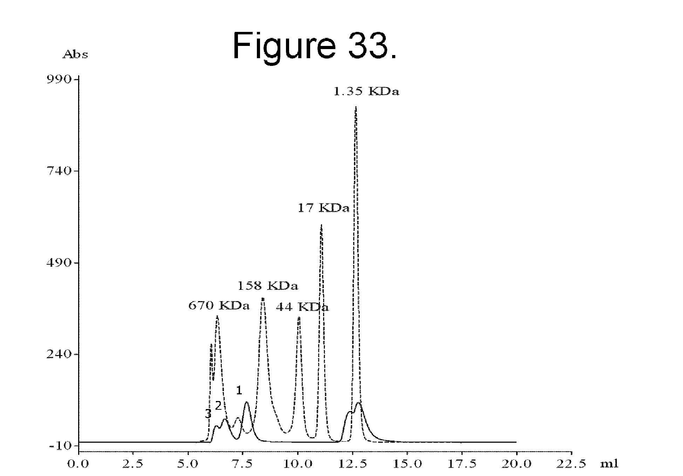

**Figure 33****.** Presented here are the results from a Size-Exclusion Chromatography (SEC) analysis of multispecific epitope binding protein "P3". This construct, which is described in Figure 4H, comprises three distinct epitope binding regions. The epitope binding protein was expressed and analyzed by SEC. The dotted tracing represents a set of defined molecular weight components used to determine the molecular weight of the P3 protein. The solid tracing represents the elution profile of P3. Peak 1 represents about 70% of the protein at an estimated molecular weight of about 240 kDa (monomer). Peak 2 and 3 represent higher order structures (e.g. dimers) or aggregates.

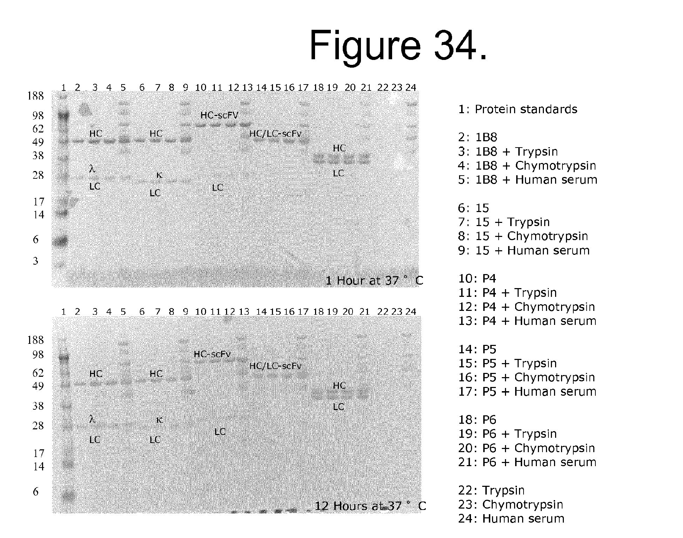

**Figure 34****.** Presented here are the results from a protease sensitivity assay performed on epitope binding proteins of various formats. Specifically, the proteins (parental antibodies and epitope binding proteins of various formats outlined herein) were expressed, purified and incubated without or with Trypsin (20 ng Trypsin/1 µg of antibody/epitope binding protein), Chymotrypsin (20 ng Chymotrypsin/1 µg of antibody/epitope binding protein) or Human serum (1 µg of serum/1 µg of antibody/epitope binding protein) for either (A) 1 hour or (B) 20 hours at 37 °C. Once incubation with the proteases was complete, samples were run on a reducing PAGE gel and stained with Coomassie to determine whether proteolysis had occurred. As presented, a 1 hour incubation at 37 °C does not result in proteolytic degradation of the various parental antibodies or epitope binding proteins. In an extended incubation (12 hours at 37 °C) no detectable proteolysis of the epitope binding proteins was observed.

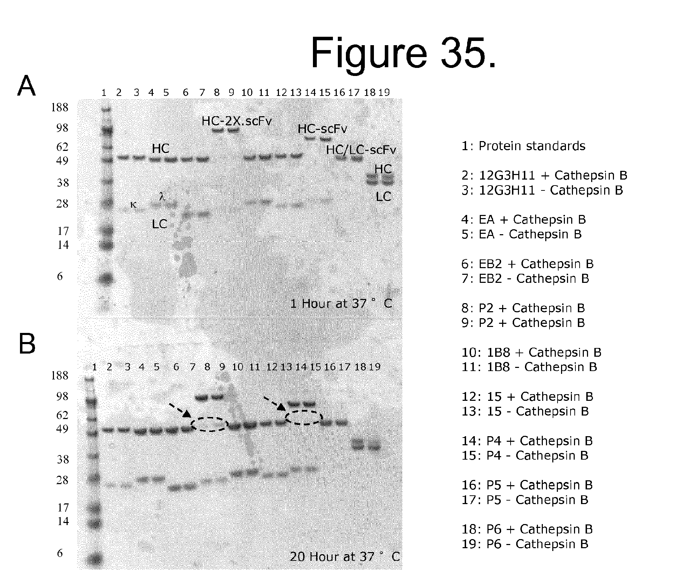

**Figure 35****.** Presented here are the results from a protease sensitivity assay performed on epitope binding proteins of various formats. Specifically, the proteins (parental antibodies and epitope binding proteins of various formats outlined herein) were expressed, purified and incubated for either (A) 1 hour or (B) 20 hours at 37 °C without (odd numbers) or with (even numbers) Cathepsin B (20 ng protease/1 µg of antibody/epitope binding protein). Once incubation with the protease was complete, samples were run on a reducing PAGE gel and stained with Coomassie to determine whether proteolysis had occurred. As presented, a 1 hour incubation at 37 °C does not result in proteolytic degradation of the various parental antibodies or epitope binding proteins. In an extended incubation (20 hours at 37 °C) some proteolysis of the epitope binding proteins in lanes 8 and 9 (protein P2) and lanes 14 and 15 is (protein P4) evident (see dashed circles).

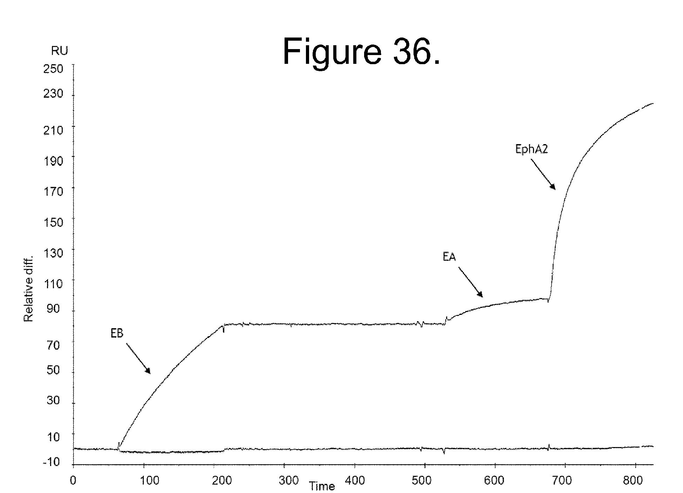

**Figure 36****.** Presented here are the results from a BIAcore experiment which demonstrate the simultaneous binding of three distinct antigens by a multispecific epitope binding protein. The upper curve represents the binding activity of three distinct epitope binding domains present on multispecific epitope binding protein "P1" represented in Figure 4D. Soluble EB, EA, and EphA2 antigens were added to immobilized P1 and the relative binding was measured. The three distinct inflections referenced by the arrows corresponding to the three antigens indicates binding to the immobilized P1. Ovalbumin (bottom curve) was used as a negative control. No specific binding of soluble EB, EA, and EphA2 was observed for the immobilized ovalbumin.

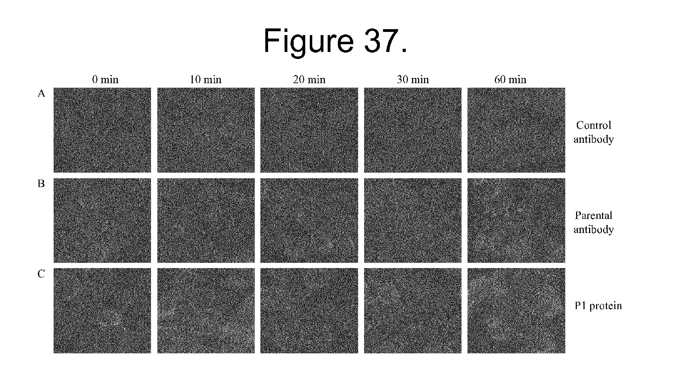

**Figure 37****.** Presented here are the results of a study analyzing the internalization of the multispecific epitope binding protein, P1. The images represent a time course experiment of receptor mediated internalization of the P1 protein, 12G3H11 antibody and a control antibody (R347). The multispecific epitope binding proteins and antibodies were detected using AlexaFluor488 (fluorescent green color) goat-γ-human IgG antibody ofpermeabilized PC3 cells following incubation with 5 µg/ml of P1 protein and antibodies for 0, 10, 20, 30 and 60 minutes. The cell nuclei were stained with DAPI. Images were analyzed by confocal laser-scanning microscopy as described in the corresponding Examples.

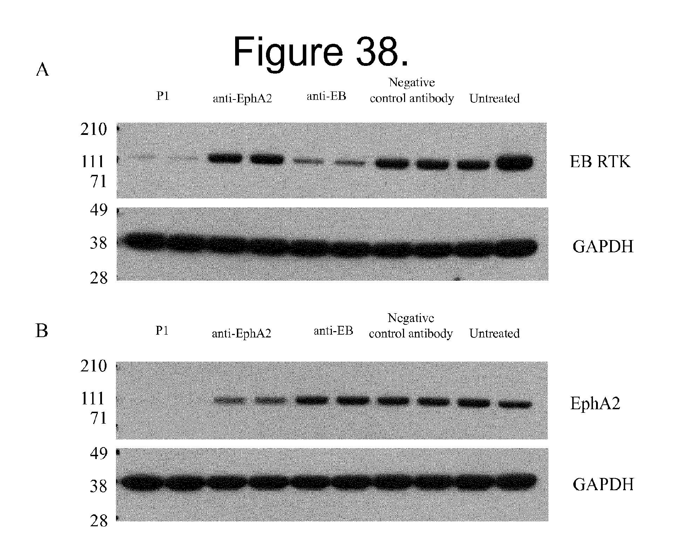

**Figure 38****.** Presented here are the results of a study investigating the ability of the P1 protein to direct the degradation of an EB family RTK and EphA2 in PC-3 cells. Briefly, 3x 10⁵ PC-3 (prostate adenocarcinoma cells) cells were plated in 6-well plates and allowed to attach overnight. The cells were then treated with the P1 protein or control antibodies (anti-EphA2, anti-EB1, negative control antibody (R347) or untreated) at 67 nM, as schematically shown. P1 protein and control antibodies were incubated with PC-3 cells for 4 hours for EB degradation (A), and 24 hours for EphA2 degradation (B). Western blots were probed with anti-EphA2, anti-EB1 and anti-GAPDH specific antibodies. The position of the EB family RTK, EphA2 and GAPDH (Glyceraldehyde 3-phosphate dehydrogenase) is schematically shown. The molecular weights are reported in KDa.

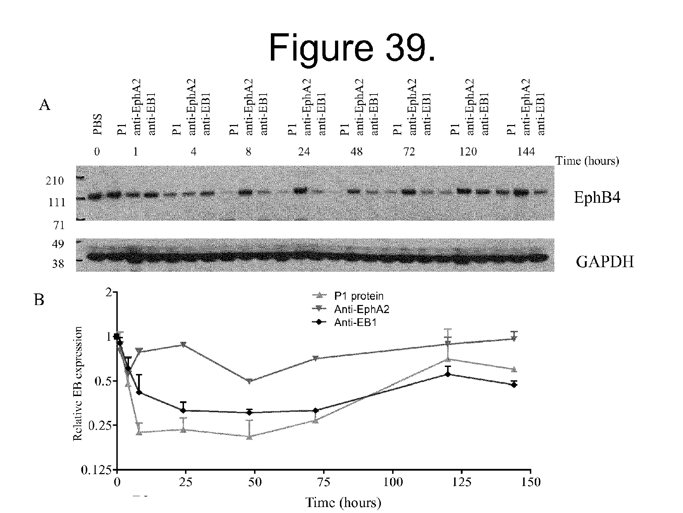

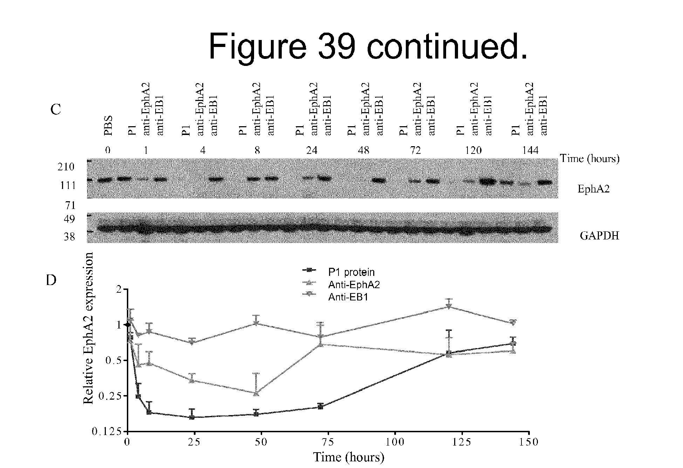

**Figure 39****.** Presented here are the results from an *In vivo* time course degradation of an EB family RTK and EphA2 in PC-3 tumor-bearing nude mice dosed with the P1 protein and control antibodies. Briefly, 5 x 10⁶ PC-3 (prostate adenocarcinoma cells) were implanted subcutaneously on the right flank of nude mice. Tumors were allowed to progress to approximately 100 mm³ and dosed intraperitoneally with the P1 protein, the parental anti-EphA2 or anti-EB1 antibodies at 67 nmol/kg body weight. Tumors were harvested from 3 mice per time point per dose group at 0, 1, 4, 8, 24, 48, 72, 120 and 144 hours post-dose. Tumors lysates were analyzed by western blot for EB protein expression (A) and EphA2 (C). GAPDH (Glyceraldehyde 3-phosphate dehydrogenase) was used as expression control. Each tumor lysate was loaded onto one of three separate gels with one sample from each time point loaded on each gel. In this figure only one representative gel is shown. The protein bands in the three gels were quantified by densitometry analysis and normalized against the protein band from the 0-hour time point. One PBS treated (0 hours) control tumor was used as a control on each of the three blots. The normalized densitometry values were plotted as mean of relative EB (B) or EphA2 (D) expression using GraphPad Prism® software.

**Figure 40****.** Presented here are the pharmacokinetic analyses of the P1 protein and control antibodies in PC-3 tumor-bearing nude mice. Briefly, 3 x 10⁵ PC-3 prostate adenocarcinoma cells were implanted subcutaneously on the right flank of nude mice. Tumors were allowed to progress to approximately 100 mm³ and dosed with the P1 protein or the parental anti-EphA2 or anti-EB 1 antibodies at 67 nmol/kg body weight. Blood was collected via the tail vein and serum separated from 3 mice per time point per dose group at 1, 4, 8, 24, 48, 72, 120 and 144 hours post-dose. Serum from one additional group of 3 mice, dosed with PBS, was harvested immediately after dosing (0 hours). Serum samples were analyzed for the presence of the P1 protein (green and red curves, respectively for anti-EphA2 and anti-EphB4 binding), parental anti-EphA2 (black curve) or anti-EB1 (blue curve) control antibodies using EphA2 and EB1 binding ELISA.

### 5. Terminology

As used herein, the terms "antibody" and "antibodies" refer to, for example, monoclonal antibodies, human antibodies, humanized antibodies, camelised antibodies, chimeric antibodies, single-chain Fvs (scFv), disulfide-linked Fvs (sdFv), Fab fragments, F (ab') fragments, and anti-idiotypic (anti-Id) antibodies (including, e.g., anti-Id antibodies to antibodies of the invention), and epitope-binding fragments of any of the above. In particular, antibodies include immunoglobulin molecules and immunologically active fragments of immunoglobulin molecules, *i.e.,* molecules that contain an antigen binding site, these fragments may or.may not be fused to another immunoglobulin domain including but not limited to, an Fc region or fragment thereof. Immunoglobulin molecules can be of any type *(e.g.,* IgG, IgE, IgM, IgD, IgA and IgY), class (*e.g.,* IgG1, IgG2, IgG3, IgG4, IgA1 and IgA2) or subclass.

Native antibodies are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies between the heavy chains of different immunoglobulin isotypes. Each heavy and light chain also has regularly spaced intrachain disulfide bridges. Each heavy chain has at one end a variable domain (VH) followed by a number of constant domains. Each light chain has a variable domain at one end (VL) and a constant domain at its other end; the constant domain of the light chain is aligned with the first constant domain of the heavy chain, and the light chain variable domain is aligned with the variable domain of the heavy chain. The term "variable region" may also be used to describe the variable domain of a heavy chain or light chain. Particular amino acid residues are believed to form an interface between the light and heavy chain variable domains. Such antibodies may be derived from any mammal, including, but not limited to, humans, monkeys, pigs, horses, rabbits, dogs, cats, mice, etc.

The term "variable" refers to the fact that certain portions of the variable domains differ extensively in sequence among antibodies and are responsible for the binding specificity of each particular antibody for its particular antigen. However, the variability is not evenly distributed through the variable domains of antibodies. It is concentrated in segments called Complementarity Determining Regions (CDRs) both in the light chain and the heavy chain variable domains. The more highly conserved portions of the variable domains are called the framework regions (FW). The variable domains of native heavy and light chains each comprise four FW regions, largely adopting a β-sheet configuration, connected by three CDRs, which form loops connecting, and in some cases forming part of, the β-sheet structure. The CDRs in each chain are held together in close proximity by the FW regions and, with the CDRs from the other chain, contribute to the formation of the antigen-binding site of antibodies (see, Kabat et al., Sequences of Proteins of Immunological Interest, 5th Ed. Public Health Service, National Institutes of Health, Bethesda, MD (1991)). The constant domains are generally not involved directly in antigen binding, but may influence antigen binding affinity and may exhibit various effector functions, such as participation of the antibody in ADCC, CDC, and/or apoptosis.

The term "hypervariable region" when used herein refers to the amino acid residues of an antibody which are associated with its binding to antigen. The hypervariable regions encompass the amino acid residues of the "complementarity determining regions" or "CDRs" (e.g., residues 24-34 (L1), 50-56 (L2) and 89-97 (L3) of the light chain variable domain and residues 31-35 (H1), 50-65 (H2) and 95-102 (H3) of the heavy chain variable domain; Kabat et al., Sequences of Proteins of Immunological Interest, 5th Ed. Public Health Service, National Institutes of Health, Bethesda, MD (1991)) and/or those residues from a "hypervariable loop" (e.g., residues 26-32 (L), 50-52 (L2) and 91-96 (L3) in the light chain variable domain and 26-32 (H1), 53-55 (H2) and 96-101 (H3) in the heavy chain variable domain; Chothia and Lesk, J. Mol. Biol., 196:901-917 (1987)). "Framework" or "FW" residues are those variable domain residues flanking the CDRs. FW residues are present in chimeric, humanized, human, domain antibodies, single chain diabodies, vaccibodies, linear antibodies, and bispecific antibodies.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, monoclonal antibodies are advantageous in that they can be synthesized by hybridoma cells that are uncontaminated by other immunoglobulin producing cells. Alternative production methods are known to those trained in the art, for example, a monoclonal antibody may be produced by cells stably or transiently transfected with the heavy and light chain genes encoding the monoclonal antibody.

The modifier "monoclonal" indicates the character of the antibody as being obtained from a substantially homogeneous population of antibodies, and is not to be construed as requiring engineering of the antibody by any particular method. The term "monoclonal" is used herein to refer to an antibody that is derived from a clonal population of cells, including any eukaryotic, prokaryotic, or phage clone, and not the method by which the antibody was engineered. For example, the monoclonal antibodies to be used in accordance with the present invention may be made by the hybridoma method first described by Kohler et al., Nature, 256:495 (1975), or may be made by any recombinant DNA method (see, e.g., U.S. Patent No. 4,816,567), including isolation from phage antibody libraries using the techniques described in Clackson et al., Nature, 352:624-628 (1991) and Marks et al., J. Mol. Biol., 222:581-597 (1991), for example. These methods can be used to produce monoclonal mammalian, chimeric, humanized, human, domain antibodies, single chain diabodies, vaccibodies, and linear antibodies.

The term "chimeric" antibodies includes antibodies in which at least one portion of the heavy and/or light chain is identical with or homologous to corresponding sequences in antibodies derived from a particular species or belonging to a particular antibody class or subclass, and at least one other portion of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al., Proc. Natl. Acad. Sci. USA, 81:6851-6855 (1984)). Chimeric antibodies of interest herein include "primatized" antibodies comprising variable domain antigen-binding sequences derived from a nonhuman primate (e.g., Old World Monkey, such as baboon, rhesus or cynomolgus monkey) and human constant region sequences (U.S. Patent No. 5,693,780).

"Humanized" forms of nonhuman (e.g., murine) antibodies are chimeric antibodies that contain minimal sequence derived from nonhuman immunoglobulin. For the most part, humanized antibodies are human immunoglobulins (recipient antibody) in which the native CDR residues are replaced by residues from the corresponding CDR of a nonhuman species (donor antibody) such as mouse, rat, rabbit or nonhuman primate having the desired specificity, affinity, and capacity. In some instances, FW region residues of the human immunoglobulin are replaced by corresponding nonhuman residues. Furthermore, humanized antibodies may comprise residues that are not found in the recipient antibody or in the donor antibody. These modifications are made to further refine antibody performance. In general, a humanized antibody heavy or light chain will comprise substantially all of at least one or more variable domains, in which all or substantially all of the CDRs correspond to those of a nonhuman immunoglobulin and all or substantially all of the FWs are those of a human immunoglobulin sequence. In certain embodiments, the humanized antibody will comprise at least a portion of an immunoglobulin constant region (Fc), typically that of a human immunoglobulin. For further details, see, Jones et al., Nature, 321:522-525 (1986); Riechmann et al., Nature, 332:323-329 (1988); and Presta, Curr. Op. Struct. Biol., 2:593-596 (1992).

A "human antibody" can be an antibody derived from a human or an antibody obtained from a transgenic organism that has been "engineered" to produce specific human antibodies in response to antigenic challenge and can be produced by any method known in the art. In certain techniques, elements of the human heavy and light chain loci are introduced into strains of the organism derived from embryonic stem cell lines that contain targeted disruptions of the endogenous heavy chain and light chain loci. The transgenic organism can synthesize human antibodies specific for human antigens, and the organism can be used to produce human antibody-secreting hybridomas. A human antibody can also be an antibody wherein the heavy and light chains are encoded by a nucleotide sequence derived from one or more sources of human DNA. A fully human antibody also can be constructed by genetic or chromosomal transfection methods, as well as phage display technology, or in vitro activated B cells, all of which are known in the art.

"Antibody-dependent cell-mediated cytotoxicity" and "ADCC" refer to a cell-mediated reaction in which non-specific cytotoxic cells (e.g., Natural Killer (NK) cells, neutrophils, and macrophages) recognize bound antibody on a target cell and subsequently cause lysis of the target cell. In one embodiment, such cells are human cells. While not wishing to be limited to any particular mechanism of action, these cytotoxic cells that mediate ADCC generally express Fc receptors (FcRs). The primary cells for mediating ADCC, NK cells, express FcγRIII, whereas monocytes express FcγRI, FcγRII, FcγRIII and/or FcγRIV. FcR expression on hematopoietic cells is summarized in Ravetch and Kinet, Annu. Rev. Immunol., 9:457-92 (1991). To assess ADCC activity of a molecule, an in vitro ADCC assay, such as that described in U.S. Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecules of interest may be assessed in vivo, e.g., in an animal model such as that disclosed in Clynes et al., Proc. Natl. Acad. Sci. (USA), 95:652-656 (1998).

"Complement dependent cytotoxicity" or "CDC" refers to the ability of a molecule to initiate complement activation and lyse a target in the presence of complement. The complement activation pathway is initiated by the binding of the first component of the complement system (C1q) to a molecule (e.g., an antibody) complexed with a cognate antigen. To assess complement activation, a CDC assay, e.g., as described in Gazzano-Santaro et al., J. Immunol. Methods, 202:163 (1996), may be performed.

"Effector cells" are leukocytes which express one or more FcRs and perform effector functions. The cells express at least FcγRI, FcγRII, FcγRIII and/or FcγRIV and carry out ADCC effector function. Examples of human leukocytes which mediate ADCC include peripheral blood mononuclear cells (PBMC), natural killer (NK) cells, monocytes, cytotoxic T cells and neutrophils.

An "epitope" is a term well understood in the art and means any chemical moiety that exhibits specific binding to an antibody. An "antigen" is a moiety or molecule that contains an epitope, and, as such, also specifically binds to antibody.

By "stringent hybridization conditions" is intended as overnight incubation at 42°C in a solution comprising: 50% formamide, 5x SSC (750 mM NaCl, 75 mM trisodium cirate), 50 mM sodium phosphate (pH 7.6), 5x Denhardt's solution, 10% dextran sulfate, and 20 µg/ml denatured, sheared salmon sperm DNA, followed by washing the filters in 0.1 x SSC at about 65° C. The polynucleotides may be obtained, and the nucleotide sequence of the polynucleotides determined, by any method known in the art. For example, if the nucleotide sequence of the antibody is known, a polynucleotide encoding the antibody may be assembled from chemically synthesized oligonucleotides (*e.g*., as described in Kutmeier et al., BioTechniques 17:242 (1994)), which, briefly, involves the synthesis of overlapping oligonucleotides containing portions of the sequence encoding the antibody, annealing and ligating of those oligonucleotides, and then amplification of the ligated oligonucleotides by PCR.

### 6. Detailed Description of the Invention

The present invention provides novel multispecific epitope binding proteins comprising an Fc region of an antibody constant domain. Specifically, the Fc region may comprise a CH3, CH2, a hinge region (or a portion thereof) from a constant domain of an antibody. In one embodiment, multispecific epitope binding proteins of the invention comprise an Fc region and a CH1 region from an antibody constant domain. In other embodiments the multispecific epitope binding proteins of the invention further comprises a Ckappa/lambda region. In other embodiments, multispecific epitope binding proteins of the invention comprise at least 1, at least 2, at least 3, at least 4, at least 5, at least 6, at least 7, at least 8 or more CH1 and/or Ckappa/lambda regions. In other embodiments, multispecific epitope binding proteins of the invention comprise 1, 2, 3, 4, 5, 6, 7, 8 or more CH1 and/or Ckappa/lambda regions. In another embodiment, the Fc region, CH1 region or Ckappa/lambda region are derived from any antibody subtype known in the art. In other embodiments, the Fc region is a chimera derived from multiple antibody subtypes known in the art.

In alternative embodiments, multispecific epitope binding proteins of the invention may comprise a CH1 or a Ckappa/lambda region in the absence of an Fc region. In other embodiments, multispecific epitope binding proteins of the invention comprise at least 1, at least 2, at least 3, at least 4, at least 5, at least 6, at least 7, at least 8 or more CH1 and/or Ckappa/lambda regions in the absence of an Fc region. In other embodiments, multispecific epitope binding proteins of the invention may comprise all or a portion of the hinge region of an antibody in the absence of an Fc region.

It is known that variants of the Fc region (*e.g.,* amino acid substitutions and/or additions and/or deletions) enhance or diminish effector function (see Presta et al., 2002, Biochem Soc Trans 30:487-490; U.S. patents 5,624,821, 5,885,573 and PCT publication Nos. WO 00/42072, WO 99/58572 and WO 04/029207). Accordingly, in one embodiment, the amino acid sequence of the multispecific epitope binding proteins of the invention comprises variant Fc regions. In one embodiment, the variant Fc regions of multispecific epitope binding proteins exhibit a similar level of inducing effector function as compared to the native Fc. In another embodiment, the variant Fc region exhibits a higher induction of effector function as compared to the native Fc. In another embodiment, the variant Fc region exhibits lower induction of effector function as compared to the native Fc. In another embodiment, the variant Fc region exhibits higher induction of ADCC as compared to the native Fc. In another embodiment, the variant Fc region exhibits lower induction of ADCC as compared to the native Fc. In another embodiment, the variant Fc region exhibits higher induction of CDC as compared to the native Fc. In another embodiment, the variant Fc region exhibits lower induction of CDC as compared to the native Fc. Specific embodiments of variant Fc regions are detailed *infra.*

It is also known in the art that the glycosylation of the Fc region can be modified to increase or decrease effector function (see for examples, Umana et al, 1999, Nat. Biotechnol 17:176-180; Davies et al., 2001, Biotechnol Bioeng 74:288-294; Shields et al, 2002, J Biol Chem 277:26733-26740; Shinkawa et al., 2003, J Biol Chem 278:3466-3473) U.S. Pat. No. 6,602,684; U.S. Ser. No. 10/277,370; U.S. Ser. No. 10/113,929; PCT WO 00/61739A1; PCT WO 01/292246A1; PCT WO 02/311140A1; PCT WO 02/30954A1; Potillegent™ technology (Biowa, Inc. Princeton, N.J.); GlycoMAb™ glycosylation engineering technology (GLYCART biotechnology AG, Zurich, Switzerland). Accordingly, in one embodiment the Fc regions of multispecific polypeptides of the invention comprise altered glycosylation of amino acid residues. In another embodiment, the altered glycosylation of the amino acid residues results in lowered effector function. In another embodiment, the altered glycosylation of the amino acid residues results in increased effector function. In a specific embodiment, the Fc region has reduced fucosylation. In another embodiment, the Fc region is afucosylated (see for examples, U.S. Patent Application Publication No.2005/0226867).

Recent research suggests that the addition of sialic acid to the oligosaccharides on IgG molecules enhances their and-inflammatory activity and alter their cytotoxicity (Keneko et al., Science 313, 670-673(2006), Scallon et al., Mol. Immuno. 2007 Mar;44(7):1524-34). Thus, the efficacy of antibody therapeutics may be optimized by selection of a glycoform that is best suited to the intended application. The two oligosaccharide chains interposed between the two CH2 domains of antibodies are involved in the binding of the Fc region to its receptors. The studies referenced above demonstrate that IgG molecules with increased sialylation have anti-inflammatory properties whereas IgG molecules with reduced sialylation have increased immunostimulatory properties. Therefore, an antibody therapeutic can be "tailor-made" with an appropriate sialylation profile for a particular application. Methods for modulating the sialylation state of antibodies are presented in WO2007/005786 entitled "Methods And Compositions With Enhanced Therapeutic Activity", and W02007/117505 entitled "Polypeptides With Enhanced Anti-Inflammatory And Decreased Cytotoxic Properties And Related Methods" each of which are incorporated by reference in their entireties for all purposes.

In one embodiment, the Fc regions of multispecific polypeptides of the invention comprise an altered sialylation profile compared to a reference unaltered Fc region. In one embodiment, the Fc regions of multispecific polypeptides of the invention comprise an increased sialylation profile compared to a reference unaltered Fc region. In some embodiments the Fc regions of multispecific polypeptides of the invention comprise an increase in sialylation of about 5%, about 10%, about 15%, about 20%, about 25%, about 30%, about 35%, about 40%, about 45%, about 50%, about 60%, about 65%, about 70%, about 80%, about 85%, about 90%, about 95%, about 100%, about 125%, or about 150% or more as compared to a reference unaltered Fc region. In some embodiments the Fc regions of multispecific polypeptides of the invention comprise an increase in sialylation of about 2 fold, about 3 fold, about 4 fold, about 5 fold, about 10 fold, about 20 fold, about 50 fold or more as compared to an unaltered reference Fc region.

In another embodiment, the Fc regions of multispecific polypeptides of the invention comprise a decreased sialylation profile compared to a reference unaltered Fc region. In some embodiments, the Fc regions of multispecific polypeptides of the invention comprise a decrease in sialylation of about 5%, about 10%, about 15%, about 20%, about 25%, about 30%, about 35%, about 40%, about 45%, about 50%, about 60%, about 65%, about 70%, about 80%, about 85%, about 90%, about 95%, about 100%, about 125%, about 150% or more as compared to a reference unaltered Fc region. In some embodiments the Fc regions of multispecific polypeptides of the invention comprise a decrease in sialylation of about 2 fold, about 3 fold, about 4 fold, about 5 fold, about 10 fold, about 20 fold, about 50 fold or more as compared to an unaltered reference Fc region.

It is also known in the art that the Fc region can be modified to increase the half-lives of proteins. The increase in half-life allows for the reduction in amount of drug given to a patient as well as reducing the frequency of administration. Accordingly, multispecific epitope binding proteins of the invention with increased half-lives may be generated by modifying (for example, substituting, deleting, or adding) amino acid residues identified as involved in the interaction between the Fc and the FcRn receptor (see, for examples, PCT publication Nos. 97/34631 and 02/060919 each of which are incorporated by reference in their entireties). In addition, the half-life of multispecific epitope binding proteins of the invention may be increase by conjugation to PEG or Albumin by techniques widely utilized in the art. In some embodiments the Fc regions of multispecific polypeptides of the invention comprise an increase in half-life of about 5%, about 10%, about 15%, about 20%, about 25%, about 30%, about 35%, about 40%, about 45%, about 50%, about 60%, about 65%, about 70%, about 80%, about 85%, about 90%, about 95%, about 100%, about 125%, about 150% or more as compared to a reference unaltered Fc region. In some embodiments the Fc regions of multispecific polypeptides of the invention comprise an increase in half-life of about 2 fold, about 3 fold, about 4 fold, about 5 fold, about 10 fold, about 20 fold, about 50 fold or more as compared to an unaltered reference Fc region.

### A. Polypeptide chain orientation

The multispecific epitope binding proteins of the invention comprise one, two, three, four, or more polypeptide chains. The multispecific epitope binding proteins of the invention may comprise two to four polypeptide chains (may hereinafter be referred to as "polypeptide chains of the invention"). Each polypeptide chain of the multispecific epitope binding protein of the invention may comprise at least 1, at least 2, at least 3, at least 4, or more than 4 epitope binding domains (also referred to herein as "EBDs") and further comprises one or more of the following regions, a Fc region, a CH1 region, a Ckappa/lambda region. The polypeptide chains of the invention comprise one or more epitope binding domains, which may be scFvs, single chain diabodies, variable regions of antibodies, or another type of epitope binding domain. The epitope binding domains may be linked N-terminal and/or C-terminal to one or more of the following regions, an Fc region, a CH1 region, a Ckappa/lambda region. In other embodiments, polypeptide chains of the invention may comprise 1, 2, 3, 4, 5, 6, 7, 8 or more Fc, CH1 or Ckappa/lambda regions.

In one embodiment a polypeptide chain of the invention comprises an Fc region. When describing the polypeptide chains of the invention in the following sections, it is understood that the term "Fc region" encompasses a polypeptide chain comprising a hinge region or a portion thereof, a CH2 region and a CH3 region. In another embodiment, a polypeptide chain of the invention may further comprise a CH1 region from the constant region of an antibody. In certain embodiments, the CH1 region is linked N-terminal and/or C-terminal to the Fc region.

### A.1. Epitope binding domain orientation

Epitope binding domains (also referred to as "EBDs") include for example, antibody variable regions, antibody fragments, scFvs, single chain diabodies, or other binding domains known in the art. Epitope binding domains also include bispecific single chain diabodies, or single chain diabodies designed to bind two distinct epitopes. Also included are antibody-like molecules or antibody mimetics, for example, but not limited to minibodies, maxybodies, "A" domain oligomers (also known as Avimers) (See for example, US. Patent Application Publication Nos. 2005/0164301, 2005/0048512, and 2004/017576 each of which are incorporated by reference), Fn3 based protein scaffolds (see for example, US Patent Application Publication 2003/0170753 which is incorporated by reference), Ankrin repeats (also known as DARpins), VASP polypeptides, Avian pancreatic polypeptide (aPP), Tetranectin (based on CTLD3), Affililin (based on γB-crystallin/ubiquitin), Knottins, SH3 domains, PDZ domains, Tendamistat, Neocarzinostatin, Protein A domains, Lipocalins, Transferrin, and Kunitz domains that specifically bind epitopes. In one embodiment, epitope binding domains useful in the construction of multispecific epitope binding proteins of the invention are exemplified in US Provisional Patent Application 60/984,206 filed October 31, 2007 entitled "Proteins Scaffolds" which is hereby incorporated by reference for all purposes.

In an embodiment, one, two, three, or more epitope binding domains of the polypeptide chains of the invention are linked to the C-terminus of the Fc region. In other embodiments one, two, three, or more epitope binding domains are linked to the N-terminus of the Fc region. In other embodiments, one, two, three, or more epitope binding domains are linked to both the N-terminus and C-terminus of the Fc region.

In some embodiments, polypeptide chains of the invention may comprise an orientation (N-terminus to C-terminus) according to the following formula: EBDₙ-antibody variable domainₙ-Xₙ-Fc regionₙ-EBDₙ, wherein X is a CH1 or a Ckappa/lambda and n is an integer from 0 to 10 and may vary for each structural element.

### A.2. C-terminal Epitope Binding Domain Containing Polypeptide Chains

In one embodiment, an epitope binding domain (e.g., an antibody variable region, an scFv, a single chain diabody) is linked to the C-terminus of the Fc region of polypeptide chains of the invention. In another embodiment, multiple epitope binding domains (e.g., an antibody variable region, an scFv, a single chain diabody) are linked to the C-terminus of the Fc region. In another embodiment, polypeptide chains of the invention comprise multiple epitope binding domains (e.g., an antibody variable region, an scFv, a single chain diabody) linked to the C-terminus of the Fc region. In another embodiment, polypeptide chains of the invention comprises at least 1, 2, 3, 4, 5, 6, 7, 8 or more epitope binding domains (e.g., an antibody variable region, an scFv, a single chain diabody) linked to the C-terminus of the Fc region. In another embodiment, polypeptide chains of the invention comprise multiple epitope binding domains linked C-terminus of the Fc region, wherein the domains are selected from the group consisting of an antibody variable region, an scFv, a single chain diabody, or another epitope binding domain known in the art.

In certain embodiments, polypeptide chains of the invention comprise more then one type of epitope binding domain linked to the C-terminus of the Fc region. For example, but not by way of limitation a polypeptide chain of the invention comprising 2 epitope binding domains may comprise an scFv and a single chain diabody, or an scFv and an antibody variable region, or an scFv and another epitope binding domain known in the art, or a single chain diabody and an antibody variable region, or a single chain diabody and another epitope binding domain known in the art, or an antibody variable region and another epitope binding domain known in the art, linked to the C-terminus of the Fc region.

In a specific embodiment, one or more epitope binding domains are scFvs. In one embodiment, one scFv is linked to the C-terminus of the Fc region of polypeptide chains of the invention (see for example Figure 1B). In another embodiment, multiple scFvs are linked to the C-terminus of the Fc region (see for examples Figures 2B, 3B, 4B). In another embodiment, polypeptide chains of the invention comprise multiple scFvs linked to the C-terminus of the Fc region. In another embodiment, polypeptide chains of the invention comprise at least 1, 2, 3, 4, 5, 6, 7, 8 or more scFvs linked to the C-terminus of the Fc region.

In a specific embodiment, one or more epitope binding domains are single chain diabodies. In one embodiment, a single chain diabody is linked to the C-terminus of the Fc region of the polypeptide chains of the invention (see for example Figure 3F). In another embodiment, multiple single chain diabodies are linked to the C-terminus of the Fc region. In another embodiment, polypeptide chains of the invention comprise multiple single chain diabodies linked to the C-terminus of the Fc region. In another embodiment, one or both polypeptide chains of the invention comprise at least 1, 2, 3, 4, 5, 6, 7, 8 or more single chain diabodies linked to the C-terminus of the Fc region.

In a specific embodiment, one or more epitope binding domains are antibody variable regions. In one embodiment, an antibody variable region is linked to the C-terminus of the Fc region of polypeptide chains of the invention. In another embodiment, multiple antibody variable regions are linked to the C-terminus of the Fc region. In another embodiment, polypeptide chains of the invention comprise multiple antibody variable regions linked to the C-terminus of the Fc region. In another embodiment, polypeptide chains of the invention comprises at least 1, 2, 3, 4, 5, 7, 8 or more antibody variable regions linked to the C-terminus of the Fc region.

### A.3. N-terminal Epitope Binding Domain Containing Polypeptide Chains

In another embodiment, an epitope binding domain (e.g., an antibody variable region, an scFv, a single chain diabody) is linked to the N-terminus of the Fc region of the polypeptide chains of the invention. In another embodiment, multiple epitope binding domains (e.g., an antibody variable region, an scFv, a single chain diabody) are linked to the N-terminus of the Fc region. In another embodiment, polypeptide chains of the invention comprise multiple epitope binding domains (e.g., an antibody variable region, an scFv, a single chain diabody) linked to the N-terminus of the Fc region. In another embodiment, polypeptide chains of the invention comprise at least 1, 2, 3, 4, 5, 6, 7, 8 or more epitope binding domains (e.g., an antibody variable region, an scFv, a single chain diabody) linked to the N-terminus of the Fc region. In another embodiment, the multiple polypeptide chains of the invention comprise multiple epitope binding domains wherein the domains are selected from the group consisting of an antibody variable region, an scFv, a single chain diabody, and another epitope binding domain known in the art.

In certain embodiments, polypeptide chains of the invention comprise more than one type of epitope binding domain linked to the N-terminus of the Fc region. For example, but not by way of limitation a polypeptide chain of the invention comprising 2 epitope binding domains may comprise an scFv and a single chain diabody, or an scFv and an antibody variable region, or an scFv and another epitope binding domain known in the art, or a single chain diabody and an antibody variable region, or a single chain diabody and another epitope binding domain known in the art, or an antibody variable region and another epitope binding domain known in the art, linked to the N-terminus of the Fc region.

In a specific embodiment, one or more epitope binding domains are scFvs. In one embodiment, an scFv is linked to the N-terminus of the Fc region of polypeptide chains of the invention. In another embodiment, multiple scFvs are linked to the N-terminus of the Fc region. In another embodiment, polypeptide chains of the invention comprise multiple scFvs linked to the N-terminus of the Fc region. In another embodiment, polypeptide chains of the invention comprise at least 1, 2, 3, 4, 5, 6, 7, 8 or more scFvs linked to the N-terminus of the Fc region.

In a specific embodiment, one or more epitope binding domains are single chain diabodies. In one embodiment, a single chain diabody is linked to the N-terminus of the Fc region of polypeptide chains of the invention. In another embodiment, multiple single chain diabodies are linked to the N-terminus of the Fc region. In another embodiment, polypeptide chains of the invention comprise multiple single chain diabodies linked to the N-terminus of the Fc region. In another embodiment, polypeptide chains of the invention comprise at least 1, 2, 3, 4, 5, 6, 7, 8 or more single chain diabodies linked to the N-terminus of the Fc region.

In a specific embodiment, one or more epitope binding domains are antibody variable regions. In one embodiment, an antibody variable region is linked to the N-terminus of the Fc region of polypeptide chains of the invention. In another embodiment, multiple antibody variable regions are linked to the N-terminus of the Fc region (see for example Figure 4D and 5B). In another embodiment, polypeptide chains of the invention comprise multiple antibody variable regions linked to the N-terminus of the Fc region. In another embodiment, polypeptide chains of the invention comprise at least 1, 2, 3, 4, 5, 7, 8 or more antibody variable regions linked to the N-terminus of the Fc region.

### A.4. N-terminal and C-terminal Epitope Binding Domain Containing Polypeptide Chains

In another embodiment, an epitope binding domain known in the art (e.g., an antibody variable region, an scFv, a single chain diabody) is linked to the N-terminus and C-terminus of the Fc region of the polypeptide chains of the invention. In another embodiment, multiple epitope binding domains known in the art are linked to the N-terminus and C-terminus of the Fc region. In another embodiment, the polypeptide chains of the invention comprise multiple epitope binding domains known in the art linked to the N-terminus and C-terminus of the Fc region. In another embodiment, the polypeptide chains of the invention comprise at least 1, 2, 3, 4, 5, 6, 7, 8 or more epitope binding domains known in the art linked to the N-terminus and C-terminus of the Fc region. In another embodiment, the polypeptide chains of the invention comprise multiple epitope binding domains wherein the domains are selected from the group consisting of an antibody variable region, an scFv, a single chain diabody, and another epitope binding domain known in the art.

In certain embodiments, polypeptide chains of the invention comprise more then one type of epitope binding domain linked to the N-terminus and C-terminus of the Fc region. For example, but not by way of limitation a polypeptide chain of the invention comprising 2 epitope binding domains may comprise an scFv and a single chain diabody, or an scFv and an antibody variable region, or an scFv and another epitope binding domain known in the art, or a single chain diabody and an antibody variable region, or a single chain diabody and another epitope binding domain known in the art, or an antibody variable region and another epitope binding domain known in the art, linked to the N-terminus and C-terminus of the Fc region.

In a specific embodiment, one or more epitope binding domains are scFvs. In one embodiment, an scFv is linked to the N-terminus and C-terminus of the Fc region of the epitope binding polypeptide chain of the invention. In another embodiment, multiple scFvs are linked to the N-terminus and C-terminus of the Fc region (see for example Figure 4B). In another embodiment, the polypeptide chains of the invention comprise multiple scFvs linked to the N-terminus and C-terminus of the Fc region. In another embodiment, the polypeptide chains of the invention comprise at least 1, 2, 3, 4, 5, 6, 7, 8 or more scFvs linked to the N-terminus and C-terminus of the Fc region.

In a specific embodiment, one or more epitope binding domains are single chain diabodies. In one embodiment, a single chain diabody is linked to the N-terminus and C-terminus of the Fc region of the epitope binding polypeptide chain of the invention (see for example, Figure 3F). In another embodiment, multiple single chain diabodies are linked to the N-terminus and C-terminus of the Fc region. In another embodiment, the polypeptide chains of the invention comprise multiple single chain diabodies linked to the N-terminus and C-terminus of the Fc region. In another embodiment, the polypeptide chains of the invention comprise at least 1, 2, 3, 4, 5, 6, 7, 8 or more single chain diabodies linked to the N-terminus and C-terminus of the Fc region.

In a specific embodiment, one or more epitope binding domains are antibody variable regions. In one embodiment, an antibody variable region is linked to the N-terminus and C-terminus of the Fc region of the epitope binding polypeptide chain of the invention (see for example Figure 3F). In another embodiment, multiple antibody variable regions are linked to the N-terminus and C-terminus of the Fc region. In another embodiment, the polypeptide chains of the invention comprise multiple antibody variable regions linked to the N-terminus and C-terminus of the Fc region. In another embodiment, the polypeptide chains of the invention comprise at least 1, 2, 3, 4, 5, 7, 8 or more antibody variable regions linked to the N-terminus and C-terminus of the Fc region.

### A.5. Specific Embodiments Of Polypeptide Chain Orientation

In one embodiment, the polypeptide chains of the invention comprise 3 scFvs linked to an Fc region. In a specific embodiment, polypeptide chains of the invention are arranged N-terminus to C-terminus: scFv-scFv-Fc region-scFv (see Figure 1B) or vice versa (scFv-Fc region-scFv-scFv, see Figure 1A inset b). In another specific embodiment, polypeptide chains of the invention are arranged N-terminus to C-terminus: Fc region-scFv-scFv-scFv (see Figure 1A inset c) or vice versa (scFv-scFv-scFv-Fc region).

In another embodiment, polypeptide chains of the invention comprise 4 scFvs linked to an Fc region. In another specific embodiment, polypeptide chains of the invention are arranged N-terminus to C-terminus: scFv-scFv-Fc region-scFv-scFv (see Figure 2B) or vice versa. In still another specific embodiment, polypeptide chains of the invention are arranged N-terminus to C-terminus: scFv-Fc region-scFv-scFv-scFv (see Figure 2A inset b). In yet another specific embodiment, polypeptide chains of the invention are arranged N-terminus to C-terminus: scFv-scFv-scFv-Fc region-scFv (see Figure 2A inset d) or vice versa (see Figure 2A inset c).

In another embodiment, polypeptide chains of the invention comprise an scFv, an antibody variable region and an Fc region. In one embodiment, polypeptide chains of the invention are arranged N-terminus to C-terminus: scFv-antibody variable region-Fc region (see Figure 4E inset a, Figure 4G inset a).

In another embodiment, polypeptide chains of the invention comprise an scFv, an antibody variable region and a Ckappa/lambda region. In one embodiment, polypeptide chains of the invention are arranged N-terminus to C-terminus: scFv-antibody variable region-Ckappa/lambda region (see Figure 4D inset b, Figure 4G inset b).

In another embodiment, polypeptide chains of the invention comprise an scFv, an antibody variable region, an Fc region and an scFv. In one embodiment, polypeptide chains of the invention are arranged N-terminus to C-terminus: scFv-antibody variable region-Fc region- scFv. In another embodiment, polypeptide chains of the invention comprise an scFv, an antibody variable region, an Fc region and two scFvs. In one embodiment, polypeptide chains of the invention are arranged N-terminus to C-terminus: scFv-antibody variable region-Fc region- scFv-scFv (see Figure 4I inset a).

In another embodiment, polypeptide chains of the invention comprise an scFv, an antibody variable region, an Fc region and an scFv. In one embodiment, polypeptide chains of the invention are arranged N-terminus to C-terminus: scFv-antibody variable region-Fc region- scFv. In another embodiment, polypeptide chains of the invention comprise an scFv, an antibody variable region, an Fc region and an scFv. In one embodiment, polypeptide chains of the invention are arranged N-terminus to C-terminus: scFv-antibody variable region-Fc region- scFv (see Figure 4K inset a).

In another embodiment, polypeptide chains of the invention comprise an antibody variable region, an Fc region, and two scFvs. In another specific embodiment, polypeptide chains of the invention are arranged N-terminus to C-terminus: antibody variable region-Fc region-scFv-scFv (see Figure 3B) or vice versa. In another embodiment, polypeptide chains of the invention comprise two antibody variable regions and two scFvs. In a specific embodiment, polypeptide chains of the invention are arranged N-terminus to C-terminus: antibody variable region-antibody variable region-Fc region-scFv-scFv (see Figure 4B) or vice versa. In one embodiment, multispecific epitope binding proteins of the invention comprise two polypeptide chains of the invention.

In some embodiments, polypeptide chains of the invention comprise 1, 2, 3, 4, 5, 6, 7, 8, or more antibody variable domains and one or more CH1, Ckappa/lambda or Fc regions. In some embodiments, polypeptide chains of the invention comprise 2 antibody variable regions with one or more CH1, Ckappa/lambda, or Fc regions. In some embodiments, polypeptide chains of the invention comprise antibody variable regions that are antibody heavy chain variable regions or domains (VH) and/or antibody light chain variable regions or domains (VL). In some embodiments, polypeptide chains of the invention comprise a mixture of antibody variable domain types, such as, but not limited to VH and VL antibody variable regions.

In a specific embodiment, polypeptide chains of the invention comprise 2 antibody variable domains and 2 Ckappa/lambda regions (see for example, Figure 4N.). In another specific embodiment, polypeptide chains of the invention comprise 2 antibody variable domains and 2 CH1 domains (see for example, Figure 4N.). In a specific embodiment, polypeptide chains of the invention comprise 2 antibody variable domains, a CH1 and a Ckappa/lambda (see for example, Figures 4P. and 4T.).

In another embodiment, polypeptide chains of the invention comprise an antibody heavy chain which further comprises a light chain variable domain (VL), a Ckappa/lambda, and an Fc region (see, for example, Figure 4V.) In another embodiment, polypeptide chains of the invention comprise an antibody light chain which further comprises a heavy chain variable domain (VH) and a Ckappa/lambda (see, for example Figure 4V.).

In some embodiments, polypeptide chains of the invention do not comprise antibody variable domains that are identical. In other embodiments, polypeptide chains of the invention do not comprise antibody variable regions with the same epitope binding specificities. In some embodiments, polypeptide chains of the invention do not comprise tandem antibody variable heavy chain domains (VH). In some embodiments, polypeptide chains of the invention do not comprise tandem antibody variable light chain domains (VL).

Monospecific multivalent antibodies comprising Fab domains linked to the heavy chain of an IgG1 molecule are described in PCT publication WO 01/77342 filed March 20, 2001. In some embodiments, proteins of the invention do not comprise Fab domains linked to the heavy chain of an IgG1 molecule are shown in Figure 4 of PCT publication WO 01/77342. In some embodiments, polypeptide chains of the invention are not as shown in Figure 4 of PCT publication WO 01/77342.

In another embodiment, polypeptide chains of the invention do not comprise an Fc region. In one embodiment, polypeptide chains of the invention comprise a Ckappa/lambda region. In another embodiment polypeptide chains of the invention comprise a CH1 domain. In one embodiment, polypeptide chains of the invention comprise antibody variable regions linked to a Ckappa/lambda region and/or a CH1 region. In another embodiment, polypeptide chains of the invention comprise antibody variable regions linked to the N-terminus and/or C-terminus of a Ckappa/lambda region and/or a CH1 region. In a specific embodiment, polypeptide chains of the invention comprise two antibody variable regions linked to a Ckappa/lambda region in the following format N-terminus to C-terminus: VL1-Ckappa/lambda-VL2 (see Figure 2D). In another embodiment, the polypeptide chains of the invention comprise an antibody variable region and 2 scFvs linked to a Ckappa/lambda in the following format, N-terminus to C-terminus: scFv-VL1-Ckappa/lambda-scFv (See Figure 5D). In another embodiment, the polypeptide chains of the invention comprise two antibody variable regions linked to a CH1 region in the following format, N-terminus to C-terminus: VH1-CH1-VH2 (see Figure 2D). In another embodiment, the polypeptide chains of the invention comprise an antibody variable region and two scFvs linked to a CH1 region in the following format, N-terminus to C-terminus: scFv-VH1-CH1-scFv (*i.e*. lacking an Fc domain, see Figure 5D).

### A.6. Linker length and single chain diabody format

It is known that linker length can greatly affect how the variable regions of an scFv fold and interact. In fact, if a short linker is employed (between 5-10 amino acids) intrachain folding is prevented and interchain folding is required to bring the two variable regions together to form a functional epitope binding site. The resulting structure, commonly termed a single chain diabody, has a variety of orientations such as those represented in Figure 6. For more examples of linker orientation and size see, e.g., Hollinger et al. 1993 Proc Natl Acad Sci. U.S.A. 90:6444-6448, U.S. Patent Application Publication Nos. 2005/0100543, 2005/0175606, 2007/0014794, and PCT publication Nos. WO2006/020258 and WO2007/024715 each of which is incorporated by reference for all purposes.

It is also understood that the domains and/or regions of the polypeptide chains of the invention may be separated by linker regions of various lengths. In some embodiments, the epitope binding domains are separated from each other, a Ckappa/lambda, CH1, Hinge, CH2, CH3, or the entire Fc region by a linker region. Such linker region may comprise a random assortment of amino acids, or a restricted set of amino acids. Such linker region may be flexible or rigid.

In some instances, the choice of linker sequences is based on crystal structure analysis of several Fab molecules. There is a natural flexible linkage between the variable domain and the CH1/Ckappa/lambda constant domain in Fab or antibody molecular structure. This natural linkage comprises approximately 10-12 amino acid residues, contributed by 4-6 residues from C-terminus of V domain and 4-6 residues from the N- terminus of Ckappa/lambda/CH1 domain. The N-terminal residues of Ckappa/lambda or CH1 domains, particularly the first 5-6 amino acid residues, adopt a loop conformation without strong secondary structures, therefore can act as flexible linkers between the two variable domains. The N- terminal residues of Ckappa/lambda or CH1 domains are natural extension of the variable domains, as they are part of the Ig sequences, therefore minimize to a large extent any immunogenicity potentially arising from the linkers and junctions. The linker sequences may include any sequence of any length of Ckappa/lambda/CH1 domain but not all residues of Ckappa/lamda/CH1 domain; for example the first 5-12 amino acid residues of the Ckappa/lambda/CH1 domains; and the heavy chain linkers can be derived from CH1 of any isotypes, including Cγ1, Cγ2, Cγ3, Cγ4, Cα1, Cα2, Cδ, Cε, and Cµ. Linker sequences may also be derived from other proteins such as Ig-like proteins, (e.g.TCR, FcR, KIR); hinge region-derived sequences; and other natural sequences from other proteins.

In one embodiment of the invention, polypeptide chains of the invention comprise a linker region of at least 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, or more amino acid residues between one or more of its epitope binding domains, Ckappa/lambda domains, CH1 domains, Hinge region, CH2 domains, CH3 domains, or Fc regions. The linker region may be comprised of any naturally occurring amino acid. In some embodiments, the amino acids glycine and serine comprise the amino acids within the linker region. In another embodiment, the linker region orientation comprises sets of glycine repeats (Gly-Gly-Gly-Gly-Ser)ₓ, where X is a positive integer equal to or greater than 1.

### A.7. Disulfide bond formation and location

It is understood in the art that antibodies comprise interchain disulfide bonds between the Ckappa/lambda and CH1 domains. In some embodiments, polypeptide chains of the invention comprise at least one cysteine residue that may facilitate an interchain disulfide bond. At least one cysteine residue may be present in the VL, VH, CH1, Hinge, CH2, or CH3 regions of the polypeptide chain of the invention. In some embodiments, polypeptide chains of the invention do not comprise a cysteine residue that may facilitate an interchain disulfide bond. In other embodiments, polypeptide chains of the invention may be engineered to remove at least one cysteine residue capable of forming an interchain disulfide bond.

In some embodiments, polypeptide chains of the invention may comprise all or at least a portion of an antibody Hinge region. The Hinge region or portion thereof may be connected directly to an epitope binding domain, a CH1, a Ckappa/lambda, a CH2, or a CH3. In other embodiments, the Hinge region, or portion thereof may be connected through a variable length linker region to an epitope binding domain, a CH1, a Ckappa/lambda, a CH2, or a CH3.

In some embodiments, polypeptide chains of the invention comprise 1, 2, 3, 4, 5, 6, or more Hinge regions or portions thereof. In other embodiments, polypeptide chains of the invention comprise Hinge regions or portions thereof that are identical. In other embodiments, the Hinge regions or portions thereof are not identical. In yet other embodiments, the polypeptide chains of the invention comprise a Hinge region or portion thereof from a human IgG1 molecule. In further embodiments, the Hinge region or portion thereof may be engineered to remove a naturally occurring cysteine residue, introduce a non-naturally occurring cysteine residue, or substitute a naturally occurring residue for a non-naturally occurring cysteine residue. In some embodiments, polypeptide chains of the invention contain at least one Hinge region or portion thereof that comprises the following amino acids sequence: EPKSC (SEQ ID No:1). In other embodiments, polypeptide chains of the invention contain at least one Hinge region or portion thereof that comprises the following amino acids sequence: EPKSCDKTHTCPPCP (SEQ ID No:2). In some embodiments, at least one Hinge region or portion thereof is engineered to substitute at least one naturally occurring cysteine residue with another amino acid residue. In some embodiments, at least one naturally occurring cysteine residue is substituted with serine. In a specific embodiment, polypeptide chains of the invention comprise at least one Hinge region or portion thereof that comprises the following amino acid sequence: EPKSS(Seq ID No:3).

In additional embodiments, polypeptide chains of the invention may comprise non-naturally occurring cysteine residues, useful for site-specific conjugation. Such approaches, compositions and methods are exemplified in U.S. Provisional Patent Application Serial No. 61/022,073 filed January 18, 2008, entitled "Cysteine Engineered Antibodies for Site-Specific Conjugation" and U.S. Patent Application Publication No. 20070092940, filed September 22, 2005, each of which are hereby incorporated by reference in its entirety for all purposes.

### A.8. Dimerization/multimerization domains

The assembly of multispecific epitope binding proteins of the invention rely on domains present in the polypeptide chains that allow for multimerization. For example, conventional antibodies employ the interaction of the CH2 and CH3 regions of the Fc to form a homodimeric molecule. Within the same molecule, antibodies utilize the CH1 and Ckappa/lambda regions from the heavy and light chain subunits to form a heterodimers. It is also possible to employ naturally occurring protein multimerization domains to bring polypeptide chains of the invention to form multispecific epitope binding proteins. In some embodiments, polypeptide chains of the invention comprise a protein dimerization/multimerization domain selected from: CH1, CH2, CH3, Ckappa/lambda, leucine zipper domain (bZIP), helix-loop-helix motif, an EF hand, a phosphotyrosine binding (PTB) domain, Src homology domains (SH2, SH3), or other domains known in the art. In other embodiments, polypeptide chains of the invention comprise multimerization domains as presented in U.S. Patent Publication No.20070140966 filed December 5, 2006 and incorporated by reference in its entirety.

### B. Vectors encoding polypeptide chains of the invention

The multispecific epitope binding proteins of the invention may comprise two-four polypeptide chains. In other embodiments, multispecific epitope binding proteins of the invention may comprise 5, 6, 7, 8, or more polypeptide chains. Each polypeptide chain of the multispecific epitope binding protein of the invention may comprise 1, 2, 3, 4, 5, 6, 7, 8, or more epitope binding domains. The polypeptide chains comprise epitope binding domains that may be scFvs, single chain diabodies, variable regions of antibodies, or other known epitope binding domains known in the art. The Fc region and the epitope binding domains may be linked together in many different orientations (See, for example, Section A and Figures 1-5). The invention also provides polynucleotide vectors for generating and/or expressing the polypeptide chains and multispecific epitope binding proteins of the invention. In one embodiment, the vectors for generating the polypeptide chains encode epitope binding domains that are linked to the C-terminus of the Fc region. In another embodiment, the vectors for generating the polypeptide chains encode epitope binding domains that are linked to the N-terminus of the Fc region. In another embodiment, the vectors for generating the polypeptide chains encode epitope binding domains that are linked to the N-terminus and the C-terminus of the Fc region.

In one embodiment, the multispecific epitope binding polypeptide chains of the invention are expressed from a vector comprising a promoter, a polynucleotide sequence encoding the polypeptide chain of the invention, and a poly A tail. In another embodiment, the expression vector comprises a promoter, a polynucleotide sequence encoding an epitope binding polypeptide chain comprising an Fc region, and a poly A tail. In another embodiment, the expression vector comprises a promoter, a polynucleotide sequence encoding an epitope binding polypeptide chain comprising an Fc region linked N-terminal to 1, 2, 3, 4, 5, 6, 7, 8, or more epitope binding domains, and a poly A tail. In another embodiment, the expression vector comprises a promoter, a polynucleotide sequence encoding an epitope binding polypeptide chain comprising an Fc region linked **C-terminal** to 1, 2, 3, 4, 5, 6, 7, 8, or more epitope binding domains, and a poly A tail. In still another embodiment, the expression vector comprises a promoter, a polynucleotide sequence encoding 1, 2, 3, 4, 5, 6, 7, 8, or more epitope binding domains linked N-terminal and C-terminal to an Fc region.

In other embodiments, vectors of the invention further comprise a polynucleotide sequence encoding multispecific epitope binding polypeptide chain of the invention that comprises at least 1, at least 2, at least 3, at least 4, at least 5, at least 6, at least 7, at least 8 or more CH1 and/or Ckappa/lambda regions. In other embodiments, multispecific epitope binding proteins of the invention comprise 1, 2, 3, 4, 5, 6, 7, 8 or more CH1 and/or Ckappa/lambda regions. In another embodiment, the Fc region, CH1 region or Ckappa/lambda region are derived from any antibody subtype known in the art. In other embodiments, the Fc region is a chimera derived from multiple antibody subtypes known in the art.

In alternative embodiments, multispecific epitope binding proteins of the invention may comprise a CH1 or a Ckappa/lambda region in the absence of an Fc region. In other embodiments, multispecific epitope binding proteins of the invention comprise at least 1, at least 2, at least 3, at least 4, at least 5, at least 6, at least 7, at least 8 or more CH1 and or Ckappa/lambda regions in the absence of an Fc region. In other embodiments, multispecific epitope binding proteins of the invention may comprise all or a portion of the hinge region of an antibody in the absence of an Fc region.

### B.1. Vectors encoding polypeptide chains with C-terminal epitope binding domains

In one embodiment, the expression vector comprises a promoter, a polynucleotide sequence encoding an scFv linked to the C-terminus of the Fc region, and a poly A tail (see for example Figure 1A (inset a, b, c)). In another embodiment, the expression vector comprises a promoter, a polynucleotide sequence encoding multiple scFvs linked to the C-terminus of the Fc region, and a poly A tail (see for example Figure 2A (inset a, b, c). In another embodiment, the expression vector comprises a promoter, a polynucleotide sequence encoding 1, 2, 3, 4, 5, 6, 7, 8 or more scFvs linked to the C-terminus of the Fc region and a poly A tail.

In another embodiment, the expression vector comprises a promoter, a polynucleotide sequence encoding a single chain diabody linked to the C-terminus of the Fc region, and a poly A tail (see for example Figure 3E (inset a)). In another embodiment, the expression vector comprises a promoter, a polynucleotide sequence encoding multiple single chain diabodies linked to the C-terminus of the Fc region, and a poly A tail. In another embodiment, the expression vector comprises a promoter, a polynucleotide sequence encoding 1, 2, 3, 4, 5, 6, 7, 8 or more single chain diabodies linked to the C-terminus of the Fc region and a poly A tail.

In another embodiment, the expression vector comprises a promoter, a polynucleotide sequence encoding an antibody variable region linked to the C-terminus of the Fc region, and a poly A tail. In another embodiment, the expression vector comprises a promoter, a polynucleotide sequence encoding multiple antibody variable regions linked to the C-terminus of the Fc region, and a poly A tail. In another embodiment, the expression vector comprises a promoter, a polynucleotide sequence encoding 1, 2, 3, 4, 5, 6, 7, 8 or more antibody variable regions linked to the C-terminus of the Fc region and a poly A tail.

In another embodiment, the expression vector comprises a promoter, a polynucleotide sequence encoding an epitope binding domain known in the art linked to the C-terminus of the Fc region, and a poly A tail. In another embodiment, the vector comprises, a promoter, a polynucleotide sequence encoding multiple epitope binding domains known in the art linked to the C-terminus of the Fc region, and a poly A tail. In another embodiment, the vector comprises a promoter, a polynucleotide sequence encoding 1, 2, 3, 4, 5, 6, 7, 8 or more epitope binding domains known in the art linked to the C-terminus of the Fc region, and a poly A tail. In another embodiment, the vector comprises a promoter, a polynucleotide sequence encoding multiple epitope binding domains wherein the domains are selected from the group consisting of an antibody variable region, an scFv, a single chain diabody, and another epitope binding domain known in the art, linked to the C-terminus of an Fc region, and a poly A tail.

### B.2. Vectors encoding polypeptide chains with N-terminal epitope binding domains

In another embodiment, the expression vector comprises a promoter, a polynucleotide sequence encoding an scFv linked to the N-terminus of the Fc region, and a poly A tail (see for example Figure 1A (inset a, b, d, e, and f)). In another embodiment, the expression vector comprises a promoter, a polynucleotide sequence encoding multiple scFvs linked to the N-terminus of the Fc region, and a poly A tail (see for example Figure 2A (inset a, d, e, and f)). In another embodiment, the expression vector comprises a promoter, a polynucleotide sequence encoding 1, 2, 3, 4, 5, 6, 7, 8 or more scFvs linked to the N-terminus of the Fc region and a poly A tail.

In another embodiment, the expression vector comprises a promoter, a polynucleotide sequence encoding a single chain diabody linked to the N-terminus of the Fc region, and a poly A tail. In another embodiment, the expression vector comprises a promoter, a polynucleotide sequence encoding multiple single chain diabodies linked to the N-terminus of the Fc region, and a poly A tail. In another embodiment, the expression vector comprises a promoter, a polynucleotide sequence encoding 1, 2, 3, 4, 5, 6, 7, 8 or more single chain diabodies linked to the N-terminus of the Fc region and a poly A tail.

In another embodiment, the expression vector comprises a promoter, a polynucleotide sequence encoding an antibody variable region (*e.g.*, heavy and or light chain variable region) linked to the N-terminus of the Fc region, and a poly A tail (see for example Figure 3A (inset a and c)). In another embodiment, the expression vector comprises a promoter, a polynucleotide sequence encoding multiple antibody variable regions linked to the N-terminus of the Fc region, and a poly A tail. In another embodiment, the expression vector comprises a promoter, a polynucleotide sequence encoding 1, 2, 3, 4, 5, 6, 7, 8 or more antibody variable regions linked to the N-terminus of the Fc region and a poly A tail.

In another embodiment, the expression vector comprises a promoter, a polynucleotide sequence encoding an epitope binding domain known in the art linked to the N-terminus of the Fc region, and a poly A tail. In another embodiment, the vector comprises, a promoter, a polynucleotide sequence encoding multiple epitope binding domains known in the art linked to the N-terminus of the Fc region, and a poly A tail. In another embodiment, the vector comprises a promoter, a polynucleotide sequence encoding 1, 2, 3, 4, 5, 6, 7, 8 or more epitope binding domains known in the art linked to the N-terminus of the Fc region, and a poly A tail. In another embodiment, the vector comprises a promoter, a polynucleotide sequence encoding multiple epitope binding domains wherein the domains are selected from the group consisting of an antibody variable region, an scFv, a single chain diabody, and another epitope binding domain known in the art, linked to the N-terminus of an Fc region, and a poly A tail.

### B.3. Vectors encoding polypeptide chains with N-terminal and C-terminal epitope binding domains

Also encompassed by the present invention are expression vectors comprising a promoter and one or more epitope binding domains linked to both the N-terminus and the C-terminus of the Fc region, and a poly A tail. In one embodiment, the expression vector comprises: (a) a promoter; (b) a polynucleotide sequence encoding at least one epitope binding domain linked to the N-terminus and at least one epitope binding domain linked to the C-terminus and (c) a poly A tail, wherein each epitope binding domain is selected from the group consisting of are selected from the group consisting of an antibody variable region, an scFv, a single chain diabody, or another epitope binding domain known in the art. In another embodiment, same type of epitope binding domain (*e.g*., scFv) is linked to both the N-terminus and the C-terminus of the Fc region. In still another embodiment, different type of epitope binding domains are linked to both the N-terminus and the C-terminus of the Fc region. For example, but not by way of limitation, one or more scFvs may be linked to the N-terminus and one or more single chain diabodies may be linked C-terminus or one or more scFvs and one or more single chain diabodies may be linked to the N-terminus and the C-terminus.

In a specific embodiment, the expression vector comprises a promoter, a polynucleotide sequence encoding at least one scFv linked to the N-terminus and C-terminus of the Fc region, and a poly A tail (see for example Figure 1A (inset a and b) and Figure 2A (inset a and b)). In another embodiment, the expression vector comprises a promoter, a polynucleotide sequence encoding multiple scFvs linked to the N-terminus and C-terminus of the Fc region, and a poly A tail (see for example Figure 2A (inset a)). In another embodiment, the expression vector comprises a promoter, a polynucleotide sequence encoding 1, 2, 3, 4, 5, 6, 7, 8 or more scFvs linked to the N-terminus and C-terminus of the Fc region and a poly A tail.

In another embodiment, the expression vector comprises a promoter, a polynucleotide sequence encoding at least one single chain diabody linked to the N-terminus and C-terminus of the Fc region, and a poly A tail. In another embodiment, the expression vector comprises a promoter, a polynucleotide sequence encoding multiple single chain diabodies linked to the N-terminus and C-terminus of the Fc region, and a poly A tail. In another embodiment, the expression vector comprises a promoter, a polynucleotide sequence encoding 1, 2, 3, 4, 5, 6, 7, 8 or more single chain diabodies linked to the N-terminus and C-terminus of the Fc region and a poly A tail.

In another embodiment, the expression vector comprises a promoter, a polynucleotide sequence encoding at least one antibody variable region linked to the N-terminus and C-terminus of the Fc region, and a poly A tail. In another embodiment, the expression vector comprises a promoter, a polynucleotide sequence encoding multiple antibody variable regions linked to the N-terminus and C-terminus of the Fc region, and a poly A tail. In another embodiment, the expression vector comprises a promoter, a polynucleotide sequence encoding 1, 2, 3, 4, 5, 6, 7, 8 or more antibody variable regions linked to the N-terminus and C-terminus of the Fc region and a poly A tail.

In another embodiment, the expression vector comprises a promoter, a polynucleotide sequence encoding an epitope binding domain known in the art linked to the N-terminus and the C-terminus of the Fc region, and a poly A tail. In another embodiment, the vector comprises, a promoter, a polynucleotide sequence encoding multiple epitope binding domains known in the art linked to the N-terminus and the C-terminus of the Fc region, and a poly A tail. In another embodiment, the vector comprises a promoter, a polynucleotide sequence encoding 1, 2, 3, 4, 5, 6, 7, 8 or more epitope binding domains known in the art linked to the N-terminus and the C-terminus of the Fc region, and a poly A tail. In another embodiment, the vector comprises a promoter, a polynucleotide sequence encoding multiple epitope binding domains wherein the domains are selected from the group consisting of an antibody variable region, an scFv, a single chain diabody, and another epitope binding domain known in the art, linked to the N-terminus and the C-terminus of an Fc region, and a poly A tail.

### B.4. Specific embodiments of vectors encoding polypeptide chains of the invention

In one embodiment, vectors of the invention comprise a promoter, a polynucleotide sequence encoding 3 scFvs linked to an Fc region, and a poly a tail. In a specific embodiment, vectors of the invention comprise a promoter, a polynucleotide sequence encoding 3 scFvs linked to an Fc region arranged N-terminus to C-terminus: scFv-scFv-Fc region-scFv, and a poly A tail (see Figure 1A (inset a)). In another embodiment, vectors of the invention comprise a promoter, a polynucleotide sequence encoding 4 scFvs linked to an Fc region, and a poly A tail. In another specific embodiment, vectors of the invention comprise a promoter, a polynucleotide sequence encoding 4 scFvs linked to an Fc region arranged scFv-scFv-Fc region-scFv-scFv, and a poly A tail (see Figure 2A (inset a)). In another embodiment, vectors of the invention comprise a promoter, a polynucleotide sequence encoding an antibody variable region and two scFvs linked to an Fc region, and a poly A tail. In another specific embodiment, vectors of the invention comprise a promoter, a polynucleotide sequence encoding an antibody variable region, 2 scFvs and an Fc region arranged antibody variable region-Fc region-scFv-scFv, and a poly A tail (see Figure 3A (inset a and c)). In another embodiment, vectors of the invention comprise a promoter, a polynucleotide sequence encoding two antibody variable regions and two scFvs linked to an Fc region and a poly A tail. In a specific embodiment, vectors of the invention comprise a promoter, a polynucleotide sequence encoding 2 antibody variable regions and 2 scFvs linked to an Fc region arranged N-terminus to C-terminus; antibody variable region-antibody variable region-Fc region-scFv-scFv, and a poly A tail (see Figure 4A(inset a and c)). In yet another specific embodiment, vectors of the invention comprise a promoter, a polynucleotide sequence encoding an antibody variable region and 2 scFvs linked to an Fc region arranged N-terminus to C-terminus; scFv-antibody variable region-Fc region-scFv, and a poly A tail (see Figure 4K(inset a).

In another embodiment, vectors of the invention comprise a promoter, a polynucleotide sequence encoding two antibody variable regions linked to a Ckappa/lambda region, and a poly A tail. In a specific embodiment, vectors of the invention comprise a promoter, a polynucleotide sequence encoding two antibody light chain variable domains linked to a Ckappa/lambda region arranged N-terminus to C-terminus in the following orientation: VL1-Ckappa/lambda-VL2, and a poly A tail (see Figure 2C inset A). In another specific embodiment, vectors of the invention comprise a promoter, a polynucleotide sequence encoding an antibody variable region and 2 scFvs linked to a Ckappa/lambda region arranged N-terminus to C-terminus in the following orientation: scFv-VL1-Ckappa/lambda-scFv, and a poly A tail (see Figure 5C inset b).

In another embodiment, vectors of the invention comprise a promoter, a polynucleotide sequence encoding two antibody variable regions linked to a CH1 region, and a poly A tail. In a specific embodiment, vectors of the invention comprise a promoter, a polynucleotide sequence encoding two antibody heavy chain variable domains linked to a CH1 region arranged N-terminus to C-terminus in the following orientation: VH1-CH1-VH2, and a poly A tail (see Figure 2C inset b). In another specific embodiment, vectors of the invention comprise a promoter, a polynucleotide sequence encoding an antibody variable region and 2 scFvs linked to a CH1 region arranged N-terminus to C-terminus in the following orientation: scFv-VH1-CH1-scFv, and a poly A tail (see Figure 5C inset a).

In another embodiment, vectors of the invention comprise a promoter, a polynucleotide sequence encoding 2 antibody variable domains flanking a CH1, a Ckappa/lambda, or an Fc region. In some embodiments, the antibody variable domains are heavy chain variable domains and/or light chain variable domains. In a specific embodiment, vectors of the invention comprise a promoter, a polynucleotide sequence encoding 2 heavy chain antibody variable domains, 2 CH1 domains, and an Fc region arranged N-terminus to C-terminus in the following orientation: VH2-first CH1-VH1- second CH1-Fc region, and a poly A tail (see Figure 4M, inset A). In another specific embodiment, vectors of the invention comprise a promoter, a polynucleotide sequence encoding 2 antibody light chain variable domains (VL) and 2 Ckappa/lambda regions arranged N-terminus to C-terminus in the following orientation: VL2-first Ckappa/lambda-VL1-second Ckappa/lambda, and a poly A tail (See Figure 4M inset B). In another specific embodiment, vectors of the invention comprise a promoter, a polynucleotide sequence encoding a VL domain, a Ckappa/lambda, a VH domain, a CH1 domain, and an Fc region arranged N-terminus to C-terminus in the following orientation: VL-Ckappa/lambda-VH-CH1-Fc region, and a poly A tail (see Figure 40, inset a). In another specific embodiment, vectors of the invention comprise a promoter, a sequence encoding a VH domain, a CH1 domain, a VL domain, a Ckappa/lambda domain arranged N-terminus to C-terminus in the following orientation:VH-CH1-VL-Ckappa/lambda, and a poly A tail (see Figure 40, inset b). In yet another specific embodiment, vectors of the invention comprise a promoter, a polynucleotide sequence encoding 2 antibody light chain variable domains, 2 Ckappa/lambda regions, and an Fc region arranged N-terminus to C-terminus in the following orientation:VL-Ckappa/lambda-VL-Ckappa/lambda-Fc region, and a poly A tail (see Figure 4Q, inset a). In another specific embodiment, vectors of the invention comprise a promoter, a polynucleotide sequence encoding 2 antibody heavy chain variable domains, and 2 CH1 domains, arranged N-terminus to C-terminus in the following orientation: VH2-CH1-VH1-CH1, and a poly A tail (see Figure 4Q, inset b). In another specific embodiment, vectors of the invention comprise a polynucleotide sequence encoding an antibody light chain variable domain, a Ckappa/lambda, an antibody heavy chain, a CH1, and an Fc region arranged N-terminus to C-terminus in the following orientation: VL-Ckappa/lambda-VH-CH1-Fc region, and a poly A tail (see Figure 4S., inset a). In yet another specific embodiment, vectors of the invention comprise a promoter, a polynucleotide sequence encoding an antibody heavy chain variable domain, a CH1, an antibody light chain variable domain, and a Ckappa/lambda arranged N-terminus to C-terminus in the following orientation:VH-CH1-VL-Ckappa/lambda, and a poly A tail (see Figure 4S, inset b).

In other specific embodiments, vectors of the invention comprise a promoter, a polynucleotide sequence encoding an antibody light chain variable domain and a Ckappa/lambda arranged N-tenninus to C-terminus in the following orientation: VL-Ckappa/lambda, and a poly A tail (see Figure 4M, inset c and d; Figure 40, inset d; and Figure 4S, inset d) In yet another specific embodiment, vectors of the invention comprise a promoter, a polynucleotide sequence encoding an antibody heavy chain variable domain and a CH1 arranged N-terminus to C-terminus in the following orientation: VH-CH1, and a poly A tail (see Figure 40, inset c; Figure 4Q insets c and d; Figure 4S inset c; and Figure 4U, inset b).

In another specific embodiment, vectors of the invention comprise a promoter, a polynucleotide sequence encoding an antibody light chain variable domain, a Ckappa/lambda, and an Fc region arranged N-terminus to C-terminus in the following orientation: VL-Ckappa/lambda-Fc region, and a poly A tail (see Figure 4U, inset a).

In some embodiments, vectors of the invention are not vectors as presented in Figure 5. of PCT publication WO 01/77342 filed March 20, 2001.

### C. Multispecific epitope binding proteins - two chains

Described in the following sections are the assembly and orientation of multispecific epitope binding proteins of the invention comprising two polypeptide chains.

In some embodiments, the multispecific epitope binding proteins of the invention comprise a first and a second polypeptide chain of the invention, wherein the first and/or second polypeptide chain comprises an Fc region linked to 1, 2, 3, 4, 5, 6, 7, 8, or more epitope binding domains. The polypeptide chains of the invention comprise epitope binding domains which may be scFvs, single chain diabodies, variable regions of antibodies, or other epitope binding domains known in the art. The Fc region and the epitope binding domains may be linked together in many different orientations (See section A, *supra*)*.* In one embodiment, the epitope binding domains are linked to the C-terminus of the Fc region. In other embodiments the epitope binding domains are linked to the N-terminus of the Fc region. In other embodiments, the epitope binding domains are linked to both the N-terminus and C-terminus of the Fc region. In some embodiments, the multispecific epitope binding proteins of the invention comprise a first and a second polypeptide chain of the invention, wherein the first and/or second polypeptide chain comprises 1, 2, 3, 4, 5, 6, 7, 8, or more Fc regions linked to 1, 2, 3, 4, 5, 6, 7, 8, or more epitope binding domains. In one embodiment, the epitope binding domains are linked to the C-terminus of at least one Fc region. In other embodiments the epitope binding domains are linked to the N-terminus of at least one Fc region. In other embodiments, the epitope binding domains are linked to both the N-terminus and C-terminus of at least one Fc region.

In alternate embodiments, the multispecific epitope binding proteins of the invention do not comprise an Fc region. In such proteins of the invention, the epitope binding domains may be linked N-terminus, C-terminus, or N- and C-terminus to a CH1 domain and/or a Ckappa/lambda domain. It is to be understood that term Fc region may be replaced by CH1 domain and/or Ckappa/lambda in the following sections as to encompass proteins of the invention that do not comprise an Fc region. In some alternate embodiments, the multispecific epitope binding proteins of the invention comprise a first and a second polypeptide chain of the invention, wherein the first and/or second polypeptide chain comprises 1, 2, 3, 4, 5, 6, 7, 8, or more CH1 and/or Ckappa/lambda regions linked to 1, 2, 3, 4, 5, 6, 7, 8, or more epitope binding domains. In one embodiment, the epitope binding domains are linked to the C-terminus of at least one CH1 and/or Ckappa/lambda region. In other embodiments the epitope binding domains are linked to the N-terminus of at least one CH1 and/or Ckappa/lambda region. In other embodiments, the epitope binding domains are linked to both the N-terminus and C-terminus of at least one CH1 and/or Ckappa/lambda region.

### C.1. Multispecific epitope binding proteins with epitope binding domains linked to the C-terminus of the Fc region

In one embodiment, multispecific epitope binding proteins of the invention comprise a first and a second polypeptide chain wherein the first and/or second polypeptide chain comprise an Fc region. In one embodiment, multispecific epitope binding proteins comprises a first and a second polypeptide chain wherein the first and/or second chain comprises an scFv linked to the C-terminus of the Fc region (see for example Figure 1B). In another embodiment, the first and/or second polypeptide chain comprises multiple scFvs linked to the C-terminus of the Fc region (see for example Figure 2B). In another embodiment, epitope binding proteins of the invention comprise a first and/or second chain with at least 1, 2, 3, 4, 5, 6, 7, 8 or more scFvs linked to the C-terminus of the Fc region.

In one embodiment, the multispecific epitope binding protein comprises a first and a second polypeptide chain wherein the first and/or second chain comprises a single chain diabody linked to the C-terminus of the Fc region (see for example Figure 3F). In another embodiment, the first and/or second polypeptide chain comprises multiple single chain diabodies linked to the C-terminus of the Fc region. In another embodiment, the epitope binding protein of the invention comprises a first and/or second chain with at least 1, 2, 3, 4, 5, 6, 7, 8 or more single chain diabodies linked to the C-terminus of the Fc region.

In one embodiment, multispecific epitope binding proteins comprise a first and a second polypeptide chain wherein the first and/or second chain comprises an antibody variable region linked to the C-terminus of the Fc region. In another embodiment, the first and/or second polypeptide chain comprises multiple antibody variable regions linked to the C-terminus of the Fc region. In another embodiment, epitope binding proteins of the invention comprise a first and/or second chain with at least 1, 2, 3, 4, 5, 6, 7, 8 or more antibody variable regions linked to the C-terminus of the Fc region.

In one embodiment, multispecific epitope binding proteins comprise a first and a second polypeptide chain wherein the first and/or second chain comprise an epitope binding domain known in the art linked to the C-terminus of the Fc region. In another embodiment, the first and/or second polypeptide chain comprises multiple epitope binding domains known in the art linked to the C-terminus of the Fc region. In another embodiment, epitope binding proteins of the invention comprise a first and/or second chain with at least 1, 2, 3, 4, 5, 6, 7, 8 or more epitope binding proteins known in the art wherein the domains are selected from the group consisting of an antibody variable region, an scFv, a single chain diabody, and another epitope binding domain known in the art, linked to the C-terminus of the Fc region.

### C.2. Multispecific epitope binding proteins with epitope binding domains linked to the N-terminus of the Fc region

In one embodiment, multispecific epitope binding proteins of the invention comprise a first and a second polypeptide chain, wherein the first and/or second polypeptide chain comprises an Fc region. In one embodiment, multispecific epitope binding proteins of the invention comprise a first and a second polypeptide chain wherein the first and/or second chain comprises an scFv linked to the N-terminus of the Fc region (see for example Figure 1A (inset b)). In another embodiment, the first and/or second polypeptide chain comprises multiple scFvs linked to the N-terminus of the Fc region (see for example Figure 1A (insets a and c)). In another embodiment, the epitope binding protein of the invention comprises a first and/or second chain with at least 1, 2, 3, 4, 5, 6, 7, 8 or more scFvs linked to the N-terminus of the Fc region.

In one embodiment, the multispecific epitope binding proteins comprise a first and a second polypeptide chain wherein the first and/or second chain comprises a single chain diabody linked to the N-terminus of the Fc region. In another embodiment, the first and/or second polypeptide chain comprises multiple single chain diabodies linked to the N-terminus of the Fc region. In another embodiment, epitope binding proteins of the invention comprise a first and/or second chain with at least 1, 2, 3, 4, 5, 6, 7, 8 or more single chain diabodies linked to the N-terminus of the Fc region.

In one embodiment, the multispecific epitope binding protein comprises a first and a second polypeptide chain wherein the first and/or second chain comprises an antibody variable region linked to the N-terminus of the Fc region (see for example Figure 3B). In another embodiment, the first and/or second polypeptide chain comprises multiple antibody variable regions linked to the N-terminus of the Fc region (see for example Figure 4B). In another embodiment, the epitope binding protein of the invention comprises a first and/or second chain with at least 1, 2, 3, 4, 5, 6, 7, 8 or more antibody variable regions linked to the N-terminus of the Fc region.

In one embodiment, the multispecific epitope binding protein comprises a first and a second polypeptide chain wherein the first and/or second chain comprises an epitope binding domain known in the art linked to the N-terminus of the Fc region. In another embodiment, the first and/or second polypeptide chain comprises multiple epitope binding domains known in the art linked to the N-terminus of the Fc region. In another embodiment, the epitope binding protein of the invention comprises a first and/or second chain with at least 1, 2, 3, 4, 5, 6, 7, 8 or more epitope binding proteins known in the art wherein the domains are selected from the group consisting of an antibody variable region, an scFv, a single chain diabody, and another epitope binding domain known in the art, linked to the N-terminus of the Fc region.

### C.3. Multispecific epitope binding proteins with epitope binding domains linked to the N-terminus or C-terminus of the Fc region

In one embodiment, multispecific epitope binding proteins of the invention comprise a first and a second polypeptide chain, wherein the first and/or second polypeptide chain comprises an Fc region. In one embodiment, the multispecific epitope binding protein comprises a first and a second polypeptide chain wherein the first and/or second chain comprises an scFv linked to the N-terminus or C-terminus of the Fc region. In another embodiment, the first and/or second polypeptide chain comprises multiple scFvs linked to the N-terminus or C-terminus of the Fc region. In another embodiment, the epitope binding protein of the invention comprises a first and/or second chain with at least 1, 2, 3, 4, 5, 6, 7, 8 or more scFvs linked to the N-terminus or C-terminus of the Fc region.

In one embodiment, the multispecific epitope binding protein comprises a first and a second polypeptide chain wherein the first and second chain comprises a single chain diabody linked to the N-terminus or C-terminus of the Fc region. In another embodiment, the first and/or second polypeptide chain comprises multiple single chain diabodies linked to the N-terminus or C-terminus of the Fc region. In another embodiment, the epitope binding protein of the invention comprises a first and/or second chain with at least 1, 2, 3, 4, 5, 6, 7, 8 or more single chain diabodies linked to the N-terminus or C-terminus of the Fc region.

In one embodiment, the multispecific epitope binding protein comprises a first and a second polypeptide chain wherein the first and/or second chain comprises an antibody variable region linked to the N-terminus or C-terminus of the Fc region. In another embodiment, the first and/or second polypeptide chain comprises multiple antibody variable regions linked to the N-terminus or C-terminus of the Fc region. In another embodiment, the epitope binding protein of the invention comprises a first and/or second chain with at least 1, 2, 3, 4, 5, 6, 7, 8 or more antibody variable regions linked to the N-terminus or C-terminus of the Fc region.

In one embodiment, the multispecific epitope binding protein comprises a first and a second polypeptide chain wherein the first and/or second chain comprises an epitope binding domain known in the art linked to the N-terminus or C-terminus of the Fc region. In another embodiment, the first and/or second polypeptide chain comprises multiple epitope binding domains known in the art linked to the N-terminus or C-terminus of the Fc region. In another embodiment, the epitope binding protein of the invention comprises a first and/or second chain with at least 1, 2, 3, 4, 5, 6, 7, 8 or more epitope binding proteins known in the art wherein the domains are selected from the group consisting of an antibody variable region, an scFv, a single chain diabody, and another epitope binding domain known in the art, linked to the N-terminus or C-terminus of the Fc region.

### C.4. Multispecific epitope binding proteins with epitope binding domains linked to the N-terminus and C-terminus of the Fc region

In one embodiment, multispecific epitope binding proteins of the invention comprise a first and a second polypeptide chain, wherein the first and/or second polypeptide chain comprises an Fc region. In one embodiment, the multispecific epitope binding protein comprises a first and a second polypeptide chain wherein the first and/or second chain comprises an scFv linked to the N-terminus and C-terminus of the Fc region (see for example Figure 1B). In another embodiment, the first and/or second polypeptide chain comprises multiple scFvs linked to the N-terminus and C-terminus of the Fc region (see for example Figure 2B). In another embodiment, the epitope binding protein of the invention comprises a first and/or second chain with at least 1, 2, 3, 4, 5, 6, 7, 8 or more scFvs linked to the N-terminus and C-terminus of the Fc region.

In one embodiment, the multispecific epitope binding protein comprises a first and a second polypeptide chain wherein the first and/or second chain comprises a single chain diabody linked to the N-terminus and C-terminus of the Fc region. In another embodiment, the first and/or second polypeptide chain comprises multiple single chain diabodies linked to the N-terminus and C-terminus of the Fc region. In another embodiment, the epitope binding protein of the invention comprises a first and/or second chain with at least 1, 2, 3, 4, 5, 6, 7, 8 or more single chain diabodies linked to the N-terminus and C-terminus of the Fc region.

In one embodiment, the multispecific epitope binding protein comprises a first and a second polypeptide chain wherein the first and/or second chain comprises an antibody variable region linked to the N-terminus and C-terminus of the Fc region. In another embodiment, the first and/or second polypeptide chain comprises multiple antibody variable regions linked to the N-terminus and C-terminus of the Fc region. In another embodiment, the epitope binding protein of the invention comprises a first and/or second chain with at least 1, 2, 3, 4, 5, 6, 7, 8 or more antibody variable regions linked to the N-terminus and C-terminus of the Fc region.

In one embodiment, the multispecific epitope binding protein comprises a first and a second polypeptide chain wherein the first and/or second chain comprises an epitope binding domain known in the art linked to the N-terminus and C-terminus of the Fc region. In another embodiment, the first and/or second polypeptide chain comprises multiple epitope binding domains known in the art linked to the N-terminus and C-terminus of the Fc region. In another embodiment, the epitope binding protein of the invention comprises a first and/or second chain with at least 1, 2, 3, 4, 5, 6, 7, 8 or more epitope binding proteins known in the art wherein the domains are selected from the group consisting of an antibody variable region, an scFv, a single chain diabody, and another epitope binding domain known in the art, linked to the N-terminus and C-terminus of the Fc region.

### C.5. Multispecific epitope binding protein assembly

In another embodiment, the multispecific epitope binding protein comprises a first and a second polypeptide chain, each chain comprising an Fc region. In a further embodiment, the first and/or second polypeptide chain comprises any epitope binding domain, including scFvs, single chain diabodies, antibody variable regions, and any epitope binding domains known in the art. In another embodiment, the multispecific epitope binding protein comprises a first and a second polypeptide chain dimerized by the Fc region. In another embodiment, the multispecific epitope binding protein comprises a first and a second polypeptide chain wherein the first and second polypeptide chains are not identical in amino acid sequence. In another embodiment, the multispecific epitope binding protein comprises a first and a second polypeptide chain wherein the first and second polypeptide chains are identical in amino acid sequence. In another embodiment, multispecific epitope binding proteins of the invention are heterodimers. In another embodiment, multispecific epitope binding proteins of the invention are homodimers.

### C.6. Specific Embodiments - Two Chains

In one embodiment, multispecific epitope binding proteins of the invention comprise a first and a second polypeptide chain wherein, the first and/or second polypeptide chain comprises an Fc region. In another embodiment, multispecific epitope binding proteins of the invention comprise a first and a second polypeptide chain, wherein the first and/or second polypeptide chain comprises 3 scFvs linked to an Fc region. In a specific embodiment, multispecific epitope binding proteins of the invention comprise a first and a second polypeptide chain, wherein the first and/or second polypeptide chain comprises 3 scFvs linked to an Fc region arranged N-terminus to C-terminus scFv-scFv-Fc region-scFv (see Figure 1B) or vice versa. In another embodiment, multispecific epitope binding proteins of the invention comprise a first and a second polypeptide chain, wherein the first and/or second polypeptide chain comprises 4 scFvs linked to an Fc region. In a specific embodiment, multispecific epitope binding proteins of the invention comprise a first and a second polypeptide chain, wherein the first and/or second polypeptide chain comprises 4 scFvs linked to an Fc region arranged N-terminus to C-terminus scFv-scFv-Fc region-scFv-scFv (see Figure 2B) or vice versa (C-terminus to N-terminus scFv-scFv-Fc region-scFv-scFv).

In one embodiment, multispecific epitope binding proteins of the invention comprise a first and a second polypeptide chain wherein, the first and/or second polypeptide chain comprises a Ckappa/lambda region. In another embodiment, multispecific epitope binding proteins of the invention comprise a first and a second polypeptide chain, wherein the first and/or second chain comprises two antibody variable regions linked to a Ckappa/lambda domain. In another embodiment, multispecific epitope binding proteins of the invention comprise a first and a second polypeptide chain, wherein the first and/or second chain comprises two antibody variable regions linked to a Ckappa/lambda region arranged N-terminus to C-terminus: VL1-Ckappa/lambda-VL2 (see Figure 2D). In another embodiment, multispecific epitope binding proteins of the invention comprise a first and a second polypeptide chain wherein, the first and/or second chain comprises an antibody variable region and 2 scFvs linked to a Ckappa/lambda region. In a specific embodiment, multispecific epitope binding proteins of the invention comprise a first and a second polypeptide chain, wherein the first and/or second chain comprises an antibody variable region and 2 scFvs linked to a Ckappa/lambda region arranged N-terminus to C-terminus: scFv-VL1-Ckappa/lambda-scFv (see Figure 5C).

In another embodiment, multispecific epitope binding proteins of the invention comprise a first and/or a second polypeptide chain, wherein the first and/or second chain comprises two antibody variable regions linked to a CH1. In another embodiment, multispecific epitope binding proteins of the invention comprise a first and/or a second polypeptide chain, wherein the first and/or second chain comprises two antibody variable regions linked to a CH1 arranged N-terminus to C-terminus: VH1-CH1-VH2 (see Figure 2D). In another embodiment, multispecific epitope binding proteins of the invention comprise a first and a second polypeptide chain wherein, the first and/or second chain comprises an antibody variable region and 2 scFvs linked to a CH1 region. In a specific embodiment, multispecific epitope binding proteins of the invention comprise a first and a second polypeptide chain, wherein the first and/or second chain comprises an antibody variable region and 2 scFvs linked to a CH1 region arranged N-terminus to C-terminus: scFv-VH1-CH1-scFv (see Figure 5C).

### D. Multispecific epitope binding proteins - 4 chains

The invention provides multispecific epitope binding proteins comprising four polypeptide chains, namely, a first chain, a second chain, a third chain, and a fourth chain respectively (hereinafter may be referred to collectively as "polypeptide chains of the invention" See, e.g., Figures 3B, 3D, 3F, 4B and 5B). In the case of four chain multispecific epitope binding proteins of the invention, as exemplified herein two of the four chains comprise an Fc region as described in the foregoing sections; however, two of the four chains will not comprise an Fc region. Accordingly, two of the four chains may comprise polypeptides of the invention as described in Section A, and two chains may be polypeptides as disclosed below.

### D.1. Ckappa/lambda regions

The multispecific epitope binding proteins of the invention may further comprise a Ckappa/lambda region from the constant region of an antibody. The orientation of the Ckappa/lambda region may be varied within the protein. In one embodiment, the polypeptide chains of the invention comprise a Ckappa/lambda region in any orientation with other components (for example scFvs, Single chain diabodies, antibody variable regions, etc.).

In one embodiment, the polypeptide chains of the invention comprise a Ckappa/lambda region of an antibody. In one embodiment, the polypeptide chains of the invention comprise a Ckappa/lambda region fused to an epitope binding domain. In one embodiment, the Ckappa/lambda region is fused to an scFv, single chain diabody, antibody variable region, or another epitope binding protein known in the art. In another embodiment, the polypeptide chains of the invention comprise an epitope binding domain linked to the N-terminus of the Ckappa/lambda region. In another embodiment, multiple epitope binding domains are linked to the N-terminus of the Ckappa/lambda region. In yet another embodiment, the polypeptide chains of the invention comprise multiple scFvs, single chain diabodies, antibody variable regions, and/or other epitope binding domains known in the art linked to the N-terminus of the Ckappa/lambda region.

### a. Vectors encoding polypeptide chains comprising a Ckappa/lambda region

The invention also provides polynucleotide vectors for generating or expressing polypeptide chains of the invention comprising Ckappa/lambda domains (as described above). In one embodiment, polypeptide chains of the invention are expressed from a vector comprising a promoter, a polynucleotide sequence encoding an epitope binding polypeptide chain comprising a Ckappa/lambda and a poly A tail. In one embodiment, the expression vector comprises a promoter, a polynucleotide sequence encoding an epitope binding polypeptide chain comprising an epitope binding domain linked to a Ckappa/lambda region and a poly A tail. In another embodiment, the expression vector comprises a promoter, a polynucleotide sequence encoding an scFv, a single chain diabody, an antibody variable region or another epitope binding domain known in the art linked to a Ckappa/lambda region and a poly A tail. In another embodiment, the expression vector comprises a promoter, a polynucleotide sequence encoding an scFv, a single chain diabody, an antibody variable region or another epitope binding domain known in the art linked to the N-terminus of a Ckappa/lambda region and a poly A tail. In another embodiment, the expression vector comprises a promoter, a polynucleotide sequence encoding multiple scFvs, single chain diabodies, antibody variable regions or another epitope binding domains known in the art linked to the N-terminus of a Ckappa/lambda region and a poly A tail.

In a specific embodiment, the expression vectors of the invention comprise a promoter, a polynucleotide sequence encoding an antibody variable region linked to the N-terminus of a Ckappa/lambda region and a poly A tail (see for example Figure 3A (inset b and d). In another specific embodiment, the expression vectors of the invention comprise a promoter, a polynucleotide sequence encoding two antibody variable regions linked to the N-terminus of a Ckappa/lambda domain and a poly A tail (see for example Figure 4A (inset b and d). In another specific embodiment, the expression vectors of the invention comprise a promoter, a polynucleotide sequence encoding 2 scFvs linked to the N-terminus of a Ckappa/lambda region, and a poly A tail (see for example Figure 5A(inset a).

In another specific embodiment, expression vectors of the invention comprise a promoter, a polynucleotide sequence encoding an scFv and an antibody variable region linked to the N-terminus of a Ckappa/lambda region and a poly A tail (see for example Figure 4C inset b). In another specific embodiment, expression vectors of the invention comprise a promoter, a polynucleotide sequence encoding an antibody variable region linked to the N-terminus of a CH1 region and a poly A tail (see for example Figure 4C inset a).

### D.2. Multispecific epitope binding proteins - 4 chains

Described in the following sections are the assembly and orientation of multispecific epitope binding proteins of the invention comprising four polypeptide chains.

In one embodiment, the multispecific epitope binding proteins of the invention comprise a first, and/or a second, and/or a third, and/or a fourth polypeptide chain, wherein the first, and/or the second, and/or the third, and/or the fourth polypeptide chain comprises CH1 and Fc regions (also referred to herein jointly as "CH1/Fc region"), or Ckappa/lambda region linked to 1, 2, 3, 4, 5, 6, 7, 8, or more epitope binding domains. The proteins of the invention comprise epitope binding domains which may be scFvs, single chain diabodies, variable regions of antibodies, or other known epitope binding domains known in the art.

In one embodiment, the CH1/Fc region, or Ckappa/lambda region and the epitope binding domains may be linked together in many different orientations. In one embodiment, the epitope binding domains are linked to the C-terminus of the CH1/Fc region, or Ckappa/lambda region. In other embodiments the epitope binding domains are linked to the N-terminus of the CH1/Fc region, or Ckappa/lambda region. In other embodiments, the epitope binding domains are linked to the N-terminus or the C-terminus of the CH1/Fc region, or Ckappa/lambda region. In other embodiments, the epitope binding domains are linked to both the N-terminus and C-terminus of the CH1/Fc region, or Ckappa/lambda region.

In other embodiments, the Fc region of multispecific epitope binding proteins comprising 4 chains is not associated with a CH1 domain. In alternative embodiments, the Fc region of multispecific epitope binding proteins comprising 4 chains is associated with a Ckappa/lambda region.

### D.3. Inverted antibodies

In other embodiments, the invention provides inverted antibodies (herein after referred to as ("inverted antibodies" or "inverted antibody proteins of the invention") which comprise "antibody-like" chains. Inverted antibodies of the invention comprise at least four chains with at least two chains being antibody "heavy-like" chains and at least two antibody "light-like" chains. It is to be understood that polypeptide chains that form an inverted antibody of the invention are sometimes referred to herein as "inverted antibody polypeptide chains of the invention" and are included as a type of polypeptide chain of the invention.

In some embodiments, heavy-like polypeptide chains of the invention may comprise at least one antibody light chain variable region and at least one Ckappa/lambda region. In other embodiments, heavy-like chains of the invention comprise at least one or more antibody light chain variable regions (VL) in tandem with a Ckappa/lambda domain. In some embodiments, heavy-like polypeptide chains of the invention may comprise at least one or more Fc regions. In some embodiments, heavy-like polypeptide chains of the invention comprise all or part of a hinge region. In other embodiments, heavy-like polypeptide chains of the invention may further comprise other epitope binding domains as described herein,

In some embodiments, light-like polypeptide chains of the invention may comprise at least one antibody heavy chain variable region and at least one CH1 domain. In some embodiments, light-like chains of the invention further comprise at least one addition antibody heavy chain variable region in tandem with a CH1 domain, or at least one addition antibody light chain variable domain in tandem with a Ckappa/lambda region. In some embodiments, light-like polypeptide chains of invention may comprise all or part of a hinge region. In other embodiments, light-like polypeptide chains of invention may further comprise other epitope binding domains as described herein.

Inverted antibodies are formed with the antibody heavy-like chains and light-like chains associating to bring the antibody variable domains of one heavy-like chain in proximity of the antibody variable domains of a light-like chain to form a functional epitope binding site. For example, the VL domains from the antibody heavy-like chains will form a functional binding site with the VH domains from the antibody light-like chains (see, for example, Figure 4U.). In some embodiments, the light-like chains are disulfide linked to the heavy-like chains of an inverted antibody. In other embodiments, the antibody variable regions from one chain form an interchain disulfide bond with another chain. In yet other embodiments, the antibody variable regions of the light-like chains form interchain disulfide bonds with the heavy-like chains. In other embodiments, the antibody variable regions of the heavy-like chains form interchain disulfide bonds with the light-like chains.

### D.4. Multispecific epitope binding proteins with epitope binding domains linked to the C-terminus of the CH1/Fc region or the Ckappa/lambda region

In one embodiment, the multispecific epitope binding protein comprises a first, a second, a third and a fourth polypeptide chain, wherein at least the first, the second, the third, or the fourth chain further comprises an scFv linked to the C-terminus of the CH1/Fc region, or Ckappa/lambda region (see for example Figure 4B). In another embodiment, the epitope binding protein of the invention comprises multiple scFvs linked to the C-terminus of the CH1/Fc region, , or Ckappa/lambda region (see for example Figure 4B). In another embodiment, the epitope binding protein of the invention comprises at least a first, a second, a third, or a fourth chain with 1, 2, 3, 4, 5, 6, 7, 8 or more scFvs linked to the C-terminus of the CH1/Fc region, , or Ckappa/lambda region.

In one embodiment, the multispecific epitope binding protein comprises a first, a second, a third and a fourth polypeptide chain, wherein at least the first, the second, the third, or the fourth chain further comprises a single chain diabody linked to the C-terminus of the CH1/Fc region, or Ckappa/lambda region. In another embodiment, the epitope binding protein of the invention comprises multiple single chain diabodies linked to the C-terminus of the CH1/Fc region, or Ckappa/lambda region. In another embodiment, the epitope binding protein of the invention comprises at least a first, a second, a third, or a fourth chain with 1, 2, 3, 4, 5, 6, 7, 8 or more single chain diabodies linked to the C-terminus of the CH1/Fc region, or Ckappa/lambda region.

In one embodiment, the multispecific epitope binding protein comprises a first, a second, a third and a fourth polypeptide chain, wherein at least the first, the second, the third, or the fourth chain further comprises an antibody variable domain linked to the C-terminus of the CH1/Fc region, or Ckappa/lambda region. In another embodiment, the epitope binding protein of the invention comprises at least a first, a second, a third, or a fourth chain with multiple antibody variable regions linked to the C-terminus of the CH1/Fc region, or Ckappa/lambda region. In another embodiment, the epitope binding protein of the invention comprises at least a first, a second, a third, or a fourth chain with 1, 2, 3, 4, 5, 6, 7, 8 or more antibody variable regions linked to the C-terminus of the CH1/Fc region, or Ckappa/lambda region.

In one embodiment, the multispecific epitope binding protein comprises a first, a second, a third and a fourth polypeptide chain, wherein at least the first, the second, the third, or the fourth chain further comprises an epitope binding protein known in the art linked to the C-terminus of the CH1/Fc region, or Ckappa/lambda region. In another embodiment, the epitope binding protein of the invention comprises at least a first, a second, a third, or a fourth chain with multiple epitope binding domains known in the art linked to the C-terminus of the CH1/Fc region, or Ckappa/lambda region. In another embodiment, the epitope binding protein of the invention comprises at least a first, a second, a third, or a fourth chain with 1, 2, 3, 4, 5, 6, 7, 8 or more epitope binding domains known in the art, wherein the epitope binding domain is selected from the group consisting of scFvs, single chain diabodies, antibody variable regions of other epitope binding domains known in the art, linked to the C-terminus of the CH1/Fc region, or Ckappa/lambda region.

### D.5. Multispecific epitope binding proteins with epitope binding domains linked to the N-terminus of the CH1/Fc region, or the Ckappa/lambda region

In one embodiment, the multispecific epitope binding protein comprises a first, a second, a third and a fourth polypeptide chain, wherein at least the first, the second, the third, or the fourth chain further comprises an scFv linked to the N-terminus of the CH1/Fc region, or Ckappa/lambda region (see for example Figure 5B). In another embodiment, the epitope binding protein of the invention comprises multiple scFvs linked to the N-terminus of the CH1/Fc region, or Ckappa/lambda region (see for example Figure 5B). In another embodiment, the epitope binding protein of the invention comprises at least a first, a second, a third, or a fourth chain with 1, 2, 3, 4, 5, 6, .7, 8 or more scFvs linked to the N-terminus of the CH1/Fc region, or Ckappa/lambda region.

In one embodiment, the multispecific epitope binding protein comprises a first, a second, a third and a fourth polypeptide chain, wherein at least the first, the second, the third, or the fourth chain further comprises a single chain diabody linked to the N-terminus of the CH1/Fc region, or Ckappa/lambda region. In another embodiment, multiple single chain diabodies are linked to the N-terminus of the CH1/Fc region, or Ckappa/lambda region. In another embodiment, the epitope binding protein of the invention comprises at least a first, a second, a third, or a fourth chain with 1, 2, 3, 4, 5, 6, 7, 8 or more single chain diabody domains linked to the N-terminus of the CH1/Fc region, or Ckappa/lambda region.

In one embodiment, the multispecific epitope binding protein comprises a first, a second, a third and a fourth polypeptide chain, wherein at least the first, the second, the third, or the fourth chain further comprises an antibody variable domain linked to the N-terminus of the CH1/Fc region, or Ckappa/lambda region. In another embodiment, the epitope binding protein of the invention comprises at least a first, a second, a third, or a fourth chain with multiple antibody variable regions linked to the N-terminus of the CH1/Fc region, or Ckappa/lambda region. In another embodiment, the epitope binding protein of the invention comprises at least a first, a second, a third, or a fourth chain with 1, 2, 3, 4, 5, 6, 7, 8 or more antibody variable regions linked to the N-terminus of the CH1/Fc region, or Ckappa/lambda region.

In one embodiment, the multispecific epitope binding protein comprises a first, a second, a third and a fourth polypeptide chain, wherein at least the first, the second, the third, or the fourth chain further comprises an epitope binding protein known in the art linked to the N-terminus of the CH1/Fc region, or Ckappa/lambda region. In another embodiment, the epitope binding protein of the invention comprises at least a first, a second, a third, or a fourth chain with multiple epitope binding domains known in the art linked to the N-terminus of the CH1/Fc region, or Ckappa/lambda region. In another embodiment, the epitope binding protein of the invention comprises at least a first, a second, a third, or a fourth chain with 1, 2, 3, 4, 5, 6, 7, 8 or more epitope binding domains known in the art, wherein the epitope binding domains are selected from the group consisting of scFvs, single chain diabodies, antibody variable regions or other epitope binding domains known in the art, linked to the N-terminus of the CH1/Fc region, or Ckappa/lambda region.

### D.6. Multispecific epitope binding proteins with epitope binding domains linked to the N-terminus or C-terminus of the Fc region, CH1 region or the Ckappa/lambda region

In one embodiment, the multispecific epitope binding protein comprises a first, a second, a third and a fourth polypeptide chain, one chain further comprising an scFv linked to the N-terminus or C-terminus of the CH1/Fc region, or Ckappa/lambda region (see for example Figure 4B). In another embodiment, the epitope binding protein of the invention comprises multiple scFvs linked to the N-terminus or C-terminus of the CH1/Fc region, , or Ckappa/lambda region (see for example Figure 5B). In another embodiment, the epitope binding protein of the invention comprises at least a first, a second, a third, or a fourth chain with 1, 2, 3, 4, 5, 6, 7, 8 or more scFvs linked to the N-terminus or C-terminus of the CH1/Fc region, or Ckappa/lambda region.

In one embodiment, the multispecific epitope binding protein comprises a first, a second, a third and a fourth polypeptide chain, wherein at least the first, the second, the third, or the fourth chain further comprises a single chain diabody linked to the N-temninus or C-terminus of the CH1/Fc region or Ckappa/lambda region. In another embodiment, multiple single chain diabodies are linked to the N-terminus or C-terminus of the CH1/Fc region, or Ckappa/lambda region. In another embodiment, the epitope binding protein of the invention comprises multiple single chain diabodies linked to the N-terminus or C-terminus of the CH1/Fc region, or Ckappa/lambda region. In another embodiment, the epitope binding protein of the invention comprises at least a first, a second, a third, or a fourth chain with 1, 2, 3, 4, 5, 6, 7, 8 or more single chain diabodies linked to the N-terminus or C-terminus of the CH1/Fc region, or Ckappa/lambda region.

In one embodiment, the multispecific epitope binding protein comprises a first, a second, a third and a fourth polypeptide chain, wherein at least the first, the second, the third, or the fourth chain further comprises an antibody variable domain linked to the N-tenninus or C-terminus of the CH1/Fc region, or Ckappa/lambda region (see for example Figure 4B). In another embodiment, the epitope binding protein of the invention comprises at least a first, a second, a third, or a fourth chain with multiple antibody variable regions linked to the N-terminus or C-terminus of the CH1/Fc region, or Ckappa/lambda region (see for example Figure 4B). In another embodiment, the epitope binding protein of the invention comprises at least a first, a second, a third, or a fourth chain with 1, 2, 3, 4, 5, 6, 7, 8 or more antibody variable regions linked to the N-terminus or C-terminus of the CH1/Fc region, or Ckappa/lambda region.

In one embodiment, the multispecific epitope binding protein comprises a first, a second, a third and a fourth polypeptide chain, wherein at least the first, the second, the third, or the fourth chain further comprises an epitope binding protein known in the art linked to the N-terminus or C-terminus of the CH1/Fc region, or Ckappa/lambda region. In another embodiment, the epitope binding protein of the invention comprises at least a first, a second, a third, or a fourth chain with multiple epitope binding domains known in the art linked to the N-terminus or C-terminus of the CH1/Fc region, or Ckappa/lambda region. In another embodiment, the epitope binding protein of the invention comprises at least a first, a second, a third, or a fourth chain with 1, 2, 3, 4, 5, 6, 7, 8 or more epitope binding domains, wherein the domains are selected from the group consisting of an antibody variable region, an scFv, a single chain diabody, or another epitope binding domain known in the art, linked to the N-terminus or C-terminus of the CH1/Fc region, or Ckappa/lambda region.

### D.7. Multispecific epitope binding proteins with epitope binding domains linked to the N-terminus and C-terminus of the CH1/Fc region or the Ckappa/lambda region

In one embodiment, the multispecific epitope binding protein comprises a first, a second, a third and a fourth polypeptide chain, wherein at least the first, the second, the third, or the fourth chain further comprises an scFv linked to the N-terminus and C-terminus of the CH1/Fc region, or Ckappa/lambda region. In another embodiment, the epitope binding protein of the invention comprises multiple scFvs linked to the N-terminus and C-terminus of the CH1/Fc region, or Ckappa/lambda region. In another embodiment, the epitope binding protein of the invention comprises at least a first, a second, a third, or a fourth chain with 1, 2, 3, 4, 5, 6, 7, 8 or more scFvs linked to the N-terminus and C-terminus of the CH1/Fc region, or Ckappa/lambda region.

In one embodiment, the multispecific epitope binding protein comprises a first, a second, a third and a fourth polypeptide chain, wherein at least the first, the second, the third, or the fourth chain further comprises a single chain diabody linked to the N-terminus and C-terminus of the CH1/Fc region, or Ckappa/lambda region. In another embodiment, the epitope binding protein of the invention comprises multiple single chain diabodies linked to the N-terminus and C-terminus of the CH1/Fc region, or Ckappa/lambda region. In another embodiment, the epitope binding protein of the invention comprises at least a first, a second, a third, or a fourth chain with 1, 2, 3, 4, 5, 6, 7, 8 or more single chain diabodies linked to the N-terminus and C-terminus of the CH1/Fc region, or Ckappa/lambda region.

In one embodiment, the multispecific epitope binding protein comprises a first, a second, a third and a fourth polypeptide chain, wherein at least the first, the second, the third, or the fourth chain further comprises an antibody variable domain linked to the N-terminus and C-terminus of the CH1/Fc region, or Ckappa/lambda region. In another embodiment, the epitope binding protein of the invention comprises at least a first, a second, a third, or a fourth chain with multiple antibody variable regions linked to the N-terminus and C-terminus of the CH1/Fc region or Ckappa/lambda region. In another embodiment, the epitope binding protein of the invention comprises at least a first, a second, a third, or a fourth chain with 1, 2, 3, 4, 5, 6, 7, 8 or more antibody variable regions linked to the N-terminus and C-terminus of the CH1/Fc region, or Ckappa/lambda region.

In one embodiment, the multispecific epitope binding protein comprises a first, a second, a third and a fourth polypeptide chain, wherein at least the first, the second, the third, or the fourth chain further comprises an epitope binding protein known in the art linked to the N-terminus and C-terminus of the CH1/Fc region, or Ckappa/lambda region. In another embodiment, the epitope binding protein of the invention comprises at least a first, a second, a third, or a fourth chain with multiple epitope binding domains known in the art linked to the N-terminus and C-terminus of the CH1/Fc region, or Ckappa/lambda region. In another embodiment, the epitope binding protein of the invention comprises at least a first, a second, a third, or a fourth chain with at least 1, 2, 3, 4, 5, 6, 7, 8 or more epitope binding domains known in the art, wherein the domains are selected from the group consisting of scFvs, single chain diabodies, antibody variable regions, or an epitope binding domain known in the art, linked to the N-terminus and C-terminus of the CH1/Fc region, or Ckappa/lambda region.

### D.8. Four chain multispecific epitope binding proteins assembly

In another embodiment, the multispecific epitope binding protein comprises four chains, each chain comprising an CH1/Fc region, or Ckappa/lambda region. In a further embodiment, each polypeptide chain comprises any epitope binding domain, including scFvs, single chain diabodies, antibody variable regions, or any epitope binding domains known in the art. In another embodiment, the multispecific epitope binding protein comprises four chains containing dimers formed by the CH1/Fc region, or Ckappa/lambda region. In another embodiment, the multispecific epitope binding protein comprises two or more non-identical polypeptide chains. In an embodiment, multispecific epitope binding proteins of the invention comprise multimers of two or more polypeptide chains. In another embodiment, multispecific epitope binding proteins of the invention comprise two identical chains dimerized by the CH1/Fc region, or Ckappa/lambda region. In another embodiment, multispecific epitope binding proteins of the invention comprise four chains, two chains dimerized by an Fc region and two chains dimerized by a CH1 and a Ckappa/lambda region.

### D.9. Specific Embodiments of Four Chain Multispecific Epitope Binding Proteins

In another embodiment, multispecific epitope binding proteins of the invention comprise at least a first, a second, a third, or a fourth polypeptide chain, said first chain comprising an antibody variable region and 2 scFvs linked to a CH1/Fc region, said second chain comprising an antibody variable region linked to a Ckappa/lambda region. In a specific embodiment, multispecific epitope binding proteins of the invention comprise a first, a second, a third, and a fourth polypeptide chain, said first chain comprising an antibody variable region and 2 scFvs linked to a CH1/Fc region arranged N-terminus to C-terminus: antibody variable region-CH1/Fc region-scFv-scFv, said second chain comprising an antibody variable region linked to a Ckappa/lambda region (see Figure 3B).

In another embodiment, multispecific epitope binding proteins of the invention comprise at least a first, a second, a third, or a fourth polypeptide chain, said first chain comprising an antibody variable region and a single chain diabody linked to a CH1/Fc region, said second chain comprising an antibody variable region linked to a Ckappa/lambda region. In a specific embodiment, multispecific epitope binding proteins of the invention comprise a first, a second, a third, and a fourth polypeptide chain, said first chain comprising an antibody variable region and a single chain diabody linked to a CH1/Fc region arranged N-terminus to C-terminus: antibody variable region-CH1/Fc region-single chain diabody, said second chain comprising an antibody variable region linked to a Ckappa/lambda region (see Figure 3F).

In another embodiment, multispecific epitope binding proteins of the invention comprise at least a first, a second, a third, or a fourth polypeptide chain, said first chain comprising 2 antibody variable regions and 2 scFvs linked to a CH1/Fc region, said second chain comprising 2 antibody variable regions linked to a Ckappa/lambda region. In a specific embodiment, multispecific epitope binding proteins of the invention comprise a first, a second, a third, and a fourth polypeptide chain, said first chain comprising 2 antibody variable regions and 2 scFvs linked to a CH1/Fc region arranged N-terminus to C-terminus: antibody variable region-antibody variable region-CH1/Fc region-scFv-scFv, said second chain comprising 2 antibody variable regions and a Ckappa/lambda region arranged N-terminus to C-terminus: antibody variable region-antibody-variable region-Ckappa/lambda (see Figure 4B).

In another embodiment, multispecific epitope binding proteins of the invention comprise at least a first, a second, a third, or a fourth chain, said first chain comprising 2 scFvs linked to a CH1 region, said second chain comprising 2 scFvs linked to a Ckappa/lambda region. In a specific embodiment, multispecific epitope binding proteins of the invention comprise a first, a second, a third, and a fourth polypeptide chain, said first chain comprising 2 scFvs linked to a CH1 region arranged N-terminus to C-terminus: scFv-scFv-CH1, said second chain comprising 2 scFvs linked to a Ckappa/lambda region arranged N-terminus to C-terminus: scFv-scFv-Ckappa/lambda (see Figure 5B).

In a specific embodiment multispecific epitope binding proteins of the invention comprise a first, a second, a third, and a fourth polypeptide chain, said first chain comprising 2 antibody variable regions linked to a CH1/Fc region, said second chain comprising 2 antibody variable regions linked to a Ckappa/lambda region.

In another embodiment, multispecific epitope binding proteins of the invention comprise at least a first, a second, a third, or a fourth chain, said first chain comprising an scFv and antibody variable region linked to a Ckappa/lambda region in the following N-terminus to C-terminus orientation: scFv-antibody variable region-Ckappa/lambda, said second chain comprising an antibody variable region linked to a CH1/Fc region in the following N-terminus to C-terminus orientation: antibody variable region-CH1/Fc region (see Figure 4D).

In another embodiment, multispecific epitope binding proteins of the invention comprise at least a first, a second, a third, or a fourth chain, said first chain comprising an scFv and antibody variable region linked to a CH1 region in the following N-terminus to C-terminus orientation: scFv-antibody variable region-CH1 region , said second chain comprising an antibody variable region linked to a Ckappa/lambda region in the following N-terminus to C-terminus orientation: antibody variable region-Ckappa/lambda region (see Figure 4F).

In another embodiment, multispecific epitope binding proteins of the invention comprise at least a first, a second, a third, or a fourth chain, said first chain comprising an scFv and antibody variable region linked to a Ckappa/lambda region in the following N-terminus to C-terminus orientation: scFv-antibody variable region-Ckappa/lambda, said second chain comprising 3 scFvs and antibody variable region linked to a CH1/Fc region in the following N-terminus to C-terminus orientation: scFv-antibody variable region-CH1/Fc region-scFv-scFv (see Figure 4J).

In another embodiment, multispecific epitope binding proteins of the invention comprise at least a first, a second, a third, or a fourth chain, said first chain comprising an scFv and antibody variable region linked to a Ckappa/lambda region in the following N-terminus to C-terminus orientation: scFv-antibody variable region-Ckappa/lambda, said second chain comprising 2 scFvs and antibody variable region linked to a **CH1/Fc** region in the following N-terminus to C-terminus orientation: scFv-antibody variable region-CH1/Fc region-scFv (see Figure 4K).

In another embodiment, multispecific epitope binding proteins of the invention comprise at least a first, a second, a third, or a fourth chain, said first chain comprising 2 antibody light chain variable regions and 2 Ckappa/lambda regions in the following N-terminus to C-terminus orientation: antibody variable region (VL2)- first Ckappa/lambda-antibody variable region (VL1)- second Ckappa/lambda, said second chain comprising a 2 antibody heavy chain variable regions, 2CH1, and an Fc region in the following N-terminus to C-terminus orientation:antibody variable region (VH2)-first CH1-antibody variable region (VH1)-second CH1-Fc region (see Figure 4N).

In another embodiment, multispecific epitope binding proteins of the invention comprise at least a first, a second, a third, or a fourth chain, said first chain comprising an antibody heavy chain variable region, a CH1, an antibody light chain variable region and a Ckappa/lambda in the following N-terminus to C-terminus orientation: antibody heavy chain variable region(VH2)-CH1-antibody light chain variable region (VL1)-Ckappa/lambda, said second chain comprising an antibody light chain variable region, a Ckappa/lambda, an antibody heavy chain variable region, a CH1, and an Fc region in the following N-terminus to C-terminus orientation: antibody light chain variable region (VL2)-Ckappa/lambda-antibody heavy chain variable region (VH1)-CH1-Fc region (see Figure 4P).

In another embodiment, multispecific epitope binding proteins of the invention comprise at least a first, a second, a third, or a fourth chain, said first chain comprising two antibody variable heavy regions and two CH1 domains in the following N-terminus to C-terminus orientation: antibody heavy chain variable region (VH2)-CH1-antibody variable region (VH1)-CH1, said second chain comprising two antibody light chain variable regions, 2 Ckappa/lambda regions, and an Fc region arranged N-terminus to C-terminus in the following orientation: antibody light chain variable region (VL2)-Ckappa/lambda-antibody light chain variable region (VL1)-Ckappa/lambda-Fc region (see Figure 4R).

In another embodiment, multispecific epitope binding proteins of the invention comprise at least a first, a second, a third, or a fourth chain, said first chain comprising an antibody heavy chain variable region, a CH1, an antibody light chain variable region, and a Ckappa/lambda arranged N-terminus to C-terminus in the following orientation, antibody heavy chain variable region (VH2)-CH1-antibody light chain variable region (VL1)-Ckappa/lambda, and a second chain comprising an antibody light chain variable region, a Ckappa/lambda, an antibody variable heavy chain, a CH1, and an Fc region arranged N-terminus to C-terminus in the following orientation: antibody light chain variable region (VL2)-Ckappa/lambda-antibody heavy chain variable region (VH1)-CH1-Fc region (see Figure 4T).

In other embodiments, the invention also encompasses inverted antibodies comprising at least a first, a second, a third or a fourth polypeptide chain, said first chain comprising an antibody heavy chain variable region and a CH1 domain arranged N-terminus to C-terminus in the following orientation: antibody heavy chain variable region-CH1, and a second chain comprising an antibody light chain variable region, a Ckappa/lambda, and an Fc region arranged N-terminus to C-terminus in the following orientation: antibody light chain variable region-Ckappa/lambda-Fc region (see Figure 4V).

In some embodiments, the multispecific epitope binding polypeptides of the invention comprise a structural format in any of the figures presented in the application. In some embodiments, the multispecific epitope binding proteins of the invention comprise a structural format in any of the figures presented in the application. In some embodiments, the vectors of the invention comprise a format in any of the figures presented in the application.

### D.10. Six chain multispecific epitope binding protein assembly

Described in the following sections are the assembly and orientation of multispecific epitope binding proteins of the invention comprising six polypeptide chains.

In one embodiment, the multispecific epitope binding proteins of the invention comprise a first, and/or a second, and/or a third, and/or a fourth, and/or a fifth, and/or a sixth polypeptide chain, wherein the first, and/or the second, and/or the third, and/or the fourth, and/or the fifth, and/or the sixth polypeptide chain comprises CH1 and Fc regions (also referred to herein jointly as "CH1/Fc region"), or Ckappa/lambda region linked to 1, 2, 3, 4, 5, 6, 7, 8, or more epitope binding domains. The proteins of the invention comprise epitope binding domains which may be scFvs, single chain diabodies, variable regions of antibodies, or other known epitope binding domains known in the art.

In one embodiment, the CH1/Fc region, or Ckappa/lambda region and the epitope binding domains may be linked together in many different orientations. In one embodiment, the epitope binding domains are linked to the C-terminus of the CH1/Fc region, or Ckappa/lambda region. In other embodiments the epitope binding domains are linked to the N-terminus of the CH1/Fc region, or Ckappa/lambda region. In other embodiments, the epitope binding domains are linked to the N-terminus or the C-terminus of the CH1/Fc region, or Ckappa/lambda region. In other embodiments, the epitope binding domains are linked to both the N-terminus and C-terminus of the CH1/Fc region, or Ckappa/lambda region.

In other embodiments, the Fc region of multispecific epitope binding proteins comprising 4 chains is not associated with a CH1 domain. In alternative embodiments, the Fc region of multispecific epitope binding proteins comprising six chains is associated with a Ckappa/lambda region.

### D.11. Specific embodiments for six-chain multispecific epitope binding proteins

In another embodiment, multispecific epitope binding proteins of the invention comprise at least a first, a second, a third, a fourth, a fifth, or a sixth chain, said first chain comprising an antibody light chain variable region and a Ckappa/lambda region in the following N-terminus to C-terminus orientation:antibody light chain variable region (VL2)-Ckappa/lambda, said second chain comprising an antibody light chain variable region and a Ckappa/lambda arranged N-terminus to C-terminus in the following orientation: antibody light chain variable region (VL1)-Ckappa/lambda, said third chain comprising a 2 antibody heavy chain variable regions, 2CH1, and an Fc region in the following N-terminus to C-terminus orientation:antibody variable region (VH2)-first CH1-antibody variable region (VH1)-second CH1-Fc region (see Figures 4M and N).

In another embodiment, multispecific epitope binding proteins of the invention comprise at least a first, a second, a third, a fourth, a fifth, or a sixth chain, said first chain comprising an antibody heavy chain variable region and a CH1, arranged N-terminus to C-terminus in the following orientation, antibody heavy chain variable region(VH2)-CH1, said second chain comprising an antibody light chain variable region and Ckappa/lambda in the following N-terminus to C-terminus orientation: antibody light chain variable region(VL1)-Ckappa/lambda, said third chain comprising an antibody light chain variable region, a Ckappa/lambda, an antibody heavy chain variable region, a CH1, and an Fc region in the following N-terminus to C-terminus orientation: antibody light chain variable region (VL2)-Ckappa/lambda-antibody heavy chain variable region (VH1)-CH1-Fc region (see Figures 40 and P).

In another embodiment, multispecific epitope binding proteins of the invention comprise at least a first, a second, a third, a fourth, a fifth, or a sixth chain, said first chain comprising an antibody variable heavy region and a CH1 in the following N-terminus to C-terminus orientation: antibody heavy chain variable region(VH2)-CH1, said second chain comprising an antibody heavy chain variable region and a CH1 arranged N-terminus to C-terminus in the following orientation: antibody heavy chain variable region(VH1)-CH1, said third chain comprising two antibody light chain variable regions, 2 Ckappa/lambda regions, and an Fc region arranged N-terminus to C-terminus in the following orientation: antibody light chain variable region (VL2)-Ckappa/lambda-antibody light chain variable region (VL1)-Ckappa/lambda-Fc region (see Figures 4Q and R).

In another embodiment, multispecific epitope binding proteins of the invention comprise at least a first, a second, a third, a fourth, a fifth, or a sixth chain, said first chain comprising an antibody heavy chain variable region and a CH1, arranged N-terminus to C-terminus in the following orientation: antibody heavy chain variable region (VH2)-CH1, said second chain comprising an antibody light chain variable region and a Ckappa/lambda arranged N-terminus to C-terminus in the following orientation: antibody light chain variable region (VL1)-Ckappa/lambda, and a said third chain comprising an antibody light chain variable region, a Ckappa/lambda, an antibody variable heavy chain, a CH1, and an Fc region arranged N-terminus to C-terminus in the following orientation: antibody light chain variable region (VL2)-Ckappa/lambda-antibody heavy chain variable region (VH1)-CH1-Fc region (see Figures 4S and T).

Monospecific multivalent antibodies comprising Fab domains linked to an IgG1 molecule are described in PCT publication WO 01/77342 filed March 20, 2001. In some embodiments, multispecific epitope binding proteins of the invention do not comprise Fab domains linked to an IgG1 molecule. In some embodiments, multispecific epitope binding proteins of the invention do not comprise a Fab linked N-terminally to an Fc region. In other embodiments, multispecific epitope binding proteins of the invention do comprise at least one or more Fab domains linked to an IgG1 molecule. In other embodiments, multispecific epitope binding proteins of the invention do not comprise identical Fabs linked to an IgG1 molecule. In other embodiments, multispecific epitope do not comprise Fabs linked to an IgG1 molecule wherein said Fabs comprise antibody variable regions identical to said IgG1 molecule. In some embodiments, multispecific epitope binding proteins of the invention comprise at least one Fab linked to an IgG1 molecule wherein said Fab and IgG1 do not share the same binding specificity. In some embodiments, multispecific epitope binding proteins of the invention comprise at least one Fab linked C-terminally to the Fc region of an IgG1 molecule. In some embodiments, proteins of the invention are not proteins as presented in Figure 4. of PCT publication WO 01/77342 filed March 20, 2001.

### E. Specific Embodiments for Multispecific Epitope Binding Protein Assemblies

In some embodiments, proteins of the invention comprise at least one epitope binding domain from, or an epitope binding domain that competes for binding with an epitope binding domain from abagovomab, abatacept (also know as ORENCIA®), abciximab (also known as REOPRO®, c7E3 Fab), adalimumab (also known as HUMIRA®), adecatumumab, alemtuzumab (also known as CAMPATH®, MabCampath or Campath-1H), altumomab, afelimomab, anatumomab mafenatox, anetumumab, anrukizumab, apolizumab, arcitumomab, aselizumab, atlizumab, atorolimumab, bapineuzumab, basiliximab (also known as SIMULECT®), bavituximab, bectumomab (also known as LYMPHOSCAN®), belimumab (also known as LYMPHO-STAT-B®), bertilimumab, besilesomab, bevacizumab (also known as AVASTIN®), biciromab brallobarbital, bivatuzumab mertansine, campath, canakinumab (also known as ACZ885), cantuzumab mertansine, capromab (also known as PROSTASCINT®), catumaxomab (also known as REMOVAB®), cedelizumab (also known as CIMZIA®), certolizumab pegol, cetuximab (also known as ERBITUX®), clenoliximab, dacetuzumab, dacliximab, daclizumab (also known as ZENAPAX®), denosumab (also known as AMG 162), detumomab, dorlimomab aritox, dorlixizumab, duntumumab, durimulumab, durmulumab, ecromeximab, eculizumab (also known as SOLIRIS®), edobacomab, edrecolomab (also known as Mab17-1A, PANOREX®), efalizumab (also known as RAPTIVA®), efungumab (also known as MYCOGRAB®), elsilimomab, enlimomab pegol, epitumomab cituxetan, efalizumab, epitumomab, epratuzumab, erlizumab, ertumaxomab (also known as REXOMUN®), etanercept (also known as ENBREL®), etaracizumab (also known as etaratuzumab, VITAXIN®, ABEGRIN™), exbivirumab, fanolesomab (also known as NEUTROSPEC®), faralimomab, felvizumab, fontolizumab (also known as HUZAF®), galiximab, gantenerumab, gavilimomab (also known as ABX-CBL®), gemtuzumab ozogamicin (also known as MYLOTARG®), golimumab (also known as CNTO 148), gomiliximab, ibalizumab (also known as TNX-355), ibritumomab tiuxetan (also known as ZEVALIN®), igovomab, imciromab, infliximab (also known as REMICADE®), inolimomab, inotuzumab ozogamicin, ipilimumab (also known as MDX-010, MDX-101), iratumumab, keliximab, labetuzumab, lemalesomab, lebrilizumab, lerdelimumab, lexatumumab (also known as, HGS-ETR2, ETR2-ST01), lexitumumab, libivirumab, lintuzumab, lucatumumab, lumiliximab, mapatumumab (also known as HGS-ETR1, TRM-1), maslimomab, matuzumab (also known as EMD72000), mepolizumab (also known as BOSATRIA®), metelimumab, milatuzumab, minretumomab, mitumomab, morolimumab, motavizumab (also known as NUMAX™), muromonab (also known as OKT3), nacolomab tafenatox, naptumomab estafenatox, natalizumab (also known as TYSABRI®, ANTEGREN®), nebacumab, nerelimomab, nimotuzumab (also known as THERACIM hR3®, THERA-CIM-hR3®, THERALOC®), nofetumomab merpentan (also known as VERLUMA®), ocrelizumab, odulimomab, ofatumumab, omalizumab (also known as XOLAIR®), oregovomab (also known as OVAREX®), otelixizumab, pagibaximab, palivizumab (also known as SYNAGIS®), panitumumab (also known as ABX-EGF, VECTIBIX®), pascolizumab, pemtumomab (also known as THERAGYN®), pertuzumab (also known as 2C4, OMNITARG®), pexelizumab, pintumomab, priliximab, pritumumab, ranibizumab (also known as LUCENTIS®), raxibacumab, regavirumab, reslizumab, rituximab (also known as RITUXAN®, MabTHERA®), rovelizumab, ruplizumab, satumomab, sevirumab, sibrotuzumab, siplizumab (also known as MEDI-507), sontuzumab, stamulumab (also known as MYO-029), sulesomab (also known as LEUKOSCAN®), tacatuzumab tetraxetan, tadocizumab, talizumab, taplitumomab paptox, tefibazumab (also known as AUREXIS®), telimomab aritox, teneliximab, teplizumab, ticilimumab, tocilizumab (also known as ACTEMRA®), toralizumab, tositumomab, trastuzumab (also known as HERCEPTIN®), tremelimumab (also known as CP-675,206), tucotuzumab celmoleukin, tuvirumab, urtoxazumab, ustekinumab (also known as CNTO 1275), vapaliximab, veltuzumab, vepalimomab, visilizumab (also known as NUVION®), volociximab (also known as M200), votumumab (also known as HUMASPECT®), zalutumumab, zanolimumab (also known as HuMAX-CD4), ziralimumab, or zolimomab aritox.

In another embodiment, multispecific epitope binding proteins of the invention comprise at least one epitope binding domain that comprises an epitope binding protein disclosed in US. Patent Application Publication No. 20050215767 filed February 11, 2005, and US. Patent Application Publication No. 20080014141 filed November 26, 2004, each are hereby incorporated by reference in their entireties.

In other embodiments, the multispecific epitope binding proteins of the invention comprise at least one epitope binding domain that binds to the same antigen as the antibodies listed above.

In other embodiments, the multispecific epitope binding proteins of the invention comprise at least one epitope binding domain that specifically binds to an antigen selected from: PDGFRalpha, PDGFRbeta, PDGF, VEGF, VEGF-A, VEGF-B, VEGF-C. VEGF-D, VEGF-E, VEGF-F, VEGFR-1, VEGFR-2, VEGFR-3, FGF, FGF2, HGF, KDR, flt-1, FLK-1 Ang-2, Ang-1, PLGF, CEA, CXCL13, Baff, IL-21, CCL21, TNF-alpha, CXCL12, SDF-1, bFGF, MAC-1, IL23p19, FPR, IGFBP4, CXCR3, TLR4, CXCR2, EphA2, EphA4, EphrinB2, EGFR(ErbB1), HER2(ErbB2 or p185^{neu}), HER3(ErbB3), HER4 ErbB4 or tyro2), SC1, LRP5, LRP6, RAGE, Nav1.7, GLP1, RSV, RSV F protein, Influenza HA protein, Influenza NA protein, HMGB1, CD16, CD19, CD20, CD21, CD28, CD32, CD32b, CD64, CD79, CD22, ICAM-1, FGFR1, FGFR2, HDGF, EphB4, GITR, β-amyloid, hMPV, PIV-1, PIV-2, OX40L, IGFBP3, cMet, PD-1, PLGF, Neprolysin, CTD, IL-18, IL-6, CXCL-13, IL-1R1, IL-15, IL-4R, IgE, PAI-1, NGF, EphA2, CEA, uPARt, DLL-4, αvβ6, α5β1, interferon receptor type I and type II. CD19, ICOS, IL-17, Factor II, Hsp90, IGF, CD19, GM-CSFR, PIV-3, CMV, IL-13, IL-9, and EBV..

In other embodiments, the multispecific epitope binding proteins of the invention comprise at least one epitope binding domain that specifically binds to a member (receptor or ligand) of the TNF superfamily. Various molecules include, but are not limited to Tumor Necrosis Factor-alpha ("TNF-alpha"), Tumor Necrosis Factor-beta ("TNF-beta"), Lymphotoxin-alpha ("LT-alpha"), CD30 ligand, CD27 ligand, CD40 ligand, 4-1 BB ligand, Apo-1 ligand (also referred to as Fas ligand or CD95 ligand), Apo-2 ligand (also referred to as TRAIL), Apo-3 ligand (also referred to as TWEAK), osteoprotegerin (OPG), APRIL, RANK ligand (also referred to as TRANCE), TALL-1 (also referred to as BlyS, BAFF or THANK), DR4, DR5 (also known as Apo-2, TRAIL-R2, TR6, Tango-63, hAPO8, TRICK2, or KILLER), DR6, DcR1,DcR2, DcR3 (also known as TR6 or M68), CAR1, HVEM (also known as ATAR or TR2), GITR, ZTNFR-5, NTR-1, TNFL1, CD30, LTBr, 4-1BB receptor and TR9.

In another embodiment the binding proteins of the invention are capable of binding one or more targets selected from the group consisting of 5T4, ABL, ABCF1, ACVR1, ACVR1B, ACVR2, ACVR2B, ACVRL1, ADORA2A, Aggrecan, AGR2, AICDA, AIFI, AIG1, AKAP1, AKAP2, AMH, AMHR2, ANGPT1, ANGPT2, ANGPTL3, ANGPTL4, ANPEP, APC, APOCI, AR, aromatase, ATX, AX1, AZGP1 (zinc-a-glycoprotein), B7.1, B7.2, B7-H1, BAD, BAFF, BAG1, BAI1, BCR, BCL2, BCL6, BDNF, BLNK, BLR1 (MDR15), BIyS, BMP1, BMP2, BMP3B (GDF1O), BMP4, BMP6, BMP8, BMPR1A, BMPR1B, BMPR2, BPAG1 (plectin), BRCA1, C19orf1O (IL27w), C3, C4A, C5, C5R1, CANT1, CASP1, CASP4, CAV1, CCBP2 (D6 / JAB61), CCL1 (1-309), CCLI1 (eotaxin), CCL13 (MCP-4), CCL15 (MIP-Id), CCL16 (HCC-4), CCL17 (TARC), CCL18 (PARC), CCL19 (MIP-3b), CCL2 (MCP -1), MCAF, CCL20 (MIP-3a), CCL21 (MEP-2), SLC, exodus-2, CCL22(MDC / STC-I), CCL23 (MPIF-I), CCL24 (MPIF-2 / eotaxin-2), CCL25 (TECK), CCL26(eotaxin-3), CCL27 (CTACK / ILC), CCL28, CCL3 (MIP-Ia), CCL4 (MIP-Ib), CCL5(RANTES), CCL7 (MCP-3), CCL8 (mcp-2), CCNA1, CCNA2, CCND1, CCNE1, CCNE2, CCR1 (CKR1 /HM145), CCR2 (mcp-1RB / RA),CCR3 (CKR3 / CMKBR3), CCR4, CCR5(CMKBR5 /ChemR13), CCR6 (CMKBR6 / CKR-L3 / STRL22 / DRY6), CCR7 (CKR7 / EBI1),CCR8 (CMKBR8 / TER1 / CKR-L1), CCR9 (GPR-9-6), CCRL1 (VSHK1), CCRL2 (L-CCR),CD164, CD19, CD1C, CD20, CD200, CD-22, CD24, CD28, CD3, CD33, CD35, CD37, CD38, CD3E, CD3G,CD3Z, CD4, CD40, CD40L, CD44, CD45RB, CD52, CD69, CD72, CD74, CD79A, CD79B, CD8, CD80, CD81, CD83, CD86, CD137, CDH1 (E-cadherin), CDH1O, CDH12, CDH13; CDH18,CDH19, CDH20, CDH5, CDH7, CDH8, CDH9, CDK2, CDK3, CDK4, CDK5, CDK6, CDK7,CDK9, CDKN1A (p21Wap1/Cip1), CDKN1B (p27Kip1), CDKN1C, CDKN2A (p16INK4a),CDKN2B, CDKN2C, CDKN3, CEBPB, CER1, CHGA, CHGB, Chitinase, CHST1O, CKLFSF2,CKLFSF3, CKLFSF4, CKLFSF5, CKLFSF6, CKLFSF7, CKLFSF8, CLDN3, CLDN7 (claudin- 7), CLN3, CLU (clusterin), CMKLR1, CMKOR1 (RDC1), CNR1, COL18A1, COL1A1,COL4A3, COL6A1, CR2, Cripto, CRP, CSF1 (M-CSF), CSF2 (GM-CSF), CSF3 (GCSF), CTLA4, CTL8, CTNNB1 (b-catenin), CTSB (cathepsin B), CX3CL1 (SCYD1), CX3CR1 (V28), CXCL1(GRO1), CXCL1O (IP-IO), CXCLI1 (I-TAC / IP-9), CXCL12 (SDF1), CXCL13, CXCL14,CXCL16, CXCL2 (GRO2), CXCL3 (GRO3), CXCL5 (ENA-78 / LIX), CXCL6 (GCP-2), CXCL9 (MIG), CXCR3 (GPR9/CKR-L2), CXCR4, CXCR6 (TYMSTR /STRL33 / Bonzo),CYB5, CYCl, CYSLTR1, DAB2IP, DES, DKFZp451J0118, DNCL1, DPP4, E2F1, ECGF1,EDG1, EFNA1, EFNA3, EFNB2, EGF, EGFR, ELAC2, ENG, ENO1, ENO2, ENO3, EPHA1, EPHA2, EPHA3, EPHA4, EPHA5, EPHA6, EPHA7, EPHA8, EPHA9, EPHA10, EPHB1, EPHB2, EPHB3, EPHB4, EPHB5, EPHB6, EPHRIN-A1, EPHRIN-A2, EPHRIN-A3, EPHRIN-A4, EPHRIN-A5, EPHRIN-A6, EPHRIN-B1, EPHRIN-B2, EPHRIN-B3, EPHB4,EPG, ERBB2 (Her-2), EREG, ERK8, Estrogen receptor, ESR1, ESR2, F3 (TF), FADD, farnesyltransferase, FasL, FASNf, FCER1A,FCER2, FCGR3A, FGF, FGF1 (aFGF), FGF1O, FGF11, FGF12, FGF12B, FGF13, FGF 14, FGF16, FGF 17, FGF18, FGF 19, FGF2 (bFGF), FGF20, FGF21 , FGF22, FGF23, FGF3 (int-2),FGF4 (HST), FGF5, FGF6 (HST-2), FGF7 (KGF), FGF8, FGF9, FGFR3, FIGF (VEGFD), FIL1(EPSILON), FBL1 (ZETA), FLJ12584, FLJ25530, FLRT1 (fibronectin), FLT1, FLT-3, FOS, FOSL1(FRA-I), FY (DARC), GABRP (GABAa), GAGEB1, GAGEC1, GALNAC4S-6ST, GATA3, GD2, GDF5, GFI1, GGT1, GM-CSF, GNAS1, GNRH1, GPR2 (CCR1O), GPR31, GPR44, GPR81 (FKSG80), GRCC1O (CIO), GRP, GSN (Gelsolin), GSTP1, HAVCR2, HDAC, HDAC4, HDAC5,HDAC7A, HDAC9, Hedgehog, HGF, HIFIA, HIP1, histamine and histamine receptors, HLA-A, HLA-DRA, HM74, HMOX1, HSP90, HUMCYT2A, ICEBERG, ICOSL, ID2, IFN-a, IFNA1, IFNA2, IFNA4,IFNA5, EFNA6, BFNA7, IFNB1, IFNgamma, IFNW1, IGBP1, IGFI, IGF1R, IGF2, IGFBP2,IGFBP3, IGFBP6, DL-I, IL1O, IL1ORA, IL1ORB, IL-1, IL1R1 (CD121a), IL1R2(CD121b), IL- IRA, IL-2, IL2RA (CD25), IL2RB(CD122), IL2RG(CD132), IL-4, IL-4R(CD123), IL-5, IL5RA(CD125), IL3RB(CD131), IL-6, IL6RA (CD126), IR6RB(CD130), IL-7, IL7RA(CD127), IL-8, CXCR1 (IL8RA), CXCR2 (IL8RB/CD128), IL-9, IL9R (CD129), IL-10, IL10RA(CD210), IL10RB( CDW210B), IL-11, IL11RA, IL-12, IL-12A, IL-12B, IL-12RB1, IL-12RB2, IL-13, IL13RA1, IL13RA2, IL14, IL15, IL15RA, 1L16, IL17, IL17A, IL17B, IL17C, IL17R, IL18, IL18BP, IL18R1, IL18RAP, IL19, ILIA, IL1B, IL1F10, IL1F5, IL1F6, IL1F7, IL1F8, DL1F9, IL1HY1, IL1R1, IL1R2, IL1RAP, IL1RAPL1, IL1RAPL2, IL1RL1, IL1RL2, IL1RN, IL2, IL20, IL20RA, IL21R, IL22, IL22R, IL22RA2, IL23,DL24, IL25, IL26, IL27, IL28A, IL28B, IL29, IL2RA, IL2RB, IL2RG, IL3, IL30, IL3RA, IL4,IL4R, IL6ST (glycoprotein 130), ILK, INHA, INHBA, INSL3, INSL4, IRAKI, IRAK2, ITGA1, ITGA2,ITGA3, ITGA6 (α6 integrin), ITGAV, ITGB3, ITGB4 (β4 integrin), JAGI, JAK1, JAK3, JTB, JUN,K6HF, KAI1, KDR, KITLG, KLF5 (GC Box BP), KLF6, KLK1O, KLK12, KLK13, KLK14, KLK15, KLK3, KLK4, KLK5, KLK6, KLK9, KRT1, KRT19 (Keratin 19), KRT2A, KRTHB6(hair-specific type II keratin), LAMA5, LEP (leptin), Lingo-p75, Lingo-Troy, LPS, LTA (TNF- b), LTB, LTB4R (GPR16), LTB4R2, LTBR, MACMARCKS, MAG or Omgp , MAP2K7 (c-Jun), MCP-1, MDK, MIB1, midkine, MIF, MISRII, MJP-2,MK, MKI67 (Ki-67), MMP2, MMP9, MS4A1, MSMB,MT3 (metallothionectin-UI), mTOR, MTSS1, MUC1 (mucin), MYC, MYD88, NCK2, neurocan,NFKB1, NFKB2, NGFB (NGF), NGFR, NgR-Lingo, NgR-Nogo66 (Nogo), NgR-p75, NgR-Troy, NMEI (NM23A), NOTCH, NOTCH1, NOX5, NPPB, NROB1, NR0B2, NR1D1, NR1D2, NR1H2, NR1H3, NR1H4, NR1I2, NR1I3, NR2C1, NR2C2, NR2E1, NR2E3, NR2F1, NR2F2, NR2F6, NR3C1, NR3C2, NR4A1, NR4A2, NR4A3, NR5A1, NR5A2, NR6A1, NRP1, NRP2, NT5E, NTN4,ODZ1, OPRD1, P2RX7, PAP, PARTI, PATE, PAWR, PCA3, PCDGF, PCNA, PDGFA, PDGFB, PDGFRA, PDGFRB, PECAM1, peg-asparaginase, PF4 (CXCL4), PGF, PGR, phosphacan, PIAS2, PI3 Kinase, PIK3CG, PLAU (uPA), PLG,PLXDC1, PKC, PKC-beta, PPBP (CXCL7), PPID, PR1, PRKCQ, PRKD1, PRL, PROC, PROK2, PSAP, PSCA, PTAFR, PTEN, PTGS2 (COX-2), PTN, RAC2 (P21Rac2), RANK, RANK ligand, RARB, RGS1, RGS13, RGS3,RNFI1O (ZNF144), Ron, ROBO2, RXR, S100A2, SCGB 1D2 (Iïpophilin B), SCGB2A1 (mammaglobin 2),SCGB2A2 (mammaglobin 1), SCYE1 (endothelial Monocyte-activating cytokine), SDF2,SERPENA1, SERPINA3, SERPINB5 (maspin), SERPINE1 (PAI-I), SERPINF1, SHIP-1, SHIP-2, SHB1, SHB2, SHBG, SfcAZ,SLC2A2, SLC33A1, SLC43A1, SLIT2, SPPI, SPRR1B (Spr1), ST6GAL1, STAB1, STAT6, STEAP, STEAP2, TB4R2, TBX21, TCP1O, TDGF1, TEK, TGFA, TGFB1, TGFB1I1, TGFB2,TGFB3, TGFBI, TGFBR1, TGFBR2, TGFBR3, TH1L, THBS1 (thrombospondin-1), THBS2,THBS4, THPO, TIE (Tie-1), TIMP3, tissue factor, TLR1O, TLR2, TLR3, TLR4, TLR5, TLR6,TLR7, TLR8, TLR9, TNF, TNF-a, TNFAIP2 (B94), TNFAIP3, TNFRSFI1A, TNFRSF1A, TNFRSF1B, TNFRSF21, TNFRSF5, TNFRSF6 (Fas), TNFRSF7, TNFRSF8, TNFRSF9, TNFSF1O (TRAIL), TNFSF1 1 (TRANCE), TNFSF12 (APO3L), TNFSF13 (April), TNFSF13B,TNFSF14 (HVEM-L), TNFSF15 (VEGI), TNFSF18, TNFSF4 (OX40 ligand), TNFSF5 (CD40 ligand), TNFSF6 (FasL), TNFSF7 (CD27 ligand), TNFSF8 (CD30 ligand), TNFSF9 (4-1BB ligand), TOLLIP, Toll-like receptors, TOP2A (topoisomerase Iia), TP53, TPM1, TPM2,TRADD, TRAF1, TRAF2, TRAF3, TRAF4, TRAF5, TRAF6, TRKA, TREM1, TREM2, TRPC6, TSLP, TWEAK, Tyrosinase, uPAR, VEGF, VEGFB, VEGFC, versican, VHL C5, VLA-4, Wnt-1, XCL1 (lymphotactin),XCL2 (SCM-Ib), XCR1 (GPR5 / CCXCR1), YY1, and ZFPM2.

### F. Binding characteristics of multispecific epitope binding proteins of the invention

### b. Binding specificities

The invention provides epitope binding proteins comprising multiple binding sites. The proteins of the invention comprise two to four polypeptide chains comprising multiple epitope binding sites. The multiple epitope binding sites comprise binding domains from scFvs, single chain diabodies, antibody variable regions, or other epitope binding domains known in the art.

In one embodiment, multispecific epitope binding proteins of the invention comprise scFvs with identical binding specificities (see for example Figure 1A inset f). In another embodiment, multispecific epitope binding proteins of the invention comprise scFvs with non-identical binding specificities (see for example Figure 1A inset a). In another embodiment, multispecific epitope binding proteins of the invention comprise scFvs which share the same binding specificity with other scFvs within the protein (see for example Figure 1A inset e). In another embodiment, the multispecific epitope binding proteins of the invention comprise scFvs wherein at least 1, 2, 3, 4, 5, 6, 7, 8, or more share the same binding specificity. In another embodiment, multispecific epitope binding proteins of the invention comprise scFvs wherein at least 1, 2, 3, 4, 5, 6, 7, 8 or more do not share the same binding specificity. In another embodiment, multispecific epitope binding proteins of the invention comprise scFvs which are specific for another epitope on the same antigen as other scFvs within the protein. In another embodiment, multispecific epitope binding proteins of the invention comprise at least 1, 2, 3, 4, 5, 6, 7, 8 or more scFvs which are specific for another epitope on the same antigen as another scFv within the protein.

In one embodiment, multispecific epitope binding proteins of the invention comprise single chain diabodies with the identical binding specificities. In another embodiment, multispecific epitope binding proteins of the invention comprise single chain diabodies with non-identical binding specificities. In another embodiment, multispecific epitope binding proteins of the invention comprise single chain diabodies which may share the same binding specificity with other single chain diabodies within the protein. In another embodiment, the multispecific epitope binding proteins of the invention comprise single chain diabodies wherein at least 1, 2, 3, 4, 5, 6, 7, 8, or more share the same binding specificity. In another embodiment, multispecific epitope binding proteins of the invention comprise single chain diabodies wherein at least 1, 2, 3, 4, 5, 6, 7, 8 or more do not share the same binding specificity. In another embodiment, multispecific epitope binding proteins of the invention comprise single chain diabodies which are specific for another epitope on the same antigen as other single chain diabodies within the protein. In another embodiment, multispecific epitope binding proteins of the invention comprise at least 1, 2, 3, 4, 5, 6, 7, 8 or more single chain diabodies which are specific for another epitope on the same antigen as another single chain diabody within the protein.

In one embodiment, multispecific epitope binding proteins of the invention comprise antibody variable regions with the identical binding specificities. In another embodiment, multispecific epitope binding proteins of the invention comprise antibody variable regions with non-identical binding specificities. In another embodiment, multispecific epitope binding proteins of the invention comprise antibody variable regions which may share the same binding specificity with other antibody variable regions within the protein. In another embodiment, the multispecific epitope binding proteins of the invention comprise antibody variable regions wherein at least 1, 2, 3, 4, 5, 6, 7, 8, or more share the same binding specificity. In another embodiment, multispecific epitope binding proteins of the invention comprise antibody variable regions wherein at least 1, 2, 3, 4, 5, 6, 7, 8 or more do not share the same binding specificity. In another embodiment, multispecific epitope binding proteins of the invention comprise antibody variable regions which are specific for another epitope on the same antigen as other variable regions within the protein. In another embodiment, multispecific epitope binding proteins of the invention comprise at least 1, 2, 3, 4, 5, 6, 7, 8 or more antibody variable regions which are specific for another epitope on the same antigen as another antibody variable region within the protein.

In one embodiment, multispecific epitope binding proteins of the invention comprise epitope binding regions known in the art with the identical binding specificities. In another embodiment, multispecific epitope binding proteins of the invention comprise epitope binding regions known in the art with non-identical binding specificities. In another embodiment, multispecific epitope binding proteins of the invention comprise epitope binding regions known in the art which may share the same binding specificity with other epitope binding regions known in the art within the protein. In another embodiment, the multispecific epitope binding proteins of the invention comprise epitope binding regions known in the art wherein at least 1, 2, 3, 4, 5, 6, 7, 8, or more share the same binding specificity. In another embodiment, multispecific epitope binding proteins of the invention comprise epitope binding regions known in the art wherein at least 1, 2, 3, 4, 5, 6, 7, 8 or more do not share the same binding specificity. In another embodiment, multispecific epitope binding proteins of the invention comprise epitope binding regions known in the art which are specific for another epitope on the same antigen as other epitope binding regions known in the art within the protein. In another embodiment, multispecific epitope binding proteins of the invention comprise at least 1, 2, 3, 4, 5, 6, 7, 8 or more epitope binding regions known in the art which are specific for another epitope on the same antigen as another epitope binding region known in the art within the protein.

In one embodiment, multispecific epitope binding proteins of the invention comprise more then one type of epitope binding domain selected from the group consisting of scFvs, single chain diabodies, antibody variable regions or epitope binding domains known in the art. In one embodiment, multispecific epitope binding proteins of the invention comprise different epitope binding domains with the identical binding specificities. In another embodiment, multispecific epitope binding proteins of the invention comprise different epitope binding domains with non-identical binding specificities. In another embodiment, multispecific epitope binding proteins of the invention comprise scFvs, single chain diabodies, antibody variable regions or epitope binding domains known in the art which may share the same binding specificity with other scFvs, single chain diabodies, antibody variable regions or epitope binding domains known in the art within the protein. In another embodiment, multispecific epitope binding proteins of the invention comprise scFvs, single chain diabodies, antibody variable regions or epitope binding domains known in the art wherein at least 1, 2, 3, 4, 5, 6, 7, 8, or more share the same binding specificity. In another embodiment, the multispecific epitope binding proteins of the invention comprises scFvs, single chain diabodies, antibody variable regions or epitope binding domains known in the art wherein at least 1, 2, 3, 4, 5, 6, 7, 8 or more do not share the same binding specificity. In another embodiment, multispecific epitope binding proteins of the invention comprise scFvs, single chain diabodies, antibody variable regions or epitope binding domains known in the art which are specific for another epitope on the same antigen as other scFvs, single chain diabodies, antibody variable regions or epitope binding domains known in the art within the protein. In another embodiment, multispecific epitope binding proteins of the invention comprise at least 1, 2, 3, 4, 5, 6, 7, 8 or more scFvs, single chain diabodies, antibody variable regions or epitope binding domains known in the art which are specific for another epitope on the same antigen as another scFvs, single chain diabodies, antibody variable regions or epitope binding domains known in the art within the protein.

In one embodiment, multispecific epitope binding proteins of the invention comprise scFvs, single chain diabodies, antibody variable regions, or epitope binding domains known in the art, capable of binding epitopes concurrently. In another embodiment, multispecific epitope binding proteins of the invention comprise scFvs, single chain diabodies, antibody variable regions, or epitope binding domains known in the art, with non-identical binding specificities, capable of binding epitopes concurrently. In another embodiment, multispecific epitope binding proteins of the invention comprise scFvs, single chain diabodies, antibody variable regions, or epitope binding domains known in the art, which may or may not share the same binding specificity with other scFvs, single chain diabodies, antibody variable regions or epitope binding domains known in the art, within the protein, capable of binding epitopes concurrently. In another embodiment, the multispecific epitope binding proteins of the invention comprise scFvs, single chain diabodies, antibody variable regions, or epitope binding domains known in the art, wherein at least 1, 2, 3, 4, 5, 6, 7, 8, or more epitopes are bound concurrently.

In one embodiment, multispecific epitope binding proteins of the invention comprise scFvs, single chain diabodies, antibody variable regions, or epitope binding domains known in the art, capable of binding epitopes sequentially. In another embodiment, multispecific epitope binding proteins of the invention comprise scFvs, single chain diabodies, antibody variable regions, or epitope binding domains known in the art, with non-identical binding specificities, capable of binding epitopes concurrently. In another embodiment, multispecific epitope binding proteins of the invention comprise scFvs, single chain diabodies, antibody variable regions, or epitope binding domains known in the art, which may or may not share the same binding specificity with other scFvs, single chain diabodies, antibody variable regions or epitope binding domains known in the art, within the protein, capable of binding epitopes sequentially. In another embodiment, the multispecific epitope binding proteins of the invention comprise scFvs, single chain diabodies, antibody variable regions, or epitope binding domains known in the art, wherein at least 1, 2, 3, 4, 5, 6, 7, 8, or more epitopes are bound sequentially. To attain various epitope binding domains that bind antigens sequentially, one method employs adjusting the affinity and/or disassociation constant for the epitope binding domain for a specific antigen. Modulation of the affinity and/or disassociation constant values may be performed by known, art accepted techniques. Such affinity and/or disassociation constant modified domains may exhibit values represented in the following section.

### F.1. Multispecific Epitope Binding Protein Affinity

Epitope binding proteins of the invention may have a high binding affinity to one or more of its cognate antigens. For example, an epitope binding protein described herein may have an association rate constant or kₒₙ rate (epitope binding protein (EBP) + antigen->EBP-Ag) of at least 2 X 10⁵ M⁻¹s⁻¹, at least 5 X 10⁵ M⁻¹s⁻¹, at least 10⁶ M⁻¹s⁻¹, at least 5 X 10⁶ M⁻¹s⁻¹, at least 10⁷ M⁻¹s⁻¹, at least 5 X 10⁷ M⁻¹s⁻¹, or at least 10⁸ M⁻¹s⁻¹.

In another embodiment, an epitope binding protein may have a k_{off} rate (EBP-Ag -> EBP + Ag) of less than 5x10⁻¹ s⁻¹, less than 10⁻¹ s⁻¹, less than 5x10⁻² s⁻¹, less than 10⁻² s⁻¹, less than 5x10⁻³ s⁻¹, less than 10⁻³ s⁻¹, less than 5x10⁻⁴ s⁻¹, or less than 10⁻⁴ s⁻¹. In a another embodiment, an epitope binding protein of the invention has a k_{off} of less than 5x10⁻⁵ s⁻¹, less than 10⁻⁵ s⁻¹, less than 5x10⁻⁶ s⁻¹, less than 10⁻⁶ s⁻¹, less than 5x10⁻⁷ s⁻¹, less than 10⁻⁷ s⁻¹, less than 5x10⁻⁸ s⁻¹, less than 10⁻⁸ s⁻¹, less than 5x10⁻⁹ s⁻¹, less than 10⁻⁹ s⁻¹, or less than 10⁻¹⁰ s⁻¹.

In another embodiment, an epitope binding protein may have an affinity constant or Kₐ (kₒₙ/k_{off}) of at least 10² M⁻¹, at least 5 X 10² M⁻¹, at least 10³ M⁻¹, at least 5 X 10³ M⁻¹, at least 10⁻⁴ M⁻¹, at least 5 X 10⁻⁴ M⁻¹, at least 10⁵ M⁻¹, at least 5 X 10⁵ M⁻¹, at least 10⁶ M⁻¹, at least 5 X 10⁶ M⁻¹, at least 10⁷ M⁻¹, at least 5 X 10⁷ M⁻¹, at least 10⁸ M⁻¹, at least 5 X 10⁸ M⁻¹, at least 10⁹ M⁻¹, at least 5 X 10⁹ M⁻¹, at least 10¹⁰ M⁻¹, at least 5 X 10¹⁰ M⁻¹, at least 10¹¹ M⁻¹, at least 5 X 10¹¹ M⁻¹, at least 10¹² M⁻¹, at least 5 X 10¹² M⁻¹, at least 10¹³ M⁻¹, at least 5 X 10¹³ M⁻¹, at least 10¹⁴ M⁻¹, at least 5 X 10¹⁴ M⁻¹, at least 10¹⁵ M⁻¹, or at least 5 X 10¹⁵ M⁻¹. In yet another embodiment, an epitope binding protein may have a dissociation constant or K_{d} (k_{off}/kₒₙ) of less than 5x10⁻² M, less than 10⁻² M, less than 5x10⁻³ M, less than 10⁻³ M, less than 5x10⁻⁴ M, less than 10⁻⁴ M, less than 5x10⁻⁵ M, less than 10⁻⁵ M, less than 5x10⁻⁶ M, less than 10⁻⁶ M, less than 5x10⁻⁷ M, less than 10⁻⁷ M, less than 5x10⁻⁸ M, less than 10⁻⁸ M, less than 5x10⁻⁹ M, less than 10⁻⁹ M, less than 5x10⁻¹⁰ M, less than 10⁻¹⁰ M, less than 5x10⁻¹¹ M, less than 10⁻¹¹ M, less than 5x10⁻¹² M, less than 10⁻¹² M, less than 5x10⁻¹³ M, less than 10⁻¹³ M, less than 5x10⁻¹⁴ M, less than 10⁻¹⁴ M, less than 5x10⁻¹⁵ M, or less than 10⁻¹⁵ M.

An epitope binding protein used in accordance with a method described herein may have a dissociation constant (K_{d}) of less than 3000 pM, less than 2500 pM, less than 2000 pM, less than 1500 pM, less than 1000 pM, less than 750 pM, less than 500 pM, less than 250 pM, less than 200 pM, less than 150 pM, less than 100 pM, less than 75 pM as assessed using a method described herein or known to one of skill in the art (e.g., a BIAcore assay, ELISA) (Biacore International AB, Uppsala, Sweden). In a specific embodiment, an epitope binding protein used in accordance with a method described herein may have a dissociation constant (K_{d}) of between 25 to 3400 pM, 25 to 3000 pM, 25 to 2500 pM, 25 to 2000 pM, 25 to 1500 pM, 25 to 1000 pM, 25 to 750 pM, 25 to 500 pM, 25 to 250 pM, 25 to 100 pM, 25 to 75 pM, 25 to 50 pM as assessed using a method described herein or known to one of skill in the art (*e.g.*, a BIAcore assay, ELISA). In another embodiment, an epitope binding protein used in accordance with a method described herein may have a dissociation constant (K_{d}) of 500 pM, 100 pM, 75 pM or 50 pM as assessed using a method described herein or known to one of skill in the art (*e.g.*, a BIAcore assay, ELISA).

### F.2. Relative Binding Affinities Of Multispecific Epitope Binding Proteins

It is to be understood that the invention provides proteins carrying multiple epitope binding domains that may retain functionality within the protein in a similar fashion to the functionalities exhibited in an isolated state (i.e. the epitope binding domain exhibits similar properties as part of the multispecific epitope binding protein as compared to the domain if expressed or isolated independently). For example, an isolated scFv specific for epitope Y exhibits a specific functional profile including binding affinity, agonistic or antagonistic functions. It is to be understood that the same scFv expressed as a binding domain within a multispecific epitope binding protein of the invention would exhibit similar binding affinity and/or agonistic or antagonistic properties as compared to the isolated scFv.

In one embodiment multispecific epitope binding proteins of the invention comprise epitope binding domains from scFvs, single chain diabodies, antibody variable regions, or other epitope binding domains known in the art, with binding affinities lower than the same isolated (free from other components of the multispecific protein) epitope binding domains from scFvs, single chain diabodies, antibody variable regions, or other epitope binding domains known in the art. In another embodiment, multispecific epitope binding proteins of the invention comprise epitope binding domains from scFvs, single chain diabodies, antibody variable regions, or other epitope binding domains known in the art, with binding affinities higher than the same isolated (free from other components of the multispecific protein) epitope binding domains from scFvs, single chain diabodies, antibody variable regions, or other epitope binding domains known in the art. In another embodiment, multispecific epitope binding proteins of the invention comprise epitope binding domains from scFvs, single chain diabodies, antibody variable regions, or other epitope binding domains known in the art, with binding affinities essentially the same as the corresponding isolated (free from other components of the multispecific protein) epitope binding domains from scFvs, single chain diabodies, antibody variable regions, or other epitope binding domains known in the art.

Binding affinities can be routinely assayed by many techniques known in the art, such as ELISA, BiaCore™, KinExA™, cell surface receptor binding, competitive inhibition of binding assays. In a specific embodiment, the binding affinities of the multispecific epitope binding proteins of the invention can be assayed by the techniques presented in Example 10. In another embodiment, the multispecific epitope binding protein exhibits a lower binding affinity to a specific epitope than the functional isolated binding domain as measured by the techniques presented in Example 10. In another embodiment, the multispecific epitope binding protein exhibits a higher binding affinity to a specific epitope than the functional isolated binding domain as measured by the techniques presented in Example 10. In another embodiment, the multispecific epitope binding protein exhibits a similar binding affinity to a specific epitope than the functional isolated binding domain as measured by the techniques presented in Example 10.

In one embodiment, an epitope binding domain of a multispecific epitope binding protein exhibits a binding affinity less than 99%, less than 95%, less than 90%, less than 80%, less than 70%, less than 60%, less than 50%, less than 40%, less than 30%, less than 20%, or less than 10% to a specific epitope than the affinity of an identical functional isolated epitope binding domain as measured by any assay known in the art. In another embodiment, an epitope binding domain of a multispecific epitope binding protein exhibits a binding affinity less than 99%, less than 95%, less than 90%, less than 80%, less than 70%, less than 60%, less than 50%, less than 40%, less than 30%, less than 20%, or less than 10% to a specific epitope than the affinity of an identical functional isolated binding domain as measured by the techniques presented in Example 10.

In another embodiment, an epitope binding domain of a multispecific epitope binding protein exhibits a binding affinity more than 99%, more than 95%, more than 90%, more than 80%, more than 70%, more than 60%, more than 50%, more than 40%, more than 30%, more than 20%, or more than 10% to a specific epitope than the affinity of an identical functional isolated binding domain as measured by any assay known in the art. In another embodiment, the multispecific epitope binding protein exhibits a binding affinity more than 99%, more than 95%, more than 90%, more than 80%, more than 70%, more than 60%, more than 50%, more than 40%, more than 30%, more than 20%, or more than 10% to a specific epitope than the affinity of an identical functional isolated binding domain as measured by the techniques presented in Example 10.

Binding affinities can be routinely assayed by many techniques known in the art, such as ELISA, BiaCore™, KinExA™, cell surface receptor binding, competitive inhibition of binding assays . In a specific embodiment, the binding affinities of epitope binding domains of multispecific epitope binding proteins of the invention can be assayed by the techniques presented in any of Examples 13-20. In another embodiment, an epitope binding domain of a multispecific epitope binding protein exhibits a lower binding affinity to a specific epitope than an identical functional isolated epitope binding domain as measured by the techniques presented in any of Examples 13-20. In another embodiment, an epitope binding domain of a multispecific epitope binding protein exhibits a higher binding affinity to a specific epitope than an identical functional isolated epitope binding domain as measured by the techniques presented in any of Examples 13-20. In another embodiment, an epitope binding domain of a multispecific epitope binding protein exhibits a similar binding affinity to a specific epitope than an identical functional isolated epitope binding domain as measured by the techniques presented in any of Examples 13-20.

In another embodiment, an epitope binding domain of a multispecific epitope binding protein exhibits a binding affinity less than 99%, less than 95%, less than 90%, less than 80%, less than 70%, less than 60%, less than 50%, less than 40%, less than 30%, less than 20%, or less than 10% to a specific epitope than an identical functional isolated epitope binding domain as measured by any assay known in the art. In another embodiment, an epitope binding domain of a multispecific epitope binding protein exhibits a binding affinity less than 99%, less than 95%, less than 90%, less than 80%, less than 70%, less than 60%, less than 50%, less than 40%, less than 30%, less than 20%, or less than 10% to a specific epitope than an identical functional isolated epitope binding domain as measured by the techniques presented in any of Examples 13-20.

In another embodiment, an epitope binding domain of a multispecific epitope binding protein exhibits a binding affinity more than 99%, more than 95%, more than 90%, more than 80%, more than 70%, more than 60%, more than 50%, more than 40%, more than 30%, more than 20%, or more than 10% to a specific epitope than an identical functional isolated epitope binding domain as measured by any assay known in the art. In another embodiment, an epitope binding domain of a multispecific epitope binding protein exhibits a binding affinity more than 99%, more than 95%, more than 90%, more than 80%, more than 70%, more than 60%, more than 50%, more than 40%, more than 30%, more than 20%, or more than 10% to a specific epitope than an identical functional isolated epitope binding domain as measured by the techniques presented in any of Examples 13-20.

In a specific embodiment, multispecific epitope binding proteins of the invention comprise 3 scFvs wherein the most N-terminal scFv has a lower binding affinity than the identical functional isolated scFvs, wherein the second most N-terminal scFv has a lower binding affinity than the identical functional isolated scFv and wherein the third most N-terminal scFv has a lower binding affinity than the identical functional isolated scFv .

In another specific embodiment, multispecific epitope binding proteins of the invention comprise 4 scFvs wherein the most N-terminal scFv binds an epitope with a lower binding affinity than an identical functional isolated scFv, wherein the second most N-terminal scFv binds an epitope with a lower binding affinity than an identical functional isolated scFv , wherein the third most N-terminal scFv binds an epitope with a lower binding affinity than an identical functional isolated scFv, wherein the third most N-terminal scFv binds an epitope with a lower binding affinity than an identical functional isolated scFv , and the fourth most N-terminal scFv binds an epitope with a lower binding affinity than an identical functional isolated scFv.

In another specific embodiment, the multispecific epitope binding polypeptide chain of the invention comprises 2 scFvs linked to an antibody chain wherein the antibody binds an epitope with a similar binding affinity than an identical functional isolated antibody, wherein the most N-terminal scFv binds an epitope with a lower affinity than an identical functional isolated scFv, and the second most N-terminal scFv binds an epitope with a lower affinity than an identical functional isolated scFv.

In a specific embodiment, multispecific epitope binding proteins of the invention comprise two polypeptide chains, the first chain comprising 2 scFvs wherein the most N-terminal scFv binds an epitope with a lower affinity than an identical functional isolated scFv, wherein the second most N-terminal scFv binds an epitope with a lower affinity than an identical functional isolated scFv, and the second chain comprising 2 scFvs wherein the most N-terminal scFv binds an epitope with a lower affinity than an identical functional isolated scFv, wherein the second most N-terminal scFv binds an epitope with a lower affinity than an identical functional isolated scFv.

In another specific embodiment, multispecific epitope binding proteins of the invention comprise two epitope binding sites formed by two antibody variable regions wherein the first antigen variable region binds the epitope with an affinity less than an identical functional isolated antigen variable region, and where the second antigen variable region binds the epitope with an affinity less than an identical functional isolated antigen variable region.

n another embodiment, multispecific epitope binding proteins of the invention can selectively bind and inhibit distinct receptors on the surface of cells (e.g., *in vivo* in a mammal (e.g., a human) and/or *in vitro).* In one embodiment, multispecific epitope binding proteins of the invention can selectively bind and inhibit at least 1, 2, 3, 4, 5, 6, 7, 8, or more distinct cell surface receptors (e.g., *in vivo* in a mammal (e.g., a human) and/or *in vitro).* In another embodiment, multispecific epitope binding proteins of the invention can selectively bind and activate distinct receptors on the surface of cells (e.g., *in vivo* in a mammal (e.g., a human) and/or *in vitro).* In one embodiment, multispecific epitope binding proteins of the invention can selectively bind and activate at least 1, 2, 3, 4, 5, 6, 7, 8, or more distinct cell surface receptors (e.g., *in vivo* in a mammal (e.g., a human) and/or *in vitro).* In another embodiment, multispecific epitope binding proteins of the invention can selectively bind distinct receptors on the surface of cells and activate or inhibit said receptor (e.g., *in vivo* in a mammal (e.g., a human) and/or *in vitro).* In one embodiment, multispecific epitope binding proteins of the invention can selectively bind and activate or inhibit at least 1, 2, 3, 4, 5, 6, 7, 8, or more distinct cell surface receptors (e.g., *in vivo* in a mammal (e.g., a human) and/or *in vitro).* In a further embodiment, the multispecific epitope binding proteins of the invention bind distinct receptors on the surface of cells simultaneously (e.g., *in vivo* in a mammal (e.g., a human) and/or *in vitro).*

In another embodiment, multispecific epitope binding proteins of the invention can selectively bind and neutralize distinct soluble ligands (e.g., *in vivo* in a mammal (e.g., a human) and/or *in vitro).* In one embodiment, multispecific epitope binding proteins of the invention can selectively bind and/or neutralize at least 1, 2, 3, 4, 5, 6, 7, 8 or more distinct soluble ligands (e.g., *in vivo* in a mammal (e.g., a human) and/or *in vitro).* In a further embodiment, multispecific epitope binding proteins of the invention can selectively bind distinct soluble ligands simultaneously (e.g., *in vivo* in a mammal (e.g., a human) and/or *in vitro).*

In another embodiment, multispecific epitope binding proteins of the invention can selectively bind and inhibit distinct target proteins (e.g., *in vivo* in a mammal (e.g., a human) and/or *in vitro).* In one embodiment, multispecific epitope binding proteins of the invention can selectively bind and inhibit at least 1, 2, 3, 4, 5, 6, 7, 8, or more distinct target proteins (e.g., *in vivo* in a mammal (e.g., a human) and/or *in vitro).* In another embodiment, multispecific epitope binding proteins of the invention can selectively bind and activate distinct target proteins. In one embodiment, multispecific epitope binding proteins of the invention can selectively bind and activate at least 1, 2, 3, 4, 5, 6, 7, 8, or more distinct target proteins (e.g., *in vivo* in a mammal (e.g., a human) and/or *in vitro).* In another embodiment, multispecific epitope binding proteins of the invention can selectively bind distinct target proteins and activate or inhibit said target protein (e.g., *in vivo* in a mammal (e.g., a human) and/or *in vitro).* In one embodiment, multispecific epitope binding proteins of the invention can selectively bind and activate or inhibit at least 1, 2, 3, 4, 5, 6, 7, 8, or more distinct target proteins (e.g., *in vivo* in a mammal (e.g., a human) and/or *in vitro).* In a further embodiment, multispecific epitope binding proteins of the invention can selectively bind distinct target proteins simultaneously (e.g., *in vivo* in a mammal (e.g., a human) and/or *in vitro).*

### G. Uses of the multispecific epitope binding proteins of the invention and formulations of the same

The invention also provides methods of using multispecific epitope binding proteins of the invention. The present invention also encompasses the use of multispecific epitope binding proteins of the invention for the prevention, diagnosis, management, treatment or amelioration of one or more symptoms associated with diseases, disorders of diseases or disorders, including but not limited to cancer, inflammatory and autoimmune diseases either alone or in combination with other therapies. The invention also encompasses the use of multispecific epitope binding proteins of the invention conjugated or fused to a moiety (*e.g.*, therapeutic agent or drug) for prevention, management, treatment or amelioration of one or more symptoms associated with diseases, disorders or infections, including but not limited to cancer, inflammatory and autoimmune diseases either alone or in combination with other therapies.

Many cell types express various common cell surface antigens and it is the specific combination of antigens that distinguish a defined subset of cells. Using the multispecific epitope binding proteins of the invention, it is possible to target specific subsets of cells without cross-reacting with other unrelated populations of cells. Further, it is possible that multispecific epitope binding proteins of the invention comprise one to several (two, three, four, five, six, seven, eight, nine, ten, etc.) epitope binding domains that bind cell surface antigens present on non-target cell populations, however, it is the combined avidity of the set of epitope binding domains, which confer an effective level of binding (i.e., a therapeutically effective level of binding) to the target cell population. In other words, several of the epitope binding domains are engaged to facilitate the targeting of a particular cell type, which would not be achieved by binding of an individual isolated domain (e.g., isolated from the multispecific protein) or not achieved by one or more, but not all (i.e., a subset) of epitope binding domains (control peptides) exposed to the same cell, but not as part of a multispecific protein. It is envisioned that the relative avidity contribution of each epitope binding domain may be tailored to only target the specific cell population of interest. Such affinity alterations may be performed by art-accepted techniques, such as affinity maturation, site-specific mutagenesis, and others known in art. It is also contemplated that the relative avidity contribution of each epitope binding domain may also be altered by changing the relative orientation of said epitope binding domains in the multispecific epitope binding protein.

Accordingly, provided herein in one embodiment are methods of using multispecific epitope binding proteins of the invention to identify, deplete, modulate (e.g., activate, inhibit) a cell population (e.g., *in vivo* in a mammal (e.g., a human) and/or *in vitro).* In one particular embodiment, the multispecific epitope binding proteins of the invention do not significantly bind normal tissue (e.g., non-cancerous tissue or non-diseased tissue, i.e., non-target tissue) when administered to a mammal (e.g., a human). Avoidance of significant binding to non-target tissue may avoid or minimize depletion, modulation (activation, inhibition) of one or more non-targeted cell population. In some embodiments, the increase in avidity exhibited by the multispecific epitope binding proteins of the invention for a target cell population *in vitro* or when administered to a mammal (e.g., a human) is at least 2 fold, at least 3 fold, at least 4 fold, at least 5 fold, at least 6, fold, at least 7 fold, at least 8 fold, at least 9 fold, at least 10 fold, at least 15 fold, at least 20 fold, or at least 25 fold as compared to a "control epitope binding protein" which comprises one or more, but not all (i.e., a subset) of epitope binding domains isolated from said multispecific epitope binding protein. In some embodiments, the increase in avidity exhibited by the multispecific epitope binding proteins of the invention is at least 5%, at least 10%, at least 15%, at least 20%, at least 30%, fold, at least 40%, at least 50%, at least 75%, at least 85%, at least 90%, at least 95%, or at least 150% over said "control epitope binding protein".

In some embodiments, multispecific epitope binding proteins of the invention have an increase in avidity for a particular antigen compared to a control epitope binding protein, which comprises one or more, but not all (i.e., a subset) of epitope binding domains isolated from said multispecific epitope binding protein. In some embodiments, the increase in avidity exhibited by the multispecific epitope binding proteins of the invention is at least 2 fold, at least 3 fold, at least 4 fold, at least 5 fold, at least 6, fold, at least 7 fold, at least 8 fold, at least 9 fold, at least 10 fold, at least 15 fold, at least 20 fold, or at least 25 fold over said control epitope binding protein. In some embodiments, the increase in avidity exhibited by the multispecific epitope binding proteins of the invention is at least 5%, at least 10%, at least 15%, at least 20%, at least 30%, fold, at least 40%, at least 50%, at least 75%, at least 85%, at least 90%, at least 95%, or at least 150% over said control epitope binding protein.

In other embodiments, multispecific epitope binding proteins of the invention comprising X number of epitope binding domains (where X is any positive integer from 1 through 20) exhibit an increase in avidity (*in vitro* or when administered to a mammal (e.g., a human)for a particular antigen compared to a control epitope binding protein (such as, but not limited to an antibody, another multispecific epitope binding protein, an scFv, or a single chain diabody) wherein said control epitope binding protein comprises X-Y (Where X and Y are any positive integer from 1 through 20 and X is greater than Y) epitope binding domains, wherein at least one epitope binding domain in the control epitope binding domain protein is specific for the same epitope as the epitope binding domain present in the protein of the invention. In other words, the invention provides multispecific epitope binding proteins with increased avidity for a particular target as compared to control epitope binding proteins with greater or fewer epitope binding domains wherein at least one epitope binding domain is specific for a common antigen with the protein of the invention.

Such avidity changes in multispecific epitope binding proteins allow for the decrease of toxicity of such therapeutic proteins (for example, combinations of any antibodies listed in Section E. "Specific embodiments of multispecific epitope binding assemblies") in an animal. It is understood that the proteins of the invention may exhibit a "tailor-fit" avidity and/or affinity to decrease toxicity *in vivo.* As such, the invention also provides multispecific epitope binding proteins of the invention that exhibit a lower toxicity in an animal, than a control epitope binding protein. It is also understood that the invention also provides methods of reducing toxicity of a protein of the invention by the methods described herein.

It is also appreciated that avidity changes may be evaluated by readily available *in vitro* methods such as functional assays (including but not limited to cytokine expression/release/binding, gene expression, morphology changes, chemotaxis, calcium flux, and the like), binding measurements determined by BIAcore or KinExa measurements with control epitope binding domain proteins. In some embodiments, control epitope binding proteins may contain a subset of epitope binding domains from the proteins of the invention. In other embodiments, control epitope binding proteins may comprise at least one or more isolated epitope binding domains from the multispecific epitope binding proteins of the invention. For example, for a protein of the invention with 8 epitope binding domains, a control epitope binding protein may comprise 1, 2, 3, 4, 5, 6, or 7 epitope binding domains, with at least one epitope binding domain having specificity for an antigen recognized by both the protein of the invention and the control protein. In other embodiments, the control epitope binding protein comprises an isolated epitope binding domain having specificity for an antigen recognized by both the isolated epitope binding domain and the protein of the invention.

In one embodiment, the multispecific epitope binding proteins of the invention are used to specifically identify, deplete, activate, inhibit, or target for neutralization cells which are defined by the expression of multiple cell surface antigens. In another embodiment, invention contemplates use of proteins of the invention to purify cells expressing multiple antigens recognized by the multiple epitope binding sites in the protein. As such, methods of the invention include modulating the relative avidity contributions of each individual epitope binding domain within the multispecific epitope binding protein.

In some embodiments, the methods of the invention used to identify, deplete, activate, inhibit, or target for neutralization cells do not significantly deplete, activate, or inhibit a non-target cell population. In such embodiments, the administration of proteins of the invention does not exhibit a negative outcome that outweighs the benefits achieved from the treatment itself. Such negative outcomes may include, but are not limited to, binding to non-target tissues, increased toxicity, pathological depletion of cells, increased risk of infection, and the like. Negative outcomes presented by therapies by traditional epitope binding domains (including but not limited to antibodies, scFvs, single chain diabodies and the like) may also be minimized by the implementation of therapies employing multispecific epitope binding proteins of the invention.

Also, it is well understood that cancer cells differentially express cell surface molecules during the tumor progression. For example, a neoplastic cell may express a cell surface antigen in a benign state, yet down-regulate that particular cell surface antigen upon metastasis. It is understood that this process may occur for many different tumor cell types. As such, it is envisioned that the multispecific-epitope binding proteins of the invention may be designed to comprise epitope binding domains that could target such a tumor cell at many different stages of cancer progression In other words, the multispecific epitope binding proteins of the invention may be used to target a tumor cell type irrespective of its stage of progression. The multispecific epitope binding proteins of the invention may comprise multiple epitope binding domains that would bind specific tumor cell surface antigens expressed at various stages of tumor progression.

In one embodiment, multispecific epitope binding proteins of the invention comprise epitope binding domains that are specific for tumor cell surface antigens which may or may not be displayed concurrently. In other embodiments, multispecific epitope binding proteins of the invention comprise at least one, at least two, at least three, at least four, at least five, at least six, at least seven, or at least eight epitope binding domains that are specific for tumor cell surface antigens, wherein said antigens may not be displayed on the surface of the tumor cell concurrently. In other embodiments, multispecific epitope binding proteins of the invention comprise at least one, at least two, at least three, at least four, at least five, at least six, at least seven, or at least eight epitope binding domains that are specific for tumor cell surface antigens, wherein at least one, at least two, at least three, at least four, at least five, at least six, at least seven, or at least eight tumor cell surface antigens are engaged by said protein to elicit a response, such as effector function, internalization, neutralization, and others.

In other embodiments, the invention provides methods of depleting, killing, neutralizing, and/or sequestering cell types that are defined by the expression of multiple cell surface antigens. Such cell types include, but are not limited to T cells (such as cytotoxic, memory and NK cells), B cells, Mast cells, eosinophils, basophils, neutrophils, stem cells (such as cancer stem cells), and macrophages.

The multispecific epitope binding proteins of the invention are employed in an amount that is effective for both producing the desired therapeutic effect and for reducing side effects. A cytokine storm is a potentially fatal immune reaction consisting of a positive feedback loop between cytokines and immune cells which is caused for example, when the immune system is fighting pathogens. The cytokine storm (hypercytokinemia) is the systemic expression of a healthy and vigorous immune system resulting in the release of more than 150 inflammatory mediators (cytokines, oxygen free radicals, and coagulation factors). Both pro-inflammatory cytokines (such as Tumor necrosis factor-alpha, Interleukin-1, and Interleukin-6) and anti-inflammatory cytokines (such as interleukin 10, and interleukin 1 receptor antagonist) are elevated in the serum of patients experiencing a cytokine storm. Cytokine storms can occur in a number of infectious and non-infectious diseases including graft versus host disease (GVHD), adult respiratory distress syndrome (ARDS), sepsis, avian influenza, smallpox, stroke, allergic reaction (hypersensitivity), cardiac arrest, toxic shock syndrome, and systemic inflammatory response syndrome (SIRS). A cytokine storm may also be induced as a result of traditional small molecule, or biologic therapy. In some embodiments, multispecific epitope binding proteins of the invention are useful in the prevention, management, suppression, and/or amelioration of a cytokine storm by inhibiting/antagonizing/neutralizing and/or depleting the released cytokines. Such multispecific epitope binding proteins would comprise at least one epitope binding domain specific for a cytokine present in the cytokine storm. Such a protein would represent a single agent therapy for multiple elevated cytokines in a patient. In other embodiments, such proteins and formulations thereof could be administered in a prophylactic manner to prevent or suppress a cytokine storm. In other embodiments, such proteins and formulations thereof could be administered to a patient in need, such as a patient currently experiencing a cytokine storm. In further embodiments, multispecific epitope binding proteins of the invention may be administered to a patient in need thereof in conjunction with OX40 based therapies (OX40-Ig, Ox40 ligand), angiotensin converting enzyme (ACE) inhibitors, angiotensin II receptor blockers (ARBs), corticosteroids, NSAIDS, and/or free radical scavengers (antioxidants). In other embodiments, multispecific epitope binding proteins of the invention may inhibit, antagonize, suppress, and/or neutralize a cytokine storm in a mammal (e.g. a human, or no-human primate) by at least about 10%, about 20%, about 30%, about 40%, about 50%, about 60%, about 70%, about 80%, about 90%, or about 95% as compared to prior to or the absence of the administration of a protein of the invention.

In other embodiments, multispecific epitope binding proteins are useful in the targeting of breakdown products of particular antigen. It is well understood that the β-amyloid protein is subjected to proteolysis into various fragments and it is these fragments that are thought to be a causative agent in disease progression. Multispecific epitope binding proteins of the invention may be useful in the reduction of various fragments of a breakdown product, such as, β-amyloid protein fragments. Proteins of the invention may remove the various fragments by exposing at least one epitope binding domain specific for each fragment. In an additional embodiment, multispecific epitope binding proteins of the invention may also comprise at least one EBD specific for an epitope only exposed after modification, for example, a cleavage product. Proteins of the invention may be useful in suppressing, inhibiting, antagonizing, or neutralizing such cryptic epitopes.

Also, many cell surface receptors activate or deactivate as a consequence of crosslinking of subunits. The proteins of the invention may be used to stimulate or inhibit a response in a target cell by crosslinking of cell surface receptors. In another embodiment, the multispecific epitope binding protein of the invention may be used to block the interaction of multiple cell surface receptors with antigens. In another embodiment, the multispecific epitope binding protein of the invention may be used to strengthen the interaction of multiple cell surface receptors with antigens. In another example, it may be possible to crosslink homodimers of a cell surface receptor using multispecific epitope binding proteins of the invention containing binding domains that share specificity for the same antigen. In another embodiments, multispecific epitope binding proteins of the invention are useful to crosslink subunits or a heteromultimeric receptor (for example, but not limited to a heterodimeric receptor). In other embodiments, proteins of the invention may antagonize, inhibit, or suppress heteromultimer formation by binding at least two distinct antigens. In other embodiments, proteins of the invention are useful for the targeting, agonizing, antagonizing, suppression, and/or stimulation ofmultimeric receptors through the interaction of multiple epitope binding domains present in the protein with the various protein component of the multimeric receptor.

In another embodiment, the proteins of the invention may be used to deliver a ligand, or ligand analogue to a specific cell surface receptor. In some embodiments, proteins of the invention may be useful to increase the stoichiometry of ligands present at a receptor. In this situation, it is possible that the multispecific epitope binding protein of the invention may present more than one ligand isoform over others through multiple epitope binding domains that bind a specified ligand. In other embodiments, the proteins of the invention may coordinate one or more ligands to properly engage a receptor through the interaction of multiple epitope binding domains with the ligand.

The invention also provides methods of targeting epitopes not easily accomplished with traditional antibodies. For example, in one embodiment, the multispecific epitope binding proteins of the invention may be used to first target an adjacent antigen and while binding, another binding domain may engage the cryptic antigen.

The invention also provides methods of targeting epitopes not present on the cell surface through the use of the multiple epitope binding domains. It is to be understood that the proteins of the invention are useful in delivering epitope binding domains to the interior of a cell. Using at least on epitope binding domain specific for a cell surface antigen, proteins of the invention may be targeted directly (through internalization of the bound antigen) or indirectly through membrane permeable structures of sequences (ie. intrabodies) present on the protein of the invention. In such a scenario, it is possible to target intracellular targets with the proteins of the invention.

The invention also provides methods of binding, antagonizing, suppressing, and/or neutralizing circulating soluble antigens (e.g., *in vivo* in a mammal (e.g., a human) and/or *in vitro).* Such antigens include various cytokines, inflammatory mediators, hormones, albumin, vitamins, triglycerides, small molecule drugs, and the like. It is also envisaged that the proteins of the invention may comprise epitope binding domains specific for various distinct soluble ligands. In some embodiments, proteins of the invention are useful in a method to bind, neutralize, and/or suppress various drugs given to a patient in an effort to control a particular interaction or effect. In some embodiments, proteins of the invention may increase or decrease the half-life of circulating soluble antigens. In other embodiments, proteins of the invention have no effect on the half-life od circulating soluble antigens.

Proteins of the invention are also useful in the clearance of unwanted agents circulating in an individual. For example, multispecific epitope binding proteins of the invention may be employed to bind and target for clearance agents selected from pathogens, cytokines, inflammatory mediators, hormones, albumin, vitamins, triglycerides, and small molecule drugs.

The invention also provides methods of reducing therapy-induced toxicity associated with one or more antibody or antibody fragment (or any peptide) based therapy using the proteins of the invention. For instance, if two polypeptides are toxic when administered together (or administered at times where they may interact *in vivo),* the toxicity associated with such interaction may be avoided by engineering the binding properties of the individual proteins in a multispecific protein of the invention. In some embodiments, reducing toxicity using methods of the invention comprise targeting the same antigens of the individual therapies with a protein of the invention. In some embodiments, proteins of the invention comprise the same epitope binding domains as the individual therapies. In other embodiments, proteins of the invention comprise epitope binding domains directed at the same epitope and/or antigen as the individual therapies. In some embodiments, methods of the invention reduce toxicity associated with one or more antibody and/or antibody fragment based therapy (for example, combinations of any antibodies listed in Section E. "Specific embodiments of multispecific epitope binding assemblies") by at least about 10%, about 20%, about 30%, about 40%, about 50%, about 60%, about 70%, about 80%, about 90%, or about 95% as compared to the toxicity associated with one or more antibody and/or antibody fragment based therapy.

The invention also provides methods of using multispecific epitope binding proteins to bring together distinct cell types. In one embodiment, the proteins of the invention may bind a target cell with one binding domain and recruit another cell via another binding domain. In another embodiment, the first cell may be a cancer cell and the second cell is an immune effector cell such as an NK cell. In another embodiment, the multispecific epitope binding protein of the invention may be used to strengthen the interaction between two distinct cells, such as an antigen presenting cell and a T cell to possibly boost the immune response.

The invention also provides methods of using multispecific epitope binding proteins to ameliorate, treat, or prevent cancer or symptoms thereof. In one embodiment, methods of the invention are useful in the treatment of cancers of the head, neck, eye, mouth, throat, esophagus, chest, skin, bone, lung, colon, rectum, colorectal, stomach, spleen, kidney, skeletal muscle, subcutaneous tissue, metastatic melanoma, endometrial, prostate, breast, ovaries, testicles, thyroid, blood, lymph nodes, kidney, liver, pancreas, brain, or central nervous system. Examples of cancers that can be prevented, managed, treated or ameliorated in accordance with the methods of the invention include, but are not limited to, cancer of the head, neck, eye, mouth, throat, esophagus, chest, bone, lung, colon, rectum, stomach, prostate, breast, ovaries, kidney, liver, pancreas, and brain. Additional cancers include, but are not limited to, the following: leukemias such as but not limited to, acute leukemia, acute lymphocytic leukemia, acute myelocytic leukemias such as myeloblastic, promyelocytic, myelomonocytic, monocytic, erythroleukemia leukemias and myclodysplastic syndrome, chronic leukemias such as but not limited to, chronic myelocytic (granulocytic) leukemia, chronic lymphocytic leukemia, hairy cell leukemia; polycythemia vera; lymphomas such as but not limited to Hodgkin's disease, non-Hodgkin's disease; multiple myelomas such as but not limited to smoldering multiple myeloma, nonsecretory myeloma, osteosclerotic myeloma, plasma cell leukemia, solitary plasmacytoma and extramedullary plasmacytoma; Waldenstrom's macroglobulinemia; monoclonal gammopathy of undetermined significance; benign monoclonal gammopathy; heavy chain disease; bone cancer and connective tissue sarcomas such as but not limited to bone sarcoma, myeloma bone disease, multiple myeloma, cholesteatoma-induced bone osteosarcoma, Paget's disease of bone, osteosarcoma, chondrosarcoma, Ewing's sarcoma, malignant giant cell tumor, fibrosarcoma of bone, chordoma, periosteal sarcoma, soft-tissue sarcomas, angiosarcoma (hemangiosarcoma), fibrosarcoma, Kaposi's sarcoma, leiomyosarcoma, liposarcoma, lymphangiosarcoma, neurilemmoma, rhabdomyosarcoma, and synovial sarcoma; brain tumors such as but not limited to, glioma, astrocytoma, brain stem glioma, ependymoma, oligodendroglioma, non-glial tumor, acoustic neurinoma, craniopharyngioma, medulloblastoma, meningioma, pineocytoma, pineoblastoma, and primary brain lymphoma; breast cancer including but not limited to adenocarcinoma, lobular (small cell) carcinoma, intraductal carcinoma, medullary breast cancer, mucinous breast cancer, tubular breast cancer, papillary breast cancer, Paget's disease (including juvenile Paget's disease) and inflammatory breast cancer; adrenal cancer such as but not limited to pheochromocytom and adrenocortical carcinoma; thyroid cancer such as but not limited to papillary or follicular thyroid cancer, medullary thyroid cancer and anaplastic thyroid cancer; pancreatic cancer such as but not limited to, insulinoma, gastrinoma, glucagonoma, vipoma, somatostatin-secreting tumor, and carcinoid or islet cell tumor; pituitary cancers such as but limited to Cushing's disease, prolactin-secreting tumor, acromegaly, and diabetes insipius; eye cancers such as but not limited to ocular melanoma such as iris melanoma, choroidal melanoma, and cilliary body melanoma, and retinoblastoma; vaginal cancers such as squamous cell carcinoma, adenocarcinoma, and melanoma; vulvar cancer such as squamous cell carcinoma, melanoma, adenocarcinoma, basal cell carcinoma, sarcoma, and Paget's disease; cervical cancers such as but not limited to, squamous cell carcinoma, and adenocarcinoma; uterine cancers such as but not limited to endometrial carcinoma and uterine sarcoma; ovarian cancers such as but not limited to, ovarian epithelial carcinoma, borderline tumor, germ cell tumor, and stromal tumor; esophageal cancers such as but not limited to, squamous cancer, adenocarcinoma, adenoid cyctic carcinoma, mucoepidermoid carcinoma, adenosquamous carcinoma, sarcoma, melanoma, plasmacytoma, verrucous carcinoma, and oat cell (small cell) carcinoma; stomach cancers such as but not limited to, adenocarcinoma, fungating (polypoid), ulcerating, superficial spreading, diffusely spreading, malignant lymphoma, liposarcoma, fibrosarcoma, and carcinosarcoma; colon cancers; rectal cancers; liver cancers such as but not limited to hepatocellular carcinoma and hepatoblastoma, gallbladder cancers such as adenocarcinoma; cholangiocarcinomas such as but not limited to pappillary, nodular, and diffuse; lung cancers such as non-small cell lung cancer, squamous cell carcinoma (epidermoid carcinoma), adenocarcinoma, large-cell carcinoma and small-cell lung cancer; testicular cancers such as but not limited to germinal tumor, seminoma, anaplastic, classic (typical), spermatocytic, nonseminoma, embryonal carcinoma, teratoma carcinoma, choriocarcinoma (yolk-sac tumor), prostate cancers such as but not limited to, adenocarcinoma, leiomyosarcoma, and rhabdomyosarcoma; penal cancers; oral cancers such as but not limited to squamous cell carcinoma; basal cancers; salivary gland cancers such as but not limited to adenocarcinoma, mucoepidermoid carcinoma, and adenoidcystic carcinoma; pharynx cancers such as but not limited to squamous cell cancer, and verrucous; skin cancers such as but not limited to, basal cell carcinoma, squamous cell carcinoma and melanoma, superficial spreading melanoma, nodular melanoma, lentigo malignant melanoma, acral lentiginous melanoma; kidney cancers such as but not limited to renal cell cancer, adenocarcinoma, hypernephroma, fibrosarcoma, transitional cell cancer (renal pelvis and/or ureter); Wilms' tumor; bladder cancers such as but not limited to transitional cell carcinoma, squamous cell cancer, adenocarcinoma, carcinosarcoma. In addition, cancers include myxosarcoma, osteogenic sarcoma, endotheliosarcoma, lymphangioendotheliosarcoma, mesotheliorna, synovioma, hemangioblastoma, epithelial carcinoma, cystadenocarcinoma, bronchogenic carcinoma, sweat gland carcinoma, sebaceous gland carcinoma, papillary carcinoma and papillary adenocarcinomas (for a review of such disorders, see Fishman et al., 1985, Medicine, 2d Ed., J. B. Lippincott Co., Philadelphia and Murphy et al., 1997, Informed Decisions: The Complete Book of Cancer Diagnosis, Treatment, and Recovery, Viking Penguin, Penguin Books U.S.A., inc., United States of America). It is also contemplated that cancers caused by aberrations in apoptosis can also be treated by the methods and compositions of the invention. Such cancers may include, but not be limited to, follicular lymphomas, carcinomas with p53 mutations, hormone dependent tumors of the breast, prostate and ovary, and precancerous lesions such as familial adenomatous polyposis, and myelodysplastic syndromes. The invention also provides methods of using multispecific epitope binding proteins to deplete a cell population. In one embodiment, methods of the invention are useful in the depletion of the following cell types: eosinophil, basophil, neutrophil, T cell, B cell, mast cell, monocytes, cancer stem cell, and tumor cell.

The invention also provides methods of using multispecific epitope binding proteins to inactivate, inhibit, or deplete cytokines. In one embodiment, methods of the invention are useful in the inactivation, inhibition, or depletion of at least one of the following cytokines: TGF-β, TNF-α, C5a, fMLP, Leukotrienes A4 and B4, Prostaglandins D, E, and F, Thromboxane A₂, Interferon alpha (including subtypes 1, 2a, 2b, 4, 4b, 5, 6, 7, 8, 10, 14, 16, 17 and 21), Interferon beta, Interferon omega, Interferon gamma, interleukins IL-1-33, CCL1-28, CXCL 1-17, and CX3CL1.

The invention also provides methods of using multispecific epitope binding proteins as synthetic receptors. At least one or more epitope binding domains may be engineered to bind various molecules, such as, but not limited to drug molecules, imaging agents and others to be used as receptors for such molecules. For example, a multispecific epitope binding protein specific for a population of cells (such as, but not limited to cancer cells) would bind the cell surface antigens and provide a platform for specific targeting of small molecule agents direct to the population of cells. This method may lead to increased agent concentration at the target site. The method may decrease toxicity to normal tissues due to a lowered systemic dose of an agent. The methods allows for altered drug/agent pharmacodynamic profiles and therapeutic windows. In some embodiments, the agent would be administered prior to, concominantly, or after administration of the multispecific epitope binding protein.

The invention also provides methods of using proteins of the invention to reduce toxicity in a mammal (e.g. a human or non-human primate) associated with exposure to one or more agents selected from the group consisting of abrin, brucine, cicutoxin, diphtheria toxin, botulism toxin, shiga toxin, endotoxin, tetanus toxin, pertussis toxin, anthrax toxin, cholera toxin, falcarinol, alfa toxin, geldanamycin, gelonin, lotaustralin, ricin, strychnine, snake venom toxin and tetradotoxin.

In some embodiments, multispecific epitope binding proteins of the invention may utilize one or more uses described herein to accomplish the required task. For example, multispecific epitope binding proteins of the invention may block receptor dimerization and neutralize the cognate antigen concominantly. For example, multispecific epitope binding proteins of the invention may comprise at least one epitope directed against the IFNAR1 receptor to block dimerization which is required for activity, while using at least one other epitope binding domain to bind and neutralize the soluble interferon alpha subtypes that bind IFNAR1. As such, the proteins of the invention provide methods of performing multiple tasks by the administration of multiple epitope binding domains directed at various properties of an interaction.

The invention also provides methods of using multispecific epitope binding proteins as diagnostic reagents either *in vivo* (e.g. when administered to a mammal, for example, a human) or *in vitro* (for example, in a patient-derived sample),. The multiple binding specificities may be useful in kits or reagents where different antigens need to be efficiently captured concurrently. As such, in some embodiments, the invention provides methods of detecting and/or purifying at least one soluble compound from a solution using a protein of the invention. In some embodiments, such a solution may be a bodily fluid, cell culture media, fermentation media fluid, biological sample, potable water. Bodily fluids may include, for example, blood, sweat, lymph, urine, tears, bile, saliva, serum, amniotic fluid, cerumen (earwax), Cowper's fluid, semen, chyle, chime, cerebrospinal fluid, stool, stool water, pancreatic juice, synovial fluid, aqeous humor, interstitial fluid, breast milk, mucus, pleural fluid, pus, sebum, and vomit.

The proteins of the invention and compositions comprising the same are useful for many purposes, for example, as therapeutics against a wide range of chronic and acute diseases and disorders including, but not limited to, autoimmune and/or inflammatory disorders, which include Sjogren's syndrome, rheumatoid arthritis, lupus psoriasis, atherosclerosis, diabetic and other retinopathies, retrolental fibroplasia, age-related macular degeneration, neovascular glaucoma, hemangiomas, thyroid hyperplasias (including Grave's disease), corneal and other tissue transplantation, and chronic inflammation, sepsis, rheumatoid arthritis, peritonitis, Crohn's disease, reperfusion injury, septicemia, endotoxic shock, cystic fibrosis, endocarditis, psoriasis, arthritis (*e.g.*, psoriatic arthritis), anaphylactic shock, organ ischemia, reperfusion injury, spinal cord injury and allograft rejection. Other Examples of autoimmune and/or inflammatory disorders include, but are not limited to, alopecia areata, ankylosing spondylitis, antiphospholipid syndrome, autoimmune Addison's disease, autoimmune diseases of the adrenal gland, autoimmune hemolytic anemia, autoimmune hepatitis, autoimmune oophoritis and orchitis, Sjogren's syndrome, psoriasis, atherosclerosis, diabetic and other retinopathies, retrolental fibroplasia, age-related macular degeneration, neovascular glaucoma, hemangiomas, thyroid hyperplasias (including Grave's disease), corneal and other tissue transplantation, and chronic inflammation, sepsis, rheumatoid arthritis, peritonitis, Crohn's disease, reperfusion injury, septicemia, endotoxic shock, cystic fibrosis, endocarditis, psoriasis, arthritis (*e.g.*, psoriatic arthritis), anaphylactic shock, organ ischemia, reperfusion injury, spinal cord injury and allograft rejection. autoimmune thrombocytopenia, Behcet's disease, bullous pemphigoid, cardiomyopathy, celiac sprue-dermatitis, chronic fatigue immune dysfunction syndrome (CFIDS), chronic inflammatory demyelinating polyneuropathy, Churg-Strauss syndrome, cicatrical pemphigoid, CREST syndrome, cold agglutinin disease, Crohn's disease, discoid lupus, essential mixed cryoglobulinemia, fibromyalgia-fibromyositis, glomerulonephritis, Graves' disease, Guillain-Barre, Hashimoto's thyroiditis, idiopathic pulmonary fibrosis, idiopathic thrombocytopenia purpura (ITP), IgA neuropathy, juvenile arthritis, lichen planus, lupus erythematosus, Meniere's disease, mixed connective tissue disease, multiple sclerosis, type 1 or immune-mediated diabetes mellitus, myasthenia gravis, pemphigus vulgaris, pernicious anemia, polyarteritis nodosa, polychrondritis, polyglandular syndromes, polymyalgia rheumatica, polymyositis and dermatomyositis, primary agammaglobulinemia, primary biliary cirrhosis, psoriasis, psoriatic arthritis, Raynauld's phenomenon, Reiter's syndrome, Rheumatoid arthritis, sarcoidosis, scleroderma, Sjogren's syndrome, stiff-man syndrome, systemic lupus erythematosus, lupus erythematosus, takayasu arteritis, temporal arteristis/giant cell arteritis, ulcerative colitis, uveitis, vasculitides such as dermatitis herpetiformis vasculitis, vitiligo, and Wegener's granulomatosis. Examples of inflammatory disorders include, but are not limited to, asthma, encephilitis, inflammatory bowel disease, chronic obstructive pulmonary disease (COPD), allergic disorders, septic shock, pulmonary fibrosis, undifferentitated spondyloarthropathy, undifferentiated arthropathy, arthritis, inflammatory osteolysis, and chronic inflammation resulting from chronic viral or bacteria infections. The compositions and methods of the invention can be used with one or more conventional therapies that are used to prevent, manage or treat the above diseases.

In one embodiment, the invention comprises compositions capable of treating chronic inflammation. In one embodiment, the compositions are useful in the targeting of immune cells for destruction or deactivation. In one embodiment, the compositions are useful in targeting activated T cells, dormant T cells, B cells, neutrophils, eosinophils, basophils, mast cells, dendritic cells, or macrophages. In another embodiment, the invention comprises compositions capable of decreasing immune cell function. In another embodiment, the compositions are capable of ablating immune cell function.

T cells play a central role in cell-mediated immunity and collectively represent a set of cells including Helper, Cytotoxic, Memory, and NK T cells. Once activated, Helper T cells divide rapidly and secrete cytokines to regulate the immune response. In one embodiment, the invention comprises compositions capable of inhibiting the division and/or proliferation of Helper T cells. In some embodiments, compositions of the invention are capable of reducing division and/or proliferation of Helper T cells by at least 5%, at least 10%, at least 15%, at least 20%, at least 25%, at least 30%, at least 35%, at least 40%, at least 45%, at least 50%, at least 55%, at least 60%, at least 60%, at least 65%, at least 70%, at least 75%, at least 80%, at least 85%, at least 90%, at least 95%, or at least 100% or more as compared to untreated activated Helper T cells. In another embodiment compositions of the invention are capable of reducing division and/or proliferation by at least 2 fold, at least 3 fold, at least 4 fold, at least 5 fold, at least 10 fold, at least 15 fold, at least 20 fold, at least 50 fold or more as compared to untreated activated Helper T cells. In another embodiment, compositions of the invention can inhibit or reduce cytokine production and/or secretion from activated Helper T cells. In some embodiments, compositions of the invention are capable of inhibiting and or reducing cytokine production and/or secretion from Helper T cells by at leat 5%, at least 10%, at least 15%, at least 20%, at least 25%, at least 30%, at least 35%, at least 40%, at least 45%, at least 50%, at least 55%, at least 60%, at least 60%, at least 65%, at least 70%, at least 75%, at least 80%, at least 85%, at least 90%, at least 95%, or at least 100% or more as compared to untreated activated Helper T cells. In some embodiments, compositions of the invention are capable of inhibiting and/or reducing cytokine production and/or secretion from Helper T cells by at least 2 fold, at least 3 fold, at least 4 fold, at least 5 fold, at least 10 fold, at least 15 fold, at least 20 fold, or at least 50 fold or more as compared to untreated activated Helper T cells.

Cytotoxic T cells are capable of inducing cell death in, for example, tumor cells or cells infected with viruses or other pathogens. Once activated, Cytotoxic T cells undergo clonal expansion with the help of the IL-2 cytokine. Also, upon activation, the cytotoxins, perforin and granulysin are released from the Cytotoxic T cell to aide in the destruction of the target cell. In one embodiment, the invention comprises compositions capable of inhibiting the division and/or proliferation of Cytotoxic T cells. In some embodiments, compositions of the invention are capable of reducing division and/or proliferation of Cytotoxic T cells by at least 5%, at least 10%, at least 15%, at least 20%, at least 25%, at least 30%, at least 35%, at least 40%, at least 45%, at least 50%, at least 55%, at least 60%, at least 60%, at least 65%, at least 70%, at least 75%, at least 80%, at least 85%, at least 90%, at least 95%, or at least 100% or more as compared to untreated activated Cytotoxic T cells. In another embodiment, compositions of the invention are capable of reducing division and/or proliferation by at least 2 fold, at least 3 fold, at least 4 fold, at least 5 fold, at least 10 fold, at least 15 fold, at least 20 fold, at least 50 fold or more as compared to untreated activated Cytotoxic T cells. In another embodiment, compositions of the invention can inhibit or reduce cytotoxin production and/or secretion from activated Cytotoxic T cells. In some embodiments, compositions of the invention are capable of inhibiting and or reducing cytotoxin production and/or secretion from Cytotoxic T cells by at least 5%, at least 10%, at least 15%, at least 20%, at least 25%, at least 30%, at least 35%, at least 40%, at least 45%, at least 50%, at least 55%, at least 60%, at least 60%, at least 65%, at least 70%, at least 75%, at least 80%, at least 85%, at least 90%, at least 95%, or at least 100% or more as compared to untreated activated Cytotoxic T cells. In some embodiments, compositions of the invention are capable of inhibiting and/or reducing cytotoxin production and/or secretion from Helper T cells by at least 2 fold, at least 3 fold, at least 4 fold, at least 5 fold, at least 10 fold, at least 15 fold, at least 20 fold, or at least 50 fold or more as compared to untreated activated Cytotoxic T cells. In a further embodiment, the cytotoxins are selected from the group consisting of perforin and granulysin. In another embodiment, the production and/or secretion of perforin is reduced. In another embodiment, the production and/or secretion of granulysin is reduced. In another embodiment, the production and/or secretion of perforin and granulysin are reduced.

Memory T cells represent a class of T cells that can recognize foreign invaders such as bacteria and viruses that were encountered during a prior infection or vaccination. At a second encounter with the invader, memory T cells can reproduce to mount a faster and stronger immune response that the first encounter. Memory T cells produce and secrete cytokines that stimulate the immune response. In some embodiments, compositions of the invention are capable of reducing division and/or proliferation of Memory T cells by at least 5%, at least 10%, at least 15%, at least 20%, at least 25%, at least 30%, at least 35%, at least 40%, at least 45%, at least 50%, at least 55%, at least 60%, at least 60%, at least 65%, at least 70%, at least 75%, at least 80%, at least 85%, at least 90%, at least 95%, or at least 100% or more as compared to untreated activated Memory T cells. In another embodiment compositions of the invention are capable of reducing division and/or proliferation by at least 2 fold, at least 3 fold, at least 4 fold, at least 5 fold, at least 10 fold, at least 15 fold, at least 20 fold, at least 50 fold or more as compared to untreated activated Memory T cells. In another embodiment, compositions of the invention can inhibit or reduce cytokine production and/or secretion from activated Memory T cells. In some embodiments, compositions of the invention are capable of inhibiting and or reducing cytokine production and/or secretion from Memory T cells by at least 5%, at least 10%, at least 15%, at least 20%, at least 25%, at least 30%, at least 35%, at least 40%, at least 45%, at least 50%, at least 55%, at least 60%, at least 60%, at least 65%, at least 70%, at least 75%, at least 80%, at least 85%, at least 90%, at least 95%, or at least 100% or more as compared to untreated activated Memory T cells. In some embodiments, compositions of the invention are capable of inhibiting and/or reducing cytokine production and/or secretion from Memory T cells by at least 2 fold, at least 3 fold, at least 4 fold, at least 5 fold, at least 10 fold, at least 15 fold, at least 20 fold, or at least 50 fold or more as compared to untreated activated Memory T cells. In some embodiments, the compositions of the invention reduce the production of Il-2, Interferon gamma, and/or IL-4.

Natural Killer (otherwise known as NK cells) are a type of cytotoxic lymphocyte which plays a role in the targeted destruction of tumor cells and cells infected with viruses. The NK cells kill target cells by releasing small cytoplasmic granules containing perforin and granzyme which trigger apoptosis in the target cell. In some embodiments, compositions of the invention inhibit or reduce the release of perforin and/or granzyme from NK cells. In some embodiments, compositions of the invention are capable of inhibiting and or reducing the release of perforin and/or granzyme from NK cells by at least 5%, at least 10%, at least 15%, at least 20%, at least 25%, at least 30%, at least 35%, at least 40%, at least 45%, at least 50%, at least 55%, at least 60%, at least 60%, at least 65%, at least 70%, at least 75%, at least 80%, at least 85%, at least 90%, at least 95%, or at least 100% or more as compared to untreated activated NK cells. In some embodiments, compositions of the invention are capable of inhibiting and/or reducing release of perforin and/or granzyme from NK cells by at least 2 fold, at least 3 fold, at least 4 fold, at least 5 fold, at least 10 fold, at least 15 fold, at least 20 fold, at least 50 fold or more as compared to untreated activated NK cells.

B cells are lymphocytes that play a role in the humoral immune response. The principle function of B cells is the generation of antibodies against soluble antigens. Each B cell has a unique receptor that will bind one particular antigen. Once the B-cell engages its cognate antigen and receives additional signals from Helper T cells, it can become an antibody producing cell. In some embodiments, compositions of the invention can inhibit or reduce the activation of B cells. In some embodiments, compositions of the invention may inhibit or reduce the activation of B cells by at least 5%, at least 10%, at least 15%, at least 20%, at least 25%, at least 30%, at least 35%, at least 40%, at least 45%, at least 50%, at least 55%, at least 60%, at least 60%, at least 65%, at least 70%, at least 75%, at least 80%, at least 85%, at least 90%, at least 95%, or at least 100% or more as compared to untreated B cells. In some embodiments, compositions of the invention can inhibit or reduce the activation of B cells. In some embodiments, compositions of the invention may inhibit or reduce the activation of B cells by at least 2 fold, at least 3 fold, at least 4 fold, at least 5 fold, at least 10 fold, at least 15 fold, at least 20 fold, or at least 50 fold or more as compared to untreated B cells.

In another embodiment, compositions of the invention can inhibit or reduce the production and/or secretion of antibodies from B cells. In some embodiments, compositions of the invention may inhibit or reduce the production and/or secretion of antibodies from B cells by at least 5%, at least 10%, at least 15%, at least 20%, at least 25%, at least 30%, at least 35%, at least 40%, at least 45%, at least 50%, at least 55%, at least 60%, at least 60%, at least 65%, at least 70%, at least 75%, at least 80%, at least 85%, at least 90%, at least 95%, or at least 100% or more as compared to untreated B cells. In some embodiments, compositions of the invention can inhibit or reduce the production and/or secretion of antibodies from B cells by at least 2 fold, at least 3 fold, at least 4 fold, at least 5 fold, at least 10 fold, at least 15 fold, at least 20 fold, or at least 50 fold or more as compared to untreated B cells.

Eosinophils are leukocytes that are involved in fighting injections by parasites. Also, Eosinophils are involved in the mechanisms associated with allergy and asthma. Eosinophils are responsive to cytokines such as IL-3, IL-5 and GM-CSF. Following activation, Eosinophils produce and secrete a number of immune system mediators such as reactive oxygen species (such as, but not limited to superoxide), lipid mediators (such as, but not limited to leukotrienes (LTB₄, LTC₄, LTD₄, LTE₄) prostaglandins (PGE₂, Thromboxane A₂)), enzymes (such as elastase), growth factors (such as, but not limited to TGF beta, VEGF, and PDGF) and cytokines (including, but not limited to IL-1, IL-2, IL-4, IL-5, IL-6, IL-8, IL-13 and TNF-alpha). In some embodiments, compositions of the invention are capable of inhibiting or reducing the production and/or secretion of immune system mediators from activated eosinophils. In some embodiments compositions of the invention are capable of inhibiting or reducing the production and/or secretion of immune system mediated from activated eosinophils by at least 5%, at least 10%, at least 15%, at least 20%, at least 25%, at least 30%, at least 35%, at least 40%, at least 45%, at least 50%, at least 55%, at least 60%, at least 60%, at least 65%, at least 70%, at least 75%, at least 80%, at least 85%, at least 90%, at least 95%, or at least 100% or more as compared to untreated activated eosinophils. In some embodiments compositions of the invention are capable of inhibiting or reducing the production and/or secretion of immune system mediators from activated eosinophils by at least 2 fold, at least 3 fold, at least 4 fold, at least 5 fold, at least 10 fold, at least 15 fold, at least 20 fold, or at least 50 fold or more as compared to untreated activated eosinophils.

Basophils are leukocytic cells which represent an important source of histamine and II-4. Upon activation, large cytoplasmic granules containing histamine, heparin, condroitin, elastase, lysophospholipase and various leukotrienes (such as, but not limited to LTB₄, LTC₄, LTD₄, LTE₄) and several cytokines (including, but not limited to IL-1, IL-2, IL-4, IL-5, IL-6, IL-8, IL-13 and TNF-alpha). In some embodiments, compositions of the invention can inhibit or reduce the production and/or release of cytoplasmic granules from an activated basophil. In some embodiments compositions of the invention are capable of inhibiting or reducing the production and/or release of cytoplasmic granules from activated basophils by at least5%, at least 10%, at least 15%, at least 20%, at least 25%, at least 30%, at least 35%, at least 40%, at least 45%, at least 50%, at least 55%, at least 60%, at least 60%, at least 65%, at least 70%, at least 75%, at least 80%, at least 85%, at least 90%, at least 95%, or at least 100% or more as compared to untreated activated basophils. In some embodiments compositions of the invention are capable of inhibiting or reducing the production and/or release of cytoplasmic granules from activated basophils by at least 2 fold, at least 3 fold, at least 4 fold, at least 5 fold, at least 10 fold, at least 15 fold, at least 20 fold, or at least 50 fold or more as compared to untreated activated basophils.

Neutrophils become activated during the acute phase of the immune response, particularly in response to bacterial infections. Neutrophils are highly responsive to many different chemokines including but not limited to, fMLP, C5a, LTB4, IL-8, and interferon gamma which trigger their recruitment to the site of inflammation. Once at the infection site, neutrophils rapidly seek out and destroy bacteria and other pathogens. The destruction of target pathogens is accomplished by phagocytosis, and/or reactive oxygen species production. Neutrophils also release an assortment of proteins (such as, but not limited to lactoferrin, cathelicidin, myeloperoxidase, defensin, serine protease, elastase, cathepsin, gelatinase) in a process called degranulation. In some embodiments, compositions of the invention can inhibit or reduce the response of activated neutrophils to chemokines. In some embodiments compositions of the invention are capable of inhibiting or reducing the response of activated neutrophils to chemokines by at least5%, at least 10%, at least 15%, at least 20%, at least 25%, at least 30%, at least 35%, at least 40%, at least 45%, at least 50%, at least 55%, at least 60%, at least 60%, at least 65%, at least 70%, at least 75%, at least 80%, at least 85%, at least 90%, at least 95%, or at least 100% or more as compared to untreated activated neutrophils. In some embodiments compositions of the invention are capable of inhibiting or reducing the response of activated neutrophils to chemokines by at least 2 fold, at least 3 fold, at least 4 fold, at least 5 fold, at least 10 fold, at least 15 fold, at least 20 fold, or at least 50 fold or more as compared to untreated activated neutrophils.

In some embodiments, compositions of the invention can inhibit or reduce the degranulation of activated neutrophils. In some embodiments compositions of the invention are capable of inhibiting or reducing the degranulation of activated neutrophils by at least5%, at least 10%, at least 15%, at least 20%, at least 25%, at least 30%, at least 35%, at least 40%, at least 45%, at least 50%, at least 55%, at least 60%, at least 60%, at least 65%, at least 70%, at least 75%, at least 80%, at least 85%, at least 90%, at least 95%, or at least 100% or more as compared to untreated activated neutrophils. In some embodiments compositions of the invention are capable of inhibiting or reducing the degranulation of activated neutrophils to chemokines by at least 2 fold, at least 3 fold, at least 4 fold, at least 5 fold, at least 10 fold, at least 15 fold, at least 20 fold, or at least 50 fold or more as compared to untreated activated neutrophils.

Mast cells play an important role in the inflammatory process. When activated, mast cells rapidly release characteristic granules and various hormonal mediators. Mast cells may be stimulated to degranulate by direct injury, cross-linking of IgE receptors, or by activated complement proteins. The mast cell granules contain immune system modulators such as, but not limited to histamine, heparin, serine proteases, prostaglandins, leukotrienes and other cytokines. In one embodiment, compositions of the invention are capable of inhibiting or reducing mast cell degranulation. In some embodiments, compositions of the invention are capable of inhibiting or reducing mast cell degranulation by at least5%, at least 10%, at least 15%, at least 20%, at least 25%, at least 30%, at least 35%, at least 40%, at least 45%, at least 50%, at least 55%, at least 60%, at least 60%, at least 65%, at least 70%, at least 75%, at least 80%, at least 85%, at least 90%, at least 95%, or at least 100% or more as compared to untreated mast cells. In some embodiments, compositions of the invention are capable of inhibiting or reducing mast cell degranulation by at least 2 fold, at least 3 fold, at least 4 fold, at least 5 fold, at least 10 fold, at least 15 fold, at least 20 fold, or at least 50 fold or more as compared to untreated mast cells.

Macrophages are cells within tissues derived from circulating blood monocytes. Their role is to phagocytose cellular debris and pathogens and to stimulate other lymphocytes and other immune cells to respond to the pathogen. Macrophages are also designated based on their location. For example, macrophages in the liver are called Kupffer cells, while macrophages in the bone are known as osteoclasts. Other defined groups of macrophages include Dust cells (lung alveoli), Histiocytes (connective tissue), Microglial cells (neural tissue), and Sinosoidal lining cells (spleen). Macrophages are responsive to hypoxic conditions and are thought to promote chronic inflammation. In some embodiments, compositions of the invention inhibit or reduce the phagocytic activity of macrophages. In some embodiments, compositions of the invention inhibit or reduce the phagocytic capacity of macrophages by at least5%, at least 10%, at least 15%, at least 20%, at least 25%, at least 30%, at least 35%, at least 40%, at least 45%, at least 50%, at least 55%, at least 60%, at least 60%, at least 65%, at least 70%, at least 75%, at least 80%, at least 85%, at least 90%, at least 95%, or at least 100% or more as compared to untreated macrophages. In some embodiments, compositions of the invention inhibit or reduce the phagocytic capacity of macrophages by at least 2 fold, at least 3 fold, at least 4 fold, at least 5 fold, at least 10 fold, at least 15 fold, at least 20 fold, or at least 50 fold or more as compared to untreated macrophages.

In another embodiment, the invention comprises compositions capable of inhibiting or reducing angiogenesis. In another embodiment, the angiogenesis is related to tumor growth, rheumatoid arthritis, SLE, Sjogren's syndrome or others.

The invention also provides methods of using the epitope binding proteins to inactivate various infectious agents such as viruses, fungi, eukaryotic microbes, and bacteria. In some embodiments the epitope binding proteins of the invention may be used to inactivate RSV, hMPV, PIV, or influenza viruses. In other embodiments, the epitope binding proteins of the invention may be used to inactivate fungal pathogens, such as, but not limited to members of *Naegleria, Aspergillus, Blastomyces, Histoplasma, Candida* or *Tinea* genera. In other embodiments, the epitope binding proteins of the invention may be used to inactivate eukaryotic microbes, such as, but not limited to members of *Giardia, Toxoplasma, Plasmodium, Trypanosoma,* and *Entamoeba* genera. In other embodiments, the epitope binding proteins of the invention may be used to inactivate bacterial pathogens, such as but not limited to members of *Staphylococcus, Streptococcus, Pseudomonas, Clostridium, Borrelia, Vibro* and *Neiserria* genera.

The proteins of the invention and compositions comprising the same are useful for many purposes, for example, as therapeutics against a wide range of chronic and acute diseases and disorders including, but not limited to, infectious disease, including viral, bacterial and fungal diseases. Examples of viral pathogens include but are not limited to: adenovirdiae (*e.g*., mastadenovirus and aviadenovirus), herpesviridae (*e.g*., herpes simplex virus 1, herpes simplex virus 2, herpes simplex virus 5, and herpes simplex virus 6), leviviridae (*e.g*., levivirus, enterobacteria phase MS2, allolevirus), poxviridae (*e.g.,* chordopoxvirinae, parapoxvirus, avipoxvirus, capripoxvirus, leporiipoxvirus, suipoxvirus, molluscipoxvirus, and entomopoxvirinae), papovaviridae (*e.g*., polyomavirus and papillomavirus), paramyxoviridae (*e.g*., paramyxovirus, parainfluenza virus 1, mobillivims (*e.g*., measles virus), rubulavirus (*e.g*., mumps virus), pneumonovirinae (*e.g*., pneumovirus, human respiratory synctial virus), and metapneumovirus (*e.g*., avian pneumovirus and human metapneumovirus)), picornaviridae (*e.g*., enterovirus, rhinovirus, hepatovirus (*e.g.*, human hepatitis A virus), cardiovirus, and apthovirus), reoviridae (*e.g*., orthoreovirus, orbivirus, rotavirus, cypovirus, fijivirus, phytoreovirus, and oryzavirus), retroviridae (*e.g*., mammalian type B retroviruses, mammalian type C retroviruses, avian type C retroviruses, type D retrovirus group, BLV-HTLV retroviruses, lentivirus (*e.g.* human immunodeficiency virus 1 and human immunodeficiency virus 2), spumavirus), flaviviridae (*e.g.*, hepatitis C virus), hepadnaviridae (*e.g.*, hepatitis B virus), togaviridae (*e.g*., alphavirus *(e.g.,* sindbis virus) and rubivirus (*e.g*., rubella virus)), rhabdoviridae (*e.g.,* vesiculovirus, lyssavirus, ephemerovirus, cytorhabdovirus, and necleorhabdovirus), arenaviridae (*e.g.,* arenavirus, lymphocytic choriomeningitis virus, Ippy virus, and lassa virus), and coronaviridae (*e.g.,* coronavirus and torovirus). Examples of bacterial pathogens include but are not limited to: but not limited to, the *Aquaspirillum* family, *Azospirillum* family, *Azotobacteraceae* family, *Bacteroidaceae* family, *Bartonella* species, *Bdellovibrio* family, *Campylobacter* species, *Chlamydia* species (*e.g., Chlamydia pneumoniae*)*,* clostridium, *Enterobacteriaceae* family (*e.g., Citrobacter* species, Edwardsiella, *Enterobacter aerogenes, Erwinia* species, *Escherichia coli,* Hafnia species, *Klebsiella* species, Morganella species, *Proteus vulgaris,* Providencia, *Salmonella* species, *Serratia marcescens,* and *Shigella flexneri*), *Gardinella* family, *Haemophilus influenzae, Halobacteriaceae* family, *Helicobacter* family, *Legionallaceae* family, *Listeria* species, *Methylococcaceae* family, mycobacteria (*e.g., Mycobacterium tuberculosis*)*, Neisseriaceae* family, *Oceanospirillum* family, *Pasteurellaceae* family, *Pneumococcus* species, *Pseudomonas* species, *Rhizobiaceae* family, *Spirillum* family, *Spirosomaceae* family, *Staphylococcuss* (*e.g.,* methicillin resistant *Staphylococcus aureus* and *Staphylococcus pyrogenes), Streptococcus* (*e.g., Streptococcus enteritidis, Streptococcus fasciae,* and *Streptococcus pneumoniae*)*, Vampirovibr Helicobacter* family, and *Vampirovibrio* family. Examples of fungal pathogens include, but are not limited to: *Absidia* species (*e.g., Absidia corymbifera and Absidia ramosa*)*, Aspergillus* species, (*e.g., Aspergillus flavus, Aspergillus fumigatus, Aspergillus nidulans, Aspergillus niger,* and *Aspergillus terreus*)*, Basidiobolus ranarum, Blastomyces dermatitidis, Candida* species (*e.g*., *Candida albicans, Candida glabrata, Candida kerr, Candida krusei, Candida parapsilosis, Candida pseudotropicalis, Candida quillermondii, Candida rugosa, Candida stellatoidea,* and *Candida tropicalis*), *Coccidioides immitis, Conidiobolus* species, *Cryptococcus neoforms, Cunninghamella* species, dermatophytes, *Histoplasma capsulatum, Microsporum gypseum, Mucor pusillus, Paracoccidioides brasiliensis, Pseudallescheria boydii, Rhinosporidium seeberi, Pneumocystis carinii, Rhizopus species* (*e.g*., *Rhizopus arrhizus, Rhizopus oryzae, and Rhizopus microsporus*)*, Saccharomyces* species, *Sporothrix schenckii,* zygomycetes, and classes such as *Zygomycetes, Ascomycetes,* the *Basidiomycetes, Deuteromycetes,* and *Oomycetes.*

In another embodiment, the invention provides methods for preventing, managing, treating or ameliorating cancer, autoimmune, inflammatory or infectious diseases or one or more symptoms thereof, said methods comprising administering to a subject in need thereof a dose of a prophylactically or therapeutically effective amount of one or more epitope binding proteins of the invention in combination with surgery, alone or in further combination with the administration of a standard or experimental chemotherapy, a hormonal therapy, a biological therapy/immunotherapy and/or a radiation therapy. In accordance with these embodiments, the epitope binding proteins of the invention utilized to prevent, manage, treat or ameliorate cancer, autoimmune, inflammatory or infectious diseases or one or more symptoms or one or more symptoms thereof may or may not be conjugated or fused to a moiety (e.g., therapeutic agent or drug).

The invention provides methods for preventing, managing, treating or ameliorating cancer, autoimmune, inflammatory or infectious diseases or one or more symptoms or one or more symptoms thereof, said methods comprising administering to a subject in need thereof one or more epitope binding proteins of the invention in combination with one or more of therapeutic agents that are not cancer therapeutics (a.k.a., non-cancer therapies). Examples of such agents include, but are not limited to, anti-emetic agents, anti-fungal agents, anti-bacterial agents, such as antibiotics, anti-inflammatory agents, and anti-viral agents. Non-limiting examples of anti-emetic agents include metopimazin and metochlopramide. Non-limiting examples of antifungal agents include azole drugs, imidazole, triazoles, polyene, amphotericin and ryrimidine. Non-limiting examples of anti-bacterial agents include dactinomycin, bleomycin, erythromycin, penicillin, mithramycin, cephalosporin, imipenem, axtreonam, vancomycin, cycloserine, bacitracin, chloramphenicol, clindamycin, tetracycline, streptomycin, tobramycin, gentamicin, amikacin, kanamycin, neomycin, spectinomycin, trimethoprim, norfloxacin, refampin, polymyxin, amphotericin B, nystatin, ketocanazole, isoniazid, metronidazole and pentamidine. Non-limiting examples of antiviral agents include nucleoside analogs (e.g., zidovudine, acyclivir, gangcyclivir, vidarbine, idoxuridine, trifluridine and ribavirin), foscaret, amantadine, rimantadine, saquinavir, indinavir, ritonavir, interferon ("IFN")-α,β or γ and AZT. Non-limiting examples of anti-inflammatory agents include non-steroidal anti-inflammatory drugs ("NSAIDs"), steroidal anti-inflammatory drugs, beta-agonists, anti-cholingenic agents and methylxanthines.

In another embodiment, the invention comprises compositions capable of inhibiting a cancer cell phenotype. In one embodiment, the cancer cell phenotype is cell growth, cell attachment, loss of cell attachment, decreased receptor expression (e.g Eph), increased receptor expression (e.g Eph), metastatic potential, cell cycle inhibition, receptor tyrosine kinase activation/inhibition or others.

In one embodiment, the invention comprises compositions capable of treating chronic inflammation. In one embodiment, the compositions are useful in the targeting of immune cells for destruction or deactivation. In one embodiment, the compositions are useful in targeting activated T cells, dormant T cells, B cells, neutrophils, eosinophils, basophils, mast cells, or dendritic cells. In another embodiment, the invention comprises compositions capable of decreasing immune cell function. In another embodiment, the compositions are capable of ablating immune cell function.

The invention also provides methods of using epitope binding proteins as diagnostic reagents. The proteins of the invention may be useful in kits or reagents where different antigens need to be efficiently captured concurrently.

### H. Generation of Polynucleotides Encoding Multispecific Epitope Binding Proteins Of The Invention

The invention further provides polynucleotides comprising a nucleotide sequence encoding a multispecific epitope binding protein of the invention and fragments thereof. The invention further provides polynucleotides comprising a nucleotide sequence encoding polypeptide chains of the invention and fragments thereof. The invention also encompasses polynucleotides that hybridize under stringent or lower stringency hybridization conditions, e.g., as defined herein, to polynucleotides that encode an epitope binding protein and/or polypeptide chains of the invention.

A polynucleotide encoding an epitope binding protein may be generated from nucleic acid from a suitable source. For instance, if a clone containing a nucleic acid encoding a particular epitope binding protein is not available, but the sequence of the epitope binding protein molecule is known, a nucleic acid encoding the protein may be chemically synthesized or obtained from a suitable source (*e.g*., a cDNA library, or a cDNA library generated from, or nucleic acid, preferably polyA+RNA, isolated from, any tissue or cells expressing the epitope binding protein, such as hybridoma cells selected to express a protein of the invention) by PCR amplification using synthetic primers hybridizable to the 3' and 5' ends of the sequence or by cloning using an oligonucleotide probe specific for the particular gene sequence to identify, e.g., a cDNA clone from a cDNA library that encodes the protein. Amplified nucleic acids generated by PCR may then be cloned into replicable cloning vectors using any method known in the art.

Once the nucleotide sequence and corresponding amino acid sequence of the epitope binding protein is determined, the nucleotide sequence of the epitope binding protein may be manipulated using methods known in the art for the manipulation of nucleotide sequences, *e.g.*, recombinant DNA techniques, site directed mutagenesis, PCR, etc. (see, for example, the techniques described in Sambrook et al., 1990, Molecular Cloning, A Laboratory Manual, 2d Ed., Cold Spring Harbor Laboratory, Cold Spring Harbor, N.Y. and Ausubel et al., eds., 1998, Current Protocols in Molecular Biology, John Wiley & Sons, NY), to generate epitope binding protein having a different amino acid sequence, for example to create amino acid substitutions, deletions, and/or insertions.

In a specific embodiment, the amino acid sequence of the heavy and/or light chain variable domains of the epitope binding protein of the invention may be inspected to identify the sequences of the complementarity determining regions (CDRs) by methods that are known in the art, *e.g.,* by comparison to known amino acid sequences of other heavy and light chain variable regions to determine the regions of sequence hypervariability. Using routine recombinant DNA techniques, one or more of the CDRs may be inserted within framework regions, e.g., into human framework regions to humanize a non-human antibody. The framework regions may be naturally occurring or consensus framework regions, and preferably human framework regions (see, *e.g.*, Chothia et al., J. Mol. Biol. 278: 457-479 (1998) for a listing of human framework regions). Preferably, the polynucleotide generated by the combination of the framework regions and CDRs encodes an epitope binding protein of the invention.

Preferably, one or more amino acid substitutions may be made within the framework regions, and, preferably, the amino acid substitutions improve binding of the epitope binding protein to its antigen. Other alterations to the polynucleotide are encompassed by the present invention and within the skill of the art.

### I. Multispecific epitope binding protein conjugates and derivatives

The proteins of the invention include derivatives that are modified (*e.g*., by the covalent attachment of any type of molecule to the protein). For example, but not by way of limitation, the derivatives include multispecific epitope binding proteins of the invention that have been modified, *e.g.,* by glycosylation, acetylation, pegylation, phosphorylation, amidation, derivatization by known protecting/blocking groups, proteolytic cleavage, linkage to a cellular ligand or other protein, etc. Any of numerous chemical modifications may be carried out by known techniques, including, but not limited to, specific chemical cleavage, acetylation, formylation, metabolic synthesis of tunicamycin, etc. Additionally, the derivative may contain one or more non-classical amino acids.

The epitope binding proteins of the invention or fragments thereof can be fused to marker sequences, such as a peptide to facilitate purification. In certain embodiments, the marker amino acid sequence is a hexa-histidine peptide, such as the tag provided in a pQE vector (QIAGEN, Inc., 9259 Eton Avenue, Chatsworth, CA, 91311), among others, many of which are commercially available. As described in Gentz et al., 1989, Proc. Natl. Acad. Sci. USA 86:821-824, for instance, hexa-histidine provides for convenient purification of the fusion protein. Other peptide tags useful for purification include, but are not limited to, the hemagglutinin "HA" tag, which corresponds to an epitope derived from the influenza hemagglutinin protein (Wilson et al., 1984, Cell 37:767) and the "flag" tag.

The present invention further encompasses multispecific epitope binding proteins conjugated to a diagnostic or therapeutic agent. The proteins can be used diagnostically to, for example, monitor the development or progression of a tumor as part of a clinical testing procedure to, *e.g.*, determine the efficacy of a given treatment regimen. Detection can be facilitated by coupling the antibody to a detectable substance. Examples of detectable substances include various enzymes, prosthetic groups, fluorescent materials, luminescent materials, bioluminescent materials, radioactive materials, positron emitting metals using various positron emission tomographies, and nonradioactive paramagnetic metal ions. The detectable substance may be coupled or conjugated either directly to the antibody (or fragment thereof) or indirectly, through an intermediate (such as, for example, a linker known in the art) using techniques known in the art. See, for example, U.S. Pat. No. 4,741,900 for metal ions which can be conjugated to antibodies for use as diagnostics according to the present invention. Examples of suitable enzymes include horseradish peroxidase, alkaline phosphatase, beta-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin; and examples of suitable radioactive material include but are not limited to, ¹²⁵I, ¹³¹I, ¹¹¹In or ⁹⁹Tc, in addition positron emitting metals using various positron emission tomographies, nonradioactive paramagnetic metal ions, and molecules that are radiolabelled or conjugated to specific radioisotopes can be conjugated to the proteins of the invention.

Further, a multispecific epitope binding protein of the invention may be conjugated to a therapeutic moiety such as a cytotoxin, *e.g.,* a cytostatic or cytocidal agent, a therapeutic agent or a radioactive metal ion, *e.g.*, alpha-emitters such as, for example, ²¹³ Bi. A cytotoxin or cytotoxic agent includes any agent that is detrimental to cells. Examples include paclitaxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents include, but are not limited to, antimetabolites (e.g., methotrexate, 6-mercaptopurine, 6-thioguanine, cytarabine, 5-fluorouracil decarbazine), alkylating agents (*e.g*., mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum(II) (DDP) cisplatin), anthracyclines (*e.g.,* daunorubicin (formerly daunomycin) and doxorubicin), antibiotics (*e.g*., dactinomycin (formerly actinomycin), bleomycin, mithramycin, and anthramycin (AMC)), and anti-mitotic agents (*e.g*., vincristine and vinblastine). A more extensive list of therapeutic moieties can be found in PCT publications WO 03/075957.

The conjugates of the invention can be used for modifying a given biological response, the therapeutic agent or drug moiety is not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a protein such as tumor necrosis factor, alpha-interferon, beta-interferon, nerve growth factor, platelet derived growth factor, tissue plasminogen activator, an apoptotic agent, *e.g.,* TNF-alpha, TNF-beta, AIM I (See, International Publication No. WO 97/33899), AIM II (See, International Publication No. WO 97/34911), Fas Ligand (Takahashi et al., Int. Immunol., 6:1567-1574 (1994)), VEGI (See, International Publication No. WO 99/23105), CD40 Ligand, a thrombotic agent or an anti-angiogenic agent, *e.g.*, angiostatin or endostatin; or, biological response modifiers such as, for example, lymphokines, interleukin-1 ("IL-1"), interleukin-2 ("IL-2"), interleukin-6 ("IL-6"), granulocyte macrophage colony stimulating factor ("GM-CSF"), granulocyte colony stimulating factor ("G-CSF"), or other growth factors.

### J. Multispecific epitope binding protein carrier molecules

The multispecific epitope binding proteins of the invention may also be used to deliver a payload to cells. For example, a multispecific epitope binding protein may be loaded with a cargo, such as, but not limited to a cytotoxic drug, in an effort to deliver the drug to a particular cell population. Such techniques have been developed for monoclonal antibodies and require a covalent modification to the antibody, usually at an unpaired cysteine, for conjugation of a drug. These chemical modifications are cumbersome, inefficient and often destabilize the antibody. Also, there is limited control on how many drug molecules are attached to a single antibody, giving rise to variability in production. Multispecific epitope binding proteins of the invention may be engineered to deliver cargos to cells in an efficient and predictable manner. It is envisioned that the multispecific epitope binding protein may comprise epitope binding domains that are specific for a cargo, or payload, suitable to be delivered to a cell. Examples of such epitope binding domains have been described before, such as antibodies against Taxol (Leu et al. 1993. Cancer Research Mar 15;53(6):1388-91) and Doxorubicin (Morelli et al. 1996 Cancer Research May 1 ;56(9);2082-5). These multispecific epitope binding protein carrier molecules could be loaded with at least one cargo bound to a epitope binding domain, and administered to a patient.

In one embodiment, multispecific epitope binding proteins of the invention may be used to deliver a cargo molecule to a cell. In other embodiments, multispecific epitope binding proteins of the invention comprise at least one epitope binding domain specific for a cargo molecule to be delivered to a cell. In other embodiments, multispecific epitope binding proteins of the invention comprise multiple epitope binding domains specific for the same cargo molecule to be delivered. In other embodiments, multispecific epitope binding proteins of the invention comprise multiple epitope binding domains specific for different cargo molecules to be delivered.

In some embodiments, the cargo molecule to be delivered is a cytotoxic drug, an anti-metabolite, a toxin, a peptide, a DNA molecule, an RNA molecule, a small molecule, a radioisotope, a fluorophore, an enzyme, an enzyme inhibitor, a prodrug, or a mitochondrial poison. In other embodiments, the cargo specific epitope binding domains may mask the active site/region of the cargo molecule prior to delivery to the cell. In other embodiments, the cargo specific epitope binding domains reversible release the cargo molecule upon internalization.

In some embodiments, the cargo specific epitope binding domains exhibit pH dependence for binding the cargo molecule. In some embodiments, the cargo specific epitope binding domains bind the cargo molecule at physiological pH, such as that found in blood, yet do not bind the cargo molecule at lysosomal pH (around pH 6.0).

In some embodiments, at least one epitope binding domain may be specific for a linker moiety, useful for conjugation to a variety of targets. In such a scenario, the user may "custom fit" the linker moiety with the cargo of choice and use the linker specific epitope binding domain to deliver the custom cargo to the cell. Such approaches for traditional antibodies have been described previously in U.S. Patent No. 6,962,702, granted Nov 8, 2005 and which is hereby incorporated by reference in its entirety.

### K. Assays For Epitope Binding and Activity

The multispecific epitope binding proteins of the invention may be assayed for specific (*i.e*., immunospecific) binding by any method known in the art. The immunoassays which can be used, include but are not limited to, competitive and non-competitive assay systems using techniques such as western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, protein A immunoassays, to name but a few. Such assays are routine and known in the art (see, e.g., Ausubel et al, eds, 1994, Current Protocols in Molecular Biology, Vol. 1, John Wiley & Sons, Inc., New York). Exemplary immunoassays are described briefly below (but are not intended by way of limitation).

Immunoprecipitation protocols generally comprise lysing a population of cells in a lysis buffer such as RIPA buffer (1% NP-40 or Triton X-100, 1% sodium deoxycholate, 0.1 % SDS, 0.15 M NaCl, 0.01 M sodium phosphate at pH 7.2, 1% Trasylol) supplemented with protein phosphatase and/or protease inhibitors (e.g., EDTA, PMSF, aprotinin, sodium vanadate), adding the epitope binding protein of interest to the cell lysate, incubating for a period of time (*e.g*., 1-4 hours) at 4°C, adding protein A and/or protein G sepharose beads to the cell lysate, incubating for about an hour or more at 4°C, washing the beads in lysis buffer and resuspending the beads in SDS/sample buffer. The ability of the protein of interest to immunoprecipitate a particular antigen can be assessed by, *e.g.,* western blot analysis. One of skill in the art would be knowledgeable as to the parameters that can be modified to increase the binding of the epitope binding protein to an antigen and decrease the background (*e.g.,* pre-clearing the cell lysate with sepharose beads). For further discussion regarding immunoprecipitation protocols see, *e.g.,* Ausubel et al, eds, 1994, Current Protocols in Molecular Biology, Vol. 1, John Wiley & Sons, Inc., New York at 10.16.1.

Western blot analysis generally comprises preparing protein samples, electrophoresis of the protein samples in a polyacrylamide gel (*e.g*., 8%-20% SDS-PAGE depending on the molecular weight of the antigen), transferring the protein sample from the polyacrylamide gel to a membrane such as nitrocellulose, PVDF or nylon, blocking the membrane in blocking solution (*e.g*., PBS with 3% BSA or non-fat milk), washing the membrane in washing buffer (*e.g*., PBS-Tween 20), blocking the membrane with primary antibody diluted in blocking buffer, washing the membrane in washing buffer, blocking the membrane with a secondary antibody (which recognizes the primary antibody) conjugated to an enzymatic substrate (*e.g*., horseradish peroxidase or alkaline phosphatase) or radioactive molecule (*e.g.*, ³²P or ¹²⁵I) diluted in blocking buffer, washing the membrane in wash buffer, and detecting the presence of the antigen. One of skill in the art would be knowledgeable as to the parameters that can be modified to increase the signal detected and to reduce the background noise. For further discussion regarding western blot protocols see, *e.g.,* Ausubel et al, eds, 1994, Current Protocols in Molecular Biology, Vol. 1, John Wiley & Sons, Inc., New York at 10.8.1.

ELISAs comprise preparing antigen, coating the well of a 96 well microtiter plate with the antigen, adding the epitope binding protein of interest conjugated to a detectable compound such as an enzymatic substrate (*e.g*., horseradish peroxidase or alkaline phosphatase) to the well and incubating for a period of time, and detecting the presence of the antigen. In ELISAs the epitope binding protein of interest does not have to be conjugated to a detectable compound; instead, a second antibody (which recognizes the protein of interest) conjugated to a detectable compound may be added to the well. Further, instead of coating the well with the antigen, the protein of interest may be coated to the well. In this case, a second antibody conjugated to a detectable compound may be added following the addition of the antigen of interest to the coated well. One of skill in the art would be knowledgeable as to the parameters that can be modified to increase the signal detected as well as other variations of ELISAs known in the art. For further discussion regarding ELISAs see, *e.g.*, Ausubel et al, eds, 1994, Current Protocols in Molecular Biology, Vol. 1, John Wiley & Sons, Inc., New York at 11.2.1.

The binding affinity and other binding properties of an epitope binding protein to an antigen may be determined by a variety *of in vitro* assay methods known in the art including for example, equilibrium methods (*e.g*., enzyme-linked immunoabsorbent assay (ELISA; or radioimmunoassay (RIA)), or kinetics *(e.g.**,*** BIACORE® analysis), and other methods such as indirect binding assays, competitive binding assays fluorescence resonance energy transfer (FRET), gel electrophoresis and chromatography *(e.g.**,*** gel filtration). These and other methods may utilize a label on one or more of the components being examined and/or employ a variety of detection methods including but not limited to chromogenic, fluorescent, luminescent, or isotopic labels. A detailed description of binding affinities and kinetics can be found in Paul, W.E., ed., Fundamental Immunology, 4th Ed., Lippincott-Raven, Philadelphia (1999), which focuses on antibody-immunogen interactions. One example of a competitive binding assay is a radioimmunoassay Comprising the incubation of labeled antigen with the epitope binding protein of interest in the presence of increasing amounts of unlabeled antigen, and the detection of the epitope binding protein bound to the labeled antigen. The affinity of the epitope binding protein of interest for a particular antigen and the binding off-rates can be determined from the data by scatchard plot analysis. Competition with a second antibody can also be determined using radioimmunoassays. In this case, the antigen is incubated with epitope binding protein of interest conjugated to a labeled compound in the presence of increasing amounts of an unlabeled second antibody.

### L. Variant Fc regions

The invention also provides multispecific epitope binding proteins with altered Fc regions (also referred to herein as "variant Fc regions"). Accordingly, in one embodiment of the invention, polypeptide chains of the invention comprise a variant Fc region (i.e., Fc regions that have been altered as discussed below). Polypeptide chain(s) of the invention comprising a variant Fc region are also referred to here as "Fc variant protein(s)."

In the description of variant Fc regions, it is understood that the Fc regions of the multispecific epitope binding proteins of the invention comprise the numbering scheme according to the EU index as in Kabat et al. (1991, NIH Publication 91-3242, National Technical Information Service, Springfield, VA).

The present invention encompasses Fc variant proteins which have altered binding properties for an Fc ligand (e.g., an Fc receptor, C1q) relative to a comparable molecule (e.g., a protein having the same amino acid sequence except having a wild type Fc region). Examples of binding properties include but are not limited to, binding specificity, equilibrium dissociation constant (K_{D}), dissociation and association rates (k_{off} and kₒₙ respectively), binding affinity and/or avidity. It is generally understood that a binding molecule (e.g., a Fc variant protein such as an antibody) with a low K_{D} may be preferable to a binding molecule with a high K_{D}. However, in some instances the value of the kₒₙ or k_{off} may be more relevant than the value of the K_{D}. One skilled in the art can determine which kinetic parameter is most important for a given epitope binding protein application.

The affinities and binding properties of an Fc region for its ligand may be determined by a variety of in vitro assay methods (biochemical or immunological based assays) known in the art for determining Fc-FcγR interactions, i.e., specific binding of an Fc region to an FcγR including but not limited to, equilibrium methods (e.g., enzyme-linked immunoabsorbent assay (ELISA), or radioimmunoassay (RIA)), or kinetics (e.g., BIACORE® analysis), and other methods such as indirect binding assays, competitive inhibition assays, fluorescence resonance energy transfer (FRET), gel electrophoresis and chromatography (e.g., gel filtration). These and other methods may utilize a label on one or more of the components being examined and/or employ a variety of detection methods including but not limited to chromogenic, fluorescent, luminescent, or isotopic labels. A detailed description of binding affinities and kinetics can be found in Paul, W.E., ed., Fundamental Immunology, 4th Ed., Lippincott-Raven, Philadelphia (1999), which focuses on antibody-immunogen interactions.

In one embodiment, the Fc variant protein has enhanced binding to one or more Fc ligand relative to a comparable molecule. In another embodiment, the Fc variant protein has an affinity for an Fc ligand that is at least 2 fold, or at least 3 fold, or at least 5 fold, or at least 7 fold, or a least 10 fold, or at least 20 fold, or at least 30 fold, or at least 40 fold, or at least 50 fold, or at least 60 fold, or at least 70 fold, or at least 80 fold, or at least 90 fold, or at least 100 fold, or at least 200 fold greater than that of a comparable molecule. In a specific embodiment, the Fc variant protein has enhanced binding to an Fc receptor. In another specific embodiment, the Fc variant protein has enhanced binding to the Fc receptor FcγRIIIA. In a further specific embodiment, the Fc variant protein has enhanced biding to the Fc receptor FcγRIIB. In still another specific embodiment, the Fc variant protein has enhanced binding to the Fc receptor FcRn. In yet another specific embodiment, the Fc variant protein has enhanced binding to C1q relative to a comparable molecule.

The serum half-life of proteins comprising Fc regions may be increased by increasing the binding affinity of the Fc region for FcRn. In one embodiment, the Fc variant protein has enhanced serum half life relative to comparable molecule.

The ability of any particular Fc variant protein to mediate lysis of the target cell by ADCC can be assayed. To assess ADCC activity an Fc variant protein of interest is added to target cells in combination with immune effector cells, which may be activated by the antigen antibody complexes resulting in cytolysis of the target cell. Cytolysis is generally detected by the release of label (e.g. radioactive substrates, fluorescent dyes or natural intracellular proteins) from the lysed cells. Useful effector cells for such assays include peripheral blood mononuclear cells (PBMC) and Natural Killer (NK) cells. Specific examples of in vitro ADCC assays are described in Wisecarver et al., 1985 79:277-282; Bruggemann et al., 1987, J Exp Med 166:1351-1361; Wilkinson et al., 2001, J Immunol Methods 258:183-191; Patel et al., 1995 J Immunol Methods 184:29-38. ADCC activity of the Fc variant protein of interest may also be assessed in vivo, e.g., in a animal model such as that disclosed in Clynes et al., 1998, Proc. Natl. Acad. Sci. USA 95:652-656.

In one embodiment, an Fc variant protein has enhanced ADCC activity relative to a comparable molecule. In a specific embodiment, an Fc variant protein has ADCC activity that is at least 2 fold, or at least 3 fold, or at least 5 fold or at least 10 fold or at least 50 fold or at least 100 fold greater than that of a comparable molecule. In another specific embodiment, an Fc variant protein has enhanced binding to the Fc receptor FcγRIIIA and has enhanced ADCC activity relative to a comparable molecule. In other embodiments, the Fc variant protein has both enhanced ADCC activity and enhanced serum half life relative to a comparable molecule.

In one embodiment, an Fc variant protein has reduced ADCC activity relative to a comparable molecule. In a specific embodiment, an Fc variant protein has ADCC activity that is at least 2 fold, or at least 3 fold, or at least 5 fold or at least 10 fold or at least 50 fold or at least 100 fold lower than that of a comparable molecule. In another specific embodiment, an Fc variant protein has reduced binding to the Fc receptor FcγRIIIA and has reduced ADCC activity relative to a comparable molecule. In other embodiments, the Fc variant protein has both reduced ADCC activity and enhanced serum half life relative to a comparable molecule.

In one embodiment, an Fc variant protein has enhanced CDC activity relative to a comparable molecule. In a specific embodiment, an Fc variant protein has CDC activity that is at least 2 fold, or at least 3 fold, or at least 5 fold or at least 10 fold or at least 50 fold or at least 100 fold greater than that of a comparable molecule. In other embodiments, the Fc variant protein has both enhanced CDC activity and enhanced serum half life relative to a comparable molecule. In one embodiment, the Fc variant protein has reduced binding to one or more Fc ligand relative to a comparable molecule. In another embodiment, the Fc variant protein has an affinity for an Fc ligand that is at least 2 fold, or at least 3 fold, or at least 5 fold, or at least 7 fold, or a least 10 fold, or at least 20 fold, or at least 30 fold, or at least 40 fold, or at least 50 fold, or at least 60 fold, or at least 70 fold, or at least 80 fold, or at least 90 fold, or at least 100 fold, or at least 200 fold lower than that of a comparable molecule. In a specific embodiment, the Fc variant protein has reduced binding to an Fc receptor. In another specific embodiment, the Fc variant protein has reduced binding to the Fc receptor FcγRIIIA. In a further specific embodiment, an Fc variant described herein has an affinity for the Fc receptor FcγRIIIA that is at least about 5 fold lower than that of a comparable molecule, wherein said Fc variant has an affinity for the Fc receptor FcγRIIB that is within about 2 fold of that of a comparable molecule. In still another specific embodiment, the Fc variant protein has reduced binding to the Fc receptor FcRn. In yet another specific embodiment, the Fc variant protein has reduced binding to C1q relative to a comparable molecule.

In one embodiment, the present invention provides Fc variants, wherein the Fc region comprises a non naturally occurring amino acid residue at one or more positions selected from the group consisting of 234, 235, 236, 237, 238, 239, 240, 241, 243, 244, 245, 247, 251, 252, 254, 255, 256, 262, 263, 264, 265, 266, 267, 268, 269, 279, 280, 284, 292, 296, 297, 298, 299, 305, 313, 316, 325, 326, 327, 328, 329, 330, 331, 332, 333, 334, 339, 341, 343, 370, 373, 378, 392, 416, 419, 421, 440 and 443 as numbered by the EU index as set forth in Kabat. Optionally, the Fc region may comprise a non naturally occurring amino acid residue at additional and/or alternative positions known to one skilled in the art (see, e.g., U.S. Patents 5,624,821; 6,277,375; 6,737,056; PCT Patent Publications WO 01/58957; WO 02/06919; WO 04/016750; WO 04/029207; WO 04/035752; WO 04/074455; WO 04/099249; WO 04/063351; WO 05/070963; WO 05/040217, WO 05/092925 and WO 06/020114).

In a specific embodiment, the present invention provides an Fc variant, wherein the Fc region comprises at least one non naturally occurring amino acid residue selected from the group consisting of 234D, 234E, 234N, 234Q, 234T, 234H, 234Y, 234I, 234V, 234F, 235A, 235D, 235R, 235W, 235P, 235S, 235N, 235Q, 235T, 235H, 235Y, 235I, 235V, 235F, 236E, 239D, 239E, 239N, 239Q, 239F, 239T, 239H, 239Y, 240I, 240A, 240T, 240M, 241W, 241 L, 241Y, 241E, 241 R. 243W, 243L 243Y, 243R, 243Q, 244H, 245A, 247L, 247V, 247G, 251F, 252Y, 254T, 255L, 256E, 256M, 262I, 262A, 262T, 262E, 263I, 263A, 263T, 263M, 264L, 264I, 264W, 264T, 264R, 264F, 264M, 264Y, 264E, 265G, 265N, 265Q, 265Y, 265F, 265V, 265I, 265L, 265H, 265T, 266I, 266A, 266T, 266M, 267Q, 267L, 268E, 269H, 269Y, 269F, 269R, 270E, 280A, 284M, 292P, 292L, 296E, 296Q, 296D, 296N, 296S, 296T, 296L, 296I, 296H, 269G, 297S, 297D, 297E, 298H, 298I, 298T, 298F, 299I, 299L, 299A, 299S, 299V, 299H, 299F, 299E, 305I, 313F, 316D, 325Q, 325L, 325I, 325D, 325E, 325A, 325T, 325V, 325H, 327G, 327W, 327N, 327L, 328S, 328M, 328D, 328E, 328N, 328Q, 328F, 328I, 328V, 328T, 328H, 328A, 329F, 329H, 329Q, 330K, 330G, 330T, 330C, 330L, 330Y, 330V, 330I, 330F, 330R, 330H, 331G, 331A, 331L, 331M, 331F, 331W, 331K, 331Q, 331E, 331S, 331V, 331I, 331C, 331Y, 331H, 331R, 331N, 331D, 331T, 332D, 332S, 332W, 332F, 332E, 332N, 332Q, 332T, 332H, 332Y, 332A, 339T, 370E, 370N, 378D, 392T, 396L, 416G, 419H, 421K, 440Yand 434W as numbered by the EU index as set forth in Kabat. Optionally, the Fc region may comprise additional and/or alternative non naturally occurring amino acid residues known to one skilled in the art (see, e.g., U.S. Patents 5,624,821; 6,277,375; 6,737,056; PCT Patent Publications WO 01/58957; WO 02/06919; WO 04/016750; WO 04/029207; WO 04/035752 and WO 05/040217).

In another embodiment, the present invention provides an Fc variant, wherein the Fc region comprises at least one non-naturally occurring amino acid at one or more positions selected from the group consisting of 239, 330 and 332, as numbered by the EU index as set forth in Kabat. In a specific embodiment, the present invention provides an Fc variant, wherein the Fc region comprises at least one non-naturally occurring amino acid selected from the group consisting of 239D, 330L and 332E, as numbered by the EU index as set forth in Kabat. Optionally, the Fc region may further comprise additional-non naturally occurring amino acid at one or more positions selected from the group consisting of 252, 254, and 256, as numbered by the EU index as set forth in Kabat. In a specific embodiment, the present invention provides an Fc variant, wherein the Fc region comprises at least one non naturally occurring amino acid selected from the group consisting of 239D, 330L and 332E, as numbered by the EU index as set forth in Kabat and at least one non naturally occurring amino acid at one or more positions selected from the group consisting of 252Y, 254T and 256E, as numbered by the EU index as set forth in Kabat.

In another embodiment, the present invention provides an Fc variant, wherein the Fc region comprises at least one non-naturally occurring amino acid at one or more positions selected from the group consisting of 234, 235 and 331, as numbered by the EU index as set forth in Kabat. In a specific embodiment, the present invention provides an Fc variant, wherein the Fc region comprises at least one non-naturally occurring amino acid selected from the group consisting of 234F, 235F, 235Y, and 331S, as numbered by the EU index as set forth in Kabat. In a further specific embodiment, an Fc variant of the invention comprises the 234F, 235F, and 331 S non-naturally occurring amino acid residues, as numbered by the EU index as set forth in Kabat. In another specific embodiment, an Fc variant of the invention comprises the 234F, 235Y, and 331 S non-naturally occurring amino acid residues, as numbered by the EU index as set forth in Kabat. Optionally, the Fc region may further comprise additional non-naturally occurring amino acid at one or more positions selected from the group consisting of 252, 254, and 256, as numbered by the EU index as set forth in Kabat. In a specific embodiment, the present invention provides an Fc variant, wherein the Fc region comprises at least one non naturally occurring amino acid selected from the group consisting of 234F, 235F, 235Y, and 33 1S, as numbered by the EU index as set forth in Kabat; and at least one non naturally occurring amino acid at one or more positions are selected from the group consisting of 252Y, 254T and 256E, as numbered by the EU index as set forth in Kabat.

In other embodiments, the Fc variants of the present invention may be combined with other known Fc variants such as those disclosed in Ghetie et al., 1997, Nat Biotech. 15:637-40; Duncan et al, 1988, Nature 332:563-564; Lund et al., 1991, J. Immunol 147:2657-2662; Lund et al, 1992, Mol Immunol 29:53-59; Alegre et al, 1994, Transplantation 57:1537-1543; Hutchins et al., 1995, Proc Natl. Acad Sci U S A 92:11980-11984; Jefferis et al, 1995, Immunol Lett. 44:111-117; Lund et al., 1995, Faseb J 9:115-119; Jefferis et al, 1996, Immunol Lett 54:101-104; Lund et al, 1996, J Immunol 157:4963-4969; Armour et al., 1999, Eur J Immunol 29:2613-2624; Idusogie et al, 2000, J Immunol 164:4178-4184; Reddy et al, 2000, J Immunol 164:1925-1933; Xu et al., 2000, Cell Immunol 200:16-26; Idusogie et al, 2001, J Immunol 166:2571-2575; Shields et al., 2001, J Biol Chem 276:6591-6604; Jefferis et al, 2002, Immunol Lett 82:57-65; Presta et al., 2002, Biochem Soc Trans 30:487-490); U.S. Patent Nos. 5,624,821; 5,885,573; 5,677,425; 6,165,745; 6,277,375; 5,869,046; 6,121,022; 5,624,821; 5,648,260; 6,528,624; 6,194,551; 6,737,056; 6,821,505; 6,277,375; U.S. Patent Publication Nos. 2004/0002587 and PCT Publications WO 94/29351; WO 99/58572; WO 00/42072; WO 02/060919; WO 04/029207; WO 04/099249; WO 04/063351. Also encompassed by the present invention are Fc regions which comprise deletions, additions and/or modifications. Still other modifications/substitutions/additions/deletions of the Fc domain will be readily apparent to one skilled in the art.

### M. Glycosylation of multispecific epitope binding proteins of the invention

In another embodiment, the glycosylation of epitope binding proteins utilized in accordance with the invention is modified. For example, an aglycoslated epitope binding protein can be made (i.e., the antibody lacks glycosylation). Glycosylation can be altered to, for example, increase the affinity of the epitope binding protein for a target antigen. Such carbohydrate modifications can be accomplished by, for example, altering one or more sites of glycosylation within the protein sequence. Such aglycosylation may increase the affinity of the protein of the invention for its antigen. Such an approach is described in further detail in U.S. Patent Nos. 5,714,350 and 6,350,861. One or more amino acid substitutions can also be made that result in elimination of a glycosylation site present in the Fc region (e.g., Asparagine 297 of IgG). Furthermore, aglycosylated epitope binding proteins may be produced in bacterial cells which lack the necessary glycosylation machinery.

An epitope binding protein of the invention can also be made that has an altered type of glycosylation, such as a hypofucosylated protein having reduced amounts of fucosyl residues or a protein having increased bisecting GlcNAc structures. Such altered glycosylation patterns have been demonstrated to increase the ADCC ability of antibodies. Such carbohydrate modifications can be accomplished by, for example, expressing the protein of the invention in a host cell with altered glycosylation machinery. Cells with altered glycosylation machinery have been described in the art and can be used as host cells in which to express recombinant proteins of the invention to thereby produce a protein with altered glycosylation. See, for example, Shields, R.L. et al. (2002) J. Biol. Chem. 277:26733-26740; Umana et al. (1999) Nat. Biotech. 17:176-1, as well as, U.S. Patent No: US 6,946,292; European Patent No: EP 1,176,195; PCT Publications WO 03/035835; WO 99/54342 each of which is incorporated herein by reference in its entirety.

The presence of diversity is known regarding addition of galactose to the non-reducing end of a complex type N-glycoside-linked sugar chain binding to the Fc region of an antibody IgG molecule and addition of fucose to N-acetylglucosamine in the reducing end [Biochemistry, 36, 130 (1997)], and it has been reported that the ADCC activity of antibodies is greatly reduced particularly by adding fucose to N-acetylglucosamine in the reducing end in sugar chains [WO00/61739, J. Biol. Chem., 278, 3466 (2003)]. Thus, in an effort in maximize potency, multispecific epitope binding proteins with reduced or ablated fucosylation of the Fc region are desired.

In a specific embodiment, the multispecific epitope binding protein of the invention has reduced fucosylation compared to an unmodified multispecific epitope binding protein. In an embodiment, the fucosylation of the multispecific epitope binding protein of the invention is at least 99%, 95%, 90%, 85%, 80%, 75%, 70%, 65%, 60%, 55%, 50%, 45%, 40%, 35%, 30%, 25%, 20%, 15%, 10%, 5%, 2.5%, or 1% the level of an unmodified multispecific epitope binding protein. In another embodiment, the multispecific epitope binding protein of the invention is afucosylated. In a further embodiment, the afucosylated multispecific epitope binding protein of the invention contains less that 5%, 2.5%, 1%, 0.5%, 0.1 % or 0.05% the level of fucosylation of an unmodified multispecific epitope binding protein.

### N. Manufacture/Production of Multispecific Epitope Binding Proteins

The invention also provides methods of producing the multispecific epitope binding proteins of the invention. The multispecific epitope binding proteins may be expressed from a single vector, or from multiple vectors. The arrangement of the binding domains within the vector can be varied. The orientation of the Fc region (or CH1/Fc region) may be N-terminus or C-terminus to any of the binding domains contained within the multispecific epitope binding polypeptide chain. In some embodiments, the epitope binding domains are present both N-terminal and C-terminal to the Fc region (or CH1/Fc region) within the polypeptide chain.

Once a desired multispecific epitope binding protein is engineered, the protein can be produced on a commercial scale using methods that are known in the art for large scale manufacturing of antibodies. For example, this can be accomplished using recombinant expressing systems such as, but not limited to, those described below.

### N.1. Recombinant expression systems

Recombinant expression of an epitope binding protein of the invention requires construction of an expression vector containing a polynucleotide that encodes the epitope binding protein of the invention. Once a polynucleotide encoding protein of the invention has been obtained, the vector for the production of the epitope binding molecule may be produced by recombinant DNA technology using techniques well-known in the art. *See, e.g.,* U.S. Patent No. 6,331,415, which is incorporated herein by reference in its entirety. Thus, methods for preparing a protein by expressing a polynucleotide containing an encoding nucleotide sequence are described herein. The multispecific epitope binding proteins of the invention can be produced in many different expression systems. In one embodiment, the multispecific epitope binding proteins of the invention are produced and secreted by mammalian cells. In another embodiment, the multispecific epitope binding proteins of the invention are produced and secreted in human cells. In a specific embodiment, the multispecific epitope binding proteins of the invention are produced in cells of the 293F, CHO, or NS0 cell line.

Methods which are known to those skilled in the art can be used to construct expression vectors containing protein coding sequences and appropriate transcriptional and translational control signals. These methods include, for example, *in vitro* recombinant DNA techniques, synthetic techniques, and *in vivo* genetic recombination. The invention, thus, provides replicable vectors comprising a nucleotide sequence encoding an epitope binding protein molecule operably linked to a promoter.

Once the expression vector is transferred to a host cell by conventional techniques, the transfected cells are then cultured by conventional techniques to produce an epitope binding protein. Thus, the invention includes host cells containing a polynucleotide encoding a protein of the invention operably linked to a heterologous promoter.

A variety of host-expression vector systems may be utilized to express an epitope binding protein of the invention or portions thereof as described in U.S. Patent No. 5,807,715. For example, mammalian cells such as Chinese hamster ovary cells (CHO), in conjunction with a vector such as the major intermediate early gene promoter element from human cytomegalovirus is an effective expression system for epitope binding proteins (Foecking et al., Gene, 45:101 (1986); and Cockett et al., Bio/Technology, 8:2 (1990)). In addition, a host cell strain may be chosen which modulates the expression of inserted sequences, or modifies and processes the gene product in the specific fashion desired. Such modifications (*e.g*., glycosylation) and processing (*e.g*., cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the protein of the invention. To this end, eukaryotic host cells which possess the cellular machinery for proper processing of the primary transcript, glycosylation, and phosphorylation of the gene product may be used. Such mammalian host cells include but are not limited to CHO, VERY, BHK, Hela, COS, MDCK, 293, 3T3, W138, BT483, Hs578T, HTB2, BT2O and T47D, NS0, CRL7O3O and HsS78Bst cells.

In bacterial systems, a number of expression vectors may be advantageously selected depending upon the use intended for the protein molecule being expressed. For example, when a large quantity of such an epitope binding protein is to be produced, for the generation of pharmaceutical compositions comprising an epitope binding protein of the invention, vectors which direct the expression of high levels of fusion protein products that are readily purified may be desirable. Such vectors include, but are not limited to, the *E. coli* expression vector pUR278 (Ruther et al., EMBO, 12:1791 (1983)), in which the coding sequence may be ligated individually into the vector in frame with the lac Z coding region so that a fusion protein is produced; pIN vectors (Inouye & Inouye, 1985, Nucleic Acids Res. 13:3101-3109 (1985); Van Heeke & Schuster, 1989, J. Biol. Chem., 24:5503-5509 (1989)); and the like. pGEX vectors may also be used to express foreign polypeptides as fusion proteins with glutathione-S-transferase (GST). In general, such fusion proteins are soluble and can easily be purified from lysed cells by adsorption and binding to glutathione-agarose affinity matrix followed by elution in the presence of free glutathione. The pGEX vectors are designed to introduce a thrombin and/or factor Xa protease cleavage sites into the expressed polypeptide so that the cloned target gene product can be released from the GST moiety.

In an insect system, *Autographa californica* nuclear polyhedrosis virus (AcNPV) is used as a vector to express foreign genes. The virus grows in *Spodoptera frugiperda* cells. The protein coding sequence may be cloned individually into non-essential regions (for example, the polyhedrin gene) of the virus and placed under control of an AcNPV promoter (for example, the polyhedrin promoter).

In mammalian host cells, a number of virus based expression systems may be utilized. In cases where an adenovirus is used as an expression vector, the coding sequence of interest may be ligated to an adenovirus transcription/translation control complex, e.g., the late promoter and tripartite leader sequence. This chimeric gene may then be inserted in the adenovirus genome by *in vitro* or *in vivo* recombination. Insertion into a non-essential region of the viral genome (*e.g*., region El or E3) will result in a recombinant virus that is viable and capable of expressing the antibody molecule in infected hosts (*e.g., see,* Logan & Shenk, Proc. Natl. Acad. Sci. USA, 81:355-359 (1984)). Specific initiation signals may also be required for efficient translation of inserted antibody coding sequences. These signals include the ATG initiation codon and adjacent sequences. Furthermore, the initiation codon should generally be in frame with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, *etc.* (*see, e.g.*, Bittner et al., Methods in Enzymol., 153:51-544(1987)).

Stable expression can be used for long-term, high-yield production of recombinant proteins. For example, cell lines which stably express the protein molecule may be generated. Host cells can be transformed with an appropriately engineered vector comprising expression control elements (*e.g*., promoter, enhancer, transcription terminators, polyadenylation sites, etc.), and a selectable marker gene. Following the introduction of the foreign DNA, cells may be allowed to grow for 1-2 days in an enriched media, and then are switched to a selective media. The selectable marker in the recombinant plasmid confers resistance to the selection and allows cells that stably integrated the plasmid into their chromosomes to grow and form foci which in turn can be cloned and expanded into cell lines. Plasmids that encode an epitope binding protein of the invention can be used to introduce the gene/cDNA into any cell line suitable for production in culture.
A number of selection systems may be used, including, but not limited to, the herpes simplex virus thymidine kinase (Wigler et al., Cell, 11:223 (1977)), hypoxanthineguanine phosphoribosyltransferase (Szybalska & Szybalski, Proc. Natl. Acad. Sci. USA, 48:202 (1992)), and adenine phosphoribosyltransferase (Lowy et al., Cell, 22:8-17 (1980)) genes can be employed in tk⁻, hgprt⁻ or aprT⁻cells, respectively. Also, antimetabolite resistance can be used as the basis of selection for the following genes: *dhfr,* which confers resistance to methotrexate (Wigler et al., Natl. Acad. Sci. USA, 77:357 (1980); O'Hare et al., Proc. Natl. Acad. Sci. USA, 78:1527 (1981)); *gpt,* which confers resistance to mycophenolic acid (Mulligan & Berg, Proc. Natl. Acad. Sci. USA, 78:2072 (1981)); *neo,* which confers resistance to the aminoglycoside G-418 (Wu and Wu, Biotherapy 3:87-95 (1991); Tolstoshev, Ann. Rev. Pharmacol. Toxicol. 32:573-596 (1993); Mulligan, Science 260:926-932 (1993); and Morgan and Anderson, Ann. Rev. Biochem. 62:191-217 (1993); May, TIB TECH 11(5):155-2 15 (1993)); and *hygro,* which confers resistance to hygromycin (Santerre et al., Gene, 30:147 (1984)). Methods commonly known in the art of recombinant DNA technology may be routinely applied to select the desired recombinant clone, and such methods are described, for example, in Ausubel et al. (eds.), Current Protocols in Molecular Biology, John Wiley & Sons, NY (1993); Kriegler, Gene Transfer and Expression, A Laboratory Manual, Stockton Press, NY (1990); and in Chapters 12 and 13, Dracopoli et al. (eds.), Current Protocols in Human Genetics, John Wiley & Sons, NY (1994); Colberre-Garapin et al., 1981, J. Mol. Biol., 150:1, which are incorporated by reference herein in their entireties. Once an epitope binding protein of the invention has been produced by recombinant expression, it may be purified by any method known in the art for purification of an immunoglobulin molecule, for example, by chromatography (e.g., ion exchange, affinity, particularly by affinity for the specific antigens Protein A or Protein G, and sizing column chromatography), centrifugation, differential solubility, or by any other standard technique for the purification of proteins. Further, the proteins of the present invention or fragments thereof may be fused to heterologous polypeptide sequences described herein or otherwise known in the art to facilitate purification.

### O. Scalable Production of Multispecific Epitope Binding Proteins

In an effort to obtain large quantities of the multispecific epitope binding proteins of the invention, they may be produced by a scalable process (hereinafter referred to as "scalable process of the invention"). In some embodiments, multispecific epitope binding proteins may be produced by a scalable process of the invention in the research laboratory that may be scaled up to produce the proteins of the invention in analytical scale bioreactors (for example, but not limited to 5L, 10L, 15L, 30L, or 50L bioreactors) while maintaining the functional activity of the proteins. For instance, in one embodiment, proteins produced by scalable processes of the invention exhibit low to undetectable levels of aggregation as measured by HPSEC or rCGE, that is, no more than 5%, no more than 4%, no more than 3%, no more than 2%, no more than 1%, or no more than 0.5% aggregate by weight protein, and/or low to undetectable levels of fragmentation, that is, 80% or higher, 85% or higher, 90% or higher, 95% or higher, 98% or higher, or 99% or higher, or 99.5% or higher of the total peak area in the peak(s) representing intact multispecific epitope binding proteins. In other embodiments, the multispecific epitope binding proteins may be produced by a scalable process of the invention in the research laboratory that may be scaled up to produce the proteins of the invention in production scale bioreactors (for example, but not limited to 75L, 100L, 150L, 300L, or 500L). In some embodiments, the scalable process of the invention results in little or no reduction in production efficiency as compared to the production process performed in the research laboratory. In other embodiments, the scalable process of the invention produces multispecific epitope binding proteins at production efficiency of about 10 mg/L, about 20 m/L, about 30 mg/L, about 50 mg/L, about 75 mg/L, about 100 mg/ L, about 125 mg/L, about 150 mg/L, about 175 mg/L, about 200 mg/L, about 250 mg/L, or about 300 mg/L or higher.

In other embodiments, the scalable process of the invention produces multispecific epitope binding proteins at production efficiency of at least about 10 mg/L, at least about 20 mg/L, at least about 30 mg/L, at least about 50 mg/L, at least about 75 mg/L, at least about 100 mg/L, at least about 125 mg/L, at least about 150 mg/L, at least about 175 mg/L, at least about 200 mg/L, at least about 250 mg/L, or at least about 300 mg/L or higher.

In other embodiments, the scalable process of the invention produces multispecific epitope binding proteins at production efficiency from about 10 mg/L to about 300 mg/L, from about 10 mg/L to about 250 mg/L, from about 10 mg/L to about 200 mg/L, from about 10 mg/L to about 175 mg/L, from about 10 mg/L to about 150 mg/L, from about 10 mg/L to about 100 mg/L, from about 20 mg/L to about 300 mg/L, from about 20 mg/L to about 250 mg/L, from about 20 mg/L to about 200 mg/L, from 20 mg/L to about 175 mg/L, from about 20 mg/L to about 150 mg/L, from about 20 mg/L to about 125 mg/L, from about 20 mg/L to about 100 mg/L, from about 30 mg/L to about 300 mg/L, from about 30 mg/L to about 250 mg/L, from about 30 mg/L to about 200 mg/L, from about 30 mg/L to about 175 mg/L, from about 30 mg/L to about 150 mg/L, from about 30 mg/L to about 125 mg/L, from about 30 mg/L to about 100 mg/L, from about 50 mg/L to about 300 mg/L, from about 50 mg/L to about 250 mg/L, from about 50 mg/L to about 200 mg/L, from 50 mg/L to about 175 mg/L, from about 50 mg/L to about 150 mg/L, from about 50 mg/L to about 125 mg/L, or from about 50 mg/L to about 100 mg/L.

### P. Multispecific Epitope Binding Protein Purification and Isolation

When using recombinant techniques, the epitope binding proteins of the invention can be produced intracellularly, in the periplasmic space, or directly secreted into the medium. If the protein is produced intracellularly, as a first step, the particulate debris, either host cells or lysed fragments, is removed, for example, by centrifugation or ultrafiltration. Carter et al., Bio/Technology, 10:163-167 (1992) describe a procedure for isolating antibodies which are secreted into the periplasmic space of E. coli. Briefly, cell paste is thawed in the presence of sodium acetate (pH 3.5), EDTA, and phenylmethylsulfonylfluoride (PMSF) over about 30 min. Cell debris can be removed by centrifugation. Where the epitope binding protein is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

The epitope binding protein composition prepared from the cells can be purified using, for example, hydroxylapatite chromatography, hydrophobic interaction chromatography, ion exchange chromatography, gel electrophoresis, dialysis, and/or affinity chromatography either alone or in combination with other purification steps. The suitability of protein A as an affinity ligand depends on the species and isotype of any immunoglobulin Fc that is present in the epitope binding protein. Protein A can be used to purify antibodies that are based on human γ1, γ2, or γ4 heavy chains (Lindmark et al., J. Immunol. Methods, 62:1-13 (1983)). Protein G is recommended for all mouse isotypes and for human γ3 (Guss et al., EMBO J., 5:15671575 (1986)). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. Where the protein of the invention comprises a CH3 domain, the Bakerbond ABX resin (J.T. Baker, Phillipsburg, NJ) is useful for purification. Other techniques for protein purification such as fractionation on an ion-exchange column, ethanol precipitation, Reverse Phase HPLC, chromatography on silica, chromatography on heparin, SEPHAROSE chromatography on an anion or cation exchange resin (such as a poiyaspartic acid column), chromatofocusing, SDS-PAGE, and ammonium sulfate precipitation are also available depending on the antibody to be recovered.

Following any preliminary purification step(s), the mixture comprising the epitope binding protein of interest and contaminants may be subjected to low pH hydrophobic interaction chromatography using an elution buffer at a pH between about 2.5-4.5, and performed at low salt concentrations (e.g., from about 0-0.25 M salt).

Recombinant protein isolation and purification can be accomplished by many art-accepted techniques exploiting the physical characteristics of the protein of interest, such as size, charge, hydrophobicity, affinity, etc. In one embodiment, the proteins of the invention are subjected to isolation/purification methods known in the art such as size exclusion chromatography, ion-exchange chromatography, and affinity chromatography. In another embodiment, the proteins of the invention are purified through protein A affinity chromatography. In another embodiment, the proteins of the invention are purified through affinity chromatography exploiting one or more binding specificities within the protein.

It is to be understood that constituent epitope binding domains of multispecific epitope binding proteins retain substantially all of the functions exhibited by the identical isolated functional epitope binding domains. In one embodiment, the constituent epitope binding domains of multispecific epitope binding proteins retain one or more of the functions exhibited by the identical isolated functional epitope binding domains. In one embodiment, the functions exhibited by epitope binding domains of multispecific epitope binding proteins include but are not limited to specificity, affinity, agonistic (see for example Figures 29, 30, and 31) antagonistic, crosslinking characteristics essentially the same as the functions exhibited by identical isolated epitope binding domains. In another embodiment, the constituent epitope binding domains of multispecific epitope binding proteins retain at least 10%, at least 20%, at least 30%, at least 40%, at least 50%, at least 60 %, at least 70%, at least 80%, at least 90%, at least 100%, at least 110%, at least 120%, at least 130%, at least 140%, at least 150%, or at least 160% activity of one or more functions exhibited by the identical isolated functional epitope binding domains.

To ensure the functionality of the proteins of the invention, suitable binding assays have been developed. In addition to techniques well described in the art, such as ELISA, BIACore®, and KinExA™ the invention provides assays to determine the functionality of multispecific epitope binding proteins. In one embodiment, the functionality of the protein of the invention can be assayed using the methods described in the Examples section. In another embodiment, the functionality of the protein of the invention can be assayed using the methods described in Example 10. In another embodiment, the functionality of the protein of the invention can be assayed using the methods described in any of Examples 13-20.

To ensure the stability of the proteins of the invention, suitable assays have been developed. In one embodiment, the stability of proteins of the invention is characterized by known techniques in the art. In other embodiments, the stability of the proteins of the invention can be assessed by aggregation and/or fragmentation rate or profile. To determine the level of aggregation or fragmentation, many techniques may be used. In one embodiment, the aggregation and/or fragmentation profile may be assessed by the use of analytical ultracentrifugation (AUC), size-exclusion chromatography (SEC), high-performance size-exclusion chromatography (HPSEC), melting temperature (Tₘ), polyacrylamide gel electrophoresis (PAGE), capillary gel electrophoresis (CGE), light scattering (SLS), Fourier Transform Infrared Spectroscopy (FTIR), circular dichroism (CD), urea-induced protein unfolding techniques, intrinsic tryptophan fluorescence, differential scanning calorimetry, or 1-anilino-8-naphthalenesulfonic acid (ANS) protein binding techniques. In another embodiment, the stability of proteins of the invention is characterized by polyacrylamide gel electrophoresis (PAGE) analysis. In another embodiment, the stability of proteins of the invention is characterized by the methods described in Examples 3 and 6. In another embodiment, the stability of the proteins of the invention is characterized by size exclusion chromatography (SEC) profile analysis. In another embodiment, the stability of the proteins of the invention is characterized by the methods described in Example 10. In another embodiment, the stability of the proteins of the invention can be assayed using the methods described in any of Examples 13-20.

Another measure of stability is the relative resistance to protease degradation exhibited by a protein. In one embodiment, the stability of the proteins of the invention is characterized by a protease resistance assay. In one embodiment, the protease utilized in the protease resistance assay is a serine protease, threonine protease, cysteine protease, aspartic acid protease, metalloprotease , or a glutamic acid protease. In one embodiment, the proteins of the invention are subjected to a protease resistance assay in which the protease is trypsin, chymotrypsin, cathepsin B, D, L, or G, pepsin, papain, elastase, HIV-1 protease, chymosin, renin, plasmepsin, plasmin, carboxypeptidase E, caspase 1-10, or calpain. In another embodiment, multispecific epitope binding proteins of the invention exhibit a low level of protease degradation. In some embodiments, the multispecific epitope binding proteins of the invention exhibit protease (e.g., trypsin (20 ng/ 1µg of antibody/epitope binding protein or chymotrypsin (20 ng/ 1µg of antibody/epitope binding protein)) resistance in which at least 70%, at least 75%, at least 80%, at least 85%, at least 90%, at least 95% or more of the protein remains undigested after incubation with the protease at, e.g., 37°C for 1 hr, 12 hours, or 24 hours. In another embodiment, multispecific epitope binding proteins of the invention exhibit a low level of protease degradation. In some embodiments, the multispecific epitope binding proteins of the invention exhibit protease resistance in which at least 70%, at least 75%, at least 80%, at least 85%, at least 90%, at least 95% or more of the protein remains undigested after incubation with the protease as essentially described in Examples 28-29.

The invention also provides methods of testing multiple epitope binding proteins of the invention. The binding specificities of an antibody or antibody-like molecule can be assessed by many different art accepted techniques such as phage display and other ELISA based technologies. In one embodiment, the binding specificities of the proteins of the invention may be tested by any known technique in the art. In another embodiment, the proteins of the invention may be tested by any of the techniques presented in the specification. In another embodiment, the binding specificities for proteins of the invention may be tested by an ELISA based assay such as the assay described in Example 10. In another embodiment, the binding specificities for proteins of the invention may be tested by the method described in any of Examples 13-20.

### Q. Methods Of Monitoring The Stability And Aggregation Of Multispecific Epitope Binding Protein Formulations

There are various methods available for assessing the stability of protein formulations based on the physical and chemical structures of the proteins as well as on their biological activities. For example, to study denaturation of proteins, methods such as charge-transfer absorption, thermal analysis, fluorescence spectroscopy, circular dichroism, NMR, rCGE (reducing capillary gel electrophoresis) and HPSEC (high performance size exclusion chromatography), are available. See, for example, Wang et al., 1988, J. of Parenteral Science & Technology 42(Suppl):S4-S26.

The rCGE and HPSEC are the most common and simplest methods to assess the formation of protein aggregates, protein degradation, and protein fragmentation. Accordingly, the stability of the liquid formulations of the present invention may be assessed by these methods.

For example, the stability of the liquid formulations of the present invention may be evaluated by HPSEC or rCGE, wherein the percent area of the peaks represents the non-degraded multispecific epitope binding proteins. In one embodiment, approximately 250 µg of a multispecific epitope binding protein is injected onto a TosoH Biosep TSK G3000SWXL column (7.8 mm x 30 cm) fitted with a TSK SW x1 guard column (6.0 mm CX 4.0 cm). The multispecific epitope binding protein is eluted isocratically with 0.1 M disodium phosphate containing 0.1 M sodium sulfate and 0.05% sodium azide, at a flow rate of 0.8 to 1.0 ml/min. Eluted protein is detected using UV absorbance at 280 nm. Multispecific epitope binding proteins may be run in comparison to reference standards and the results are reported as the area percent of the product monomer peak compared to all other peaks excluding the included volume peak observed. Peaks eluting earlier than the monomer peak are recorded as percent aggregate.

The liquid formulations of the present invention exhibit low to undetectable levels of aggregation as measured by HPSEC or rCGE, that is, no more than 5%, no more than 4%, no more than 3%, no more than 2%, no more than 1%, or no more than 0.5% aggregate by weight protein, and/or low to undetectable levels of fragmentation, that is, 80% or higher, 85% or higher, 90% or higher, 95% or higher, 98% or higher, or 99% or higher, or 99.5% or higher of the total peak area in the peak(s) representing intact multispecific epitope binding proteins. In the case of SDS-PAGE, the density or the radioactivity of each band stained or labeled with radioisotope can be measured and the % density or % radioactivity of the band representing non-degraded multispecific epitope binding proteins can be obtained.

The liquid formulations of the present invention exhibit low to undetectable levels of non-functional (e.g., not functional *in vivo* and/or *in vitro*) dimer or multimer by-products (for example, but not limited to undesired light chain dimers). Such unwanted dimer or multimer by-products may be measured by HPSEC, rCGE, or SDS-PAGE. In some embodiments, liquid formulations of the present invention exhibit low to undetectable levels of non-functional dimer or multimer byproducts as measured by HPSEC or rCGE, that is, no more than 15%, no more than 10%, no more than 5%, no more than 4%, no more than 3%, no more than 2%, no more than 1%, or no more than 0.5% by weight of total protein, or represented by 80% or higher, 85% or higher, 90% or higher, 95% or higher, 98% or higher, or 99% or higher, or 99.5% or higher of the total peak area in the peak(s) representing intact multispecific epitope binding proteins. Alternatively, undesired byproducts may be detected by SDS-PAGE using radiolabeled amino acid incorporation, or by Western Blotting with various immunospecific reagents.

The stability of the liquid formulations of the present invention can be also assessed by any assays which measure the biological activity of the multispecific epitope binding proteins in the formulation. The biological activities of multispecific epitope binding proteins include, but are not limited to, antigen-binding activity, complement-activation activity, Fc-receptor binding activity, receptor/ligand neutralizing activity, receptor agonism or antagonism and so forth. Antigen-binding activity of the multispecific epitope binding proteins can be measured by any method known to those skilled in the art, including but not limited to ELISA, radioimmunoassay, Western blot, and the like (Also see Harlow et al., Antibodies: A Laboratory Manual, (Cold Spring Harbor Laboratory Press, 2nd ed. 1988) (incorporated by reference herein in its entirety). The purity of the liquid multispecific epitope binding proteins formulations of the invention may be measured by any method well-known to one of skill in the art such as, e.g., HPSEC. The sterility of the liquid multispecific epitope binding protein formulations may be assessed as follows: sterile soybean-casein digest medium and fluid thioglycollate medium are inoculated with a test liquid antibody formulation by filtering the liquid multispecific epitope binding protein formulation through a sterile filter having a nominal porosity of 0.45 µm. When using the Sterisure™ or Steritest™ method, each filter device is aseptically filled with approximately 100 ml of sterile soybean-casein digest medium or fluid thioglycollate medium. When using the conventional method, the challenged filter is aseptically transferred to 100 ml of sterile soybean-casein digest medium or fluid thioglycollate medium. The media are incubated at appropriate temperatures and observed three times over a 14 day period for evidence of bacterial or fungal growth.

Liquid formulations of the invention also exhibit enhanced stability *in vivo* (for example, upon administration to a mammal). As such, the component multispecific epitope binding proteins present in a formulation may be analysed by the methods described above to evaluate stability after administration to a mammal. In some embodiments, formulations of the invention are stable *in vivo* for at least 1 hour, 2 hours, 3 hours, 4 hours, 5 hours, 6 hours, 7 hours, 8 hours, 9 hours, 10 hours, 12 hours, or 24 hours, or more. In other embodiments, formulations of the invention are stable *in vivo* for at least 1 day, 2 days, 3 days, 4 days, 5 days, 6 days, 7 days, 14 days, 21 days, or 30 days or more. In other embodiments, proteins or formulations of the invention exhibit a half life of at least at least 1 hour, 2 hours, 3 hours, 4 hours, 5 hours, 6 hours, 7 hours, 8 hours, 9 hours, 10 hours, 12 hours, 24 hours, or more after administration to a mammal. In other embodiments, proteins or formulations of the invention exhibit a half life of at least 1 day, 2 days, 3 days, 4 days, 5 days, 6 days, 7 days, 14 days, 21 days, or 30 days or more after administration to a mammal.

### R. Pharmaceutical Compositions

In another aspect, the present invention provides a composition, for example, but not limited to, a pharmaceutical composition, containing one or a combination of multispecific epitope binding proteins of the present invention, formulated together with a pharmaceutically acceptable carrier. Such compositions may include one or a combination of, for example, but not limited to two or more different multispecific epitope binding proteins of the invention. For example, a pharmaceutical composition of the invention may comprise a combination of multispecific epitope binding proteins that bind to different epitopes on the target antigen or that have complementary activities.

Pharmaceutical compositions of the invention also can be administered in combination therapy, such as, combined with other agents. For example, the combination therapy can include a multispecific epitope binding protein of the present invention combined with at least one other therapy wherein the therapy may be immunotherapy, chemotherapy, radiation treatment, or drug therapy.

The pharmaceutical compounds of the invention may include one or more pharmaceutically acceptable salts. Examples of such salts include acid addition salts and base addition salts. Acid addition salts include those derived from nontoxic inorganic acids, such as hydrochloric, nitric, phosphoric, sulfuric, hydrobromic, hydroiodic, phosphorous and the like, as well as from nontoxic organic acids such as aliphatic mono- and dicarboxylic acids, phenyl-substituted alkanoic acids, hydroxy alkanoic acids, aromatic acids, aliphatic and aromatic sulfonic acids and the like. Base addition salts include those derived from alkaline earth metals, such as sodium, potassium, magnesium, calcium and the like, as well as from nontoxic organic amines, such as N,N'-dibenzylethylenediamine, N-methylglucamine, chloroprocaine, choline, diethanolamine, ethylenediamine, procaine and the like.

A pharmaceutical composition of the invention also may include a pharmaceutically acceptable anti-oxidant. Examples of pharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Examples of suitable aqueous and non-aqueous carriers that may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of presence of microorganisms may be ensured both by sterilization procedures and by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

Pharmaceutical compositions typically must be sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be suitable to include isotonic agents, for example, sugars, polyalcohols such as mannitol, sorbitol, or sodium chloride in the composition. Prolonged absorption of the injectable compositions can be brought about by including in the composition an agent that delays absorption, for example, monostearate salts and gelatin.

Sterile injectable solutions can be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

In one embodiment the compositions of the invention are pyrogen-free formulations which are substantially free of endotoxins and/or related pyrogenic substances. Endotoxins include toxins that are confined inside a microorganism and are released when the microorganisms are broken down or die. Pyrogenic substances also include fever-inducing, thermostable substances (glycoproteins) from the outer membrane of bacteria and other microorganisms. Both of these substances can cause fever, hypotension and shock if administered to humans. Due to the potential harmful effects, it is advantageous to remove even low amounts of endotoxins from intravenously administered pharmaceutical drug solutions. The Food & Drug Administration ("FDA") has set an upper limit of 5 endotoxin units (EU) per dose per kilogram body weight in a single one hour period for intravenous drug applications (The United States Pharmacopeial Convention, Pharmacopeial Forum 26 (1):223 (2000)). When therapeutic proteins are administered in amounts of several hundred or thousand milligrams per kilogram body weight it is advantageous to remove even trace amounts of endotoxin. In one embodiment, endotoxin and pyrogen levels in the composition are less then 10 EU/mg, or less then 5 EU/mg, or less then 1 EU/mg, or less then 0.1 EU/mg, or less then 0.01 EU/mg, or less then 0.001 EU/mg. In another embodiment, endotoxin and pyrogen levels in the composition are less then about 10 EU/mg, or less then about 5 EU/mg, or less then about 1 EU/mg, or less then about 0.1 EU/mg, or less then about 0.01 EU/mg, or less then about 0.001 EU/mg.

In one embodiment, the proteins of the invention and compositions comprising the same are useful in the treatments for cancer or symptoms thereof. In one embodiment, the invention comprises composition useful in the treatment of solid tumors of the head, brain, neck, skin, throat, lung, bone, breast, colon, liver, pancreas, stomach, intestine, urinary tract, thyroid, eye, testicles, central nervous system, prostate, ovary, kidney, rectum, and adrenal gland. In another embodiment, the invention comprises compositions useful in the treatment of cancer metastases. In another embodiment, the invention comprises compositions that are useful in the treatment of non-solid tumors, such as but not limited to myeloma, lymphoma, and leukemia.

In another embodiment, the invention comprises compositions capable of inhibiting a cancer cell phenotype. In one embodiment, the cancer cell phenotype is cell growth, cell attachment, loss of cell attachment, decreased receptor expression (e.g Eph), increased receptor expression (e.g Eph), metastatic potential, cell cycle inhibition, receptor tyrosine kinase activation/inhibition or others.

In another embodiment, the invention comprises administering a composition wherein said administration is oral, parenteral, intramuscular, intranasal, vaginal, rectal, lingual, sublingual, buccal, intrabuccal, intravenous, cutaneous, subcutaneous or transdermal.

In another embodiment the invention further comprises administering a composition in combination with other therapies, such as chemotherapy, hormonal therapy, biological therapy, immunotherapy or radiation therapy.

### S. Dosing/Administration

To prepare pharmaceutical or sterile compositions including a multispecific epitope binding protein of the invention, multispecific epitope binding protein is mixed with a pharmaceutically acceptable carrier or excipient. Formulations of therapeutic and diagnostic agents can be prepared by mixing with physiologically acceptable carriers, excipients, or stabilizers in the form of, e.g., lyophilized powders, slurries, aqueous solutions, lotions, or suspensions (see, e.g., Hardman, et al. (2001) Goodman and Gilman's The Pharmacological Basis of Therapeutics, McGraw-Hill, New York, N.Y.; Gennaro (2000) Remington: The Science and Practice of Pharmacy, Lippincott, Williams, and Wilkins, New York, N.Y.; Avis, et al. (eds.) (1993) Pharmaceutical Dosage Forms: Parenteral Medications, Marcel Dekker, NY; Lieberman, et al. (eds.) (1990) Pharmaceutical Dosage Forms: Tablets, Marcel Dekker, NY; Lieberman, et al. (eds.) (1990) Pharmaceutical Dosage Forms: Disperse Systems, Marcel Dekker, NY; Weiner and Kotkoskie (2000) Excipient Toxicity and Safety, Marcel Dekker, Inc., New York, N.Y.).

Selecting an administration regimen for a therapeutic depends on several factors, including the serum or tissue turnover rate of the entity, the level of symptoms, the immunogenicity of the entity, and the accessibility of the target cells in the biological matrix. In certain embodiments, an administration regimen maximizes the amount of therapeutic delivered to the patient consistent with an acceptable level of side effects. Accordingly, the amount of biologic delivered depends in part on the particular entity and the severity of the condition being treated. Guidance in selecting appropriate doses of antibodies, cytokines, and small molecules are available (see, e.g., Wawrzynczak (1996) Antibody Therapy, Bios Scientific Pub. Ltd, Oxfordshire, UK; Kresina (ed.) (1991) Monoclonal Antibodies, Cytokines and Arthritis, Marcel Dekker, New York, N.Y.; Bach (ed.) (1993) Monoclonal Antibodies and Peptide Therapy in Autoimmune Diseases, Marcel Dekker, New York, N.Y.; Baert, et al. (2003) New Engl. J. Med. 348:601-608; Milgrom, et al. (1999) New Engl. J. Med. 341:1966-1973; Slamon, at al. (2001) New Engl. J. Med. 344:783-792; Beniaminovitz, et al. (2000) New Engl. J. Med. 342:613-619; Ghosh, et al. (2003) New Engl. J. Med. 348:24-32; Lipsky, et al. (2000) New Engl. J. Med. 343:1594-1602).

Determination of the appropriate dose is made by the clinician, e.g., using parameters or factors known or suspected in the art to affect treatment or predicted to affect treatment. Generally, the dose begins with an amount somewhat less than the optimum dose and it is increased by small increments thereafter until the desired or optimum effect is achieved relative to any negative side effects. Important diagnostic measures include those of symptoms of, e.g., the inflammation or level of inflammatory cytokines produced.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors known in the medical arts.

Compositions comprising multispecific epitope binding proteins of the invention can be provided by continuous infusion, or by doses at intervals of, e.g., one day, one week, or 1-7 times per week. Doses may be provided intravenously, subcutaneously, topically, orally, nasally, rectally, intramuscular, intracerebrally, or by inhalation. A specific dose protocol is one involving the maximal dose or dose frequency that avoids significant undesirable side effects. A total weekly dose may be at least 0.05 µg/kg body weight, at least 0.2 µg/kg, at least 0.5 µg/kg, at least 1 µg/kg, at least 10 µg/kg, at least 100 µg/kg, at least 0.2 mg/kg, at least 1.0 mg/kg, at least 2.0 mg/kg, at least 10 mg/kg, at least 25 mg/kg, or at least 50 mg/kg (see, e.g., Yang, et al. (2003) New Engl. J. Med. 349:427-434; Herold, et al. (2002) New Engl. J. Med. 346:1692-1698; Liu, et al. (1999) J. Neurol. Neurosurg. Psych. 67:451-456; Portielji, et al. (20003) Cancer Immunol. Immunother. 52:133-144). The desired dose of multispecific epitope binding protein is about the same as for an antibody or polypeptide, on a moles/kg body weight basis. The desired plasma concentration of a multispecific epitope binding protein therapeutic is about the same as for an antibody, on a moles/kg body weight basis. The dose may be at least 15 µg, at least 20 µg, at least 25 µg, at least 30 µg, at least 35 µg, at least 40 µg, at least 45 µg, at least 50 µg, at least 55 ug, at least 60 µg, at least 65 µg, at least 70 µg, at least 75 µg, at least 80 µg, at least 85 µg, at least 90 µg, at least 95 µg, or at least 100 µg. The doses administered to a subject may number at least 1, 2, 3, 4, 5, 6, 7, 8, 9, 10, 11, or 12, or more.

For multispecific epitope binding proteins of the invention, the dosage administered to a patient may be 0.0001 mg/kg to 100 mg/kg of the patient's body weight. The dosage may be between 0.0001 mg/kg and 20 mg/kg, 0.0001 mg/kg and 10 mg/kg, 0.0001 mg/kg and 5 mg/kg, 0.0001 and 2 mg/kg, 0.0001 and 1 mg/kg, 0.0001 mg/kg and 0.75 mg/kg, 0.0001 mg/kg and 0.5 mg/kg, 0.0001 mg/kg to 0.25 mg/kg, 0.0001 to 0.15 mg/kg, 0.0001 to 0.10 mg/kg, 0.001 to 0.5 mg/kg, 0.01 to 0.25 mg/kg or 0.01 to 0.10 mg/kg of the patient's body weight.

The dosage of the multispecific epitope binding proteins of the invention may be calculated using the patient's weight in kilograms (kg) multiplied by the dose to be administered in mg/kg. The dosage of the multispecific epitope binding proteins of the invention may be 150 µg/kg or less, 125 µg/kg or less, 100 µg/kg or less, 95 µg/kg or less, 90 µg/kg or less, 85 µg/kg or less, 80 µg/kg or less, 75 µg/kg or less, 70 µg/kg or less, 65 µg/kg or less, 60 µg/kg or less, 55 µg/kg or less, 50 µg/kg or less, 45 µg/kg or less, 40 µg/kg or less, 35 µg/kg or less, 30 µg/kg or less, 25 µg/kg or less, 20 µg/kg or less, 15 µg/kg or less, 10 µg/kg or less, 5 µg/kg or less, 2.5 µg/kg or less, 2 µg/kg or less, 1.5 µg/kg or less, 1 µg/kg or less, 0.5 µg/kg or less, or 0.5 µg/kg or less of a patient's body weight.

Unit dose of the multispecific epitope binding proteins of the invention may be 0.1 mg to 20 mg, 0.1 mg to 15 mg, 0.1 mg to 12 mg, 0.1 mg to 10 mg, 0.1 mg to 8 mg, 0.1 mg to 7mg, 0.1 mg to 5 mg, 0.1 to 2.5 mg, 0.25 mg to 20 mg, 0.25 to 15 mg, 0.25 to 12 mg, 0.25 to 10 mg, 0.25 to 8 mg, 0.25 mg to 7 m g, 0.25 mg to 5 mg, 0.5 mg to 2.5 mg, 1 mg to 20 mg, 1 mg to 15 mg, 1 mg to 12 mg, 1 mg to 10 mg, 1 mg to 8 mg, 1 mg to 7 mg, 1 mg to 5 mg, or 1 mg to 2.5 mg.

The dosage of the multispecific epitope binding proteins of the invention may achieve a serum titer of at least 0.1 µg/ml, at least 0.5 µg/ml, at least 1 µg/ml, at least 2 µg/ml, at least 5 µg/ml, at least 6 µg/ml, at least 10 µg/ml, at least 15 µg/ml, at least 20 µg/ml, at least 25 µg/ml, at least 50 µg/ml, at least 100 µg/ml, at least 125 µg/ml, at least 150 µg/ml, at least 175 µg/ml, at least 200 µg/ml, at least 225 µg/ml, at least 250 µg/ml, at least 275 µg/ml, at least 300 µg/ml, at least 325 µg/ml, at least 350 µg/ml, at least 375 µg/ml, or at least 400 µg/ml in a subject. Alternatively, the dosage of the multispecific epitope binding proteins of the invention may achieve a serum titer of at least 0.1 µg/ml, at least 0.5 µg/ml, at least 1 µg/ml, at least, 2 µg/ml, at least 5 µg/ml, at least 6 µg/ml, at least 10 µg/ml, at least 15 µg/ml, at least 20 µg/ml, at least 25 µg/ml, at least 50 µg/ml, at least 100 µg/ml, at least 125 µg/ml, at least 150 µg/ml, at least 175 µg/ml, at least 200 µg/ml, at least 225 µg/ml, at least 250 µg/ml, at least 275 µg/ml, at least 300 µg/ml, at least 325 µg/ml, at least 350 µg/ml, at least 375 µg/ml, or at least 400 µg/ml in the subject.

Doses of multispecific epitope binding proteins of the invention may be repeated and the administrations may be separated by at least 1 day, 2 days, 3 days, 5 days, 10 days, 15 days, 30 days, 45 days, 2 months, 75 days, 3 months, or at least 6 months.

An effective amount for a particular patient may vary depending on factors such as the condition being treated, the overall health of the patient, the method route and dose of administration and the severity of side affects (see, e.g., Maynard, et al. (1996) A Handbook of SOPs for Good Clinical Practice, Interpharm Press, Boca Raton, Fla.; Dent (2001) Good Laboratory and Good Clinical Practice, Urch Publ., London, UK).

The route of administration may be by, e.g., topical or cutaneous application, injection or infusion by intravenous, intraperitoneal, intracerebral, intramuscular, intraocular, intraarterial, intracerebrospinal, intralesional, or by sustained release systems or an implant (see, e.g., Sidman et al. (1983) Biopolymers 22:547-556; Langer, et al. (1981) J. Biomed. Mater. Res. 15:167-277; Langer (1982) Chem. Tech. 12:98-105; Epstein, et al. (1985) Proc. Natl. Acad. Sci. USA 82:3688-3692; Hwang, et al. (1980) Proc. Natl. Acad. Sci. USA 77:4030-4034; U.S. Pat. Nos. 6,350466 and 6,316,024). Where necessary, the composition may also include a solubilizing agent and a local anesthetic such as lidocaine to ease pain at the site of the injection. In addition, pulmonary administration can also be employed, *e.g.,* by use of an inhaler or nebulizer, and formulation with an aerosolizing agent. See, *e.g.,* U.S. Pat. Nos. 6,019,968, 5,985, 320, 5,985,309, 5,934,272, 5,874,064, 5,855,913, 5,290,540, and 4,880,078; and PCT Publication Nos. WO 92/19244, WO 97/32572, WO 97/44013, WO 98/31346, and WO 99/66903, each of which is incorporated herein by reference their entirety. In one embodiment, an antibody, combination therapy, or a composition of the invention is administered using Alkermes AIR™ pulmonary drug delivery technology (Alkermes, Inc., Cambridge, Mass.).

A composition of the present invention may also be administered via one or more routes of administration using one or more of a variety of methods known in the art. As will be appreciated by the skilled artisan, the route and/or mode of administration will vary depending upon the desired results. Selected routes of administration for multispecific epitope binding proteins of the invention include intravenous, intramuscular, intradermal, intraperitoneal, subcutaneous, spinal or other parenteral routes of administration, for example by injection or infusion. Parenteral administration may represent modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion. Alternatively, a composition of the invention can be administered via a non-parenteral route, such as a topical, epidermal or mucosal route of administration, for example, intranasally, orally, vaginally, rectally, sublingually or topically.

If the multispecific epitope binding proteins of the invention are administered in a controlled release or sustained release system, a pump may be used to achieve controlled or sustained release (see Langer, supra; Sefton, 1987, CRC Crit. Ref. Biomed. Eng. 14:20*;* Buchwald et al., 1980, Surgery 88:507; Saudek et al., 1989, N. Engl. J. Med. 321:574*).* Polymeric materials can be used to achieve controlled or sustained release of the therapies of the invention (see *e.g.,* Medical Applications of Controlled Release, Langer and Wise (eds.), CRC Pres., Boca Raton, Fla. (1974); Controlled Drug Bioavailability, Drug Product Design and Performance, Smolen and Ball (eds.), Wiley, New York (1984); Ranger and Peppas, 1983, J., Macromol. Sci. Rev. Macromol. Chem. 23:61; see also Levy et al., 1985, Science 228:190; During et al., 1989, Ann. Neurol. 25:351; Howard et al., 1989, J. Neurosurg. 7 1:105); U.S. Pat. No. 5,679,377; U.S. Pat. No. 5,916,597; U.S. Pat. No. 5,912,015; U.S. Pat. No. 5,989,463; U.S. Pat. No. 5,128,326; PCT Publication No. WO 99/15154; and PCT Publication No. WO 99/20253. Examples of polymers used in sustained release formulations include, but are not limited to, poly(2-hydroxy ethyl methacrylate), poly(methyl methacrylate), poly(acrylic acid), poly(ethylene-co-vinyl acetate), poly(methacrylic acid), polyglycolides (PLG), polyanhydrides, poly(N-vinyl pyrrolidone), poly(vinyl alcohol), polyacrylamide, poly(ethylene glycol), polylactides (PLA), poly(lactide-co-glycolides) (PLGA), and polyorthoesters. In one embodiment, the polymer used in a sustained release formulation is inert, free of leachable impurities, stable on storage, sterile, and biodegradable. A controlled or sustained release system can be placed in proximity of the prophylactic or therapeutic target, thus requiring only a fraction of the systemic dose (see, *e.g.,* Goodson, in Medical Applications of Controlled Release, supra, vol. 2, pp. 115-138 (1984)).

Controlled release systems are discussed in the review by Langer (1990, Science 249:1527-1533). Any technique known to one of skill in the art can be used to produce sustained release formulations comprising one or more multispecific epitope binding proteins of the invention. See, *e.g.,* U.S. Pat. No. 4,526,938, PCT publication WO 91/05548, PCT publication WO 96/20698, Ning et al., 1996, "Intratumoral Radioimmunotheraphy of a Human Colon Cancer Xenograft Using a Sustained-Release Gel," Radiotherapy & Oncology 39:179-189, Song et al., 1995, "Antibody Mediated Lung Targeting of Long-Circulating Emulsions," PDA Journal of Pharmaceutical Science & Technology 50:372-397, Cleek et al., 1997, "Biodegradable Polymeric Carriers for a bFGF Antibody for Cardiovascular Application," Pro. Int'l. Symp. Control. Rel. Bioact. Mater. 24:853-854, and Lam et al., 1997, "Microencapsulation of Recombinant Humanized Monoclonal Antibody for Local Delivery," Proc. Int'l. Symp. Control Rel. Bioact. Mater. 24:759-760, each of which is incorporated herein by reference in their entirety.

If the multispecific epitope binding protein of the invention is administered topically, it can be formulated in the form of an ointment, cream, transdermal patch, lotion, gel, shampoo, spray, aerosol, solution, emulsion, or other form well-known to one of skill in the art. See, e.g., Remington's Pharmaceutical Sciences and Introduction to Pharmaceutical Dosage Forms, 19th ed., Mack Pub. Co., Easton, Pa. (1995). For non-sprayable topical dosage forms, viscous to semi-solid or solid forms comprising a carrier or one or more excipients compatible with topical application and having a dynamic viscosity, in some instances, greater than water are typically employed. Suitable formulations include, without limitation, solutions, suspensions, emulsions, creams, ointments, powders, liniments, salves, and the like, which are, if desired, sterilized or mixed with auxiliary agents (*e.g.*, preservatives, stabilizers, wetting agents, buffers, or salts) for influencing various properties, such as, for example, osmotic pressure. Other suitable topical dosage forms include sprayable aerosol preparations wherein the active ingredient, in some instances, in combination with a solid or liquid inert carrier, is packaged in a mixture with a pressurized volatile (*e.g*., a gaseous propellant, such as freon) or in a squeeze bottle. Moisturizers or humectants can also be added to pharmaceutical compositions and dosage forms if desired. Examples of such additional ingredients are well-known in the art.

If the compositions comprising multispecific epitope binding proteins are administered intranasally, it can be formulated in an aerosol form, spray, mist or in the form of drops. In particular, prophylactic or therapeutic agents for use according to the present invention can be conveniently delivered in the form of an aerosol spray presentation from pressurized packs or a nebuliser, with the use of a suitable propellant (*e.g.*, dichlorodifluoromethane, trichlorofluoromethane, dichlorotetrafluoroethane, carbon dioxide or other suitable gas). In the case of a pressurized aerosol the dosage unit may be determined by providing a valve to deliver a metered amount. Capsules and cartridges (composed of, *e.g.,* gelatin) for use in an inhaler or insufflator may be formulated containing a powder mix of the compound and a suitable powder base such as lactose or starch.

Methods for co-administration or treatment with a second therapeutic agent, e.g., a cytokine, steroid, chemotherapeutic agent, antibiotic, or radiation, are known in the art (see, e.g., Hardman, et al. (eds.) (2001) Goodman and Gilman's The Pharmacological Basis of Therapeutics, 10.sup.th ed., McGraw-Hill, New York, N.Y.; Poole and Peterson (eds.) (2001) Pharmacotherapeutics for Advanced Practice:A Practical Approach, Lippincott, Williams & Wilkins, Phila., Pa.; Chabner and Longo (eds.) (2001) Cancer Chemotherapy and Biotherapy, Lippincott, Williams & Wilkins, Phila., Pa.). An effective amount of therapeutic may decrease the symptoms by at least 10%; by at least 20%; at least about 30%; at least 40%, or at least 50%.

Additional therapies (*e.g.,* prophylactic or therapeutic agents), which can be administered in combination with the multispecific epitope binding proteins of the invention may be administered less than 5 minutes apart, less than 30 minutes apart, 1 hour apart, at about 1 hour apart, at about 1 to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, at about 12 hours to 18 hours apart, 18 hours to 24 hours apart, 24 hours to 36 hours apart, 36 hours to 48 hours apart, 48 hours to 52 hours apart, 52 hours to 60 hours apart, 60 hours to 72 hours apart, 72 hours to 84 hours apart, 84 hours to 96 hours apart, or 96 hours to 120 hours apart from the multispecific epitope binding proteins of the invention. The two or more therapies may be administered within one same patient visit.

The multispecific epitope binding proteins of the invention and the other therapies may be cyclically administered. Cycling therapy involves the administration of a first therapy (*e.g.,* a first prophylactic or therapeutic agent) for a period of time, followed by the administration of a second therapy (*e.g.,* a second prophylactic or therapeutic agent) for a period of time, optionally, followed by the administration of a third therapy (*e.g.,* prophylactic or therapeutic agent) for a period of time and so forth, and repeating this sequential administration, *i.e.,* the cycle in order to reduce the development of resistance to one of the therapies, to avoid or reduce the side effects of one of the therapies, and/or to improve the efficacy of the therapies.

In certain embodiments, the multispecific epitope binding proteins of the invention can be formulated to ensure proper distribution *in vivo.* For example, the blood-brain barrier (BBB) excludes many highly hydrophilic compounds. To ensure that the therapeutic compounds of the invention cross the BBB (if desired), they can be formulated, for example, in liposomes. For methods of manufacturing liposomes, see, *e.g.,* U.S. Patents 4,522,811; 5,374,548; and 5,399,331. The liposomes may comprise one or more moieties which are selectively transported into specific cells or organs, thus enhance targeted drug delivery (*see, e.g.,* V.V. Ranade (1989) J. Clin. Pharmacol. 29:685). Exemplary targeting moieties include folate or biotin (see, e.g., U.S. Patent 5,416,016 to Low et al.); mannosides (Umezawa et al., (1988) Biochem. Biophys. Res. Commun. 153:1038); antibodies (P.G. Bloeman et al. (1995) FEBS Lett. 357:140; M. Owais et al. (1995) Antimicrob. Agents Chemother. 39:180); surfactant protein A receptor (Briscoe et al. (1995) Am. J. Physiol. 1233:134); p120 (Schreier et al. (1994) J. Biol. Chem. 269:9090); see also K. Keinanen; M.L. Laukkanen (1994) FEBS Lett. 346:123; J.J. Killion; I.J. Fidler (1994) Immunomethods 4:273.

The invention provides protocols for the administration of pharmaceutical composition comprising multispecific epitope binding proteins of the invention alone or in combination with other therapies to a subject in need thereof. The therapies (e.g., prophylactic or therapeutic agents) of the combination therapies of the present invention can be administered concomitantly or sequentially to a subject. The therapy (e.g., prophylactic or therapeutic agents) of the combination therapies of the present invention can also be cyclically administered. Cycling therapy involves the administration of a first therapy (e.g., a first prophylactic or therapeutic agent) for a period of time, followed by the administration of a second therapy (e.g., a second prophylactic or therapeutic agent) for a period of time and repeating this sequential administration, i.e., the cycle, in order to reduce the development of resistance to one of the therapies (e.g., agents) to avoid or reduce the side effects of one of the therapies (e.g., agents), and/or to improve, the efficacy of the therapies.

The therapies (e.g., prophylactic or therapeutic agents) of the combination therapies of the invention can be administered to a subject concurrently. The term "concurrently" is not limited to the administration of therapies (e.g., prophylactic or therapeutic agents) at exactly the same time, but rather it is meant that a pharmaceutical composition comprising multispecific epitope binding proteins of the invention are administered to a subject in a sequence and within a time interval such that the multispecific epitope binding proteins of the invention can act together with the other therapy(ies) to provide an increased benefit than if they were administered otherwise. For example, each therapy may be administered to a subject at the same time or sequentially in any order at different points in time; however, if not administered at the same time, they should be administered sufficiently close in time so as to provide the desired therapeutic or prophylactic effect. Each therapy can be administered to a subject separately, in any appropriate form and by any suitable route. In various embodiments, the therapies (e.g., prophylactic or therapeutic agents) are administered to a subject less than 15 minutes, less than 30 minutes, less than 1 hour apart, at about 1 hour apart, at about 1 hour to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, 24 hours apart, 48 hours apart, 72 hours apart, or 1 week apart. In other embodiments, two or more therapies (e.g., prophylactic or therapeutic agents) are administered to a within the same patient visit.

The prophylactic or therapeutic agents of the combination therapies can be administered to a subject in the same pharmaceutical composition. Alternatively, the prophylactic or therapeutic agents of the combination therapies can be administered concurrently to a subject in separate pharmaceutical compositions. The prophylactic or therapeutic agents may be administered to a subject by the same or different routes of administration.

### T. Kits

Also within the scope of the invention are kits comprising the compositions (*e.g.* multispecific epitope binding proteins) of the invention and instructions for use. The kit can further contain a least one additional reagent, or one or more additional multispecific epitope binding proteins of the invention. Kits typically include a label indicating the intended use of the contents of the kit. The term label includes any writing, or recorded material supplied on or with the kit, or which otherwise accompanies the kit.

Equivalents

Those skilled in the art will recognize, or be able to ascertain using no more than routine experimentation, many equivalents to the specific embodiments of the invention described herein. Such equivalents are intended to be encompassed by the following claims.

All publications, patents and patent applications mentioned in this specification are herein incorporated by reference into the specification to the same extent as if each individual publication, patent or patent application was specifically and individually indicated to be incorporated herein by reference.

### U. Specific embodiments

1. An isolated multispecific epitope binding protein comprising a first and a second polypeptide chain, wherein the first and/or the second chain comprises at least two epitope binding domains (EBDs) and one or more Fc regions.
2. The protein of embodiment 1 (or any protein of the invention), wherein said first chain comprises one or more Fc regions linked N-terminal to at least one EBD.
3. The protein of embodiment 2 (or any protein of the invention), wherein said first chain comprises domains and Fc regions arranged from N-terminus to C-terminus: one or more Fc regions-EBD-EBD.
4. The protein of embodiment 2 (or any protein of the invention), wherein said first chain comprises domains and Fc regions arranged from N-terminus to C-terminus: EBD-one or more Fc regions-EBD.
5. The protein of embodiment 1 (or any protein of the invention), wherein said first chain comprises one or more Fc regions linked C-terminal to at least one EBD.
6. The protein of embodiment 5 (or any protein of the invention), wherein said first chain comprises domains and Fc regions arranged from N-terminus to C-terminus: EBD-EBD-one or more Fc regions.
7. The protein of embodiment 5 (or any protein of the invention), wherein said first chain comprises domains and Fc regions arranged from N-terminus to C-terminus: EBD-one or more Fc regions-EBD-EBD.
8. The protein of embodiment 1 (or any protein of the invention), wherein said second chain comprises one or more Cκ or Cλ regions linked N-terminal to at least one EBD.
9. The protein of embodiment 1 (or any protein of the invention), wherein said second chain comprises one or more Cκ or Cλ regions linked C-terminal to at least one EBD.
10. The protein of embodiment 1 (or any protein of the invention), wherein the first and/or the second chain comprises at least three EBDs.
11. The protein of embodiment 10 (or any protein of the invention), wherein the first and/or the second chain comprises at least four epitope binding domains EBDs.
12. The protein of embodiment 10 or 11 (or any protein of the invention), wherein each Fc region is linked N-terminal to at least three EBDs.
13. The protein of embodiment 12 (or any protein of the invention), wherein said domains and Fc region are arranged from N-terminus to C-terminus: one or more Fc regions-EBD-EBD-EBD.
14. The protein of embodiment 12 (or any protein of the invention), wherein said domains and Fc region are arranged from N-terminus to C-terminus: one or more Fc regions-EBD-EBD-EBD-EBD.
15. The protein of embodiment 10 or 11 (or any protein of the invention), wherein each Fc region is linked C-terminal to at least one EBD.
16. The protein of embodiment 15 (or any protein of the invention), wherein said domains and Fc region are arranged from N-terminus to C-terminus: EBD-EBD-EBD-one or more Fc regions.
17. The protein of embodiment 16 (or any protein of the invention), wherein said domains and Fc region are arranged from N-terminus to C-terminus EBD-EBD-EBD-EBD-one or more Fc regions.
18. The protein of embodiment 10 or 11 (or any protein of the invention), wherein each Fc region is linked N-terminal to at least one EBD and C-terminal to at least one EBD.
19. The protein of embodiment 18 (or any protein of the invention), wherein said domains and Fc region are arranged from N-terminus to C-terminus: at least one EBD-one or more Fc regions-at least one EBD.
20. The protein of embodiment 18 (or any protein of the invention), wherein said domains and Fc region are arranged from N-terminus to C-terminus: EBD-EBD-one or more Fc regions-EBD.
21. The protein of embodiment 18 (or any protein of the invention), wherein said domains and Fc region are arranged from N-terminus to C-terminus: EBD-EBD-one or more Fc regions-EBD-EBD-EBD.
22. The protein of embodiment 18 (or any protein of the invention), wherein said domains and Fc region are arranged from N-terminus to C-terminus: EBD-EBD-EBD-one or more Fc regions-EBD.
23. The protein of embodiment 18 (or any protein of the invention), wherein said domains and Fc region are arranged from N-terminus to C-terminus: EBD-EBD-one or more Fc regions-EBD-EBD.
24. The protein of embodiment 18 (or any protein of the invention), wherein said domains and Fc region are arranged from N-terminus to C-terminus: EBD-one or more Fc regions-EBD-EBD-EBD.
25. The protein of embodiment 10 (or any protein of the invention), wherein said first polypeptide chain comprises 3 scFvs and an Fc region arranged N-terminus to C-terminus: scFv-scFv-Fc region-scFv and wherein said second polypeptide chain comprises 3 scFvs and an Fc region arranged N-terminus to C-terminus: scFv-scFv-Fc region-scFv.
26. The protein of embodiment 11 (or any protein of the invention), wherein said first polypeptide chain comprises 4 scFvs and an Fc region arranged N-terminus to C-terminus: scFv-scFv-Fc region-scFv-scFv and wherein said second polypeptide chain comprises 4 scFvs and an Fc region arranged N-terminus to C-terminus: scFv-scFv-Fc region-scFv-scFv.
27. The protein of embodiment 1 (or any protein of the invention), wherein said protein comprises a first and a second chain;
   a. said first chain comprising an scFv, an antibody variable region, and an Fc region arranged N-terminus to C-terminus:scFv-antibody variable region-Fc region; and
   b. said second chain comprising an antibody variable region and a Ckappa/lambda region arranged N-terminus to C-terminus:antibody variable region- Cκ or Cλ.
28. The protein of embodiment 1 (or any protein of the invention), wherein said protein comprises a first and a second chain;
   a. said first chain comprising an antibody variable region, and an Fc region arranged N-terminus to C-terminus: antibody variable region-Fc region; and
   b. said second chain comprising an scFv, antibody variable region, and a Ckappa/lambda region arranged N-terminus to C-terminus:scFv-antibody variable region- Cκ or Cλ.
29. The protein of embodiment 1 (or any protein of the invention), wherein said protein comprises a first and a second chain;
   a. said first chain comprising an scFv, an antibody variable region, and an Fc region arranged N-terminus to C-terminus:scFv-antibody variable region-Fc region; and
   b. said second chain comprising an scFv, an antibody variable region, and a Ckappa/lambda region arranged N-terminus to C-terminus:scFv-antibody variable region- Cκ or Cλ.
30. The protein of any of embodiments 27-29 (or any protein of the invention), wherein at least two EBDs are linked C-terminal to at least one Fc region.
31. The protein of any of embodiments 27-30 (or any protein of the invention), wherein said protein comprises an antibody heavy chain and an antibody light chain.
32. A multispecific epitope binding protein comprising a first and a second polypeptide chain wherein the first and/or the second chain comprises at least two EBDs and one or more CH1 domains.
33. The protein of embodiment 32 (or any protein of the invention), wherein said protein further comprises a Ckappa/lambda domain.
34. The protein of embodiment 33 (or any protein of the invention), wherein said first chain comprises two scFvs linked to a CH1 domain arranged N-terminus to C-terminus:scFv-CH1-scFv, said second chain comprises two scFvs linked to a Ckappa/lambda domain arranged N-terminus to C-terminus:scFv-Ckappa/lambda-scFv.
35. The protein of embodiment 34 (or any protein of the invention), wherein said first and/or second chain comprises all or a portion of an antibody hinge domain.
36. The protein of embodiment 35 (or any protein of the invention), wherein said first and second polypeptide chains are linked by a disulfide bond.
37. A multispecific epitope binding protein comprising at least a first and a second chain, said first chain comprises two variable domains and a Cκ or a Cλ domain and said second chain comprises two variable domains, a CH1, a Hinge, a CH2, and a CH3 domain, wherein said variable domains in said first chain associate with the variable domains in said second chain to form at least two distinct epitope binding sites.
38. The protein of embodiment 37 (or any protein of the invention), wherein said first chain comprises variable domains arranged N-terminal to C-terminal that are specific to a first epitope and second epitope and said second chain comprises variable domains arranged N-terminal to C-terminal that are specific to said first and said second epitopes.
39. A multispecific epitope binding protein comprising a first and a second chain, wherein said first chain comprises at least 2 antibody variable domains and at least one CH1 and/or Ckappa/lambda domains, wherein said variable domains in said first chain associate with the variable domains in said second chain to form at least two different epitope binding sites.
40. The protein of embodiment 39 (or any protein of the invention), wherein said first chain comprises an antibody heavy chain variable domain (VH), a CH1 domain, an antibody light chain variable domain (VL), and a Ckappa/lambda domain.
41. The protein of embodiment 40 (or any protein of the invention), wherein said first chain is organized N-terminus to C-terminus: VH-CH1-VL-Ckappa/lambda.
42. The protein of embodiment 40 (or any protein of the invention), wherein said first chain is organized N-terminus to C-terminus: VL-Ckappa/lambda-VH1-CH1.
43. The protein of embodiment 39 (or any protein of the invention), wherein said first chain comprises 2 antibody heavy chain variable domains (VH), and 2 CH1 domains.
44. The protein of embodiment 43 (or any protein of the invention), wherein said first chain is organized N-terminus to C-terminus: VH-CH1-VH-CH1.
45. The protein of embodiment 39 (or any protein of the invention), wherein said first chain comprises 2 antibody light chain variable domains (VL), and 2 Ckappa/lambda domains.
46. The protein of embodiment 45 (or any protein of the invention), wherein said first chain is organized N-terminus to C-terminus: VL-Ckappa/lambda-VL-Ckappa/lambda.
47. The protein of any of embodiments 39-46 (or any protein of the invention), wherein said first chain further comprises an Fc region.
48. The protein of any of embodiments 39-46 (or any protein of the invention), wherein said second chain does not comprise an Fc region.
49. An inverted antibody protein comprising at least a first and a second polypeptide chain wherein said first chain comprises at least one antibody heavy chain variable region (VH) linked to a CH1 domain and said second chain comprises an antibody light chain variable region (VL) linked to a Ckappa/lambda domain, said Ckappa/lambda region further linked to an Fc region, wherein said variable region in said first chain associate with the variable region in said second chain to form an epitope binding site.
50. The protein of embodiment 49 (or any protein of the invention), wherein said first chain is disulfide linked to said second chain.
51. A multispecific epitope binding protein comprising an antibody-like light chain and an antibody-like heavy chain wherein said light chain comprises two variable domains and a Cκ or a Cλ domain and said heavy chain comprises two variable domains, a CH1, a Hinge, a CH2, and a CH3 domain, wherein said variable domains in said light chain associate with the variable domains in said heavy chain to form at least two distinct epitope binding sites.
52. The protein of embodiment 51 (or any protein of the invention), wherein said light chain comprises variable domains arranged N-terminal to C-terminal are specific to a first epitope and second epitope and said heavy chain comprises variable domains arranged N-terminal to C-terminal are specific to said first and said second epitopes.
53. The protein of embodiment 52 (or any protein of the invention), wherein said protein further comprises at least 2 linked EBDs.
54. The protein of embodiment 53 (or any protein of the invention), wherein at least one EBD is linked to the C-terminus of said heavy chain.
55. The protein of embodiment 53 (or any protein of the invention), wherein at least one EBD is linked to the N-terminus of said light chain.
56. The protein of embodiment 53 (or any protein of the invention), wherein at least one EBD is linked to the N-terminus of said heavy chain.
57. A multispecific epitope binding protein comprising an antibody light chain and an antibody heavy chain, wherein said heavy chain further comprises at least 2 linked EBDs.
58. The protein of embodiment 57 (or any protein of the invention), wherein at least one EBD is linked to the C-terminus of said heavy chain.
59. The protein of embodiment 57 (or any protein of the invention), wherein at least one EBDs is linked to the N-terminus of said heavy chain.
60. The protein of embodiment 57 (or any protein of the invention), wherein at least one EBD is linked to the N-terminus and to the C-terminus of said heavy chain.
61. A multispecific epitope binding protein comprising a first and a second polypeptide chain wherein the first and/or the second chain comprises at least three EBDs and said first chain comprises a Cκ or a Cλ domain and said second chain comprises a CH1 domain.
62. A multispecific epitope binding protein comprising a structural format presented in any of the Figures 1-5 (or any protein of the invention).
63. The protein of any one of embodiments 1-24, 30-36, or 53-61 (or any protein of the invention), wherein each EBD is specific for the same epitope.
64. The protein of any one of embodiments 1-24, 30-36, or 53-61 (or any protein of the invention), wherein at least two EBDs are specific for different epitopes.
65. The protein of embodiment 63 or 64 (or any protein of the invention), wherein each epitope is located on the same antigen.
66. The protein of embodiment 65 (or any protein of the invention), wherein each epitope is located on different antigens.
67. The protein of embodiment any one of embodiments 1-24, 30-36, or 53-61 (or any protein of the invention), wherein two or more EBDs are specific for the same epitope.
68. The protein of any one of embodiments 1-67 (or any protein of the invention), wherein the protein is capable of binding at least two, at least three, at least four, at least five, at least six epitopes concurrently upon administration to a mammal or in an in vitro.
69. The protein of any of any one of embodiments 1-24, 30-36, or 53-61 (or any protein of the invention), wherein at least one EBD specifically binds an epitope with an affinity less than the identical isolated functional EBD.
70. The protein of embodiment 69 (or any protein of the invention), wherein said EBD is selected from the group consisting of the most N-terminal EBD, the second most N-terminal EBD, and the third most N-terminal EBD.
71. The protein of any one of embodiments 1-24, 30-36, 53-61, 63-67, and 69-70 (or any protein of the invention) wherein at least one EBD is selected from the group consisting of an scFv, a single chain single chain diabody, an antibody mimetic, and an antibody variable domain.
72. The protein of embodiment 71 (or any protein of the invention), wherein said antibody mimetic is selected from the group consisting of a minibody, a maxybody, an avimer, an Fn3 based protein scaffold, an ankrin repeat, a VASP polypeptide, an avian pancreatic polypeptide (aPP), a Tetranectin, an affililin, a knottin, an SH3, a PDZ domain, a protein A domain, a lipocalin, a transferrin, and a kunitz domains.
73. The protein of any of embodiments 1-72 (or any protein of the invention), wherein said protein specifically binds distinct cell surface receptors and inhibits and/or neutralizes said cell surface receptors.
74. The protein of embodiment 73 (or any protein of the invention), wherein said cell surface receptors are identical receptors.
75. The protein of embodiment 74 (or any protein of the invention), wherein said cell surface receptors are not identical receptors.
76. The protein of any of embodiments 1-75 (or any protein of the invention), wherein said protein specifically binds distinct soluble ligands and inhibits and/or neutralizes said ligands.
77. The protein of embodiment 76 (or any protein of the invention), wherein said soluble ligands are identical.
78. The protein of embodiment 76 (or any protein of the invention), wherein said soluble ligands are not identical.
79. The protein of any of embodiments 1-78 (or any protein of the invention), wherein said protein specifically binds distinct target proteins and inhibits and/or neutralizes said target proteins.
80. The protein of embodiment 79 (or any protein of the invention), wherein said target proteins are identical.
81. The protein of embodiment 79 (or any protein of the invention), wherein said target proteins are not identical.
82. The protein of any of embodiments 1-24, 30-36, 53-61, 63-67, and 69-70 (or any protein of the invention), wherein at least one EBD retains the functional activity of the identical EBD isolated from said protein.
83. The protein of any of embodiments 1-24, 30-36, 53-61, 63-67, and 69-70 (or any protein of the invention), wherein said protein has at least equivalent functional activity as a composition comprising each EBD isolated from said protein.
84. The protein of any of embodiments 1-24, 30-36, 53-61, 63-67, and 69-70 (or any protein of the invention), wherein said protein has greater functional activity as a composition comprising each EBD isolated from said protein.
85. The protein of any of embodiments 1-24, 30-36, 53-61, 63-67, and 69-70 (or any protein of the invention), wherein said protein has 50 % less functional activity as compared to a composition comprising each EBD isolated from said protein.
86. The protein of any of embodiments 1-85 (or any protein of the invention), wherein said protein has the functional activity of depleting a cell population selected from the group consisting of: T cells, B cells, mast cells, eosinophils, basophils, neutrophils, dendritic cells, monocytes, macrophages, and tumor cells.
87. The protein of any of embodiments 1-85 (or any protein of the invention), wherein said protein has the functional activity of inhibiting or reducing proliferation of cells selected from the group consisting of: T cells, B cells, mast cells, eosinophils, basophils, neutrophils, dendritic cells, monocytes, macrophages, and tumor cells.
88. The protein of any of embodiments 1-85 (or any protein of the invention), wherein said protein has the functional activity of inhibiting or reducing secretion of inflammatory mediators from cells selected from the group consisting of: T cells, B cells, mast cells, eosinophils, basophils, neutrophils, dendritic cells, monocytes, macrophages, and tumor cells.
89. The protein of any of embodiments 1-85 (or any protein of the invention), wherein said protein has the functional activity of inhibiting or reducing secretion of cytoplasmic granules from cells selected from the group consisting of: T cells, mast cells, eosinophils, basophils, neutrophils, monocytes, and macrophages.
90. The protein of any of embodiments 1-85 (or any protein of the invention), wherein said protein has the functional activity of inhibiting or reducing the response to an activating stimuli in cells selected from the group consisting of: T cells, B cells, mast cells, eosinophils, basophils, neutrophils, dendritic cells, monocytes, macrophages, and tumor cells.
91. The protein of any of embodiments 1-85 (or any protein of the invention), wherein said protein has the functional activity activating a protein through binding of one or more epitopes.
92. The protein of any of embodiments 1-85 (or any protein of the invention), wherein said protein has the functional activity of deactivating a protein through binding of one or more epitopes.
93. The protein of any of embodiments 1-85 (or any protein of the invention), wherein said protein has effector function when administered to a mammal or *in vitro.*
94. The protein of embodiment 93 (or any protein of the invention), wherein said effector function is antibody dependent cellular cytotoxicity.
95. The protein of embodiment 93 (or any protein of the invention), wherein said effector function is complement dependent cytotoxicity.
96. An isolated nucleic acid molecule encoding the protein, or epitope-binding portion thereof, of any one of embodiments 1-95 (or any protein of the invention).
97. An expression vector comprising the nucleic acid molecule of embodiment 96.
98. A host cell comprising the expression vector of embodiment 97.
99. A method of making the protein of any of embodiments 1-95 (or any protein of the invention), wherein said method comprises a scalable process for making said protein wherein said scalable process results in a production efficiency of said protein from about 10 mg/L to about 300 mg/L and said protein retains at least one functional activity.
100. The method of embodiment 99, wherein said protein produced from said process exhibits an aggregation level of no more than 5% by weight of protein as measured by HPSEC.
101. The method of embodiment 99, wherein said protein produced by said process exhibits an aggregation level of no more than 5% by weight of protein as measured by rCGE.
102. The method of embodiment 99, wherein said protein produced by said process exhibits a low level of fragmentation as demonstrated by 80% or higher of the total peak area in the peaks representing the intact said proteins as measured by HPSEC.
103. A liquid formulation comprising the protein of any of embodiments 1-95 (or any protein of the invention), wherein said formulation exhibits an aggregation level of no more than 5% by weight of protein as measured by HPSEC.
104. A liquid formulation comprising the protein of any of embodiments 1-95 (or any protein of the invention), wherein said formulation exhibits an aggregation level of no more than 5% by weight of protein as measured by rCGE.
105. A liquid formulation comprising the protein of any of embodiments 1-95 (or any protein of the invention), wherein said formulation exhibits a low level of fragmentation as demonstrated by 80% or higher of the total peak area in the peaks representing the intact said proteins as measured by HPSEC.
106. A sterile formulation comprising a therapeutically effective amount of the protein of any of embodiments 1-95 (or any protein of the invention)in a pharmaceutically-acceptable excipient.
107. A method of ameliorating, treating or preventing cancer or a symptom thereof by administering the formulation of embodiment 106 to a patient in need thereof.
108. The method of embodiment 107, wherein said cancer is of the head, neck, eye, mouth, throat, esophagus, chest, bone, lung, colon, rectum, colorectal, stomach, spleen, renal, skeletal muscle, subcutaneous tissue, metastatic melanoma, endometrial, prostate, breast, ovaries, testicles, skin, thyroid, blood, lymph nodes, kidney, liver, pancreas, brain or central nervous system.
109. A method of depleting a cell population in a mammal wherein said cell population is selected from the group consisting of eosinophils, basophils, neutrophils, T cells, B cells, mast cells, monocytes and tumor cell comprising contacting said cell with the formulation of embodiment 106.
110. A method of killing or targeting a pathogen comprising contacting the pathogen in a mammal with the formulation of embodiment 106.
111. A method of inactivating, inhibition, or depleting a cytokine comprising contacting said cytokine in a mammal with the formulation of embodiment 106.
112. The method of embodiment 111, wherein said cytokine is C5a.
113. A method of preventing, treating managing or diagnosing an inflammatory or autoimmune disease in a patient in need thereof by administering the formulation of embodiment 106.
114. A method of inhibiting angiogenesis in a patient in need thereof by administering the formulation of embodiment 106.
115. The method of embodiment 113 or 114, wherein said patient has cancer, rheumatoid arthritis, SLE, or Sjogren's syndrome.
116. The protein of any of embodiments 1-95 (or any protein of the invention), wherein said protein identifies and/or depletes a population of cells when administered to a mammal or *in vitro,* said cells defined by the expression of distinct cell surface epitopes, said distinct cell surface epitopes selectively engaged by at least one epitope binding domain of said protein.
117. The protein of embodiment 116 (or any protein of the invention), wherein said cell population is selected from the group consisting of tumor cells, cancer stem cells, B cells, and T cells.
118. The protein of any of embodiments 1-95 (or any protein of the invention), wherein said protein comprises epitope binding domains specific for cell surface antigens presented on a tumor cell.
119. The protein of embodiment 118 (or any protein of the invention), wherein said cell surface antigens are not displayed concurrently.
120. The protein of embodiment 118 or 119 (or any protein of the invention), wherein said protein elicits antibody effector function when administered to a mammal, directed at the tumor cell when at least one epitope binding domain is engaged.
121. The protein of any of embodiments 1-95 (or any protein of the invention), wherein said protein comprises at least one epitope binding domain, said binding domain specific for a cargo molecule to be delivered to a cell when said protein is administered to a mammal, or *in vitro.*
122. The protein of embodiment 121 (or any protein of the invention), wherein said cargo molecule is selected from the group consisting of a cytotoxic drug, an anti-metabolite, a toxin, a peptide, a DNA molecule, an RNA molecule, a small molecule, a radioisotope, a fluorophore, an enzyme, an enzyme inhibitor, a prodrug, or a mitochondrial poison.
123. The protein of embodiment 121 or 122 (or any protein of the invention), wherein said protein internalizes when bound to said cell.
124. The protein of any of embodiments 121-123 (or any protein of the invention), wherein said epitope binding domain specific for a cargo molecule exhibits high binding affinity for the cargo molecule at about pH 7.4, and a low binding affinity for the cargo molecule at about pH 6.0.
125. A method of identifying, depleting, activating, or inhibiting a target cell population comprising contacting the protein of any of embodiments 1-95 (or any protein of the invention) with said target cell population when administered to a mammal or *in vitro,* wherein said protein does not significantly deplete, activate, or inhibit a non-target cell population.
126. The method of embodiment 125, wherein said protein exhibits increased avidity for the target cell population over a control epitope binding protein, wherein said control epitope binding protein comprises either:
   a. a subset of epitope binding domains present in the multispecific epitope binding protein; or
   b. at least one isolated epitope binding domain present in the multispecific epitope binding protein.
127. The method of embodiment 126, wherein said subset comprises at least one, at least two, at least three, at least four, at least five, at least six, at least seven, or at least eight epitope binding domains.
128. The method of embodiments 125-127, wherein said target cell is selected from the group consisting of: a cancerous cell, a cancer stem cell, a T cell, a B cell, a melanoma cell, a lymphoma cell, a tumor cell, a pre-B cell, a pre-T cell, a basophil, a monocyte, and a macrophage.
129. A method of prevention, management, treatment or diagnosis of acute or chronic diseases in a patient using a protein of any of embodiments 1-95 or 116-124 (or any protein of the invention).
130. A method of prevention, management, treatment or diagnosis of acute or chronic diseases in a patient using the method of any of embodiments 107-115 or 125-128.
131. A method of using the protein of any of embodiments 1-95 or 116-124 (or any protein of the invention), in a mammal to reduce toxicity associated with exposure to one or more agents selected from the group consisting of abrin, brucine, cicutoxin, diphtheria toxin, botulism toxin, shiga toxin, endotoxin, tetanus toxin, pertussis toxin, anthrax toxin, cholera toxin, falcarinol, alfa toxin, geldanamycin, gelonin, lotaustralin, ricin, strychnine, snake venom toxin and tetradotoxin.
132. A method of detecting and/or purifying at least one soluble compound from a solution using a protein of any of embodiments 1-95 or 116-124 (or any protein of the invention).
133. The method of embodiment 132, wherein said solution is a bodily fluid, cell culture media, fermentation media fluid, biological sample, or potable water.
134. The method of embodiment 133, wherein said bodily fluid is selected from the group consisting of blood, sweat, lymph, urine, tears, bile, saliva, serum, amniotic fluid, cerumen (earwax), Cowper's fluid, semen, chyle, chime, cerebrospinal fluid, stool, stool water, pancreatic juice, synovial fluid, aqeous humor, interstitial fluid, breast milk, mucus, pleural fluid, pus, sebum, and vomit.
135. A method of reducing toxicity of a cytokine storm in a mammal using the protein of any of embodiments 1-95 or 116-124 (or any protein of the invention).
136. A method of reducing therapy-induced toxicity in a mammal using the protein of any of embodiments 1-95 or 116-124 (or any protein of the invention), wherein said therapy is a biological therapy.
137. The method of embodiment 136, wherein said protein comprises at least one epitope binding domain derived from said biological therapy.
138. The method of embodiment 136, wherein said proteins comprises at least one epitope binding domain that competes with at least one component of said biological therapy.
139. The protein of any of embodiments 1-95 or 116-124 (or any protein of the invention),wherein said protein exhibits a half-life of at least 1 day when administered to a mammal.
140. The protein of any of embodiments 1-95 or 116-124 (or any protein of the invention), wherein said Fc region is directly linked to at least one additional domain.
141. The protein of any of embodiments 1-95 or 116-124 (or any protein of the invention), wherein said Fc region is indirectly linked to at least one additional domain.

In addition, the following United States provisional patent applications: 60/935,199 filed July 31, 2007,61/012,656 filed December 10, 2007 and 61/074, 330 filed June 20, 2008 are hereby incorporated by reference herein in their entireties for all purposes.

### 7. Examples

The following examples serve merely to illustrate the invention and are not intended to limit the invention in any way.

### Example 1. Expression of proteins comprising scFv domains fused to an Fc region

Purpose: To demonstrate high level expression of epitope binding proteins comprising scFv domains linked to Fc regions.

Methods: The vectors encoding 3F2-522-Fc region and 522-Fc region are used to transfect 293F cells using the 293Fectin™ reagent (Invitrogen Cat. 51-0031) and Freestyle™ media (Invitrogen Cat. 12338) + 10% fetal bovine serum following the manufacturer's recommendations. The cells are fed on the third day post transfection and the supernatant are harvested on day six. Purification of the antibodies is accomplished was via a protein A column and is followed by dialysis into PBS. The molecules are evaluated in the denatured and non-denatured forms on a protein gel to determine the size and relative purity of the protein.

Results: Presented in Figure 7 are the results of a PAGE gel experiment wherein various proteins were subjected to (A) Non-denaturing or (B) denaturing conditions. Lanes 1 and 5 represent 3F2-522-Fc region (a example of 2 scFv domains linked to an Fc region) loaded at 1 µg/well. Lanes 2 and 6 represent 3F2-522-Fc region loaded at 4 µg/well. Lanes 3 and 7 represent an scFv-Fc region protein 522-Fc (an example of one scFv linked to an Fc region) loaded at 1 µg/well. Lanes 4 and 8 represent 522-Fc loaded at 4 µg/well. Lane M represents standard molecular weight markers (SeeBlue 2™). The production and subsequent purification of the proteins yielded at considerably pure product of the predicted size. These results demonstrate that the proteins 3F2-522scFv-Fc and 522-Fc can be made and purified to homogeneity using the above described methods.

### Example 2. Multiple epitope binding proteins exhibit specificity for target antigens.

Purpose: To evaluate the ability of the certain proteins to bind target antigens

Methods: To evaluate binding of 522-Fc region and 3F2-522-Fc an ELISA based format was used. In general, the EIA/RIA ELISA plates (Costar cat. 3690) were coated with 50µl at 1 µg/ml of the capture protein in PBS (pH 7.2) and incubated at 4°C overnight. The next day, the plates were washed using an Elₓ405 auto plate washer programmed for five dispense/aspirate wash steps with 1X PBST (1X PSB, 0.1% Tween 20) separated by three second shaking intervals. The plates were patted dry on a stack of paper towels and blocked with 170µl of blocking buffer (2% BSA w/v in1X PBST) for one hour at room temperature. 522-Fc region and 3F2-522-Fc were titrated in another plate with blocking buffer through eight wells starting at 5ug/ml and 50ul were added to the blocked wells ELISA plate. After a 1 hour incubation step at room temperature, the plates were washed again using the Elₓ405 auto plate washer and patted dry. To each well, 50 µl of the secondary HRP labeled antibody was added and allowed to incubate for 1 hour. The plates were washed, rotated 180° and washed again. They were patted dry and 50 µl of SureBlue TMB peroxidase (KPL cat. 52-00-03) added to each well and allowed to develop for approximately 3 minutes. The reaction was stopped with 50 µl of 0.2M H₂SO₄ and the ELISA signal was read at 450nM. In (A) the capture protein was α_{ν}β₃ integrin while in (B) the capture protein was either EphA2 or α_{ν}β₃ integrin.

Results: The epitope binding proteins, 522-Fc and 3F2-522-Fc were analyzed for the ability to bind to the α_{ν}β₃ integrin and biotinylated EphA2-Fc, the results of which are presented in Figure 8A. Two different samples of the 3F2-522-Fc protein demonstrated the ability to bind concurrently to α_{ν}β₃ integrin (immobilized on the plate) and soluble EphA2-Fc. The 522-Fc region protein was not detected by the biotinylated EphA2 as it is only specific for the α_{ν}β₃ integrin. The dual specificity of the 3F2-522-Fc region protein for α_{ν}β₃ integrin and EphA2 was analyzed, the results of which are presented in Figure 8B. EphA2 or α_{ν}β₃ (immobilized on the plate) was allowed to bind increasing concentrations of soluble 3F2-522-Fc-biotin . After washing, the presence of 3F2-522-Fc-biotin was detected by a strepavidin-conjugated reagent. At increasing concentrations, the soluble 3F2-522-Fc specifically binds to both plate bound EphA2 and α_{ν}β₃ integrin. These results demonstrate that the epitope binding protein 3F2-522-Fc region is capable of binding both EphA2 and α_{ν}β₃ integrin.

### Example 3. High level expression of epitope binding proteins comprising scFv domains fused to Fc regions.

Purpose: To demonstrate high level expression of epitope binding proteins comprising scFv domains fused to Fc regions.

Methods: The epitope binding protein P1 was constructed by combining three epitope binding proteins, an antibody specific for EphA2 (12G3H11), an scFv specific for an EphA family RTK(EA), and an scFv specific for an EphB family RTK (EB). The resultant structure is disclosed in Figures 3C and 3D.

The vectors encoding 522-Fc region, 3F2-522-Fc region, P1, and 12G3H11 were used to transfect 293F cells using the 293Fectin™ reagent (Invitrogen Cat. 51-0031) and Freestyle™ media (Invitrogen Cat. 12338) + 10% fetal bovine serum following the manufacturer's recommendations. The cells were fed on the third day post transfection and the supernatant was harvested on day six. Purification of the epitope binding proteins were via a protein A column and followed by dialysis into PBS. The molecules were evaluated in the denatured and non-denatured forms on a protein gel to determine the size and relative purity of the proteins.

Results: Presented in Figure 9 are the results from a polyacrylamide gel electrophoresis experiment of a collection of certain epitope binding proteins. Briefly, purified samples of each of the proteins were loaded and run on a PAGE gel and subsequently stained with Coomassie Blue. The proteins presented are as follows: Lane 1 - 522-Fc region, Lane 2- 3F2-522-Fc region. Lane 3- P1 (see Figures 3C,D for a diagram of the structure), Lane 4 - 3F2-522-Fc Lane 5 -12G3H11 (antibody specific for EphA2), and Lane 6 - 3F2-522-Fc. The production and subsequent purification of the multispecific epitope binding polypeptides yielded at considerably pure product of the predicted sizes. These results demonstrate that epitope binding proteins such as those described in the Figures and detailed description can be made and purified to homogeneity using the above-described methods.

### Example 4. SEC purification of epitope binding protein P2

Purpose: To demonstrate homogeneity of protein composition by Size Exclusion Chromatography

Methods: The epitope binding protein P2 was constructed by combining two epitope binding proteins, an antibody specific for EphA2 (12G3H11) and a single chain diabody comprising two sets of variable regions, one specific for an EphA family RTK(EA), and the other specific for an EphB family RTK (EB). The resultant structure is disclosed in Figures 3E and 3F.

To purify the P2 multiple epitope binding protein (see Figures 3E, F for a diagram of the structure) based on its large size, the protein was run over and Hi Prep 16/60 Sephacryl S-200 column (Amersham cat. 17-1166-01). The P2 multiple epitope binding protein molecule was concentrated to a final volume of 1ml at 4.3mg/ml. First, using an Akta primer, the SEC column was equilibrated in 1X PBS at 1ml per minute for 1.5 hour to remove the column storage buffer. The concentrated protein was injected into a 2ml loop and then onto the SEC column. The PBS buffer was loaded onto the column at 1ml/min for 120 minutes and 1ml fractions were collected for the entire run.

Results: Presented in Figure 10 is the elution profile of the P2 protein from a SEC column. The tracing represents the relative protein concentration in each column fraction (x axis). The results demonstrate that the P2 multiple epitope binding protein elutes as a homogenous single entity.

### Example 5. Cation Exchange Chromatography profile of epitope binding proteins

Purpose: To establish a cation exchange chromatography profile of the P1 multiple epitope binding protein

Methods: The P1 protein was purified by ion exchange chromatography. With a pI of about 9.1, the multiple epitope binding protein at pH 6 would have a net positive charge. A strong cationic exchanger column, (Amersham Cat. 17-5054-01), was used to separated the different molecular species. The column was equilibrated in 50mM phosphate buffer with 20mM NaCl. After equilibration the protein was loaded onto the Hitrap SP FF column at 1ml/min, the column was washed with three column volumes of equilibration buffer. A gradient elution was setup on the Akta prime for the equilibration buffer to 50mM phosphate buffer with 20mM NaCl over 100 minutes. The gradient was held at various points to allow better sampling of the different peaks. Fractions were collected over the entire gradient.

Results: Presented in Figure 11 is the elution profile of the P1 protein from a cation exchange column. The tracing represents the relative protein concentration in each column fraction (x axis).

### Example 6. PAGE analysis of purified P1 protein fractions

Purpose: To evaluate the purity and functionality of the P1 containing fractions from the cation exchange column presented in Example 5.

Methods: The column fractions were prepared as described in Example 5. Subsequent analysis was as follows. Column fractions were pooled in accordance with the elution peaks observed from the column (Figure 11). Specifically, fractions were pooled as follows: Group 1 (5-22), Group 2 (23-28), Group 3 (29-34), Group 4 (35-41), Group 5 (42-49), Group 6 (50-54) and Group 7 (55-73). Samples from each group (original, 1-7) were loaded on both a non-denaturing (A) and denaturing (B) PAGE gels and run under standard conditions. In addition, samples (original, 1-7) were tested for the ability to bind to EphA2 and another EphA family RTK in an ELISA based binding assay similar to the assay described in Example 2. The P1 protein containing samples were bound to the ELISA plate and subsequently incubated with soluble EphA2-Fc or EphA family RTK-Fc. As presented in Figure 12C, all of the fractions exhibit specific binding to both EphA2 and another EphA family RTK as compared to the original sample. Group 6 and 7 exhibit reduced binding for the other EphA family RTK. These results demonstrate that the P1 protein retains binding specificity after subjected to Cation Exchange Chromatography as defined by the above methods section.

### Example 7. Determining binding specificities of epitope binding proteins P1 and P2

Methods: The experiment was performed as described in Example 2 with the capture antigen being designated as another EphA family RTK.

Results: Presented in Figure 13 are the results of a binding assay performed on various epitope binding proteins. Specifically demonstrated here is the specificity for EphA2 by the antibody 12G3H11 and the multispecific epitope binding proteins P1 and P2. The EA, EB1 and EB2 epitope binding proteins do not exhibit specificity for EphA2 as expected as they do not contain EphA2 specific binding motifs. These results demonstrate that the P1 and P2 proteins retain binding for EphA2 with a similar profile to the 12G3H11 base antibody.

### Example 8. Determining binding specificities of epitope binding proteins P1 and P2

Methods: The experiment was performed as described in Example 2 with the capture antigen being designated as another EphA family RTK.

Results: Presented in Figure 14 are the results of a binding assay performed on various epitope binding proteins. Specifically demonstrated here is the specificity for an EphA family RTK by the multispecific epitope binding proteins P1 and P2 and EA. The 12G3H11, EB1 and EB2 epitope binding proteins do not exhibit specificity for the EphA family RTK as expected as they do not contain specific binding motifs for the EphA family RTK. These results demonstrate that the P1 and P2 proteins retain binding for the EphA family RTK with a similar profile to the EA base binding protein.

### Example 9. Determining binding specificities of epitope binding proteins P1 and P2

Methods: The experiment was performed as described in Example 2 with the capture antigen being designated as an EphB family RTK.

Results: Presented in Figure 14 are the results of a binding assay performed on various epitope binding proteins. Specifically demonstrated here is the specificity for an EphB family RTK by EB1, EB2 and the multispecific epitope binding proteins P1 and P2. The 12G3H11 and EA epitope binding proteins do not exhibit specificity for the EphB family RTK as expected as they do not contain specific binding motifs for the EphB family RTK. The P2 protein appears to have a lower affinity for the EphB family RTK, however it does demonstrate specificity. These results demonstrate that the P1 and P2 proteins retain binding for the EphB family RTK with a similar profile to the EB1 and EB2 base binding proteins.

### Example 10. Determining multiple binding specificities of epitope binding proteins P1 and P2

Methods: To evaluate multiple binding specificities of certain epitope binding proteins, a modified ELISA based binding assay was performed. EIA/RIA ELISA plates (Costar cat. 3690) were coated with 50µl at 1 µg/ml of Eph receptor or ανβ₃ integrin in PBS (pH 7.2) and incubated at 4°C overnight. The next day, the plates were washed using an Elₓ405 auto plate washer programmed for five dispense/aspirate wash steps with 1X PBST (1X PSB, 0.1% Tween 20) separated by three second shaking intervals. The plates were patted dry on a stack of paper towels and blocked with 170µl of blocking buffer (2% BSA w/v in1X PBST) for one hour at room temperature. The antibodies were titrated in another plate with blocking buffer through eight wells start at 5ug/ml and 50ul were add to the blocked wells ELISA plate. After a 1 hour incubation at room temperature, the plates were washed again using the Elₓ405 auto plate washer and patted dry. To each well, 50 µl of the secondary HRP labeled antibody was added and allowed to incubate for an hour. The plates were washed, rotated 180° and washed again. They were patted dry and 50 µl of SureBlue TMB peroxidase (KPL cat. 52-00-03) added to each well and allowed to develop for approximately 3 minutes. The reaction was stopped with 50 µl of 0.2M H₂SO₄ and the ELISA signal was read at 450nM. The Analysis of the polyspecific and parental antibodies binding to the individual antigens were done on EphA2, an EphA family RTK, an EphB family RTK, or ανβ₃ integrin prepared in house.

To ensure that each binding domain was functional, analysis with dual antigen binding was investigated. Specifically, EIA/RIA ELISA plates (Costar cat. 3690) were coated with 50µl at 1 µg/ml of Eph receptor or ανβ3 integrin in PBS (pH 7.2) and incubated at 4°C overnight. The next day, the plates were washed using an Elₓ405 auto plate as describe above. The plates were blocked with 170 µl of blocking buffer for one hour at room temperature. Eph receptors or ανβ₃ integrin were biotinylated with EZ-link sulfo-NHS-Biotin Reagent (Pierce cat. 21335) at a challenge ratio of eight biotins per Eph receptor molecule. The free biotin was removed using a NAP5 column (Pierce Cat. 17-0853-02) and the biotinylated protein diluted to 1 µg/ml in blocking buffer. The plates were washed again using the Elₓ405 auto plate washer and patted dry. To each well, 50 µl of the diluted biotinylated Eph receptors or ανβ3 integrin were added and incubated at 37°C for one hour. The plates were washed and dry as before and 50 µl neutravidin-HRP 1:12500 (Pierce cat. 31002) added. After an hour incubation at 37°C, the plates were washed, rotated 180° and washed again. They were patted dry and 50 µl of SureBlue TMB peroxidase (KPL cat. 52-00-03) added to each well and allowed to develop for 5-10 minutes. The reaction was stopped with 50 µl of 0.2M H₂SO₄ and the ELISA signal was read at 450nM. The coated Eph receptor antigen was different from the biotinylated antigen used for later steps in the ELISA.

Results: In Figure 16(A) 12G3H11, EA, P1 and P2 proteins were analyzed for the ability to be captured on an ELISA plate by bound EphA2 and detected by a biotinylated EphA family RTK protein. The tracings demonstrate that only the P1 and P2, are capable of concurrently binding plate bound EphA2 and biotinylated EphA family RTK. 12G3H11 and EA were unable to be captured with the EphA2 and bind biotinyated EphA family RTK concurrently. In Figure 16(B), EB2, EA, P1 and P2 were analyzed for the ability to be captured on an ELISA plate by a bound EphA family RTK and detected by a biotinylated EphB family RTK protein. The tracings demonstrate that only the P1 and P2 proteins are capable of concurrently binding a plate bound EphA family RTK and biotinylated EphB family RTK. EA and EB2 were unable to be captured with the EphA family RTK and bind a biotinyated EphB family RTK concurrently.

### Example 11. Determining multiple binding specificities of the epitope binding proteins P1 and P2

Methods: The experiment was performed as described in Example 10 with the following modification: the capture antigen was the EphA family RTK.

Results: In Figure 17(A) 12G3H11, EB1, P1 and P2 were analyzed for the ability to be captured on an ELISA plate by a bound EphA family RTK and detected by a biotinylated EphA2 protein. The tracings demonstrate that only the P1 and P2 are capable of concurrently binding plate bound EphA family RTK and biotinylated EphA2. 12G3H11 and EB1 were unable to be captured with the EphA family RTK and bind biotinyated EphA2 concurrently. In Figure 17(B), 12G3H11, EB1; P1 and P2 proteins were analyzed for the ability to be captured on an ELISA plate by a bound EphA family RTK and detected by a biotinylated EphB family RTK protein. The tracings demonstrate that only the P1 and P2 proteins are capable of concurrently binding a plate bound EphA family RTK and biotinylated EphB family RTK. 12G3H11 and EB2 were unable to be captured with the EphA family RTK and bind a biotinyated EphB family RTK concurrently. These results further support the multiple binding specificities exhibited by the P1 and P2 proteins.

### Example 12. Evaluating the multispecific nature of the P1 and P2 proteins

Methods: The experiment was performed as described in Example 10 with the following modification: the capture antigen was the EphB family RTK.

Results: In Figure 18(A) 12G3H11, EB1 P1 and P2 proteins were analyzed for the ability to be captured on an ELISA plate by a bound EphB family RTK and detected by a biotinylated EphA2 protein. The tracings demonstrate that only the P1 and P2 proteins are capable of concurrently binding plate bound EphB family RTK and biotinylated EphA2. 12G3H11 and EB1 were unable to be captured with the EphB family RTK and bind biotinyated EphA2 concurrently. In Figure 18(B),EA, 12G3H11, P1, and P2 proteins were analyzed for the ability to be captured on an ELISA plate by a bound EphB family RTK and detected by a biotinylated EphA family RTK protein. The tracings demonstrate that only the P1 and P2 proteins are capable of concurrently binding a plate bound EphB family RTK and biotinylated EphA family RTK. 12G3H11 and EA2were unable to be captured with the EphB family RTK and bind a biotinyated EphA family RTK concurrently. These results further support the multiple binding specificities exhibited by the proteins P1 and P2.

### Example 13. Examining binding epitopes displayed on the cell surface.

Purpose: To establish a baseline measurement for the mono-specific binding proteins used to assemble certain multispecific epitope binding proteins.

Methods: Binding analysis was done on human pancreatic cancer cell lines, MiaPaCa2, that express all three Eph receptors on the cell surface. MiaPaCa2 cells were harvested from a confluent T-175 flask and were washed twice with PBS to remove the residual media and trypsin. Half a million cells in 200 µl of PBS were placed in a round bottom 96 well plate (Falcon Cat. 35-3077) with 200ng epitope binding protein and incubated for an hour at 4°C. The cells were washed twice with cold PBS and incubated with 200ul of a 1:1000 dilution of Immunopure Rabbit anti human IgG FC Fluorescein (Pierce Cat. 31535) 4°C for 30 minutes. Following the incubation, the cells were again washed twice with PBS. The labeled cells were resuspended in 200ul of PBS and 10 µl of 1µg/ml Propidium Iodide (Calibiochem Cat.537059). Analysis was done on a Guava FACS analyzer.

Results: Presented in Figure 19 is the FACS analysis of the binding of mono-specific epitope binding proteins to MiaPaCa2 cells. As a negative control, the detection reagent, an anti-human IgG Fc conjugated to Fluorescein was incubated with the cells and demonstrates a low level of binding to the cells. The 12G3H11 antibody demonstrates a high affinity to the MiaPaCa2 cells while the EB2 and EA mono-specific proteins exhibit a significant level of staining. These results demonstrate that MiaPaCa2 cells express and display antigens that are capable of being engaged by the binding units of 12G3H11, EA, and EB2. These binding units were utilized in the construction of proteins P1 and P2.

### Example 14. Examining binding epitopes for multispecific epitope binding proteins displayed on the cell surface.

Methods: The experiment was performed as essentially described in Example 12 above.

Results: Presented in Figure 20 is the FACS analysis of the binding of multispecific epitope binding proteins to MiaPaCa2 cells. As a negative control, the detection reagent, an anti-human IgG Fc conjugated to Fluorescein was incubated with the cells and demonstrated a low level of binding to the cells. The P1 and P2 multispecific epitope binding proteins demonstrated high affinities to the MiaPaCa2 cells. These results demonstrate that MiaPaCa2 cells express and display antigens that are capable of being engaged by the binding units of P 1 and P2. These results demonstrate that MiaPaCa2 cells display epitopes that are engaged by the proteins P1 and P2.

### Example 15. Examining binding epitopes for epitope binding proteins displayed on the cell surface.

Purpose: To establish relative binding specificities for certain epitope binding proteins.

Methods: The experiment was performed as essentially described in Example 12 above.

Results: Presented in Figure 21 is the FACS analysis of multispecific and monospecific epitope binding proteins. As a negative control, the detection reagent, an anti-human IgG Fc conjugated to Fluorascein was incubated with the cells and demonstrated a low level of binding to the cells. Also, a control antibody was incubated with the cells and also exhibited low affinity to the MiaPaCa2 cells. The P1 and P2 multispecific epitope binding proteins demonstrated high affinity to the MiaPaCa2 cells. The monospecific epitope binding proteins EB2, EA and 12G3H11 exhibited high affinities to the epitopes displayed on the cells. The monospecific epitope binding protein EB 1 displayed a lower, but specific affinity for the MiaPaCa2 cells. These results demonstrate that MiaPaCa2 cells display antigens that can be engaged by the mono and multispecific epitope binding proteins.

### Example 16. Competition assays for epitope binding proteins.

Purpose: To establish a baseline binding profile for certain proteins binding to MiaPaCa2 cells.

Methods: The binding assay was performed essentially as described in Example 12 with the following modifications. To confirm that all binding domains were actively involved in binding to the cell surface receptors, inhibition assays with free antigens were performed by pre-incubating the epitope binding proteins with 50 fold molar excess of free antigen for an hour prior to the incubation with the cells. The epitope binding protein:antigen mixture was then incubated with the MiaPaCa cells as described in the example 12. The epitope binding protein was pre-incubated with single antigens and in combination to demonstrate the functional binding of the multiple binding domains. In this example, the epitope binding protein was mixed with a vehicle control to establish a baseline for future studies.

Results: Presented in Figure 22 are the results from a sham competitive inhibition of binding assay involving particular proteins. The tracings represent the specific binding of P2, P1, EB2, EA, and 12G3H11 proteins to the surface of MiaPaCa2 cells. These results further exemplify that the P2, P1, EB2, EA, and 12G3H11 proteins are capable of binding epitopes displayed on live cells.

### Example 17. Competitive inhibition of cell surface binding.

Purpose: To evaluate the ability of soluble antigen to disrupt or compete for binding with certain epitope binding proteins

Methods: The experiment was performed essentially as described in Example 15 with the following modification: The soluble antigen, EphA2 was preincubated with solutions of epitope binding proteins in a 50 fold molar excess.

Results: Presented in Figure 23 are the results from a competitive inhibition of binding experiment involving the epitope binding proteins and soluble EphA2 ligand. The tracings represent the residual binding of certain proteins to MiaPaCa2 cells after being incubated with soluble ligand and then applied to the cells. The P2, P1 proteins contain EphA2 specific binding elements yet they remain bound to the cells, however the 12G3H11 tracing represents a protein that has monospecificity for EphA2 and resembles the non-specific anti hu-Fc tracing. This suggests that the free ligand, EphA2 has completely saturated the ability to bind epitopes displayed on the cell surface. Thus the P1 and P2 proteins retain binding likely through the specificity conferred by another binding motif. The EB2 and EA proteins retain binding as they are not specific for the soluble EphA2. These results demonstrate that soluble EphA2 is able to inhibit binding of the monospecific epitope binding protein 12G3H11, but not the multispecific epitope binding proteins P1 and P2, both of which carry an EphA2 binding domain. The P1 and P2 proteins may retain binding to the cell surface epitopes through another binding specificity. The other epitopes in the study remain available as the EA and EB2 proteins retain binding to the cell surface in the presence of soluble EphA2.

### Example 18. Competitive inhibition of binding of proteins to MiaPaCa2 cells.

Purpose: To evaluate the ability of soluble antigen to disrupt or compete for epitope binding

Methods: The experiment was performed essentially as described in Example 15 with the following modification: The soluble antigen, the EphA family RTK was preincubated with solutions of various proteins in a 50 fold molar excess.

Results: Presented in Figure 24 are the results from a competitive inhibition of binding experiment involving P2, P1, EB2, EA and 12G3H11 proteins and soluble EphA family RTK ligand. The tracings represent the residual binding of the proteins to MiaPaCa2 cells after being incubated with soluble ligand and then applied to the cells. The P2 and P1 proteins contain EphA family RTK specific binding elements yet they remain bound to the cells, however the EA tracing represents a protein that has monospecificity for EphA family RTK and resembles the non-specific anti hu-Fc tracing. This suggests that the free ligand, EphA family RTK has completely saturated the ability to bind epitopes displayed on the cell surface. The P1 and P2 proteins retain binding likely through the specificity conferred by another binding motif. The EB2 and 12G3H11 proteins retain binding as they are not specific for the soluble EphA family RTK. These results demonstrate that soluble EphA family RTK is able to inhibit binding of the monospecific epitope binding protein EA, but not the multispecific epitope binding proteins P1 and P2, both of which carry an EphA family RTK binding domain. The P1 and P2 proteins may retain binding to the cell surface epitopes through another binding specificity. The other epitopes in the study remain available as the 12G3H11 and EB2 proteins retain binding to the cell surface in the presence of soluble EphA family RTK.

### Example 19. Competitive inhibition of binding of proteins to MiaPaCa2 cells.

Purpose: To evaluate the ability of soluble antigen to disrupt or compete for binding

Methods: The experiment was performed essentially as described in Example 15 with the following modification: The soluble antigen, the EphB family RTK was preincubated with solutions of various proteins in a 50 fold molar excess.

Results: Presented in Figure 25 are the results from a competitive inhibition of binding experiment involving the proteins and soluble EphB family RTK ligand. The tracings represent the residual binding of the proteins to MiaPaCa2 cells after being incubated with soluble ligand and then applied to the cells. The P2 and P 1 proteins contain EphB family RTK specific binding elements yet they remain bound to the cells, however the EB2 tracing represents a protein that has monospecificity for EphB family RTK and resembles the non-specific anti hu-Fc tracing. This suggests that the free ligand, EphB family RTK has completely saturated the ability to bind epitopes displayed on the cell surface. The P 1 and P2 proteins retain binding likely through the specificity conferred by another binding motif. The EA and 12G3H11 proteins retain binding as they are not specific for the soluble EphB family RTK. These results demonstrate that soluble EphB family RTK is able to inhibit binding of the monospecific epitope binding protein EB2, but not the multispecific epitope binding proteins P 1 and P2, both of which carry an EphB family RTK binding domain. The P 1 and P2 proteins may retain binding to the cell surface epitopes through another binding specificity. The other epitopes in the study remain available as the 12G3H11 and EA proteins retain binding to the cell surface in the presence of soluble EphB family RTK.

### Example 20. Competitive inhibition of binding of proteins to MiaPaCa2 cells with two distinct soluble antigens.

Purpose: To evaluate the ability of soluble antigens to disrupt or compete for binding

Methods: The experiment was performed essentially as described in Example 15 with the following modification: The soluble antigens, EphA2 and another EphA family RTK were preincubated with solutions of certain proteins in a 50 fold molar excess.

Results: Presented in Figure 26 are the results from a competitive inhibition of binding experiment involving the proteins P2, P1, EB2, EA, and 12G3H11 and soluble EphA2 and the EphA family RTK ligand. The tracings represent the residual binding of the epitope binding proteins to MiaPaCa2 cells after being incubated with soluble ligands and then applied to the cells. The P1 protein contains an EphA2 and EphB family RTK specific binding elements yet shows residual binding to the cells compared to the monospecific 12G3H11 and EA with specificities to EphA2 and the EphA family RTK respectively. However, the EB2 tracing represents a protein that has monospecificity for EphB family RTK and resembles the sham tracing in Example 15. This suggests that the free ligands, EphA2 and the EphA family RTK have completely saturated the ability to bind epitopes displayed on the cell surface. The P1 protein retains binding likely through the specificity conferred by the other binding motif. The EB2 protein retains binding as it is not specific for the soluble EphA2 or the EphA family RTK.

### Example 21. Competitive inhibition of binding of proteins to MiaPaCa2 cells with two distinct soluble antigens.

Purpose: To evaluate the ability of soluble antigens to disrupt or compete for binding to cell surface receptors.

Methods: The experiment was performed essentially as described in Example 15 with the following modification: The soluble antigens, EphA2 and another EphA family RTK were preincubated with solutions of certain proteins in a 50 fold molar excess.

Results: Presented in Figure 27 are the results from a competitive inhibition of binding experiment involving the proteins P2, P1, EB2, EA, 12G3H11 and soluble EphA family RTK and the EphB family RTK ligands. The tracings represent the residual binding of the proteins to MiaPaCa2 cells after being incubated with soluble ligands and then applied to the cells. The P1 and P2 proteins contains both an EphA family RTK and EphB family RTK specific binding elements yet shows residual binding to the cells compared to the monospecific EA and EB2 with specificities to the EphA family RTK and the EphB family RTK respectively. However, the 12G3H11 tracing represents a protein that has monospecificity for EphA2 and resembles the sham tracing in Example 15. This suggests that the free ligands, the EphA family RTK and the EphB family RTK have completely saturated the ability to bind epitopes displayed on the cell surface. The P1 and P2 proteins retain binding likely through the specificity conferred by the other binding motif. The 12G3H11 protein retains binding as it is not specific for the soluble EphA family RTK or the EphB family RTK.

### Example 22. Competitive inhibition of the trispecific epitope binding proteins Purpose: To demonstrate that soluble ligands can compete for binding of proteins with the cell surface antigens.

Methods: The experiment was performed essentially as described in Example 15 with the following modification: The soluble antigens, EphA2, another EphA family RTK and the EphB family RTK were preincubated with solutions of certain proteins in a 50 fold molar excess.

Results: Presented in Figure 28 are the results from a competitive inhibition of binding experiment involving the proteins P2, P1, EB2, EA, and 12G3H 11 and soluble EphA2, the EphA family RTK and the EphB family RTK ligands. The tracings represent the residual binding of the proteins to MiaPaCa2 cells after being incubated with soluble ligands and then applied to the cells. All of the proteins studied (P1, P2, EB2, EA, and 12G3H11) demonstrated little or no residual binding after preincubation with the three ligands in excess. These results demonstrate that the proteins bind to specific epitopes expressed on MiaPaCa2 cells and not through another mechanism. The trispecific proteins P 1 and P2 bind specifically through all of the epitope binding units contained within.

### Example 23. Functional analysis of multispecific epitope binding proteins

Purpose: To demonstrate that epitope binding proteins retain functional properties derived from the parental antibodies and or identical isolated functional epitope binding domains. Specifically, many of the parental antibodies function as receptor agonists when applied to target cells.

Methods: To evaluate the ability for multispecific epitope binding proteins to agonize receptors, the following experimental protocol was employed. MiaPaCa2 cells were seeded at a density of 0.5 X 10⁶ in a six well plate and incubated for 24 hrs. The wells were washed twice with PBS. To activate the receptors, 10 µg of parental protein or trispecific epitope binding protein was add in 3ml of medium and incubated 37C for 30 minutes. The media was removed and the cells were carefully washed twice with cold PBS. The cells were then lysed by adding 200ul of triton lysis buffer (Boston Bioproducts Cat. BP 115) with 1X proteinase inhibitor cocktail (Sigma cat. P8340) and incubated for 5 minutes at room temperature. The lysate was collected and centrifuge at 6000 x g for 5minutes at 4°C to remove the cell debris. Total protein concentration in the lysate was that quantified using BCA assay (Pierce Cat 23225). To analyze receptor activation, cell surface receptors were purified and analyzed as follows: The EphB family receptors were immunoprecipitated with the 4G10 agarose (Upstate Cat. 16-199) and detected with a specific anti-EphB family antibody with goat anti-mouse IgG (Pierce Cat. 31437) was used as a secondary antibody. To immunoprecipitate EphA2, a specific EphA2 antibody (1C1) was used. To immunoprecipitate the EphA family RTK, streptavidin M280 beads (Invitrogen cat. 602-10) were conjugated to 500ug of biotinylated anti-EphA family antibody. The antibodies were biotinylated using EZ-link sulfo-NHS-Biotin Reagent (Pierce cat. 21335) at a 1:4 challenge ratio following manufacture's recommendations. The biotinylated antibodies and 500ul M280 beads were mixed and incubated at room temperature for one hour and washed with PBS to remove unconjugated antibody. Biotinylated 4G10 (1:1000) anti-phosphotyrosine (Upstate Cat. 16-204) antibody and the neutravidin-HRP 1:12500 (Pierce Cat. 31003) were used secondary in a western blot detection.

Immunoprecipitation of the Eph receptors from the treated cell lysate was done by mixing 100 µg of total lysate protein with 20 µl of 4G10 Agarose or 50 µl of the antibody conjugated M280 streptavidin beads. The lysate beads mixture was incubated on a rotator for two hour at 4°C. The mixture was then centrifuge at 2000 x g to pellet the beads and the supernatant removed. To remove unbound material ,the beads were washed twice in cold lysis buffer. The wash buffer was removed from the beads and 35ul of sample buffer (Invirogen cat. NP 0007) with 5% β-Mercaptoethanol was added following by heating to 100C for 10 minutes. The supernatant was loaded on to a 10% Nupage protein gel (Invitrogen cat.NP0301) and electrophoresis for 35 minutes at 200V constant. Protein transfer to a nitrocellulose membrane was done using the Invitrogen's transfer buffer (Invirogen cat.NP0006-1) and their recommended conditions. The membrane was incubated in blocking buffer (30% cod fish gelatin in1X PBS, 1% Tween20 and 1% BSA) for one hour. The blocking buffer was decanted and 10ml of the primary antibody was added directly to the membrane. After incubating the membrane for one hour, it was washed five times with wash buffer (1X PBS, 1% Tween20 and 1% BSA). The secondary HRP labeled reagent was then added in wash buffer and incubated for one hour on a rocker at room temperature. The blot was washed as before and soaked in ECL solution (Pierce Cat. 32106) following the manufacture suggestion before exposing to the Hyperfilm ECL film (Cat. RPN1674K).

Results: Presented in Figure 29 is the Western Blot analysis of the EphA2 receptor activation assay of MiaPaCa2 cells treated with the proteins 12G3H11, EA, EB2, EB1, P1, and P2.. As a positive control, purified phosphorylated EphA2 was included (Lane 9). As negative controls, a non-specific antibody (Lane 7) and media (Lane 8) were included. As depicted in the Figure, MiaPaCa2 cells treated with 12G3H11 (Lane 1), P1 (Lane 5), and P2(Lane 6) simulate receptor activation as measured by receptor phosphorylation. Proteins EA(Lane 2), EB2 (Lane 3), and EB 1 (Lane 4) did not stimulate EphA2. These results are expected as EA, EB1, and EB2 do not contain binding domains specific for EphA2. 12G3H11, P1 and P2 proteins are expected to retain the ability to agonist EphA2 as they contain binding domains specific for EphA2. These results suggest that the proteins P1 and P2 retain the ability to agonize EphA2 as described for the parental protein.

### Example 24. Functional analysis of various epitope binding proteins.

Purpose: To demonstrate that epitope binding proteins retain functional properties derived from the parental antibodies. Specifically, many of the parental antibodies function as receptor agonists when applied to target cells.

Methods: The experiment was performed as essentially described in Example 21 with the following alteration: The target was the EphA family RTK.

Results: Presented in Figure 30 is the Western Blot analysis of the EphA family receptor activation assay of MiaPaCa2 cells treated with the proteins 12G3H11, EA, EB1, EB2, P1 and P2. As a positive control, purified phosphorylated EphA family RTK was included (Lane 9). As negative controls, a non-specific antibody (Lane 7) and media (Lane 8) were included. As depicted in the Figure, MiaPaCa2 cells treated with EA (Lane 2), P1 (Lane 5), and P2 (Lane 6) simulate receptor activation as measured by receptor phosphorylation. Proteins 12G3H11 (Lanel), EB2 (Lane 3), and EB1 (Lane 4) did not stimulate the EphA family RTK. These results are expected as 12G3H11, EB1, and EB2 do not contain binding domains specific for the EphA family RTK. EA, P1 and P2 proteins are expected to retain the ability to agonist the EphA family RTK as they contain binding domains specific for the EphA family RTK. These results suggest that the proteins P 1 and P2 retain the ability to agonize the EphA family RTK as described for the parental protein.

### Example 25. Functional analysis of various epitope binding proteins.

Purpose: To demonstrate that multispecific epitope binding proteins retain functional properties derived from the parental antibodies. Specifically, many of the parental antibodies function as receptor agonists when applied to target cells.

Methods: The experiment was performed as essentially described in Example 21 with the following exception: The target was the EphB family RTK.

Results: Presented in Figure 31 is the Western Blot analysis of the EphB family receptor activation assay of MiaPaCa2 cells treated with the proteins 12G3H11, EA, EB2, EB1, P1,and P2. As a positive control, purified phosphorylated EphB family RTK was included (Lane 9). As negative controls, a non-specific antibody (Lane 7) and media (Lane 8) were included. As depicted in the Figure, MiaPaCa2 cells treated with EB2 (Lane 2), P1 (Lane 5), and P2 (Lane 6) simulate receptor activation as measured by receptor phosphorylation. Proteins 12G3H11 (Lane1), EA (Lane 2), and EB1 (Lane 4) did not stimulate the EphB family RTK. The results for 12G3H11 and EA are expected as these proteins do not contain binding domains specific for the EphB family RTK. The inability of EB1 to agonize the EphB family RTK is most likely due to the inefficient binding of EB1 to antigens displayed on the surface of cells (See example 14). The EB1, P1 and P2 proteins are expected to retain the ability to agonist the EphB family RTK as they contain binding domains specific for the EphB family RTK. These results suggest that the proteins P1 and P2 retain the ability to agonize the EphB family RTK as described for the parental protein.

### Example 26. Expression of the trispecific multispecific epitope binding protein P3

Purpose: To demonstrate high level expression of the trispecific epitope binding protein P3 comprising scFv domains fused to antibody heavy and light chains.

Methods: The epitope binding protein P3 was constructed by combining three epitope binding proteins, an antibody specific for C5a (1B8), an scFv specific for a C5a (15), and an scFv specific for an EphA family RTK (EA). The resultant structure is disclosed in Figures 2D. The epitope binding protein P3 retains binding specificity and affinity at a level at least comparable to the parental antibodies (data not shown).

The vectors encoding P3 were used to transfect 293F cells using the 293Fectin™ reagent (Invitrogen Cat. 51-0031) and Freestyle™ media (Invitrogen Cat. 12338) + 10% fetal bovine serum following the manufacturer's recommendations. The cells were fed on the third day post transfection and the supernatant was harvested on day six. Purification of the epitope binding protein P3 was via a Protein A column and followed by dialysis into PBS. The molecules were evaluated in the denatured and non-denatured forms on a protein gel to determine the size and relative purity of the proteins.

Results: Presented in Figure 32 is a PAGE gel documenting the expression of a trispecific epitope binding protein as presented in figure 4G. In the panel, a non-reducing (lanes 1 and 2) and a denaturing gel (lanes 3 and 4) document the relative molecule weight of the trispecific epitope binding protein under those conditions. In lane 2, the trispecific epitope binding protein exhibits a predicted molecular weight of about 240 kDa which is more than the predicted molecular weigh of a traditional antibody represented by (a) run on a PAGE gel in non-denaturing conditions. In lane 4, the trispecific epitope binding protein exhibits predicted molecular weights to about 75 kDa for the heavy chain and about 50 kDa for the light chain. These values are higher than the predicted molecular weights exhibited by a traditional antibody, including a heavy chain (b) and a light chain (c) run under similar conditions. These results demonstrate that a multispecific epitope binding protein comprised of an antibody fused to 2 scFvs one to the N-terminus of the heavy chain, the other to the N-terminus of the light chain can be expressed and displays the predicted molecular weight and composition (heavy and light chains) of the predicted structure.

### Example 27. Size exclusion chromatography (SEC) analysis of the multispecific epitope binding protein P3.

Purpose: To demonstrate the multispecific epitope binding protein P3 exhibits the correct apparent molecular weight.

Materials and methods: Size exclusion chromatography is a method known in the art to determine the apparent molecular weight of molecules (e.g proteins) in their native state. In this example, the multispecific epitope binding protein P3 was expressed and purified as described in Example 26. Purified P3 protein was loaded onto a SEC column (TSK-GEL G3000SWXL) in a buffer containing 100 mM Sodium Sulfate, 100 mM Sodium Phosphate at pH 6.8. The column was run at a flow rate of 1 ml/min. Calibration standards included for the determination of the apparent molecular weight included: Thyroglobulin (670 kDa), Bovine gamma-globulin (158 kDa), Chicken ovalbumin (44 kDa), Equine myoglobin (17 kDa), and Vitamin B12 (1.35 kDa).

Results: Presented in Figure 33 are the results from a Size-Exclusion Chromatography (SEC) analysis of multispecific epitope binding protein "P3". This construct, which is described in figure 4H, comprises three distinct epitope binding regions. The epitopes binding protein was expressed and analyzed by SEC. The dotted tracing represents a set of defined molecular weight components used to determine the molecular weight of the P3 protein. The solid tracing represents the elution profile of P3. Peak 1 represents about 70% of the protein at an estimated molecular weight of about 240 kDa (monomer). Peak 2 and 3 represent higher order structures (e.g. dimers) or aggregates. These results demonstrate that multispecific epitope binding proteins, such as P3, exhibit their predicted molecular weight in the native state.

### Example 28. Protease sensitivity of multispecific epitope binding proteins

Purpose: To determine the level of protease sensitivity of multispecific epitope binding proteins as compared to the parental antibodies.

Materials and methods: Antibodies and multispecific epitope binding proteins derived from the antibodies were expressed and purified as described above. Specifically, the multispecific epitope binding proteins P4, P5 and P6 were derived from the parental antibodies 1B8 and 15. The multispecific epitope binding protein P4 (presented in figure 4F) was constructed from the scFv derived from antibody 15 which was then fused to the N-terminus of the heavy chain of the 1B8 antibody. The multispecific epitope binding protein P5 (presented in figure 4D) was constructed from the scFv derived from antibody 15 which was then fused to the N-terminus of the light chain of the 1B8 antibody. The multispecific epitope binding protein P6 (presented in figure 2D) was constructed from the variable regions of antibody 15, fused to the Fab fragment of the 1B8 antibody as depicted in the figure. The epitope binding proteins P4, P5, and P6 retain binding specificities and affinities at a level at least comparable to the parental antibodies (data not shown). The various antibodies and multispecific epitope binding proteins were incubated without or with Trypsin (20 ng/ 1µg of antibody/epitope binding protein), Chymotrypsin (20 ng/ 1µg of antibody/epitope binding protein), or human serum (1µg serum / 1µg of antibody/epitope binding protein) for either 1 hour at 37°C (panel A) or 12 hours at 37°C (panel B). The samples were then analyzed by PAGE to determine the fragmentation profile compared to the samples incubated without protease or serum.

Results: Presented in figure 34 are the results from a protease sensitivity assay performed on epitope binding proteins of various formats. Once incubation with the proteases was complete, samples were run on a reducing PAGE gel and stained with Coomassie to determine whether proteolysis had occurred. As presented, a 1 hour incubation at 37 °C does not result in proteolytic degradation of the various parental antibodies or epitope binding proteins. In an extended incubation (12 hours at 37 °C) no detectable proteolysis of the epitope binding proteins was observed. These results demonstrate that the multispecific epitope binding proteins as described herein exhibit a high level of protease resistance, similar to that observed for antibody molecules.

### Example 29. Protease sensitivity of multispecific epitope binding proteins

Purpose: To determine the level of protease sensitivity of multispecific epitope binding proteins as compared to the parental antibodies.

Materials and methods: Antibodies and multispecific epitope binding proteins derived from the antibodies were expressed and purified as described above. The various antibodies and multispecific epitope binding proteins were incubated without or with Cathepsin B (20 ng/ 1µg of antibody/epitope binding protein for either I hour at 37°C (panel A) or 20 hours at 37°C (panel B). The samples were then analyzed by PAGE to determine the fragmentation profile compared to the samples incubated without protease or serum.

Results: Presented in Figure 35 are the results from a protease (CathepsinB) sensitivity assay performed on epitope binding proteins of various formats. Specifically, the proteins (parental antibodies and epitope binding proteins of various formats outlined herein) were expressed, purified and incubated for either (A) 1 hour or (B) 20 hours at 37 °C without (odd numbers) or with (even numbers) Cathepsin B (20 ng protease/1 µg of antibody/epitope binding protein). Once incubation with the protease was complete, samples were run on a reducing PAGE gel and stained with Coomassie to determine whether proteolysis had occurred. As presented, a 1 hour incubation at 37 °C does not result in proteolytic degradation of the various parental antibodies or epitope binding proteins. In an extended incubation (20 hours at 37 °C) some proteolysis of the epitope binding proteins in lanes 8 and 9 (protein P2) and lanes 14 and 15 is (protein P4) evident (see dashed circles). These results demonstrate that the multispecific epitope binding proteins as described herein exhibit a high level of protease (Cathepsin B) resistance, similar to that observed for antibody molecules.

### Example 30. Transient expression of multispecific epitope binding proteins

Purpose: To transiently express various multispecific epitope binding protein formats

Methods: All constructs were expressed in HEK293F cells cultivated in Invitrogen Freestyle™ media. The culture medium was collected 10 days post-transfection and all antibody formats were purified by standard protein A affinity chromatography in accordance with the manufacturer's protocol (GE Healtheare, Piscataway, NJ), and buffer exchanged into 25 mM Histidine-HCl pH 6.0. The purity of the constructs was analyzed using sodium dodecyl sulfate polyacrilamide gel electrophoresis (SDS-PAGE) under reducing and non-reducing conditions and using analytical size-exclusion chromatography. Total IgG expression was determined using an in-house developed protein A binding assay. In short, the culture media was automatically loaded onto a protein A column using an HPLC system (Agilent 1100 Capillary LC System, Foster City, CA). Unbound material was washed with a solution of 100 mM sodium phosphate buffer at pH 6.8, and antibodies were eluted with 0.1 % phosphoric acid pH 1.8. The area corresponding to the eluted peak was integrated and the total antibody concentration was determined by comparing to an IgG standard. Concentration of the purified antibodies was also determined by reading the absorbance at 280 nm using theoretical determined extinction coefficients. The results are presented in Table 1 below.

**Table 1. Transient expression of multispecific epitope binding proteins**

| Binding protein format | Transient Expression Level |
|---|---|
| Traditional Ab | 180 mg/L |
| Figure 4D | 120 mg/L |
| Figure 4F | 140 mg/L |
| Figure 4H | 115 mg/L |
| Figure 4L | 45 mg/L |
| Figure 3D | 120 mg/L |

These results demonstrate that exemplary multispecific epitope **binding** proteins, such as those detailed in Table 1, may be transiently expressed to similar levels of conventional antibodies.

### Example 31. Determination of absolute molecular mass of various multispecific epitope binding proteins

Purpose: To determine various in-solution parameters of multispecific epitope binding proteins

Methods: To determine the in solution molecular weights and other molecular parameters, such as hydrodynamic radii and intrinsic viscosities, we used analytical size-exclusion HPLC coupled with a triple detector system that simultaneously measures the differential refractive index, the differential viscosity and the light scattering. Table 2 shows the molecular parameters obtained through the triple detection analysis.

**Table 2. Biophysical characterization of multispecific epitope binding proteins**

| Format ref. | Retention time (min) | Monomeric state (%) | Theoretical molecular weight (KDa) | Experimental molecular weight (KDa) | Hydrodynamic radius (Rₕ) (nm) | Intrinsic viscosity (η) (ml/g) |
|---|---|---|---|---|---|---|
| Std. Ab | 8.5 | 99 | 144 | 151 | 5.2 | 6 |
| Fig 4D | 8.0 | 95 | 197 | 204 | 6.3 | 7 |
| Fig 4F | 8.0 | 95 | 197 | 202 | 6.3 | 8 |
| Fig 4H | 7.7 | 92 | 250 | 265 | 7.0 | 8 |
| Fig 3D | 7.7 | 95 | 250 | 262 | 7.0 | 8 |

The experimental molecular weight values presented in Table 2 correlate with the theoretical values taking into account the presence of one N-linked carbohydrate moieties in each of the two antibody C_{H}2 domain, which may account for about 3.5 KDa. The calculated values of the hydrodynamic radius (Rₕ), which is the effective size of the molecules as determined by their diffusion, were increased from 5.2 nm as predicted from the addition of at least on epitope binding domain to the construct. The intrinsic viscosity values (η), which are directly related to the length of the protein chain, were also increased from 6 ml/g for the control antibody to 7 ml/g to 8 ml/g for the multispecific constructs. The hydrodynamic radii and intrinsic viscosities values correlate to that reported for intact antibody. For instance, the reported hydrodynamic radius of intact IgG is 5.4 nm and the intrinsic viscosity is 6 ml/g. Altogether these data show that the experimental determined molecular parameters are in accordance with the size and structural topology of the engineered multispecific epitope binding formats and correlate well with the control antibody. In addition, these data indicate that the multispecific epitope binding protein formats do not form aggregates and have significant structural homogeneity. Furthermore, these biophysical properties remain unchanged upon storage at 4 °C for several weeks.

### Example 32. Thermal stability of various multispecific epitope binding protein formats analyzed by differential scanning calorimetry

In this example, the thermal denaturation profiles of various multispecific epitope binding proteins were analyzed by differential scanning calorimetry.

Methods: Differential scanning calorimetry (DSC) experiments at a heating rate of 1 °C/min were carried out using a Microcal VP-DSC ultrasensitive scanning microcalorimeter (Microcal, Northampton, MA). DSC experiments were carried out in 25 mM Histidine-HCl pH 6. All solutions and samples used for DSC were filtered using a 0.22 micron-filter and degassed prior to loading into the calorimeter. All antibody formats used for the DSC studies were > 90 % monomeric as judged by analytical gel filtration chromatography. For each set of measurements, at least four buffer-versus-baseline runs were first obtained. Immediately after, the buffer solution was removed from the sample cell and loaded with approximately 0.5 ml of sample at concentration of 1 mg/ml. For each measurement the reference cell was filled with the sample buffer. From each sample-versus-buffer experiment, the corresponding buffer-versus-buffer baseline run was subtracted. The raw data were normalized for concentration and scan rate. Data analysis and deconvolution was carried out using the Origin™ DSC software provided by Microcal. Deconvolution analysis was performed according to Privalov & Potekhin ("Scanning microcalorimetry in studying temperature-induced changes in proteins", Methods Enzymol. (1986) 131, 4-51) using a non-two-state model and best fits were obtained using 100 iteration cycles. Briefly, best deconvolution fits were analyzed by either independent or dependent schemes (non-two-state model). An independent scheme is based on the assumption that each domain in a multi-domain protein unfolds independently, regardless of the state of the neighboring domains. A dependent scheme assumes an ordered process in which interacting domains unfold sequentially, thus the unfolding of any given domain depends on the status of its neighbors. The interpretation of the DSC deconvolution results was based on the fact that the different domains in the polyspecific antibody formats unfolds independently with cooperative transitions as described for immunoglobulins (Tischenko et al. (1982) "A thermodynamic study of cooperative structures in rabbit immunoglobulin G, Eur J Biochem., 126, 517-521) or other multidomain proteins (Batey et al. (2008), "The folding pathway of a single domain in a multiple domain protein is not affected by its neighboring domain", J. Mol. Biol. In press) The denaturation temperature, Tₘ, corresponding to the maximum of the transition peaks, was determined for each construct from at least three replicate runs and did not vary more than ± 2 %. Refolding was analyzed by re-scanning the samples after denaturation. The results are summarized in Table 3 below.

**Table 3. Transition temperatures (Tₘ) exhibited by the epitope binding domains present in various multispecific epitope binding proteins as measured by deconvolution of excess heat capacity curves.**

| Protein format | Transition number | Corresponding domain | Tm (°C) |
|---|---|---|---|
| Ab | | | |
| | 1 | C_{H}2 | 69 |
| | 2 | Fab | 75 |
| | 3 | C_{H}3 | 82 |

| Figure 4D | | | |
|---|---|---|---|
| | 1 | scFv -N-term of light chain | 57 |
| | 2 | C_{H}2 | 69 |
| | 3 | Fab | 73 |
| | 4 | C_{H}3 | 82 |
| Figure 4F | | | |
| | 1 | scFv N-term of heavy chain | 63 |
| | 2 | C_{H}2 | 69 |
| | 3 | Fab | 73 |
| | 4 | C_{H}3 | 82 |
| Figure 4H | | | |
| | 1 | scFv N-term of light chain | 61 |
| | 2 | scFv -N-term of heavy chain | 63 |
| | 3 | C_{H}2 | 69 |
| | 4 | Fab | 75 |
| | 5 | C_{H}3 | 82 |
| Figure 3D | | | |
| | 1 | 1^{st} scFv -C-term of heavy chain | 57 |
| | 2 | 2^{nd} scFv -C-term of heavy chain | 65 |
| | 3 | C_{H}2 | 69 |
| | 4 | Fab | 75 |
| | 5 | C_{H}3 | 82 |

Results: The thermogram for the control antibody 1B8 shows three distinct unfolding transitions with denaturation temperatures, Tₘ, of 69°C, 75°C and 82°C. These transitions correspond to the denaturation of the C_{H}2, Fab and C_{H}3 domains, respectively. The presence of these three distinct peaks indicates that the IgG molecule is a multi-domain protein in which at least three domains, or group of domains, exist that denature under distinct conditions. It is therefore of interest to analyze the denaturation transitions of the multispecific epitope binding protein formats, in order to investigate the overall stability of the constructs and their potential multi-domain cooperative denaturation. The deconvolution analysis of the multispecific format presented in Figure 4D (Table 3) uncovers four transitions, one with a Tₘ of 57 °C, one with a Tₘ of 69 °C, one with a Tₘ of 73 °C and one with a Tₘ of 82 °C. The peak with Tₘ of 57 °C corresponds to the denaturation transition of the scFv attached to the N-terminus of the light chain (Figure 4D). Generally, scFvs have lower denaturation temperatures than any given antibody domain with unfolding characterized by a single transition event. The scFv linked to the N-terminus of the light chain in the Figure 4D format does not destabilize the overall stability of the protein scaffold. In fact, the Tₘ of 73 °C for the denaturation of the Fab domain of the Figure 4D format is not significantly different from the Tₘ observed for the parental Fab, which is 75 °C (data not shown). In addition, the denaturation temperature of the C_{H}2 domain (Tₘ = 69 °C) and C_{H}3 domain (Tₘ= 82 °C) of the Figure 4D format are unchanged compared to the control antibody. Similarly, the scFv linked at the N-terminus of the antibody heavy chain in the Figure 4F format protein (Tₘ = 63 °C) does not destabilize the overall folding of the protein scaffold. In fact, for this format, the deconvolution analysis shows that the denaturation of the Fab domain (Tₘ= 73 °C) is very similar to the denaturation of the Fab domain of the control antibody (Tₘ = 75 °C), and is the same of the denaturation transition observed for the Fab domain in the Figure 4D format (Tₘ = 73 °C). Furthermore, for this antibody format the denaturation of the C_{H}2 domain (Tₘ = 69 °C) and of the C_{H}3 domain (Tₘ = 82 °C) remain unchanged compared to the control antibody (Table 3).

The deconvoluted thermogram for the denaturation of the Figure 3D format protein show that the scFv linked to the C-terminus of the C_{H}3 domain have unique denaturation transitions, with Tₘ of 57 °C for the 1^{st} scFv linked to the C-terminus of the heavy chain in the Figure 3D format, and a Tₘ of 65 °C for the 2^{nd} scFv linked to the C-terminus of the heavy chain in the Figure 3D format. The unfolding of these scFv linked after the C_{H}3 domain do not destabilize the global unfolding of the protein scaffolds. In fact, these two constructs have similar denaturation transitions for the Fab, C_{H}2 and C_{H}3 domains as the control antibody (Table 3).

The deconvolution analysis of Figure 4H format protein thermogram uncovers five transitions, with Tₘₛ of 61°C, 63°C, 69°C, 75°C, and 82°C (Table 3). The peak with transition Tₘ of 61 °C corresponds to the denaturation transition of the scFv linked to the N-terminus of the light chain. The peak with Tₘ of 63 °C corresponds to the denaturation transition of the scFv linked to the N-terminus of the heavy chain, whereas the other three peaks with Tₘ of 69 °C, 75 °C 82 °C correspond to the denaturation transition for the Fab, C_{H}2 and C_{H}3 domains, respectively. These former Tₘ values confirm that Figure 4H format is stable to a similar extent as the control antibody.

Altogether these experiments confirm that the multispecific epitope binding protein formats characterized by multi-domain unfolding transitions, have robust thermal stability. Additionally, the independent DSC unfolding transitions are in agreement with a recent study demonstrating that the folding pathway of a single domain in a multidomain protein, like the multispecific epitope binding protein formats, is not affected by its neighboring domain.

### Example 33. Simultaneous binding of three distinct antigens exhibited by a multispecific epitope binding protein

Purpose: To demonstrate simultaneous binding of three distinct antigens on a multispecific epitope binding protein immobilized on a BIAcore instrument

Methods: Triple-specific binding ability of P1 (Figure 3D) was assessed by BIAcore by injecting the three antigens EB, EA and EphA2 sequentially over a P 1-coated chip. When the P1 epitope had been saturated with the first antigen (EB), the second antigen (EA) was injected, followed by the injection of the third antigen (EphA2). Ovalbumin was used as a negative binding control.

Results: As presented in Figure 36, the P1 epitope binding protein exhibits the ability to simultaneously bind three distinct antigens, namely EB, EA, and EphA2 (upper curve). This binding ability is specific for the P1 epitope binding protein as a similar experiment performed with ovalbumin showed no specific binding (lower curve). These results demonstrate that multispecific epitope binding proteins may simultaneously engage distinct antigens recognized by the component epitope binding domains.

### Example 34. Cellular internalization of a multispecific epitope binding protein

Purpose: To demonstrate cellular internalization of a multispecific epitope binding protein

Methods: Antibodies internalization using confocal microscopy - PC3 cells, expressing EphA2, were added to a 96 well U bottom plate at a concentration of 1 x 10⁶ per well. The cells were washed twice with PBS and labeled with 5 µg of the control (R347), parental (12G3H11) or multispecific epitope binding proteins (P1) for 30 minutes on ice. Cells were then washed twice with PBS and incubated with growth media at 37 °C for 0, 10, 20, 30, 60 minutes. The cells were then fixed in 3.7 % paraformaldehyde for 20 minutes, washed twice in PBS and permeabilized using 0.5 % Triton X-100 in PBS for 5 minutes at room temperature. The cells were again washed twice with PBS, and finally labeled with 1 µg of AlexaFluor-488 goat-γ-human IgG antibody (Invitrogen). The excess secondary antibody was removed using two washes with cold PBS. The cells were spun onto a coated cytoslide and mounted beneath a coverslip with Vectasheild Hardset mounting medium with DAPI (Vector Laboratories). Internalization was analyzed by confocal laser-scanning microscopy at 63X magnification using a Leica SP5 (Mannheim, Germany) microscope.

Results: To confirm cellular uptake of the parental antibodies and multispecific epitope binding proteins, MiaPaca tumor cell lines, which express all three target receptors (EphA2, EA and EB), were incubated with the multispecific epitope binding protein P1, parental antibody or a negative control antibody for 0, 10, 20, 30 and 60 minutes at 37 °C at a concentration of 5 µg/ml. Receptor-mediated internalization was detected via fluorescently labeled anti-Fc antibody using a confocal laser-scanning microscopy. Positive internalization is typically characterized by bright fluorescence within the cell cytoplasm, together with a decrease in membranous (extracellular) fluorescence. As seen in Figure 37B and C, the multispecific epitope binding protein P1 and parental antibodies (12G3H11) were rapidly internalized (∼10 min) as showed by the intracellular staining (green fluorescent color) with a pattern typical for a receptor mediated internalization. Control experiments with 293 cells lacked any P1 or parental antibody (12G3H11) uptake (data not shown). Moreover, we did not detect internalization in MiaPaca cells using an unrelated antibody (R347, Figure 37A), and no detectable internalization could be observed at 4 °C which is a condition not permissive for internalization. This data confirm that the multispecific epitope binding protein P 1 is efficiently internalized *in vitro* similar to an anti-EphA2 parental antibody.

### Example 35. In vitro and in vivo receptor degradation by the multispecific epitope binding protein, P1.

**Purpose:** To demonstrate that a multispecific epitope binding protein may functionally interact with its target epitope *in vivo.*

**Methods:** In vivo pharmacokinetic (PK) and pharmacodinamic (PD) analysis: PC-3 prostate adenocarcinoma cells were implanted subcutaneously on the right flank of 7-week old nude mice (Harlan Sprague Dawley) at 5 x 10⁶ cells per mouse. Tumors were allowed to progress to approximately 100 mm³ and dosed with the multispecific epitope binding protein, P1 or the parental anti-EphA2 or anti-EB antibodies at 67 nmol/kg body weight. Tumors and serum were harvested from 3 mice per time point per dose group at 1, 4, 8, 24, 48, 72, 120 and 144 hours post-dose. Tumors and serum from one additional group of 3 mice, dosed with PBS, were harvested immediately after dosing (0 hours). Tumors and serum were stored at - 80 °C prior to processing. Tumors were homogenized in 1 % Triton-lysis buffer containing 25 µg/ml Aprotinin and 10 µg/ml Leupeptin for 30 seconds in Lysing Matrix A tubes (MP Biomedicals) on a Fast Prep 24 System (MP Biomedicals). The lysates were centrifuged at 10,000 rpm for 5 minutes at 4°C. The supernatants were collected and analyzed for protein concentration by BCA Protein Assay (Pierce). 30 µg of total protein from the tumor supernatants were loaded on a 10 % bis-tris gel and analyzed by western blot for EphA2, EB and GAPDH. Tumor lysates were loaded onto three separate gels with one sample from each time point loaded on each gel. The protein bands after western blot were quantified by densitometry analysis and normalized to the single protein band from the 0-hour time point, PBS control present on each of the three blots. The serum samples were analyzed for the presence of the P1 protein or parental anti-EphA2 or anti-EB control antibodies using an EphA2 or EB binding ELISA. Briefly, 96-well Maxisorp Elisa plates (Nunc) were coated with either human EphA2 or EB at 5 µg/ml in PBS pH 7.4, overnight at 4 °C. Plates were blocked with 4 % non fat dry milk, washed, and serum samples (diluted 1:1000) were loaded and incubated for 1 hour at 22 °C. For both the EB and EphA2 binding ELISA, standard curves were prepared with serial dilutions from 1 mg/ml to 10 ng/ml. For the EB binding ELISA the P1 protein serum samples were compared to a P1 protein standard curve for quantification and the anti-EB control antibody serum samples were compared to an anti-EB control antibody standard curve. The HRP conjugated secondary antibodies used were goat-anti-mouse (Jackson Immunolabs) for the anti-EB control antibody ELISA and goat-anti-human (Jackson Immunolabs) for the P1 protein specific ELISA. For the EphA2 binding ELISA the P1 protein serum samples were compared to a P1 protein standard curve for quantification and the anti-EphA2 control antibody serum samples were compared to an anti-EphA2 control antibody standard curve. The HRP conjugated secondary antibody used for both the anti-EphA2 control antibody and trispecific antibody EphA2 ELISA was goat-anti-human (Jackson Immunolabs).

**Results:** Receptor degradation assays were carried out in order to determine if the multispecific epitope binding protein P 1 is able to induce degradation of its target receptors (EphA2, EA and EB) both *in vitro* and *in vivo.* PC-3 cells, which express EB and EphA2, were chosen because of their ability to proliferate into tumors upon injection in nude mice. Unfortunately, PC-3 cells do not express EA, therefore we could not assay for *in vitro* degradation of this receptor. However, as described for the *in vitro* receptor phosphorylation, MiaPaca cells lines do express EA, EphA2, and EB, but we could not use these cells for the receptor degradation assays because (1) they have low and variable tumor growth rate *in vivo,* and (2) the parental anti-EA antibody (and therefore the multispecific epitope binding protein P1) is not able to induce EA degradation in MiaPaca cells or in other tumor cell lines that do express EA. As seen in figure 6, upon incubation with the P1 protein, PC3 cells both EB (Figure 38A) and EphA2 (Figure 38B) are strongly degraded when compared to the individual anti-EphA2 and anti-EB parental antibodies. Next, we asked whether the P 1 protein is able to induce degradation of target receptors in an *in vivo* tumor model system following systematic administration. It is important to point out that in order to induce *in vivo* receptor degradation the trispecific antibody needs not only to reach the tumor site but also to be able to penetrate into the tumor itself. Successful *in vivo* EphA2 and EB degradation was verified by inoculating PC-3 cells into nude mice and determining the level EphA2 and EB expression upon P1 protein or parental antibody treatment. As seen in Figure 7, both EB (A,B) and EphA2 (C,D) are efficiently degraded by the P1 protein better than the respective parental anti-EphA2 and anti-EB antibodies. These results suggest the P1 protein, designed to simultaneously engage three different antigens, may be able to efficiently promote clustering of a combination of different receptors at the cell surface (better than the individual antibodies), thus improving efficiency of internalization and degradation, which in turn results in substantial receptor downregulation, leading to a significant antitumor response.

### Example 36. Pharmacokinetic analysis of multispecific epitope binding protein.

Pharmacokinetic analyses of the multispecific epitope binding protein, P1 and its parental anti-EphA2 and anti-EB antibodies were carried out using PC-3 tumor-bearing nude mice. The plasma concentration of the P1 protein and parental antibodies were measured by ELISA at specific time points. As shown in Figure 40, the P1 protein was still detectable (> 200 nM) at 144 hours (6 days) after dosing using either EphA2 or EB ELISA, and the levels of plasma concentration of the P1 protein are comparable with the levels of the individual parental antibodies. These data suggests that the half-life of the P1 protein in an *in vivo* tumor model is similar to that of its respective parental antibodies. These results also suggest that the P1 protein is highly stable *in vivo.*

## Claims

1. An inverted antibody protein comprising at least a first and a second polypeptide chain wherein said first chain is a "light-like" chain comprising at least one antibody heavy chain variable region (VH) linked to a CH1 domain and said second chain is a "heavy-like" chain comprising an antibody light chain variable region (VL) linked to a Ckappa/lambda domain, said Ckappa/lambda region further linked to an Fc region wherein said variable region in said first chain associates with the variable region in said second chain to form an epitope binding site.

2. The protein of any preceding claim, wherein said first light-like chain is disulphide linked to said second heavy-like chain.

3. An inverted antibody protein of claim 1 or claim 2 comprising at least four polypeptide chains, with at least two chains being light-like chains and at least two chains being heavy-like chains.

4. The protein of any preceding claim, wherein the heavy-like chain comprises all or part of a hinge region.

5. The protein of any preceding claim, wherein the light-like chain comprises all or part of a hinge region.

6. The protein of claim 4, wherein the protein is a multimer of four polypeptide chains wherein the heavy-like chains comprise a second VL (VL2) linked to a second Ckappa/lambda region linked N-terminal to the VL domain (VL1), and wherein the light-like chains comprise a second VH domain (VH2) linked to a second CH1 domain linked N terminal to the VH domain (VH1).

7. The protein of claim 4, wherein the protein is a multimer of six polypeptide chains, wherein the heavy-like chains comprise a second VL (VL2) linked to a second Ckappa/lambda region linked N-terminal to the VL domain (VL1), and wherein the light-like chains comprise a combination of light-like chains in which two first light-like chains comprise a first VH domain (VH1) linked to a first CH1 domain and two second light-like chains comprise a second VH domain (VH2) linked to a second CH1 domain.

8. The protein of any one of the preceding claims, wherein the heavy-like chain and/or the light-like chain further comprise an epitope binding domain (EBD).

9. The protein of claim 8, wherein the EBD is selected from the group consisting of an scFv, a single chain diabody, an antibody mimetic and an antibody variable domain.

10. The protein of any one of the preceding claims, wherein the antibody variable domains are not identical.

11. The protein of any one of the preceding claims, wherein the antibody variable regions do not have the same epitope binding specificities

12. The protein of any one of the preceding claims, wherein said protein specifically binds distinct target proteins and inhibits and/or neutralizes said target proteins.

13. An expression vector comprising a nucleic acid molecule encoding the protein of any one of claims 1 to 12.

14. A host cell comprising the expression vector of claim 13.

15. A sterile formulation comprising a therapeutically effective amount of the protein of any of claims 1 to 14 in a pharmaceutically-acceptable excipient.
